# EUROPEAN PATENT SPECIFICATION

(11) **EP 4 405 334 B1**
(45) Date of publication and mention of the grant of the patent: **12.02.2025**
(21) Application number: 22822701.3
(22) Date of filing: 01.12.2022
(51) Int. Cl.: C07D 207/34, C07D 209/42, C07D 213/78, C07D 231/14, C07D 233/18, C07D 239/557, C07D 241/18, C07D 249/08, C07D 257/04, C07D 261/10, C07D 275/03, C07D 277/56, C07D 333/12, C07D 333/24, C07D 333/38

(54) **SORTILIN INHIBITORS**
SORTILINHEMMER
INHIBITEURS DE LA SORTILINE

(30) Priority: 02.12.2021 SE 2151469
(43) Date of publication of application: 31.07.2024
(73) Proprietor: Sortina Pharma AB, 411 26 Göteborg (SE)
(72) Inventor: RHOST, Sara, 431 63 Mölndal (SE); LANDBERG, Göran, 302 70 Halmstad (SE); WESTERLUND, Christer, 431 50 Mölndal (SE); OLSSON, Thomas, 423 61 Torslanda (SE)
(74) Representative: Barker Brettell Sweden AB
(86) International application number: PCT/SE2022/051131
(87) International publication number: WO 2023/101595

(56) References cited:
- STACHEL SHAWN J ET AL: "Identification of potent inhibitors of the sortilin-progranulin interaction", BIOORGANIC & MEDICINAL CHEMISTRY LETTERS, ELSEVIER, AMSTERDAM NL, vol. 30, no. 17, 15 July 2020 (2020-07-15), XP086232499, ISSN: 0960-894X, [retrieved on 20200715], DOI: 10.1016/J.BMCL.2020.127403

## Description

### TECHNICAL FIELD

The present invention generally relates to compounds suitable as sortilin inhibitors, and to uses thereof.

### BACKGROUND

Sortilin is a type I transmembrane protein that acts both as a receptor of several ligands, and in the sorting of cargo from the trans-Golgi network (TGN) to late endosomes and lysosomes for degradation. Sortilin binds the secreted protein progranulin (PGRN) and targets it for lysosomal degradation, thus, negatively regulating extracellular levels of PGRN. PGRN is a secreted, growth factor-like, trophic, and anti-inflammatory protein, which also plays a role as an adipokine involved in diet-induced obesity and insulin resistance.

PGRN deficiency accounts for roughly 25% of all heritable forms of frontotemporal dementia (FTD), an early-onset neurodegenerative disease. Patients with heterozygous loss-of-function mutations in PGRN have about 50% reduced extracellular levels of the protein and they will invariably develop FTD, making PGRN a causal gene for the disease. In addition, PGRN mutant alleles have been identified in Alzheimer's disease patients. Importantly, PGRN acts protective in several disease models with increased PGRN levels accelerating behavioral recovery from ischemia, suppressing locomotor deficits in a Parkinson's disease model, attenuating pathology in a model of amyotrophic lateral sclerosis (ALS) and arthritis and preventing memory deficits in an Alzheimer's disease model.

Sortilin also binds pro-neurotrophins, such as pro-nerve growth factor (pro-NGF), pro-brain-derived neurotrophic factor (pro-BDNF), and pro-neurotrophin-3, which harbor a pro-domain and are typically pro-apoptotic. Such pro-neurotrophin precursors are released during stress, and sortilin is involved in regulating their release as well as stimulation of apoptosis in conjunction with p75NTR.

Sortilin also binds to p75NTR directly. Sortilin further binds neurotensin in a region that partially overlaps with PGRN binding and is therefore sometimes also referred to as NTR3 receptor. Sortilin also interacts with the Trk receptors NTRK1, NTRK2, and NTRK3 and can regulate their anterograde axonal transport and signaling. Sortilin additionally interacts with and regulates the processing and trafficking of amyloid precursor protein (APP) and the resulting production of pathological beta amyloid peptides. Sortilin has been shown to bind to apolipoproteins and lipoprotein lipase and, thus, deficiency leads to reduced very low density lipoprotein (VLDL) release from liver and reduced cholesterol. Sortilin has also been implicated in binding to APP directly and also to the APP processing enzyme beta-secretase 1 (BACE1). Sortilin also binds to apolipoprotein E (APOE), and to the amyloid beta peptide. Sortilin has also been shown to bind to and regulate extracellular levels of proprotein convertase subtilisin/kexin type 9 (PCSK9), which directs low density lipoprotein receptor for degradation in lysosomes, resulting in increased levels of low density lipoprotein (LDL) cholesterol.

When present at intracellular vesicles, such as endosomes, the amino terminal extracellular domain of sortilin is directed towards the lumen, where cargo of the vesicle is present. The carboxy terminal intracellular/cytoplasmic domain of sortilin, however, binds to a series of adaptor proteins, which regulate its trafficking from the surface and within intracellular compartments. These include clathrin adaptor protein 2 (AP2) and the retromer complex/AP1, which modulate movement from early endosomes to Golgi for recycling, and interaction with GGA (Golgi-localizing, gamma-ear containing, ADP-ribosylation factor binding) family proteins for movement from Golgi directly to early endosomes, usually for subsequent degradation through lysosomes. Thus, sortilin can bind ligands at its luminal domain, while engaging the cytoplasmic adaptors that determine its destination to determine intracellular fates, such as degradation for PGRN and other factors.

Through its various interactions with proteins, such as PGRN, sortilin and its multiple ligands have been shown to be involved in various diseases, disorders, and conditions, such as FTD, ALS, ALS-FTD phenotypes, Alzheimer's disease, Parkinson's disease, depression, neuropsychiatric disorders, vascular dementia, seizures, retinal dystrophy, age related macular degeneration, glaucoma, traumatic brain injury, aging, seizures, wound healing, stroke, dermatology related diseases, autoimmune diseases, arthritis, atherosclerotic vascular diseases and cancers (WO 2009/140972, WO 2014/114779, WO 2016/164637, Breast Cancer Research 2018 20: 137, Bioorganic & Medicinal Chemistry Letters 2020 30: 127403).

Accordingly, there is a need for therapeutic compounds that bind to sortilin and block the binding of sortilin to its ligands, such as PGRN, or otherwise modulate the effective concentration of the ligands, in order to treat one or more diseases, disorders, and conditions associated with sortilin activity.

### SUMMARY

It is a general objective to provide therapeutic compounds that bind to sortilin and block the binding of sortilin to its ligands, such as PGRN.

It is a particular objective to provide such therapeutic compounds useful in treatment of diseases, disorders, and conditions associated with sortilin activity.

These and other objectives are met by embodiments as disclosed herein.

The invention is defined in the independent claims. Further embodiments of the invention are defined in dependent claims.

An aspect of the invention relates to a compound of formula I: 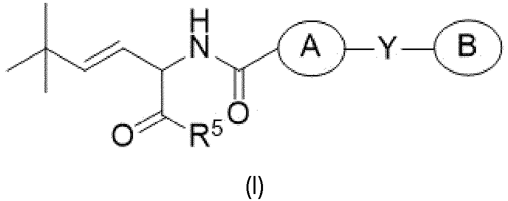 wherein
A is a 5 or 6 membered aromatic, heteroaromatic or heterocyclic ring with 0 to 2 heteroatom(s) selected among N, O and S, or a 8 to 10 membered bicyclic heterocyclic or heteroaromatic ring with 1 or 2 heteroatom(s) selected among N, O and S, the aromatic, heteroaromatic, heterocyclic or bicyclic ring A is optionally substituted with one or more substituents independently selected from the group consisting of C₁-C₄ alkyl, -NR¹R², -C(O)CZ₃, -OR⁴, halogen and =O;
Y is absent, -O-, -OCH₂-, -CH₂-, -NR³-, or -CH(NH₂)-;
B is absent or a 5 or 6 membered aromatic or heteroaromatic ring with 0 to 4 heteroatom(s) selected among N, O and S, the aromatic or heteroaromatic ring B is optionally substituted with one or more substituents independently selected from the group consisting of C₁-C₄ alkyl, -NR¹R², -C(O)CZ₃, - OR⁴, halogen and =O;
R¹, R², R³ and R⁴ are independently hydrogen or C₁-C₄ alkyl;
Z is halogen; and
R⁵ is hydroxyl or C₁-C₄ alkoxy,
or an enantiomer thereof, a diastereomer thereof, a racemate thereof, or a pharmaceutically acceptable salt thereof.

Another aspect of the invention relates to a pharmaceutical composition comprising a compound according to above and at least one pharmaceutically acceptable excipient, carrier or diluent.

Further aspects of the invention relate to a compound according to above for use as a medicament, for use in prevention or treatment of cancer and for use in prevention or treatment of a neurodegenerative disease, a psychiatric disease, a motor neuron disease, peripheral neuropathies, pain, neuroinflammation, atherosclerosis, hyperlipidemia, cardiovascular diseases, dermatology related disease, autoimmune diseases, preferably Alzheimer's disease and Parkinson's disease.

The present invention also relates to an intermediate for the production of a compound according to above. The intermediate is selected from the group consisting of:
(E)-2-{[(p-methoxyphenyl)methyl]amino}-5,5-dimethyl-3-hexenoic acid;
ethyl (*E*)-2-{[(*p*-methoxyphenyl)methyl]amino}-5,5-dimethyl-3-hexenoate; and
ethyl (*E*)-2-amino-5,5-dimethyl-3-hexenoate.

The compounds of the invention are efficient sortilin inhibitors and bind to sortilin with a high affinity. Binding of the sortilin inhibitors to sortilin blocks or at least significantly inhibits the binding of other ligands, such as progranulin (PGRN), to sortilin. The sortilin inhibitors can thereby be used in the treatment of diseases, disorders, and conditions associated with such ligand-to-sortilin binding.

### BRIEF DESCRIPTION OF THE DRAWINGS

The embodiments, together with further objects and advantages thereof, may best be understood by making reference to the following description taken together with the accompanying drawings, in which:
Figs. 1A to 1S illustrate binding of compounds to sortilin using neurotensin competitive fluorescence polarization binding assay (FPA).
Figs. 2A to 2D illustrate that progranulin induced secondary sphere formation is reduced using small molecules targeting sortilin.
Figs. 3A to 3I illustrate that the reference compound RC3 but not the sortilin inhibitors SI1, SI5, SI8, SI25, SI32, SI39, SI51 and SI62 have agonistic properties on sphere formation.
Figs. 4A to 4I illustrate viability of cells when exposed to reference compound RC3 and sortilin inhibitors SI1, SI5, SI8, SI25, SI32, SI39, SI51 and SI51.
Fig. 5 illustrates that the sortilin inhibitor SI5 reduced colon cancer cell sphere (SC) formation.
Fig. 6 illustrates that the sortilin inhibitor SI62 reduced melanoma cancer cell sphere (MelS) formation.
Fig. 7 illustrates that reference compound RC3 induces lung metastasis in MDA-MB 231 *in vivo* xenograft model.
Fig. 8 illustrates that sortilin inhibitor SI5 does not induce lung metastasis in MDA-MB 231 *in vivo* xenograft model.

### DETAILED DESCRIPTION

The present invention generally relates to sortilin inhibitors and uses thereof.

The sortilin inhibitors of the present invention are capable of binding to sortilin and block or at least significantly inhibit the binding of other ligands, such as progranulin (PGRN), to sortilin. The sortilin inhibitors can thereby be used in the treatment of diseases, disorders, and conditions associated with sortilin activity.

Sortilin inhibitors are known in the art. WO 2014/114779 and Bioorganic & Medicinal Chemistry Letters 2014, 24: 177-180 disclose structurally unrelated *N*-substituted-5-substituted phthalic acids including N-(6-methyl-pyridin-2-yl)-5-trifluoromethyl-phthalamic acid also known as AF38469. AF38469 has in the literature a reported IC₅₀ value of 330 nM for binding to sortilin. Bioorganic & Medicinal Chemistry Letters 2020, 30: 127403 discloses two series of inhibitors that disrupt PGRN-binding to sortilin. The best, optimized compounds have reported IC₅₀ values of 20-90 nM.

The sortilin inhibitors of the present invention differ from the optimized compounds disclosed in Bioorganic & Medicinal Chemistry Letters 2020, 30: 127403 by, among others, having an unsaturated aliphatic moiety instead of such a saturated aliphatic moiety. Experimental data as presented herein shows that the binding affinity of the sortilin inhibitors can be unexpectedly increased (lower IC₅₀ values) when comparing two sortilin inhibitors going from a saturated aliphatic moiety to an unsaturated aliphatic moiety.

Furthermore, AF38469 (referred to as RC3 herein) possessed sortilin agonistic properties when used alone, i.e., not in combination with PGRN. These agonistic properties resulted in increased cancer stem cell (sphere) formation *in vitro* and induced lung metastasis *in vivo.* The sortilin inhibitors of the present invention do not have these agonistic properties of AF38469 but rather have sortilin antagonistic properties when used alone resulting in no increase in sphere formation but rather a reduction in sphere formation.

### DEFINITIONS

The following abbreviations are used herein.
- ACN: Acetonitrile
- ADP: Adenosine diphosphate
- ALS: Amyotrophic lateral sclerosis
- ALS-FTD: Amyotrophic lateral sclerosis with frontotemporal dementia
- AP1: Adaptor protein 1
- AP2: Adaptor protein 2
- APOE: Apo lipoprotein E
- APP: Amyloid precursor protein
- AZQ: Diaziquone
- BACE1: Beta secretase 1
- BCNU: Carmustine
- BINAP: 2,2'-Bis(diphenylphosphino)-1,1'-binaphthyl
- Br: Bromine
- bs: Broad signal
- BSA: Bovine serum albumin
- CaCl₂: Calcium chloride
- CCNU: Lomustine
- CD₃OD: Tetra deuterated methanol
- CHO: Chinese hamster ovary
- Cl: Chlorine
- CO₂: Carbon dioxide
- d: Doublet
- dd: Doublet of doulbets
- DCM: Dichloromethane
- DIPEA: N,N-Diisopropylethylamine
- DMF: Dimethylformamide
- DMSO: Dimethyl sulfoxide
- DMSO-d₆: Hexadeuterodimethyl sulfoxide
- EGF: Epidermal growth factor
- EtOH: Ethanol
- F: Flourine
- FITC: Fluorescein isothiocyanate
- FTD: Frontotemporal dementia
- FPA: Fluorescence polarization binding assay
- g/mg: Gram/Milligram
- GGA: Golgi-localized, gamma-ear containing, ADP-ribosylation factor
- ¹H-NMR: Proton nuclear magnetic resonance
- h: Hour
- H₂: Hydrogen gas
- HATU: Hexafluorophosphate azabenzotriazole tetramethyl uranium
- HEPES: 4-(2-Hydroxyethyl)-1-piperazineethanesulfonic acid
- His6-tag: Hexa histidine tag
- H₂O: Water
- HCl: Hydrochloric acid
- HOBt: Hydroxybenzotriazole
- HPLC: High performance liquid chromatography
- Hz/MHz: Hertz/Mega hertz
- I: Iodine
- IC₅₀: Half maximal inhibitory concentration
- IC1-3: Intermediate compound 1-3
- IMAC: Immobilized metal ion affinity chromatography
- K₂CO₃: Potassium carbonate
- LC: Liquid chromatography
- LCMS: Liquid chromatography mass spectrometry
- LDL: Low density lipoprotein
- m: Multiplet
- M/mM/µM: Molar/Millimolar/Micromolar
- M⁺: Positive charged molecular mass ion
- MeCN: Acetonitrile
- MeOH: Methanol
- MeCCNU: Semustine
- ml/mL/µl/µL: Millilitre/Microlitre
- mm/µm: Millimeter/Micrometer
- MMA: Monomelic amyotrophy
- MNU: N-Nitroso-N-methylurea
- mol/mmol: Mole/Millimole
- MSD: Mass detection
- MTBE: Methyl tert-butyl ether
- N: Nitrogen
- nm: Nanometre
- NaBH₄: Sodium borohydride
- NaCl: Sodium chloride
- NaHCO₃: Sodium hydrogen carbonate
- NH₄Cl: Ammonium chloride
- NOD: Non-obese diabetic
- NTR3: Neurotensin receptor 3
- NTRK1: Neurotrophic receptor tyrosine kinase 1
- NTRK2: Neurotrophic receptor tyrosine kinase 2
- NTRK3: Neurotrophic receptor tyrosine kinase 3
- O: Oxygen
- O₂: Oxygen gas
- Pd/C: Palladium on carbon
- Pd(OAc)₂: Palladium(//) acetate
- p75NTR: p75 neurotrophin receptor
- PBP: Progressive bulbar palsy
- PBS: Phosphate buffered saline
- PCSK9: Proprotein convertase subtilisin/kexin type 9
- PLS: Primary lateral sclerosis
- PMA: Progressive muscular atrophy
- PRGN: Progranulin
- Pro-BDNF: Pro brain derived neurothropic factor
- Pro-NGF: Pro nerve growth factor
- P/S: Penicillin/Streptomycin
- PTSA: p-Toluensulfonic acid
- RC1-3: Reference compound 1-3
- RP-HPLC: Reverse phase high-performance liquid chromatography
- q: Quartet
- s: Singlet
- S: Sulfur
- SCID: Severe combined immunodeficiency
- SEM-Cl: 2-(trimethylsilyl)ethoxymethyl chloride
- SFC: Supercritical fluid chromatography
- SI1-48: Sortilin inhibitor 1-48
- SMA: Spinal muscular atrophy
- sSORT1: Secreted or soluble sortilin
- t: Triplet
- T₃P: n-Propanephosphonic acid anhydride
- TGN: Trans-golgi network
- THF: Tetrahydrofuran
- Ti(OEt)₄: Titanium (IV) ethoxide
- TLC: Thin layer chromatography
- Trk receptors: Tyrosine kinase receptors
- UPLC: Ultra performance liquid chromatography
- VLDL: Very low density lipoprotein
- Vps10p: Vacuolar protein sorting 10 protein

As used herein, the term "C₁-C₄ alkyl" includes methyl (-CH₃), ethyl (-CH₂CH₃), propyl (-CH₂CH₂CH₃), isopropyl (-CH(CH₃)₂), butyl (-CH₂CH₂CH₂CH₃), *sec*-butyl (-CH(CH₃)(CH₂CH₃)), isobutyl (-CH₂CH(CH₃)₂) and *tert*-butyl (-C(CH₃)₃).

As used herein, the term "C₁-C₄ alkoxy" includes methoxy (-OCH₃), ethoxy (-OCH₂CH₃), propoxy (-OCH₂CH₂CH₃), isopropoxy (-OCH(CH₃)₂), butoxy (-OCH₂CH₂CH₂CH₃), *sec*-butoxy (-OCH(CH₃)(CH₂CH₃)), isobutoxy (-OCH₂CH(CH₃)₂) and *tert*-butoxy (-OC(CH₃)₃).

As used herein, the term "halogen" includes fluorine (F), chlorine (Cl), bromine (Br) and iodine (I). In a particular embodiment, halogen as used herein includes F, Cl and Br.

As used herein, the term "heteroatom" relates to a non-carbon atom replacing a carbon atom in a ring structure. According to the invention, heteroatom is selected among nitrogen (N), oxygen (O) and sulfur (S). In a particular embodiment, heteroatom is selected among N and O. In a preferred embodiment, heteroatom is N.

As used herein, the term "sortilin" may refer to full length sortilin (also referred to as immature sortilin), comprising a signal peptide, a propeptide, a Vps10p domain, a 10CC domain, a transmembrane domain and a large cytoplasmic tail, having an amino acid sequence according to SEQ ID NO: 1, or it may refer to mature sortilin comprising a Vps10p domain, a 10CC domain, a transmembrane domain and a large cytoplasmic tail, having an amino acid sequence according to SEQ ID NO: 2, or a naturally occurring fragment, homologue or variant thereof. Sortilin as used herein also encompasses secreted or soluble sortilin (sSORT1), which lacks the transmembrane domain and the large cytoplasmic tail. It is understood that sortilin is capable of interacting with progranulin (PGRN) to form a sortilin/PGRN complex.

As used herein, the term "progranulin" or ""PGRN" is a 593 amino acid long (SEQ ID NO: 3) and 68.5 kDa protein. Progranulin is precursor protein for granulin. Cleavage of progranulin produces a variety of smaller active cleavage products named granulin A, granulin B, granulin C, etc.

As used herein, the terms "sortilin antagonist" or "sortilin inhibitor" refer to a compound that interferes with, blocks, or otherwise attenuates the effect of, PGRN-binding to a sortilin molecule and preventing or at least inhibiting the formation of the complex between sortilin and PGRN. Compounds of formula I as disclosed herein act as sortilin antagonists or inhibitors by binding to sortilin.

As used herein, the term "enantiomer" is one of two stereoisomers that are mirror images of each other and that are non-superposable (not identical). A single chiral atom or similar structural feature in a compound causes that compound to have two possible structures which are non-superposable, each a mirror image of the other. A sample of a compound is considered enantiopure or enantiomerically pure when it has, within the limits of detection, molecules of only one chirality.

As used herein, the terms "racemate" or "racemic mixture" is a mixture having equal amounts of both enantiomers of a chiral molecule.

As used herein, the term "diastereomers" refer to stereoisomers that are not related as mirror images and are not enantiomers. Unlike enantiomers, which are mirror images of each other and non-superimposable, diastereomers are not mirror images. Diastereomers have two or more stereocenters and can have different physical properties and reactivity.

As used herein, the term "pharmaceutically acceptable salt" comprises therapeutically active non-toxic acid and base addition salt forms that the compounds of the invention are able to form. Compounds that have basic properties can be converted to their pharmaceutically acceptable acid addition salts by treating the base form with an appropriate acid. Exemplary acids include inorganic acids, such as hydrogen chloride, hydrogen bromide, hydrogen iodide, sulphuric acid, phosphoric acid; and organic acids, such as formic acid, acetic acid, propanoic acid, hydroxyacetic acid, lactic acid, pyruvic acid, glycolic acid, maleic acid, malonic acid, oxalic acid, benzenesulphonic acid, toluenesulphonic acid, methanesulphonic acid, trifluoroacetic acid, fumaric acid, succinic acid, malic acid, tartaric acid, citric acid, salicylic acid, p-aminosalicylic acid, pamoic acid, benzoic acid, ascorbic acid and the like. Compounds that have acidic properties can be converted to their pharmaceutically acceptable base addition salts by treating the acid form with an appropriate base. Suitable base addition salt forms are the sodium, potassium, calcium salts, and salts with pharmaceutically acceptable amines such as, for example, ammonia, alkylamines, benzathine, and amino acids, such as, e.g., arginine and lysine. The term "addition salt", as used herein, also comprises solvates, which the compounds and salts thereof are able to form, such as, for example, hydrates, alcoholates and the like.

An aspect of the invention relates to a compound of formula I below 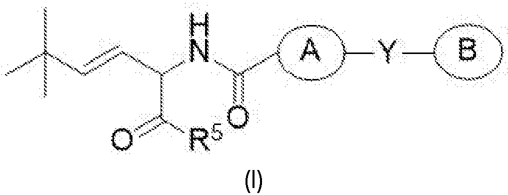 wherein,
A is a 5 or 6 membered aromatic, heteroaromatic or heterocyclic ring with 0 to 2 heteroatom(s) selected among N, O and S, or a 8 to 10 membered bicyclic heterocyclic or heteroaromatic ring with 1 or 2 heteroatom(s) selected among N, O and S, the aromatic, heteroaromatic, heterocyclic or bicyclic ring A is optionally substituted with one or more substituents independently selected from the group consisting of C₁-C₄ alkyl, -NR¹R², -C(O)CZ₃, -OR⁴, halogen, and =O;
Y is absent, -O-, -OCH₂-, -CH₂-, -NR³-, or -CH(NH₂)-;
B is absent or a 5 or 6 membered aromatic or heteroaromatic ring with 0 to 4 heteroatom(s) selected among N, O and S, the aromatic or heteroaromatic ring B is optionally substituted with one or more substituents independently selected from the group consisting of C₁-C₄ alkyl, -NR¹R², -C(O)CZ₃, -OR⁴, halogen, and =O;
R¹, R², R³ and R⁴ are independently hydrogen or C₁-C₄ alkyl;
Z is halogen; and
R⁵ is hydroxyl or C₁-C₄ alkoxy,
or an enantiomer thereof, a diastereomer thereof, a racemate thereof, a prodrug thereof, or a pharmaceutically acceptable salt thereof.

A particular embodiment relates to a compound of formula I according to above or an enantiomer thereof, a diastereomer thereof, a racemate thereof, or a pharmaceutically acceptable salt thereof.

The compounds of formula I are capable of binding to sortilin with a high affinity (IC₅₀ value in nM range). The compounds of formula I are thereby suitable as sortilin inhibitors. These sortilin inhibitors of the invention are capable of blocking or at least significantly inhibiting the binding of other ligands, such as PGRN, to sortilin. The sortilin inhibitors can thereby be used in the treatment of diseases, disorders, and conditions associated with the PGRN-sortilin axis or the interaction between sortilin and its other ligands. In fact, the sortilin inhibitors of the invention having an unsaturated aliphatic moiety, see hatched ring in formula II below, have higher binding affinity to sortilin as compared to optimized reference compounds having a saturated aliphatic moiety *(*Bioorganic & Medicinal Chemistry Letters 2020 30: 127403).

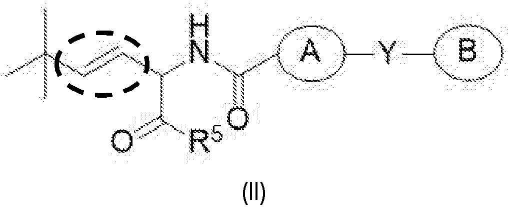

The sortilin inhibitors of the invention furthermore have biological effect as seen in reducing formation of cancer stem cells (spheres). Such cancer stem cells are a population of generally treatment resistant and aggressive cancer cells that may survive or circumvent traditional chemotherapy. Such cancer stem cells could then seed a new tumor that is resistant to chemotherapy. Hence, presence of cancer stem cells generally lead to cancer recurrences and metastasis, and thereby treatment failures. This means that there is a general need to target such aggressive cancer cells. PGRN has previously been identified as the most effective among over 500 cytokines in inducing cancer stem cells and lung metastasis in breast cancer *(*Breast Cancer Research 2018 20: 137). Furthermore, a highly malignant subgroup of cancer cells co-express PRGN and sortilin *(*BMC Cancer 2021 21: 185). Sortilin inhibitors of the invention are capable of inhibiting these effects.

Y is, as mentioned above, absent, -O-, -OCH₂-, -CH₂-, -NR³-, or -CH(NH₂)-. As used herein -OCH₂-represent -OCH₂- and -CH₂O-. Thus, according to the invention, Y is absent, -O-, -OCH₂-, -CH₂O-, - CH₂-, -NR³-, or -CH(NH₂)-.

In an embodiment, A is a 5 or 6 membered aromatic, heteroaromatic or heterocyclic ring with 0 to 2 heteroatom(s) selected among N and O or a 8 to 10 membered bicyclic heterocyclic or heteroaromatic ring with 1 or 2 heteroatom(s) selected among N and O. Hence, currently preferred heteroatoms for the heteroaromatic, heterocyclic or bicyclic ring A are nitrogen and/or oxygen atoms. In a particular embodiment, A is a 5 or 6 membered aromatic, heteroaromatic or heterocyclic ring with 0 to 2 nitrogen atom(s) or a 8 to 10 membered bicyclic heterocyclic or heteroaromatic ring with 1 or 2 nitrogen atoms. In this particular embodiment, any heteroatoms for the heteroaromatic, heterocyclic or bicyclic ring A are nitrogen atoms.

In an embodiment, A is a 5 or 6 membered aromatic, heteroaromatic or heterocyclic ring with 0 to 2 heteroatom(s) selected among N, O and S, preferably selected among N and O, and more preferably N.

In an embodiment, A is a 5 membered heteroaromatic or heterocyclic ring with 1 to 2 heteroatom(s) selected among N, O and S, preferably selected among N and O, and more preferably N. In a particular embodiment, A is 5 membered heteroaromatic ring with 1 to 2 heteroatom(s) selected among N, O and S, preferably selected among N and O, and more preferably N. In an another particular embodiment, A is a 5 membered heterocyclic ring with 1 to 2 heteroatom(s) selected among N, O and S, preferably selected among N and O, and more preferably N.

In another embodiment, A is a 6 membered aromatic, heteroaromatic or heterocyclic ring with 0 to 2 heteroatom(s) selected among N, O and S, preferably selected among N and O, and more preferably N. In a particular embodiment, A is 6 membered aromatic ring. In another particular embodiment, A is 6 membered heteroaromatic ring with 1 to 2 N as heteroatom(s). In a further particular embodiment, A is a 6 membered heterocyclic ring with 1 to 2 heteroatom(s) selected among N, O and S, preferably selected among N and O, and more preferably N.

In an embodiment, A is a 8 to 10 membered bicyclic heterocyclic or heteroaromatic ring with 1 or 2 heteroatom(s) selected among N, O and S, preferably selected among N and O, and more preferably N. In an embodiment, A is a 9 membered bicyclic heterocyclic or heteroaromatic ring with 1 or 2 heteroatom(s) selected among N, O and S, preferably selected among N and O, and more preferably N. In a particular embodiment, A is a 9 membered bicyclic heterocyclic ring with 1 or 2 heteroatom(s) selected among N, O and S, preferably selected among N and O, and more preferably N. In another particular embodiment, A is a 9 membered heteroaromatic ring with 1 or 2 heteroatom(s) selected among N, O and S, preferably selected among N and O, and more preferably N.

In the above described embodiments, the ring A is optionally substituted with one or more substituents independently selected from the group consisting of C₁-C₄ alkyl, -NR¹R², -C(O)CZ₃, -OR⁴, halogen, and =O.

In an embodiment, A is a 5 or 6 membered aromatic, heteroaromatic or heterocyclic ring with 0 to 2 heteroatom(s) selected among N and O, preferably N, or a 9 membered bicyclic heterocyclic ring with 1 or 2 heteroatom(s) selected among N and O, preferably N. The aromatic, heteroaromatic, heterocyclic or bicyclic ring A is, in this embodiment, optionally substituted with one or more substituents independently selected from the group consisting of C₁-C₄ alkyl, -NR¹R², -C(O)CZ₃, -OR⁴, halogen, and =O.

In an embodiment, any C₁-C₄ alkyl substituent of the aromatic, heteroaromatic, heterocyclic or bicyclic ring A is preferably independently selected from the group consisting of methyl, isobutyl and tert-butyl.

In an embodiment, R¹ and R² are independently selected from the group consisting of hydrogen, methyl and ethyl. In a preferred embodiment, R¹ and R² are independently selected from the group consisting of hydrogen and methyl. In a particular preferred embodiment, R¹ and R² are both methyl.

In a preferred embodiment, the aromatic, heteroaromatic, heterocyclic or bicyclic ring A is optionally substituted with one or more substituents independently selected from the group consisting of -CH₃, - CH₂CH(CH₃)₂, -C(CH₃)₃, -N(CH₃)₂, -C(O)CZ₃, halogen, and =O.

In an embodiment, Z is selected from the group consisting of F, Cl and Br. In a particular embodiment Z is F or Cl. In a particular preferred embodiment, Z is F. In such a particular embodiment, the aromatic, heteroaromatic, heterocyclic or bicyclic ring A is optionally substituted with one or more substituents independently selected from the group consisting of -CH₃, -CH₂CH(CH₃)₂, -C(CH₃)₃, -N(CH₃)₂, - C(O)CF₃ and halogen.

In an embodiment, a halogen substituent of the ring A is preferably selected from the group consisting of F, Cl and Br. In a particular embodiment, the halogen substituent of the ring A is Cl or Br. In such a particular embodiment, the aromatic, heteroaromatic, heterocyclic or bicyclic ring A is optionally substituted with one or more substituents independently selected from the group consisting of -CH₃, - CH₂CH(CH₃)₂, -C(CH₃)₃, -N(CH₃)₂, -C(O)CZ₃, -Cl and -Br.

In a currently preferred embodiment, the aromatic, heteroaromatic, heterocyclic or bicyclic ring A is optionally substituted with one or more substituents independently selected from the group consisting of -CH₃, -CH₂CH(CH₃)₂, -C(CH₃)₃, -N(CH₃)₂, -C(O)CF₃, -Cl, and -Br.

As mentioned in the foregoing, Y is absent, -O-, -OCH₂-,-CH₂-, -NR³-, or -CH(NH₂)-. In the case of absent, Y represents a direct link or bond between the aromatic, heteroaromatic, heterocyclic or bicyclic ring A and the aromatic or heteroaromatic ring B, if present.

In an embodiment, R³ is selected from the group consisting of hydrogen, methyl and ethyl. In a particular embodiment, R³ is selected from the group consisting of hydrogen and methyl. In a preferred particular embodiment, R³ is hydrogen.

In a particular embodiment, Y is absent, -O-, -OCH₂-, -CH₂-, -NH- or -CH(NH₂)-, or preferably Y is absent, -O-, -OCH₂-, -NH- or -CH(NH₂)-. In another particular embodiment, Y is absent or O. In a preferred embodiment, Y is absent. Hence, in such an embodiment, the compound is represented by formula III below 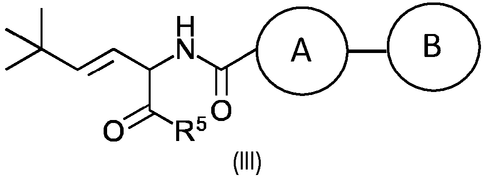 wherein
A is a 5 or 6 membered aromatic, heteroaromatic or heterocyclic ring with 0 to 2 heteroatom(s) selected among N, O and S, or a 8 to 10 membered bicyclic heterocyclic or heteroaromatic ring with 1 or 2 heteroatom(s) selected among N, O and S, the aromatic, heteroaromatic, heterocyclic or bicyclic ring A is optionally substituted with one or more substituents independently selected from the group consisting of C₁-C₄ alkyl, -NR¹R², -C(O)CZ₃, -OR⁴, halogen, and =O;
B is absent or a 5 or 6 membered aromatic or heteroaromatic ring with 0 to 4 heteroatom(s) selected among N, O and S, the aromatic or heteroaromatic ring B is optionally substituted with one or more substituents independently selected from the group consisting of C₁-C₄ alkyl, -NR¹R², -C(O)CZ₃, -OR⁴, halogen, and =O;
R¹, R², R³ and R⁴ are independently hydrogen or C₁-C₄ alkyl;
Z is halogen; and
R⁵ is hydroxyl or C₁-C₄ alkoxy,
or an enantiomer thereof, a diastereomer thereof, a racemate thereof, a prodrug thereof, or a pharmaceutically acceptable salt thereof, such as enantiomer thereof, a diastereomer thereof, a racemate thereof, or a pharmaceutically acceptable salt thereof.

In an embodiment, B is absent or a 5 or 6 membered aromatic or heteroaromatic ring with 0 to 4 heteroatom(s) selected among N and O. Hence, currently preferred heteroatoms for the heteroaromatic ring B are nitrogen and/or oxygen atoms. In a particular embodiment, B is absent or a 5 or 6 membered aromatic or heteroaromatic ring with 0 to 4 nitrogen atom(s). In this particular embodiment, any heteroatoms for the heteroaromatic ring B are nitrogen atoms. In these embodiment, the aromatic or heteroaromatic ring B is optionally substituted with one or more substituents independently selected from the group consisting of C₁-C₄ alkyl, -NR¹R², -C(O)CZ₃, -OR⁴, halogen, and =O.

In an embodiment, B is absent, a 5 membered heteroaromatic ring with 1 to 4 heteroatom(s) selected among N, O and S, preferably selected among N and O, and more preferably N, a 6 membered aromatic ring or a 6 membered heteroaromatic ring with 1 to 4 N as heteroatom(s). In this embodiment, the aromatic or heteroaromatic ring B is optionally substituted with one or more substituents independently selected from the group consisting of C₁-C₄ alkyl, -NR¹R², -C(O)CZ₃, -OR⁴, halogen, and =O.

In an embodiment, B is absent.

In another embodiment, B is a 6 membered aromatic ring. The aromatic ring B is optionally substituted with one or more substituents independently selected from the group consisting of C₁-C₄ alkyl, -NR¹R², -C(O)CZ₃, -OR⁴, halogen, and =O.

In a further embodiment, B is a 5 or 6 membered heteroaromatic ring with 1 to 4 heteroatom(s) selected among N, O and S, preferably selected among N and O, and more preferably N. The heteroaromatic ring B is optionally substituted with one or more substituents independently selected from the group consisting of C₁-C₄ alkyl, -NR¹R², -C(O)CZ₃, -OR⁴, halogen, and =O.

In an embodiment, B is absent or a 5 or 6 membered aromatic or heteroaromatic ring with 0 to 4 heteroatom(s) selected among N, O and S. The aromatic or heteroaromatic ring B is optionally substituted with one or more substituents independently selected from the group consisting of -OR⁴ and =O. In a particular embodiment, B is absent or a 5 or 6 membered aromatic or heteroaromatic ring with 0 to 4 heteroatom(s) selected among N and O, preferably N. The aromatic or heteroaromatic ring B is optionally substituted with one or more substituents independently selected from the group consisting of -OR⁴ and =O.

In an embodiment, R⁴ is selected from the group consisting of hydrogen, methyl and ethyl. Hence, in such an embodiment the substituent -OR⁴ is selected from the group consisting of hydroxyl (-OH), methoxy (-OCH₃) and ethoxy (-OCH₂CH₃). In a particular embodiment, R⁴ is selected from the group consisting of hydrogen and methyl.

In a particular embodiment, the aromatic or heteroaromatic ring B is optionally substituted with one or more substituents independently selected from the group consisting of hydroxyl, methoxy or =O.

As mentioned above, R⁵ is hydroxyl or C₁-C₄ alkoxy. In a particular embodiment, R⁵ is hydroxyl or C₁-C₂ alkoxy, i.e., R⁵ is selected from the group consisting of hydroxyl, methoxy and ethoxy. In a preferred particular embodiment, R⁵ is hydroxyl.

In an embodiment, A is a 5 or 6 membered aromatic, heteroaromatic or heterocyclic ring with 0 to 2 heteroatom(s) selected among N, O and S, preferably selected among N and O, and more preferably N. The aromatic, heteroaromatic or heterocyclic ring A is optionally substituted with one or more substituents independently selected from the group consisting of C₁-C₄ alkyl, -NR¹R², -C(O)CZ₃, -OR⁴, halogen and =O, preferably independently selected from the group consisting of methyl, isopropyl, isobutyl, -N(CH₃)₂, -N(CH₂CH₃)₂, -C(O)CF₃, Cl and Br. In this embodiment, Y is absent, -O-, -OCH₂-, - CH₂-, -NH- or -CH(NH₂)-. In this embodiment, B is absent, a 6 membered aromatic ring or a 5 or 6 membered heteroaromatic ring with 0 to 4 heteroatom(s) selected among N, O and S, preferably selected among N and O, and more preferably N. The aromatic or heteroaromatic ring B is optionally substituted with one or more substituents independently selected from the group consisting of C₁-C₄ alkyl, -NR¹R², -C(O)CZ₃, -OR⁴, halogen and =O, preferably independently selected from the group consisting of hydroxyl, methoxy and =O. In this embodiment, R⁵ is hydroxyl or C₁-C₄ alkoxy, preferably hydroxyl or C₁-C₂ alkoxy and more preferably hydroxyl.

Currently preferred compounds of formula I include the following compounds: 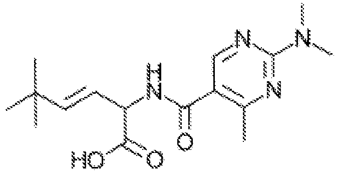
(*E*)-2-[2-(dimethylamino)-4-methyl-5-pyrimidinylcarbonylamino]-5,5-dimethyl-3-hexenoic acid (sortilin inhibitor 1 (SI1)) 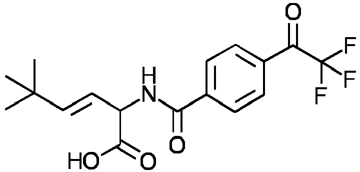
(*E*)-5,5-dimethyl-2-[*p*-(2,2,2-trifluoroacetyl)benzoylamino]-3-hexenoic acid (SI2) 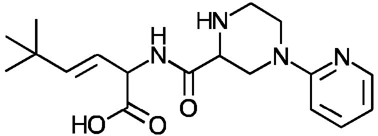
(*E*)-5,5-dimethyl-2-[4-(2-pyridyl)-2-piperazinylcarbonylamino]-3-hexenoic acid (SI3) 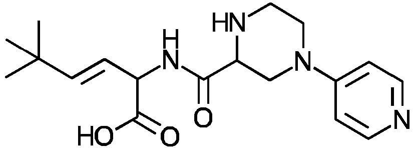
(*E*)-5,5-dimethyl-2-[4-(4-pyridyl)-2-piperazinylcarbonylamino]-3-hexenoic acid (SI4) 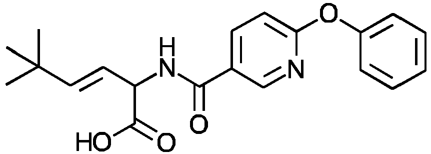
(*E*)-5,5-dimethyl-2-(6-phenoxynicotinoylamino)-3-hexenoic acid (SI5) 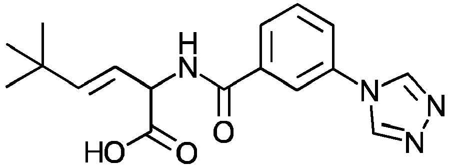
(E)-5,5-dimethyl-2-[m-(4H-1,2,4-triazol-4-yl)benzoylamino]-3-hexenoic acid (SI6) 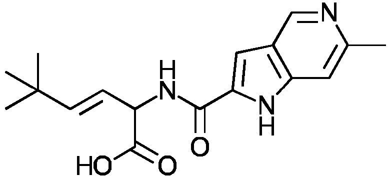
(*E*)-5,5-dimethyl-2-(6-methyl-1*H*-1,5-diazainden-2-ylcarbonylamino)-3-hexenoic acid (SI7) 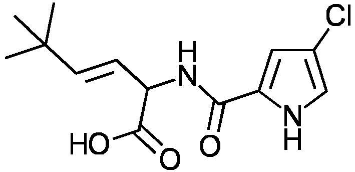
(*E*)-2-(4-chloro-2-pyrrolylcarbonylamino)-5,5-dimethyl-3-hexenoic acid (SI8) 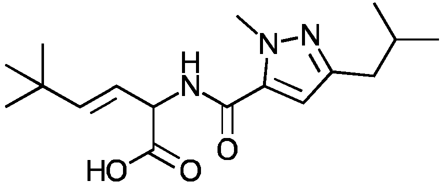
(*E*)-2-(3-isobutyl-1-methyl-5-pyrazolylcarbonylamino)-5,5-dimethyl-3-hexenoic acid (SI9) 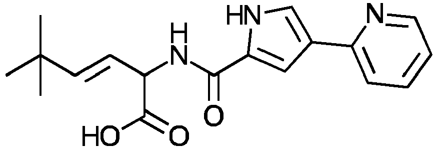
(*E*)-5,5-dimethyl-2-[4-(2-pyridyl)-2-pyrrolylcarbonylamino]-3-hexenoic acid (S110) 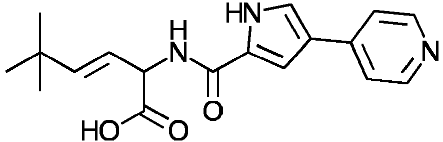
(*E*)-5,5-dimethyl-2-[4-(4-pyridyl)-2-pyrrolylcarbonylamino]-3-hexenoic acid (S111) 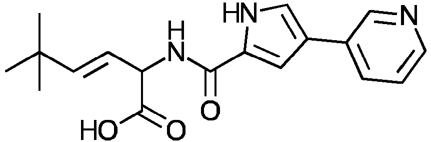
(*E*)-5,5-dimethyl-2-[4-(3-pyridyl)-2-pyrrolylcarbonylamino]-3-hexenoic acid (S112) 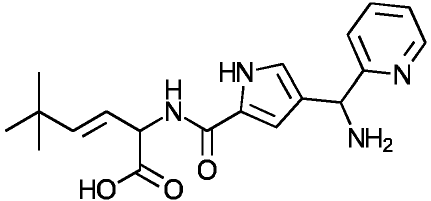
(*E*)-2-{4-[amino(2-pyridyl)methyl]-2-pyrrolylcarbonylamino}-5,5-dimethyl-3-hexenoic acid (SI13) 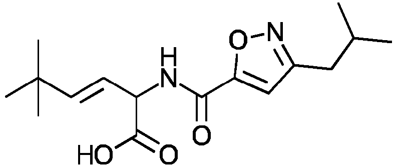
(*E*)-2-(3-isobutyl-5-isoxazolylcarbonylamino)-5,5-dimethyl-3-hexenoic acid (SI14) 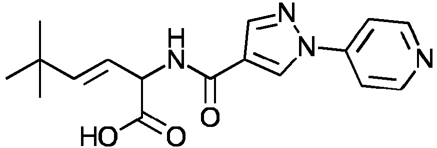
(*E*)-5,5-dimethyl-2-[1-(4-pyridyl)-4-pyrazolylcarbonylamino]-3-hexenoic acid (SI15) 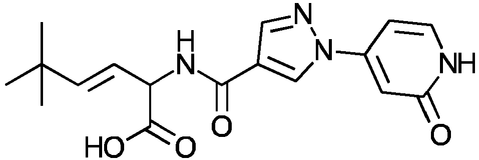
(*E*)-5,5-dimethyl-2-[1-(2-oxo-4-pyridyl)-4-pyrazolylcarbonylamino]-3-hexenoic acid (S116) 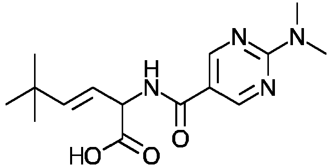
(*E*)-2-[2-(dimethylamino)-5-pyrimidinylcarbonylamino]-5,5-dimethyl-3-hexenoic acid (SI17) 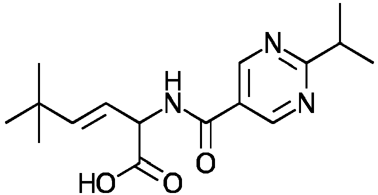
(*E*)-2-(2-isopropyl-5-pyrimidinylcarbonylamino)-5,5-dimethyl-3-hexenoic acid (SI18) 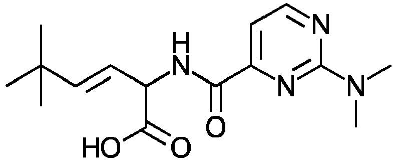
(*E*)-2-[2-(dimethylamino)-4-pyrimidinylcarbonylamino]-5,5-dimethyl-3-hexenoic acid (SI19) 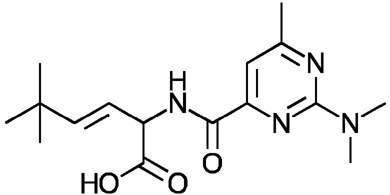
(*E*)-2-[2-(dimethylamino)-6-methyl-4-pyrimidinylcarbonylamino]-5,5-dimethyl-3-hexenoic acid (SI20) 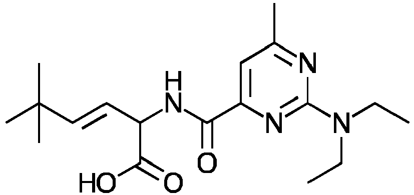
(*E*)-2-[2-(diethylamino)-6-methyl-4-pyrimidinylcarbonylamino]-5,5-dimethyl-3-hexenoic acid (SI21) 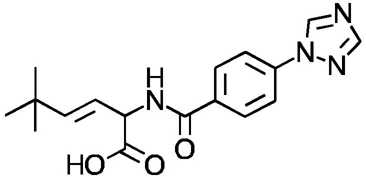
(*E*)-5,5-dimethyl-2-[*p-*(1*H*-1,2,4-triazol-1-yl)benzoylamino]-3-hexenoic acid (SI22) 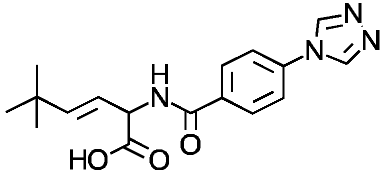
(*E*)-5,5-dimethyl-2-[*p*-(4H-1,2,4-triazol-4-yl)benzoylamino]-3-hexenoic acid (SI23) 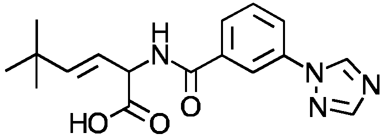
(E)-5,5-dimethyl-2-[m-(1H-1,2,4-triazol-1-yl)benzoylamino]-3-hexenoic acid (SI24) 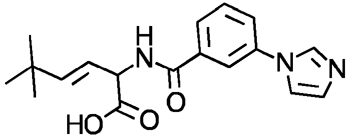
(*E*)-5,5-dimethyl-2[*m*-(1-imidazolyl)benzoylamino]--3-hexenoic acid (SI25) 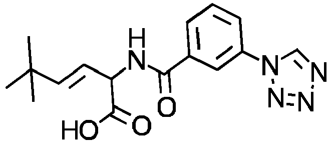
(*E*)-5,5-dimethyl-2-[m-(1*H*-tetraazol-1-yl)benzoylamino]-3-hexenoic acid (SI26) 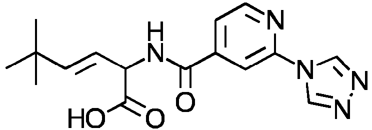
(*E*)-5,5-dimethyl-2-[2-(4*H*-1,2,4-triazol-4-yl)isonicotinoylamino]-3-hexenoic acid (SI27) 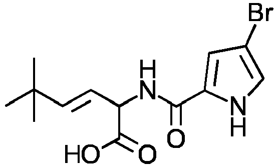
(*E*)-2-(4-bromo-2-pyrrolylcarbonylamino)-5,5-dimethyl-3-hexenoic acid (SI28) 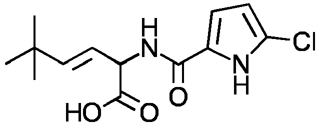
(*E*)-2-(5-chloro-2-pyrrolylcarbonylamino)-5,5-dimethyl-3-hexenoic acid (SI29) 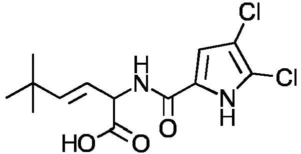
(*E*)-2-(4,5-dichloro-2-pyrrolylcarbonylamino)-5,5-dimethyl-3-hexenoic acid (SI30) 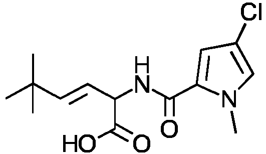
(*E*)-2-(4-chloro-1-methyl-2-pyrrolylcarbonylamino)-5,5-dimethyl-3-hexenoic acid (SI31) 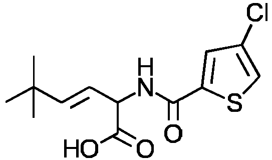
(*E*)-2-(4-chloro-2-thienylcarbonylamino)-5,5-dimethyl-3-hexenoic acid (SI32) 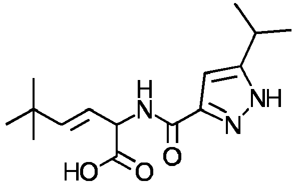
(*E*)-2-(5-isopropyl-3-pyrazolylcarbonylamino)-5,5-dimethyl-3-hexenoic acid (SI33) 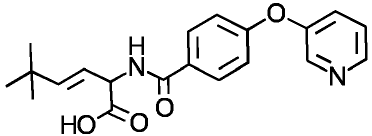
(*E*)-5,5-dimethyl-2-[*p*-(3-pyridyloxy)benzoylamino]-3-hexenoic acid (SI34) 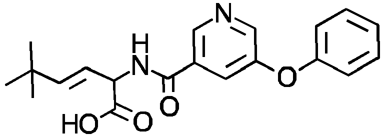
(*E*)-5,5-dimethyl-2-(5-phenoxynicotinoylamino)-3-hexenoic acid (SI35) 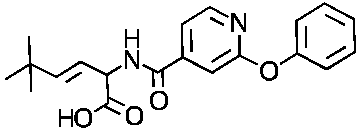
(*E*)-5,5-dimethyl-2-(2-phenoxyisonicotinoylamino)-3-hexenoic acid (SI36) 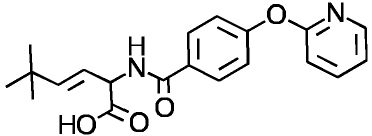
(*E*)-5,5-dimethyl-2-[*p*-(2-pyridyloxy)benzoylamino]-3-hexenoic acid (SI37) 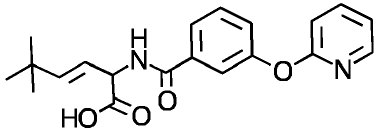
(*E*)-5,5-dimethyl-2-[m-(2-pyridyloxy)benzoylamino]-3-hexenoic acid (SI38) 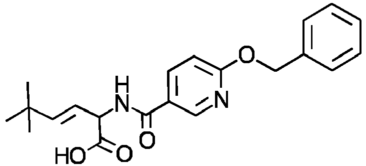
(*E*)-2-[6-(benzyloxy)nicotinoylamino]-5,5-dimethyl-3-hexenoic acid (SI39) 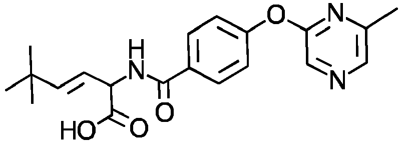
(*E*)-5,5-dimethyl-2-[*p*-(6-methyl-2-pyrazinyloxy)benzoylamino]-3-hexenoic acid (SI40) 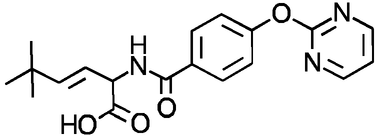
(*E*)-5,5-dimethyl-2-[*p*-(2-pyrimidinyloxy)benzoylamino]-3-hexenoic acid (SI41) 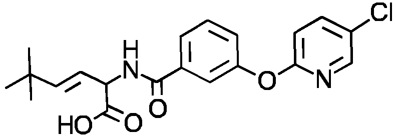
*(E)-2-[m-(5-chloro-2-pyridyloxy)benzoylamino]-5,5-dimethyl-3-hexenoic* acid (SI42) 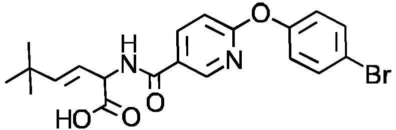
(*E*)-2-[6-(p-bromophenoxy)nicotinoylamino]-5,5-dimethyl-3-hexenoic acid (SI43) 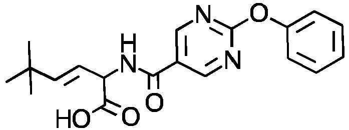
(*E*)-5,5-dimethyl-2-(2-phenoxy-5-pyrimidinylcarbonylamino)-3-hexenoic acid (SI44) 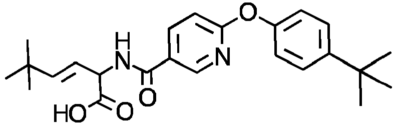
(*E*)-2-{6-[*p*-(*tert*-Butyl)phenoxy]nicotinoylamino}-5,5-dimethyl-3-hexenoic acid (SI45) 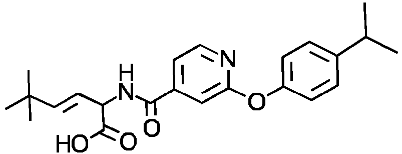
(*E*)-2-[2-(*p*-cumenyloxy)isonicotinoylamino]-5,5-dimethyl-3-hexenoic acid (SI46) 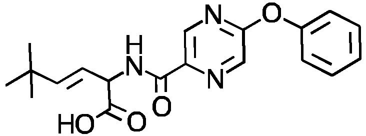
(*E*)-5,5-dimethyl-2-(5-phenoxy-2-pyrazinylcarbonylamino)-3-hexenoic acid (SI47) 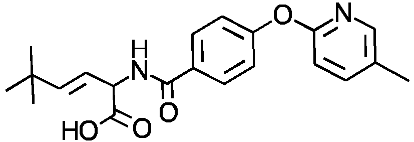
(*E*)-5,5-dimethyl-2-[*p*-(5-methyl-2-pyridyloxy)benzoylamino]-3-hexenoic acid (SI48) 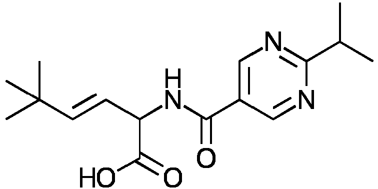
(*E*)-2-(2-isopropyl-5-pyrimidinylcarbonylamino)-5,5-dimethyl-3-hexenoic acid (SI49) 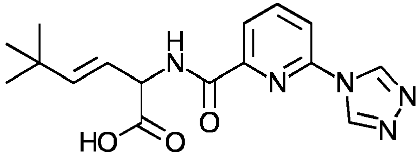
(E)-5,5-dimethyl-2-[6-(4H-1,2,4-triazol-4-yl)-2-pyridylcarbonylamino]-3-hexenoic acid (SI50) 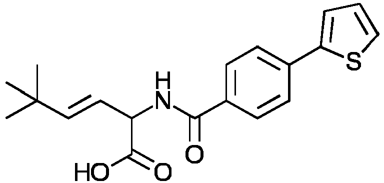
(*E*)-5,5-dimethyl-2-[*p*-(2-thienyl)benzoylamino]-3-hexenoic acid (SI51) 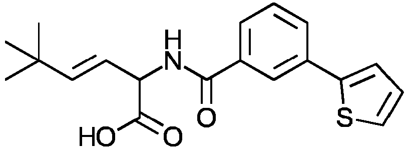
(*E*)-5,5-dimethyl-2-[*m*-(2-thienyl)benzoylamino]-3-hexenoic acid (SI52) 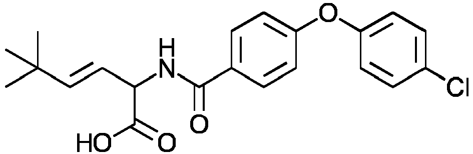
(*E*)-2-[*p*-(*p*-chlorophenoxy)benzoylamino]-5,5-dimethyl-3-hexenoic acid (SI53) 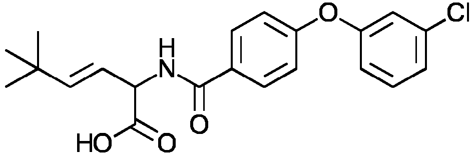
(*E*)-2-[*p*-(*m*-chlorophenoxy)benzoylamino]-5,5-dimethyl-3-hexenoic acid (SI54) 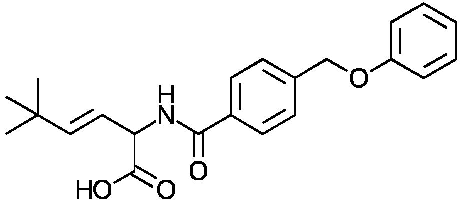
(*E*)-5,5-dimethyl-2-[*p*-(phenoxymethyl)benzoylamino]-3-hexenoic acid (SI55) 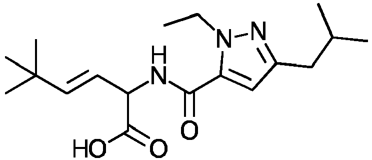
(*E*)-2-(1-ethyl-3-isobutyl-5-pyrazolylcarbonylamino)-5,5-dimethyl-3-hexenoic acid (SI56) 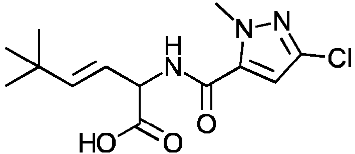
(*E*)-2-(3-chloro-1-methyl-5-pyrazolylcarbonylamino)-5,5-dimethyl-3-hexenoic acid (SI57) 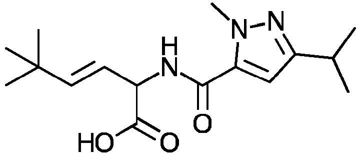
(*E*)-2-(3-isopropyl-1-methyl-5-pyrazolylcarbonylamino)-5,5-dimethyl-3-hexenoic acid (SI58) 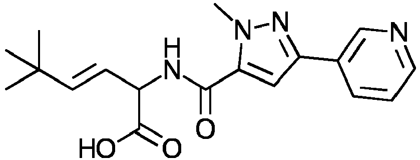
(*E*)-5,5-dimethyl-2-[1-methyl-3-(3-pyridyl)-5-pyrazolylcarbonylamino]-3-hexenoic acid (SI59) 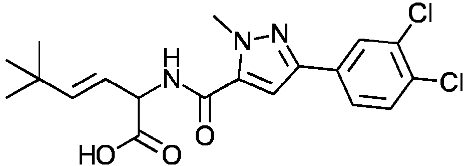
(*E*)-2-[3-(3,4-dichlorophenyl)-1-methyl-5-pyrazolylcarbonylamino]-5,5-dimethyl-3-hexenoic acid (SI60) 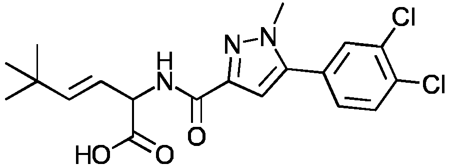
(*E*)-2-[5-(3,4-dichlorophenyl)-1-methyl-3-pyrazolylcarbonylamino]-5,5-dimethyl-3-hexenoic acid (SI61) 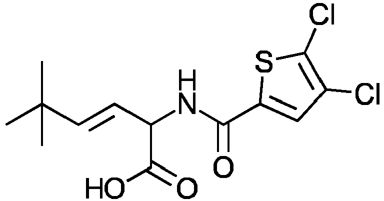
(*E*)-2-(4,5-dichloro-2-thienylamino)-5,5-dimethyl-3-hexenoic acid (SI62) 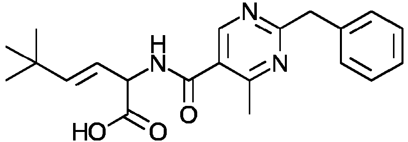
(*E*)-2-(2-benzyl-4-methyl-5-pyrimidinylcarbonylamino)-5,5-dimethyl-3-hexenoic acid (SI63) 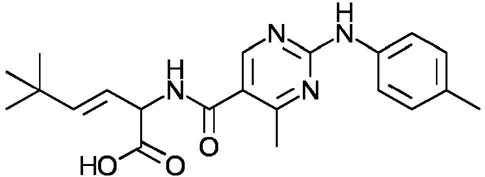
(*E*)-5,5-dimethyl-2-[4-methyl-2-(p-toluidino)-5-pyrimidinylcarbonylamino]-3-hexenoic acid (SI64) 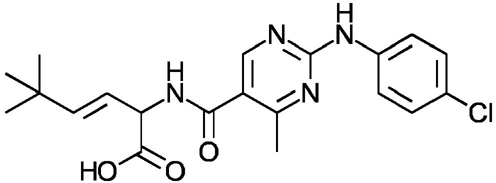
(*E*)-2-[2-(p-chlorophenylamino)-4-methyl-5-pyrimidinylcarbonylamino]-5,5-dimethyl-3-hexenoic acid (SI65) 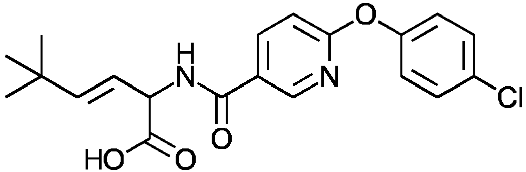
(*E*)-2-[6-(*p*-chlorophenoxy)nicotinoylamino]-5,5-dimethyl-3-hexenoic acid (SI66) 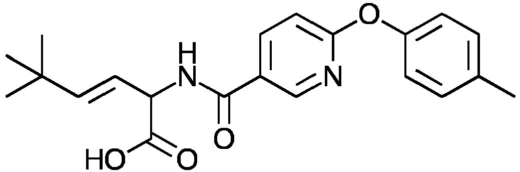
(*E*)-5,5-dimethyl-2-[6-(*p*-tolyloxy)nicotinoylamino]-3-hexenoic acid (SI67) 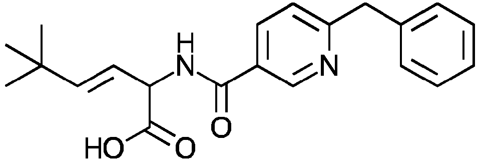
(*E*)-2-(6-benzylnicotinoylamino)-5,5-dimethyl-3-hexenoic acid (SI68) 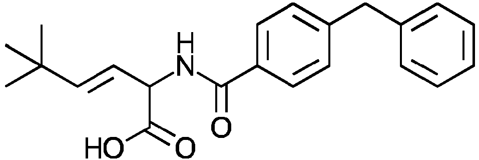
(*E*)-2-(p-benzylbenzoylamino)-5,5-dimethyl-3-hexenoic acid (SI69) 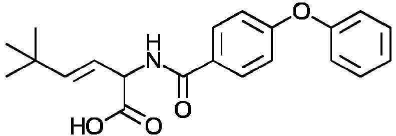
(*E*)-5,5-dimethyl-2-(p-phenoxybenzoylamino)-3-hexenoic acid (SI70) 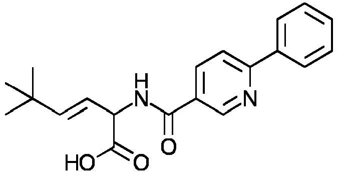
(*E*)-5,5-dimethyl-2-(6-phenylnicotinoylamino)-3-hexenoic acid (SI71) 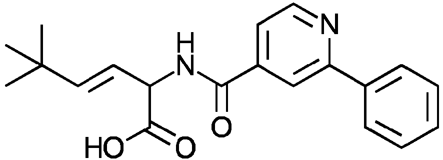
(*E*)-5,5-dimethyl-2-(2-phenylisonicotinoylamino)-3-hexenoic acid (SI72) 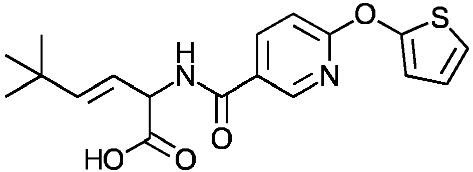
(*E*)-5,5-dimethyl-2-[6-(2-thienyloxy)nicotinoylamino]-3-hexenoic acid (SI73) 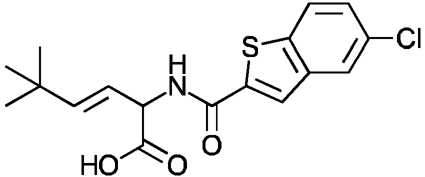
(E)-2-(5-chloro-1-benzothiophen-2-ylcarbonylamino)-5,5-dimethyl-3-hexenoic acid (SI74) 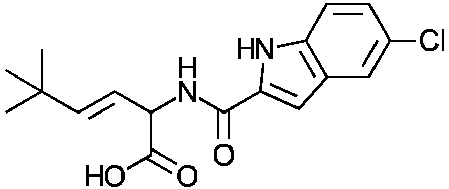
(*E*)-2-(5-chloro-2-indolylcarbonylamino)-5,5-dimethyl-3-hexenoic acid (SI75) 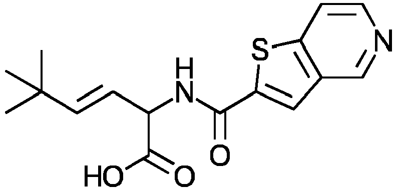
(*E*)-5,5-dimethyl-2-(1-thia-5-aza-2-indenylcarbonylamino)-3-hexenoic acid (SI76) 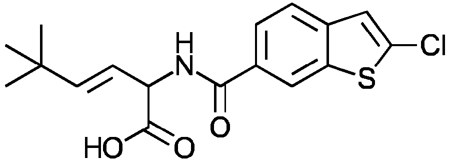
(E)-2-(2-chloro-1-benzothiophen-6-ylcarbonylamino)-5,5-dimethyl-3-hexenoic acid (SI77) 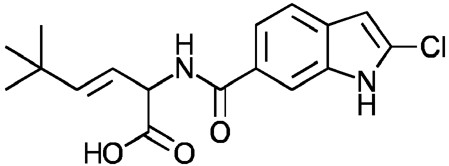
(*E*)-2-(2-chloro-6-indolylcarbonylamino)-5,5-dimethyl-3-hexenoic acid (SI78) 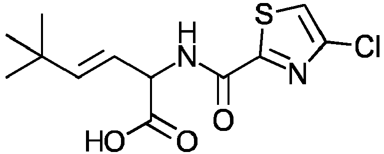
(E)-2-(4-chloro-1,3-thiazol-2-ylcarbonylamino)-5,5-dimethyl-3-hexenoic acid (SI79) 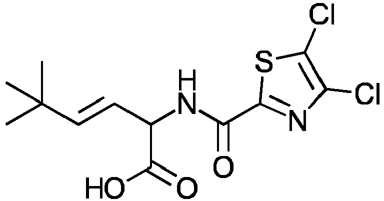
(*E*)-2-(4,5-dichloro-1,3-thiazol-2-ylcarbonylamino)-5,5-dimethyl-3-hexenoic acid (SI80) 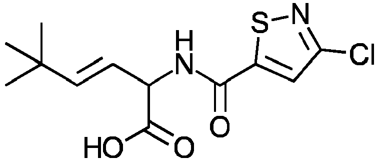
(*E*)-2-(3-chloro-5-isothiazolylcarbonylamino)-5,5-dimethyl-3-hexenoic acid (SI81) 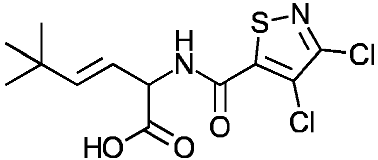
(*E*)-2-(3,4-dichloro-5-isothiazolylcarbonylamino)-5,5-dimethyl-3-hexenoic acid (SI82) 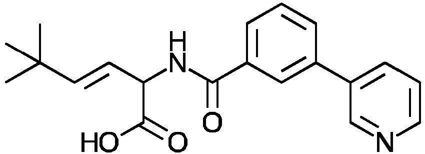
(*E*)-5,5-dimethyl-2-[m-(3-pyridyl)benzoylamino]-3-hexenoic acid (SI83) 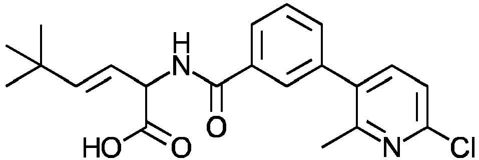
(E)-2-[m-(6-chloro-2-methyl-3-pyridyl)benzoylamino]-5,5-dimethyl-3-hexenoic acid (SI84)
or an enantiomer thereof, a diastereomer thereof, a racemate thereof, a prodrug thereof, or a pharmaceutically acceptable salt thereof, such as enantiomer thereof, a diastereomer thereof, a racemate thereof, or a pharmaceutically acceptable salt thereof.

In a particular embodiment, preferred compounds of formula I include the following compounds:
(*E*)-2-[2-(dimethylamino)-4-methyl-5-pyrimidinylcarbonylamino]-5,5-dimethyl-3-hexenoic acid (SI1);
(*E*)-5,5-dimethyl-2-[*p*-(2,2,2-trifluoroacetyl)benzoylamino]-3-hexenoic acid (SI2);
(*E*)-5,5-dimethyl-2-[4-(2-pyridyl)-2-piperazinylcarbonylamino]-3-hexenoic acid (SI3);
(*E*)-5,5-dimethyl-2-[4-(4-pyridyl)-2-piperazinylcarbonylamino]-3-hexenoic acid (SI4);
(*E*)-5,5-dimethyl-2-(6-phenoxynicotinoylamino)-3-hexenoic acid (SI5);
(*E*)-5,5-dimethyl-2-[*m*-(4*H*-1,2,4-triazol-4-yl)benzoylamino]-3-hexenoic acid (SI6);
(*E*)-5,5-dimethyl-2-(6-methyl-1*H*-1,5-diazainden-2-ylcarbonylamino)-3-hexenoic acid (SI7);
(*E*)-2-(4-chloro-2-pyrrolylcarbonylamino)-5,5-dimethyl-3-hexenoic acid (SI8);
(*E*)-2-(3-isobutyl-1-methyl-5-pyrazolylcarbonylamino)-5,5-dimethyl-3-hexenoic acid (SI9);
(*E*)-2-{4-[amino(2-pyridyl)methyl]-2-pyrrolylcarbonylamino}-5,5-dimethyl-3-hexenoic acid (SI13);
(*E*)-5,5-dimethyl-2-[1-(2-oxo-4-pyridyl)-4-pyrazolylcarbonylamino]-3-hexenoic acid (S116);
(*E*)-2-[2-(dimethylamino)-5-pyrimidinylcarbonylamino]-5,5-dimethyl-3-hexenoic acid (S117);
(E)-5,5-dimethyl-2-[*p*-(4*H*-1,2,4-triazol-4-yl)benzoylamino]-3-hexenoic acid (SI23);
(E)-5,5-dimethyl-2-[*m*-(1*H*-1,2,4-triazol-1-yl)benzoylamino]-3-hexenoic acid (SI24);
(*E*)-5,5-dimethyl-2[*m*-(1-imidazolyl)benzoylamino]--3-hexenoic acid (SI25);
(*E*)-2-(4-bromo-2-pyrrolylcarbonylamino)-5,5-dimethyl-3-hexenoic acid (SI28);
(*E*)-2-(5-chloro-2-pyrrolylcarbonylamino)-5,5-dimethyl-3-hexenoic acid (SI29);
(*E*)-2-(4-chloro-1-methyl-2-pyrrolylcarbonylamino)-5,5-dimethyl-3-hexenoic acid (SI31);
(*E*)-2-(4-chloro-2-thienylcarbonylamino)-5,5-dimethyl-3-hexenoic acid (SI32);
(*E*)-5,5-dimethyl-2-[*p*-(3-pyridyloxy)benzoylamino]-3-hexenoic acid (SI34);
(*E*)-5,5-dimethyl-2-(2-phenoxyisonicotinoylamino)-3-hexenoic acid (SI36);
(*E*)-2-[6-(benzyloxy)nicotinoylamino]-5,5-dimethyl-3-hexenoic acid (SI39);
(E)-5,5-dimethyl-2-[*p*-(2-pyrimidinyloxy)benzoylamino]-3-hexenoic acid (SI41);
(*E*)-5,5-dimethyl-2-(2-phenoxy-5-pyrimidinylcarbonylamino)-3-hexenoic acid (SI44);
(*E*)-2-(2-isopropyl-5-pyrimidinylcarbonylamino)-5,5-dimethyl-3-hexenoic acid (SI49);
(*E*)-5,5-dimethyl-2-[*p*-(2-thienyl)benzoylamino]-3-hexenoic acid (SI51);
(*E*)-5,5-dimethyl-2-[*m*-(2-thienyl)benzoylamino]-3-hexenoic acid (SI52);
(*E*)-2-[*p*-(*p*-chlorophenoxy)benzoylamino]-5,5-dimethyl-3-hexenoic acid (SI53);
((*E*)-2-[*p*-(*m*-chlorophenoxy)benzoylamino]-5,5-dimethyl-3-hexenoic acid (SI54);
(*E*)-5,5-dimethyl-2-[*p*-(phenoxymethyl)benzoylamino]-3-hexenoic acid (SI55);
(*E*)-2-(1-ethyl-3-isobutyl-5-pyrazolylcarbonylamino)-5,5-dimethyl-3-hexenoic acid (SI56);
(*E*)-2-(3-chloro-1-methyl-5-pyrazolylcarbonylamino)-5,5-dimethyl-3-hexenoic acid (SI57);
(*E*)-2-(3-isopropyl-1-methyl-5-pyrazolylcarbonylamino)-5,5-dimethyl-3-hexenoic acid (SI58);
(*E*)-5,5-dimethyl-2-[1-methyl-3-(3-pyridyl)-5-pyrazolylcarbonylamino]-3-hexenoic acid (SI59);
(*E*)-2-[5-(3,4-dichlorophenyl)-1-methyl-3-pyrazolylcarbonylamino]-5,5-dimethyl-3-hexenoic acid (SI61);
(*E*)-2-(4,5-dichloro-2-thienylamino)-5,5-dimethyl-3-hexenoic acid (SI62);
or an enantiomer thereof, a diastereomer thereof, a racemate thereof, a prodrug thereof, or a pharmaceutically acceptable salt thereof, such as enantiomer thereof, a diastereomer thereof, a racemate thereof, or a pharmaceutically acceptable salt thereof.

In another particular embodiment, preferred compounds of formula I include the following compounds:
(*E*)-2-[2-(dimethylamino)-4-methyl-5-pyrimidinylcarbonylamino]-5,5-dimethyl-3-hexenoic acid (SI1);
(*E*)-5,5-dimethyl-2-[*p*-(2,2,2-trifluoroacetyl)benzoylamino]-3-hexenoic acid (SI2);
(*E*)-5,5-dimethyl-2-[4-(2-pyridyl)-2-piperazinylcarbonylamino]-3-hexenoic acid (SI3);
(*E*)-5,5-dimethyl-2-[4-(4-pyridyl)-2-piperazinylcarbonylamino]-3-hexenoic acid (SI4);
(*E*)-5,5-dimethyl-2-(6-phenoxynicotinoylamino)-3-hexenoic acid (SI5);
(E)-5,5-dimethyl-2-[m-(4*H*-1,2,4-triazol-4-yl)benzoylamino]-3-hexenoic acid (SI6);
(*E*)-5,5-dimethyl-2-(6-methyl-1*H*-1,5-diazainden-2-ylcarbonylamino)-3-hexenoic acid (SI7);
(*E*)-2-(4-chloro-2-pyrrolylcarbonylamino)-5,5-dimethyl-3-hexenoic acid (SI8);
(*E*)-2-(3-isobutyl-1-methyl-5-pyrazolylcarbonylamino)-5,5-dimethyl-3-hexenoic acid (SI9);
or an enantiomer thereof, a diastereomer thereof, a racemate thereof, a prodrug thereof, or a pharmaceutically acceptable salt thereof, such as enantiomer thereof, a diastereomer thereof, a racemate thereof, or a pharmaceutically acceptable salt thereof.

In a preferred particular embodiment, preferred compounds of formula I include the following compounds:
(*E*)-2-[2-(dimethylamino)-4-methyl-5-pyrimidinylcarbonylamino]-5,5-dimethyl-3-hexenoic acid (SI1);
(*E*)-5,5-dimethyl-2-(6-phenoxynicotinoylamino)-3-hexenoic acid (SI5);
(*E*)-5,5-dimethyl-2-[*m-*(4*H*-1,2,4-triazol-4-yl)benzoylamino]-3-hexenoic acid (SI6);
(*E*)-5,5-dimethyl-2-(6-methyl-1*H*-1,5-diazainden-2-ylcarbonylamino)-3-hexenoic acid (SI7);
(*E*)-2-(4-chloro-2-pyrrolylcarbonylamino)-5,5-dimethyl-3-hexenoic acid (SI8);
(*E*)-5,5-dimethyl-2-[*p*-(4*H*-1,2,4-triazol-4-yl)benzoylamino]-3-hexenoic acid (SI23);
(*E*)-5,5-dimethyl-2-[*m*-(1*H*-1,2,4-triazol-1-yl)benzoylamino]-3-hexenoic acid (SI24);
(*E*)-5,5-dimethyl-2[m-(1-imidazolyl)benzoylamino]--3-hexenoic acid (SI25);
(*E*)-2-(4-bromo-2-pyrrolylcarbonylamino)-5,5-dimethyl-3-hexenoic acid (SI28);
(*E*)-2-(5-chloro-2-pyrrolylcarbonylamino)-5,5-dimethyl-3-hexenoic acid (SI29);
(*E*)-2-(4-chloro-1-methyl-2-pyrrolylcarbonylamino)-5,5-dimethyl-3-hexenoic acid (SI31);
(*E*)-2-(4-chloro-2-thienylcarbonylamino)-5,5-dimethyl-3-hexenoic acid (SI32);
(*E*)-5,5-dimethyl-2-[*p*-(3-pyridyloxy)benzoylamino]-3-hexenoic acid (SI34);
(*E*)-2-[6-(benzyloxy)nicotinoylamino]-5,5-dimethyl-3-hexenoic acid (SI39);
(*E*)-5,5-dimethyl-2-(2-phenoxy-5-pyrimidinylcarbonylamino)-3-hexenoic acid (SI44);
(*E*)-2-(2-isopropyl-5-pyrimidinylcarbonylamino)-5,5-dimethyl-3-hexenoic acid (SI49);
(*E*)-5,5-dimethyl-2-[*p*-(2-thienyl)benzoylamino]-3-hexenoic acid (SI51);
(*E*)-5,5-dimethyl-2-[*m*-(2-thienyl)benzoylamino]-3-hexenoic acid (SI52);
(*E*)-2-[*p*-(*p*-chlorophenoxy)benzoylamino]-5,5-dimethyl-3-hexenoic acid (SI53);
(*E*)-2-[*p*-(*m*-chlorophenoxy)benzoylamino]-5,5-dimethyl-3-hexenoic acid (SI54);
(E)-5,5-dimethyl-2-[*p*-(phenoxymethyl)benzoylamino]-3-hexenoic acid (SI55);
(*E*)-2-(1-ethyl-3-isobutyl-5-pyrazolylcarbonylamino)-5,5-dimethyl-3-hexenoic acid (SI56);
(*E*)-2-(3-chloro-1-methyl-5-pyrazolylcarbonylamino)-5,5-dimethyl-3-hexenoic acid (SI57);
(*E*)-2-(3-isopropyl-1-methyl-5-pyrazolylcarbonylamino)-5,5-dimethyl-3-hexenoic acid (SI58);
(*E*)-2-[5-(3,4-dichlorophenyl)-1-methyl-3-pyrazolylcarbonylamino]-5,5-dimethyl-3-hexenoic acid (SI61);
(*E*)-2-(4,5-dichloro-2-thienylamino)-5,5-dimethyl-3-hexenoic acid (SI62);
or an enantiomer thereof, a diastereomer thereof, a racemate thereof, a prodrug thereof, or a pharmaceutically acceptable salt thereof., such as enantiomer thereof, a diastereomer thereof, a racemate thereof, or a pharmaceutically acceptable salt thereof.

In another preferred particular embodiment, preferred compounds of formula I include the following compounds:
(*E*)-2-[2-(dimethylamino)-4-methyl-5-pyrimidinylcarbonylamino]-5,5-dimethyl-3-hexenoic acid (SI1);
(*E*)-5,5-dimethyl-2-(6-phenoxynicotinoylamino)-3-hexenoic acid (SI5);
(*E*)-5,5-dimethyl-2-[*m*-(4H-1,2,4-triazol-4-yl)benzoylamino]-3-hexenoic acid (SI6);
(*E*)-5,5-dimethyl-2-(6-methyl-1*H*-1,5-diazainden-2-ylcarbonylamino)-3-hexenoic acid (SI7);
(*E*)-2-(4-chloro-2-pyrrolylcarbonylamino)-5,5-dimethyl-3-hexenoic acid (SI8);
or an enantiomer thereof, a diastereomer thereof, a racemate thereof, a prodrug thereof, or a pharmaceutically acceptable salt thereof, such as enantiomer thereof, a diastereomer thereof, a racemate thereof, or a pharmaceutically acceptable salt thereof.

In a more preferred particular embodiment, preferred compounds of formula I include the following compounds:
(*E*)-2-[2-(dimethylamino)-4-methyl-5-pyrimidinylcarbonylamino]-5,5-dimethyl-3-hexenoic acid (SI1);
(*E*)-5,5-dimethyl-2-(6-phenoxynicotinoylamino)-3-hexenoic acid (SI5);
(*E*)-5,5-dimethyl-2-[*m*-(4*H*-1,2,4-triazol-4-yl)benzoylamino]-3-hexenoic acid (SI6);
(*E*)-2-(4-chloro-2-pyrrolylcarbonylamino)-5,5-dimethyl-3-hexenoic acid (SI8);
(E)-5,5-dimethyl-2-[*m*-(1*H*-1,2,4-triazol-1-yl)benzoylamino]-3-hexenoic acid (SI24);
(*E*)-5,5-dimethyl-2[*m*-(1-imidazolyl)benzoylamino]--3-hexenoic acid (SI25);
(*E*)-2-(4-bromo-2-pyrrolylcarbonylamino)-5,5-dimethyl-3-hexenoic acid (SI28);
(*E*)-2-(4-chloro-1-methyl-2-pyrrolylcarbonylamino)-5,5-dimethyl-3-hexenoic acid (SI31);
(*E*)-2-(4-chloro-2-thienylcarbonylamino)-5,5-dimethyl-3-hexenoic acid (SI32);
(*E*)-5,5-dimethyl-2-[*p*-(3-pyridyloxy)benzoylamino]-3-hexenoic acid (SI34);
(*E*)-2-[6-(benzyloxy)nicotinoylamino]-5,5-dimethyl-3-hexenoic acid (SI39);
(*E*)-2-(2-isopropyl-5-pyrimidinylcarbonylamino)-5,5-dimethyl-3-hexenoic acid (SI49);
(*E*)-5,5-dimethyl-2-[*p*-(2-thienyl)benzoylamino]-3-hexenoic acid (SI51);
(*E*)-5,5-dimethyl-2-[*m*-(2-thienyl)benzoylamino]-3-hexenoic acid (SI52);
(*E*)-2-(3-chloro-1-methyl-5-pyrazolylcarbonylamino)-5,5-dimethyl-3-hexenoic acid (SI57);
(*E*)-2-(4,5-dichloro-2-thienylamino)-5,5-dimethyl-3-hexenoic acid (SI62);
or an enantiomer thereof, a diastereomer thereof, a racemate thereof, a prodrug thereof, or a pharmaceutically acceptable salt thereof, such as enantiomer thereof, a diastereomer thereof, a racemate thereof, or a pharmaceutically acceptable salt thereof.

A currently preferred compound of the invention is (E)-2-[2-(dimethylamino)-4-methyl-5-pyrimidinylcarbonylamino]-5,5-dimethyl-3-hexenoic acid (SI1) or an enantiomer thereof, a diastereomer thereof, a racemate thereof, a prodrug thereof, or a pharmaceutically acceptable salt thereof, such as enantiomer thereof, a diastereomer thereof, a racemate thereof, or a pharmaceutically acceptable salt thereof.

Another currently preferred compound of the invention is (E)-5,5-dimethyl-2-(6-phenoxynicotinoylamino)-3-hexenoic acid (SI5) or an enantiomer thereof, a diastereomer thereof, a racemate thereof, a prodrug thereof, or a pharmaceutically acceptable salt thereof, such as enantiomer thereof, a diastereomer thereof, a racemate thereof, or a pharmaceutically acceptable salt thereof.

A further currently preferred compound of the invention is (E)-5,5-dimethyl-2[m-(1-imidazolyl)benzoylamino]--3-hexenoic acid (SI25), or an enantiomer thereof, a diastereomer thereof, a racemate thereof, a prodrug thereof, or a pharmaceutically acceptable salt thereof, such as enantiomer thereof, a diastereomer thereof, a racemate thereof, or a pharmaceutically acceptable salt thereof.

Another preferred compound of the invention is (*E*)-2-(4-chloro-1-methyl-2-pyrrolylcarbonylamino)-5,5-dimethyl-3-hexenoic acid (SI31), or an enantiomer thereof, a diastereomer thereof, a racemate thereof, a prodrug thereof, or a pharmaceutically acceptable salt thereof, such as enantiomer thereof, a diastereomer thereof, a racemate thereof, or a pharmaceutically acceptable salt thereof.

A further preferred compound of the invention is (*E*)-2-(4-chloro-2-thienylcarbonylamino)-5,5-dimethyl-3-hexenoic acid (SI32), or an enantiomer thereof, a diastereomer thereof, a racemate thereof, a prodrug thereof, or a pharmaceutically acceptable salt thereof, such as enantiomer thereof, a diastereomer thereof, a racemate thereof, or a pharmaceutically acceptable salt thereof.

Yet another preferred compound of the invention is (*E*)-2-[6-(benzyloxy)nicotinoylamino]-5,5-dimethyl-3-hexenoic acid (SI39), or an enantiomer thereof, a diastereomer thereof, a racemate thereof, a prodrug thereof, or a pharmaceutically acceptable salt thereof, such as enantiomer thereof, a diastereomer thereof, a racemate thereof, or a pharmaceutically acceptable salt thereof.

Another preferred compound of the invention is (*E*)-5,5-dimethyl-2-[*p*-(2-thienyl)benzoylamino]-3-hexenoic acid (SI51), or an enantiomer thereof, a diastereomer thereof, a racemate thereof, a prodrug thereof, or a pharmaceutically acceptable salt thereof, such as enantiomer thereof, a diastereomer thereof, a racemate thereof, or a pharmaceutically acceptable salt thereof.

A further preferred compound of the invention is (*E*)-5,5-dimethyl-2-[m-(2-thienyl)benzoylamino]-3-hexenoic acid (SI52), or an enantiomer thereof, a diastereomer thereof, a racemate thereof, a prodrug thereof, or a pharmaceutically acceptable salt thereof, such as enantiomer thereof, a diastereomer thereof, a racemate thereof, or a pharmaceutically acceptable salt thereof.

Yet another preferred compound of the invention is (*E*)-2-(4,5-dichloro-2-thienylamino)-5,5-dimethyl-3-hexenoic acid (SI62), or an enantiomer thereof, a diastereomer thereof, a racemate thereof, a prodrug thereof, or a pharmaceutically acceptable salt thereof, such as enantiomer thereof, a diastereomer thereof, a racemate thereof, or a pharmaceutically acceptable salt thereof.

Compounds of the present invention can be manufactured, as is further described in Examples 1 to 37 below, from an intermediate as synthesized as disclosed in steps 1-3 of Example 1. The present invention also encompasses such intermediates, i.e., compounds D, E and F in Example 1, and the synthesis thereof. Hence, the invention also relates to an intermediate for the production of a compound of formula I. The intermediate is selected from the group consisting of:
(*E*)-2-{[(*p*-methoxyphenyl)methyl]amino}-5,5-dimethyl-3-hexenoic acid (intermediate compound 1, IC1);
ethyl (*E*)-2-{[(*p*-methoxyphenyl)methyl]amino}-5,5-dimethyl-3-hexenoate (IC2); and
ethyl (*E*)-2-amino-5,5-dimethyl-3-hexenoate (IC3).

In an embodiment, the intermediate is (*E*)-2-{[(*p*-methoxyphenyl)methyl]amino}-5,5-dimethyl-3-hexenoic acid (IC1). IC1 can be produced by adding 2-*tert*-butyl-E-vinylboronic acid to 4-methoxybenzylamine in dry dichloromethane followed by glyoxylic acid monohydrate to obtain IC1 (step 1 of Example 1).

In another embodiment, the intermediate is ethyl (*E*)-2-{[(*p*-methoxyphenyl)methyl]amino}-5,5-dimethyl-3-hexenoate (IC2). IC2 can be produced by adding sulphuric acid to a suspension of IC1 in ethanol to obtain IC2 (step 2 of Example 1).

In a preferred embodiment, the intermediate is ethyl (*E*)-2-amino-5,5-dimethyl-3-hexenoate (IC3). IC3 can be produced by adding a pre-dissolved solution of ceric ammonium nitrate in water to a solution of IC2 in acetonitrile to obtain IC3 (step 3 of Example 1).

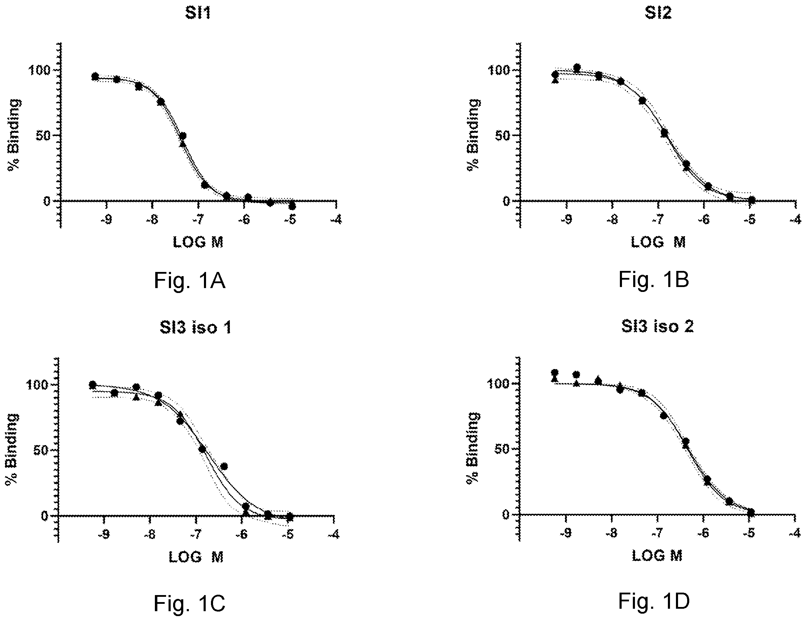

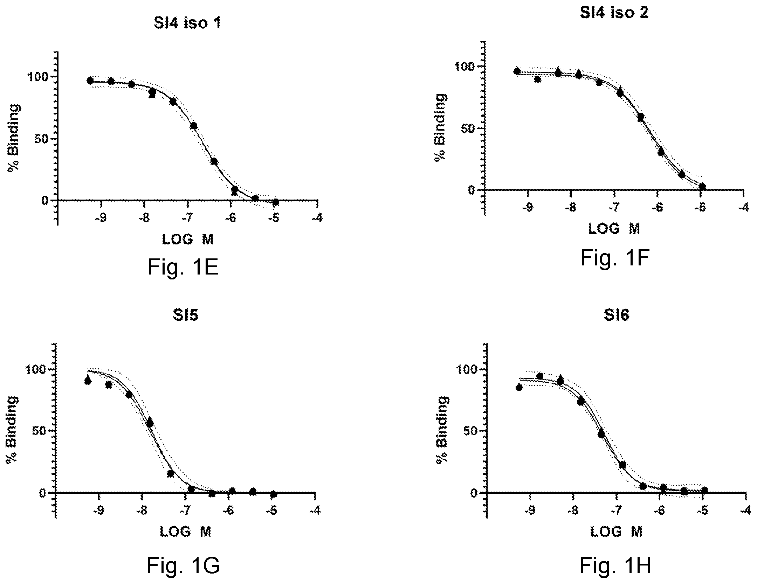

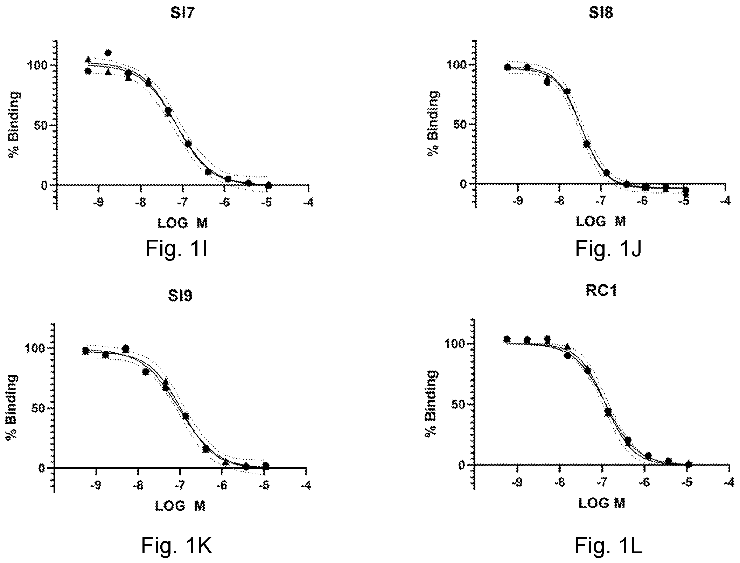

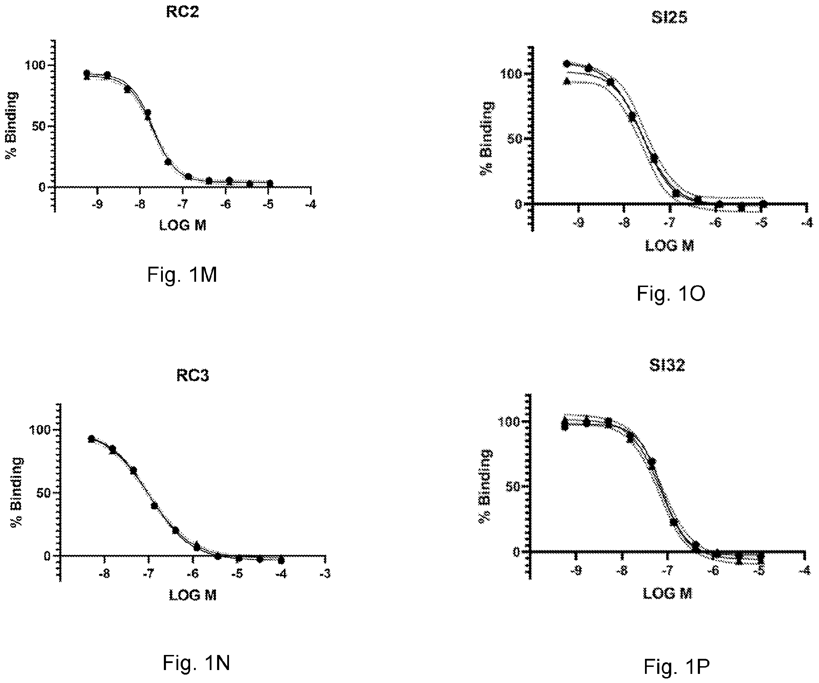

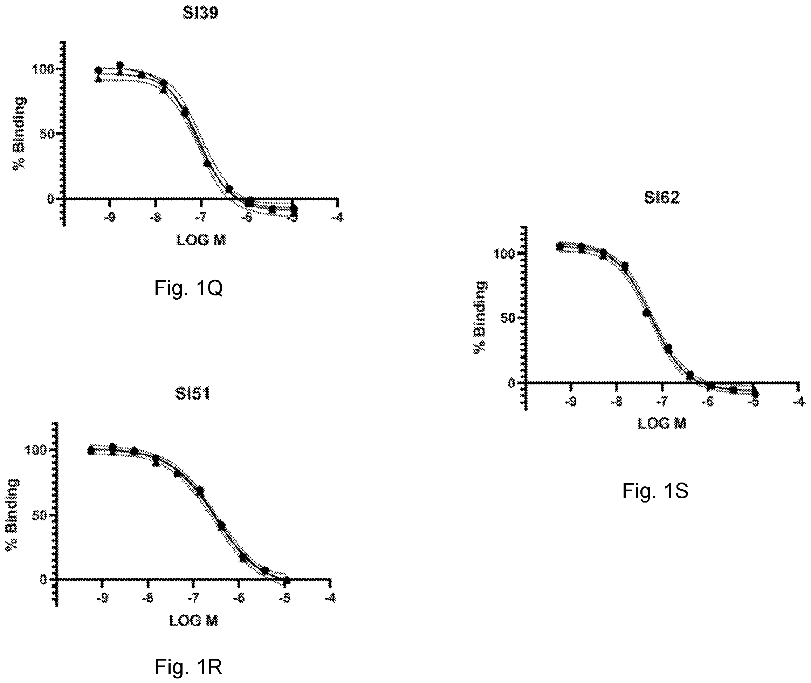

The sortilin inhibitors of the present invention are capable of binding to sortilin with a high affinity (Figs. 1A-1K, 1O-1S, Table 1). In fact, sortilin inhibitors of the invention had a higher binding affinity than optimized reference compounds (RC1 and RC2 in Figs. 1L and 1M and Table 1) having a saturated aliphatic moiety rather than an unsaturated aliphatic moiety, see hatched ring in formula II. The sortilin inhibitors of the invention are further capable of blocking or at least significantly inhibiting the binding of other ligands, such as PGRN, to sortilin. This means that the sortilin inhibitors of the invention can be used in the treatment of diseases, disorders, and conditions associated or characterized by such a ligand-to-sortilin binding and where the interruption or at least suppression of such a ligand-to-sortilin binding is beneficial for the patient in terms or treating the disease, disorder or condition.

The sortilin inhibitors of the invention can thereby be used as a medicament.

Through its various interactions with proteins, such as PGRN, sortilin and its multiple ligands have been shown to be involved in various diseases, disorders, and conditions, such as frontotemporal dementia (FTD), amyotrophic lateral sclerosis (ALS), ALS-FTD phenotypes, Alzheimer's disease, Parkinson's disease, depression, neuropsyciatric disorders, vascular dementia, seizures, retinal dystrophy, age related macular degeneration, glaucoma, traumatic brain injury, aging, seizures, wound healing, stroke, arthritis, atherosclerotic vascular diseases, dermatology related diseases and autoimmune diseases (WO 2009/140972, WO 2014/114779, WO 2016/164637, Bioorganic & Medicinal Chemistry Letters 2020 30: 127403).

The sortilin inhibitors of the invention can thereby be used in prevention or treatment of a neurodegenerative disease, a psychiatric disease, a motor neuron disease, peripheral neuropathies, pain, neuroinflammation, atherosclerosis, hyperlipidemia, cardiovascular diseases, dermatology related diseases or autoimmune diseases.

Illustrative, but non-limiting, examples of neurodegenerative diseases include FTD, ALS, ALS-FTD phenotypes, stroke, traumatic brain injury, retinal degeneration, light-induced photoreceptor degeneration, Alzheimer's disease and Parkinson's disease.

Illustrative, but non-limiting, examples of psychiatric disease include depression, neuropsychiatric disorders, epilepsy and bipolar disorder.

Illustrative, but non-limiting, examples of motor neuron diseases include ALS, progressive bulbar palsy (PBP), pseudobulbar palsy, progressive muscular atrophy (PMA), primary lateral sclerosis (PLS), spinal muscular atrophy (SMA) and monomelic amyotrophy (MMA).

Illustrative, but non-limiting, examples of peripheral neuropathies include diabetic neuropathy.

Illustrative, but non-limiting, examples of pain include acute pain, chronic pain, neuropathic pain, lower back pain, post operative pain and inflammatory pain.

Illustrative, but non-limiting, examples of neuroinflammatory diseases include rheumatoid arthritis, Crohns disease, ulcerative colitis and multiple sclerosis.

Illustrative, but non-limiting, examples of hyperlipidemias include hyperlipoproteinemia type I, such as Buerger-Gruetz syndrome, primary hyperlipoproteinemia or familial hyperchylomicronemia; hyperlipoproteinemia type Na, such as polygenic hypercholesterolemia or familial hypercholesterolemia; hyperlipoproteinemia type Nb, such as combined hyperlipidemia; hyperlipoproteinemia type III, such as familial dysbetalipoproteinemia; hyperlipoproteinemia type IV, such as endogenous hyperlipemia; hyperlipoproteinemia type V, such as familial hypertriglyceridemia. Other examples of diseases or disorder caused by hyperlipidemia or having a hyperlipidemia component include aneurysm, angina pectoris, atherosclerosis, cerebrovascular accident or disease, congenital heart disease, congestive heart failure, coronary artery disease, dilated cardiomyopathy, diastolic dysfunction, endocarditis, hypercholesterolemia, hypertension, hypertrophic cardiomyopathy, mitral valve prolapse, myocardial infarction and venous thromboembolism.

Illustrative, but non-limiting, examples of dermatology related disease include psoriasis, dermal fibrosis and dermal keratosis.

Illustrative, but non-limiting, examples of autoimmune disease include psoriasis, rheumatoid arthritis, diabetes mellitus and inflammatory bowel diseases, such as ulcerative colitis and Crohn's disease.

The PGRN-sortilin axis is also of importance for cancers. In more detail, highly malignant cancer cells co-express PGRN and sortilin resulting in sphere (cancer stem cell) formation and metastasis. Blocking or at least inhibiting the interaction between PGRN and sortilin thereby prevents or at least significantly inhibits sphere formation (Example 42) and may also reduce metastasis *(*Breast Cancer Research 2018 20: 137). High PGRN levels have been found in various cancer types including, but non-limited, to breast cancer, bladder cancer, lymphomas, biliary cancer and pancreatic cancer.

The sortilin inhibitors of the invention can thereby be used in prevention or treatment of cancer.

In an embodiment, the cancer is selected from the group consisting of breast cancer, bladder cancer, lymphomas, biliary cancer, colon cancer, melanoma and pancreatic cancer. In a preferred embodiment, the cancer is selected from the group consisting of breast cancer, colon cancer and melanoma, more preferably breast cancer.

In a particular embodiment, the sortilin inhibitors of the invention are used in prevention or treatment of metastasis. Further uses of the sortilin inhibitors of the invention include in prevention or treatment of sphere formation and in inhibition of cancer stem cell formation and/or migration.

The present invention also relates to a pharmaceutical composition comprising a sortilin inhibitor according to the invention and at least one pharmaceutically acceptable excipient, carrier or diluent.

As used herein, pharmaceutically acceptable excipient, carrier or diluent encompass various pharmaceutically acceptable additives including, but not limited to, excipients, carriers, diluents, adjuvants, colorings, aromas, preservatives etc. that the person skilled in the art would consider using when formulating a sortilin inhibitor of the invention to make a pharmaceutical composition.

Pharmaceutically acceptable excipient, carrier or diluent include, but are not limited to, lactose, dextrose, sucrose, mannitol, sorbitol, cellulose, and glycine; lubricants, such as silica, talc, stearic acid including salts thereof, and polyethylene glycol; binders, such as magnesium and aluminum silicate, starch paste, gelatin, tragacanth, methylcellulose, sodium carboxymethyl cellulose, and polyvinylpyrrolidone; disintegrants, such as starch, agar, alginic acid, and sodium alginate; absorbents; dyes; flavoring agents; sweeteners; polypropylene glycol; liquid vehicles, such as water, physiological saline solution, aqueous dextrose, glycerol, ethanol, and oil.

The at least one excipient, carrier and/or diluent is pharmaceutically acceptable in terms of being compatible with the sortilin inhibitor of the invention (compound of formula I) and any other ingredients of the pharmaceutical composition, and not deleterious to a subject when the pharmaceutical composition is administered thereto. For instance, it is preferred that the pharmaceutical composition does not contain any material that may cause an adverse reaction, such as an allergic reaction, when administered to a subject.

In an embodiment, the pharmaceutical composition comprises from 1 to 99 % by weight of the at least one pharmaceutically acceptable excipient, carrier and/or diluent and from 1 to 99 % by weight of at least one sortilin inhibitor of the invention.

The pharmaceutical composition could comprise a single sortilin inhibitor according to invention or multiple sortilin inhibitors according to the invention, i.e., a mixture of two or more different sortilin inhibitors.

In an embodiment, the pharmaceutical composition may comprise at least one second active agent in addition to the one or more sortilin inhibitors of the invention.

The at least one second active agent may be an active agent traditionally used in treatment of a disease, disorder or condition associated with sortilin activity and ligand-to-sortilin binding, such as PGRN-to-sortilin binding. Hence, the at least one active agent could be an active agent used in prevention or treatment of a neurodegenerative disease, a psychiatric disease, a motor neuron disease, peripheral neuropathies, pain, neuroinflammation, atherosclerosis, hyperlipidemia, cardiovascular diseases, dermatology related diseases or autoimmune diseases.

In another embodiment, the at least one second active agent is a cytostatic agent or other anti-cancer agent. Non-limiting examples of such cytostatic agents that could be used in the pharmaceutical composition include alkylating agents, such as mechlorethamine, cyclophosphamide, melphalan, chlorambucil, ifosfamide, busulfan, N-nitroso-N-methylurea (MNU), carmustine (BCNU), lomustine (CCNU), semustine (MeCCNU), fotemustine, streptozotocin, dacarbazine, mitozolomide, temozolomide, thiotepa, mytomycin, diaziquone (AZQ), cisplatin, carboplatin and oxaliplatin; antimetabolites, such as methotrexate, pemetrexed, fluorouracil, capecitabine, cytarabine, gemcitabine, decitabine, azacitidine, fludarabine, nelarabine, cladribine, clofarabine, pentostatin, thioguanine and mercaptopurine; anti-microtubule agents, such as Vinca alkaloids derived from *Catharanthus roseus,* vincristine, vinblastine, vinorelbine, vindesine, vinflunine, paclitaxel, docetaxel, podophyllotoxin, etoposide and teniposide; topoisomerase inhibitors, such as irinotecan, topotecan, camptothecin, etoposide, doxorubicin, mitoxantrone, teniposide, novobiocin, merbarone, and aclarubicin; and cytotoxic antibiotics, such as doxorubicin, daunorubicin, epirubicin, idarubicin, pirarubicin, aclarubicin, mitoxantrone, actinomycin, bleomycin, mitomycin C and actinomycin.

In a particular embodiment, the at least one second active agent is progranulin.

The pharmaceutical composition comprising multiple active agents may be formulated in a unit dosage form comprising the multiple active agents or may be formulated as separated dosage forms with a respective active agent. In the latter case, the multiple separate dosage forms may be administered substantially simultaneously or separately, such as sequentially.

The pharmaceutical composition could be a solid pharmaceutical composition, such as a tablet, pill, powder, or granules, a semi solid pharmaceutical composition, such as a suppositories; or a liquid pharmaceutical composition, such as soft capsule or injection solution.

The sortilin inhibitor of the invention and in particular the pharmaceutical composition comprising at least one sortilin inhibitor of the invention may be administered to a subject using various administration routes. Illustrative, but non-limiting, examples of such administration routes include oral, intravenous, topical, intraperitoneal, nasal, buccal, sublingual, or subcutaneous administration, or administration via the respiratory tract. The pharmaceutical composition of the invention may be formulated based on the particular administration route. Illustrative, but non-limiting, examples of formulations of the pharmaceutical composition include tablets, capsules, powders, nanoparticles, crystals, amorphous substances, solutions, transdermal patches, (transdermal) creams or suppositories.

The references to methods of treatment in this description are to be interpreted as references to compounds, pharmaceutical compositions and medicaments of the present invention for use in a method of treatment of the human (or animal) body by therapy (or for diagnosis).

The present invention is also a method for treating a disease, disorder or condition selected from the group consisting of a neurodegenerative disease, a psychiatric disease, a motor neuron disease, peripheral neuropathies, pain, neuroinflammation, atherosclerosis, hyperlipidemia, cardiovascular diseases, dermatology related diseases, autoimmune diseases and cancer. The method comprising administering at therapeutically effective amount of a sortilin inhibitor of the invention or a pharmaceutical composition of the invention to a subject need thereof.

"Treatment" or "treating" as used herein means the management and care of a subject for the purpose of combating a disease, a disorder or a medical condition. The term is intended to include the full spectrum of treatments for a given disease, disorder or condition, from which the subject is suffering, such as administration of the sortilin inhibitor or the pharmaceutical composition of the invention to alleviate the symptoms or complications, to delay the progression of the disease, disorder or condition, to alleviate or relief the symptoms and complications, and/or to cure or eliminate the disease, disorder or condition as well as to prevent the disease, disorder or condition. In such a case, prevention is to be understood as the management and care of a subject for the purpose of combating the disease, disorder or condition and includes the administration of the sortilin inhibitor or the pharmaceutical composition of the invention to prevent the onset of the symptoms or complications. The treatment may either be performed in an acute or in a chronic way.

"A therapeutically effective amount" of the sortilin inhibitor or the pharmaceutical composition of the invention as used herein means an amount sufficient to cure, inhibit, alleviate or partially arrest the clinical manifestations of a given disease, disorder or condition and its complications. An amount adequate to accomplish this is defined as therapeutically effective amount. Effective amounts for each purpose will depend on the severity of the disease, disorder or condition as well as parameters of the subject, such as weight, sex, age, general health status of the subject. It will be understood that determining an appropriate dosage may be achieved using routine experimentation, by constructing a matrix of values and testing different points in the matrix, which is all within the ordinary skills of a trained physician or veterinary.

A related aspect of the invention defines use of a sortilin inhibitor of the invention for the manufacture of a medicament for treatment of a disease, disorder or condition selected from the group consisting of a neurodegenerative disease, a psychiatric disease, a motor neuron disease, peripheral neuropathies, pain, neuroinflammation, atherosclerosis, hyperlipidemia, cardiovascular disease, dermatology related diseases, autoimmune diseases and cancer.

The subject treated with a sortilin inhibitor or pharmaceutical composition of the invention is preferably a human subject. The present invention can, however, be used for veterinary purposes by administering a sortilin inhibitor or pharmaceutical composition of the invention to a non-human mammal. Illustrative, but non-limiting, examples of such non-human mammals include dogs, cats, cows, horses, sheep, goat, pigs, rats, mice, rabbits and guinea pigs.

### EXAMPLES

In the syntheses of the compounds of the present invention, as described below, the following analytical and chromatographic equipment were used.
Liquid Chromatography Mass Spectrometry (LCMS)-1: Agilent LC/MSD Trap XCT Plus;
LCMS-2: Agilent 6130 Single Quadrupole LCMSC08UPD665M;
Ultra performance liquid chromatography (UPLC) MS: Waters ACQUITY UPLCMS SQD
Proton nuclear magnetic resonance (¹H-NMR): Bruker Avance III HD (400MHz)
High performance liquid chromatography (HPLC): Shimadzu/ Prominence and Prominence -I series (L2145610638 AE)
HPLC Columns: Agilent Zorbax SB-CN 250×4.6 mm 5 µm
SFC chiral purification column: Chiralpak IG 250×30 mm (preparative), 250×10 mm (semi-preparative) and Chiralpak IG 250×4.6 mm (analytical)
CombiFlash Rf, Model: RF+UV, Serial: 216C08126 from Teledyne Isco

### EXAMPLE 1 - Synthesis of (E)-2-[2-(dimethylamino)-4-methyl-5-pyrimidinylcarbonylamino]-5,5-dimethyl-3-hexenoic acid (SI1)

### Step 1: Synthesis of (E)-2-{[(p-methoxyphenyl)methyl]amino}-5,5-dimethyl-3-hexenoic acid (D)

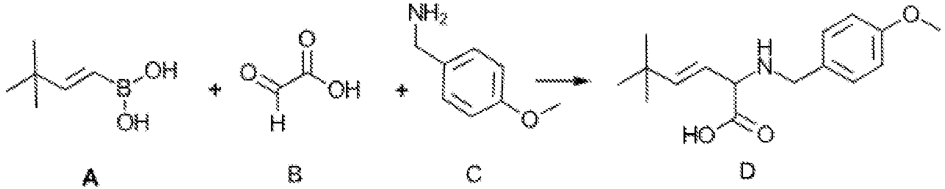

2-*tert*-Butyl-E-vinylboronic acid (A) (1.86 g, 0.014 mol) was added to a stirred solution of 4-methoxybenzylamine (C) (2 g, 0.014 mol) in dry dichloromethane (DCM, 22 mol, 0.33 M with respect to amine) followed by glyoxylic acid monohydrate (B) (1.34 g, 0.014 mol) at 25-30°C. The resulting reaction mixture was stirred over a period of 24 h at 25-30°C under argon atmosphere. Progress of the reaction was monitored by thin layer chromatography (TLC).

After completion of the reaction, the reaction mixture was filtered off, the solid obtained was washed with DCM and dried at 40°C to obtain the expected product (D) as a white solid.
Yield: 1.78 g, (44.5 %)
¹H-NMR-CD₃OD (400 MHz): δ: 1.04 (9H, s), 3.78 (3H, s), 3.86-3.89 (1H, m), 4.0-4.1 (2H, m), 5.38-5.45 (1H, m), 5.98 (1H, d, *J*=16.0 Hz), 6.95 (2H, dd, *J*₁=2.4 Hz, *J*₂=6.8 Hz), 7.35 (2H, dd, *J*₁=2.0 Hz, *J*₂=6.4 Hz);
LCMS: 278.2 [M+H]⁺

### Step 2: Synthesis of ethyl (E)-2-{[(p-methoxyphenyl)methyl]amino}-5,5-dimethyl-3-hexenoate (E)

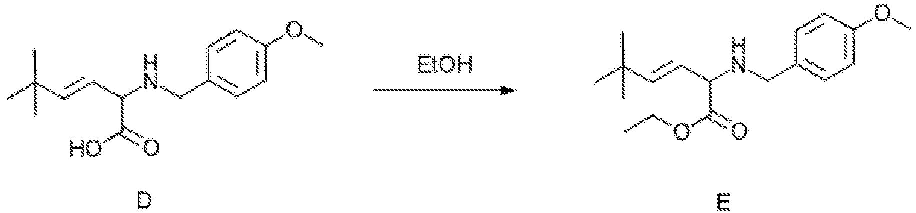

Sulphuric acid (3.4 ml) was added dropwise to a stirred suspension of compound D (1.7 g, 0.006 mol) in ethanol (34 ml) at 0-5°C. The resulting reaction mixture was stirred over a period of 24 h at 90°C. The progress of the reaction was monitored by TLC.

After completion of the reaction, the reaction mixture was evaporated at 40°C to remove ethanol. The remaining mixture was basified to pH ~7-8 using saturated NaHCO₃ solution, and the product was extracted into ethyl acetate (3 × 20 ml). The combined organic layer was dried over anhydrous sodium sulphate and concentrated at 40°C under reduced pressure to obtain the crude product (E) as a pale brown oil. The crude product was used as such for the next reaction without any purification.
Yield: 1.3 g, (crude product)
¹H-NMR-CD₃OD (400 MHz): δ: 1.04 (9H, s), 1.22 (3H, t, J=7.2 Hz), 3.60-3.63 (2H, m), 3.69-3.71 (1H, m), 3.75 (3H, s), 4.15 (2H, q, *J*=7.2 Hz), 5.27-5.33 (1H, m), 5.71-5.77 (1H, m), 6.83-6.87 (2H, m), 7.17-7.21 (2H, m);
LCMS: 306.3 [M+H]⁺

### Step 3: Synthesis of ethyl (E)-2-amino-5,5-dimethyl-3-hexenoate (F)

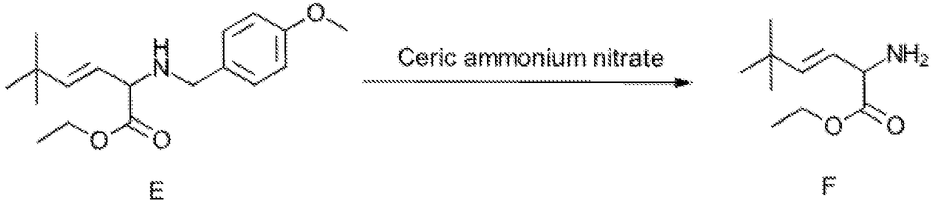

A pre-dissolved solution of ceric ammonium nitrate (14.0 g, 0.0255 mol) in water (15.6 ml) was added dropwise over a period of 15 min to a stirred solution of compound E (1.3 g, 0.0042 mol) in acetonitrile (15.6 ml) at 0-5°C. The resulting reaction mixture was stirred over a period of 18 h at 25-30°C. The progress of the reaction was monitored by TLC.

After completion of the reaction, the reaction mixture was evaporated at 25°C to remove acetonitrile, basified to pH ~7-8 using saturated NaHCO₃ solution. The thick suspension was filtered through a celite bed, and the celite bed was washed with ethyl acetate and the resulting layers were separated out. The combined organic layer was dried over anhydrous sodium sulphate and concentrated at 35°C under reduced pressure to obtain the crude product. The crude product was purified by CombiFlash^{®} using methanol/DCM as eluent to obtain the expected product (F) as a pale brown oil.
Yield: 255 mg, (32.3 %)
¹H-NMR- CD₃OD (400 MHz): δ: 1.04 (9H, s), 1.25 (3H, t, J=7.2 Hz), 3.93-3.95 (1H, m), 4.18 (2H, q, *J=7.2* Hz), 5.40-5.46 (1H, m), 5.77-5.82 (1H, m);
LCMS: 186.1 [M+H]⁺

### Step 4: Synthesis of ethyl (E)-2-[2-(dimethylamino)-4-methyl-5-pyrimidinylcarbonylamino]-5,5-dimethyl-3-hexenoate (H)

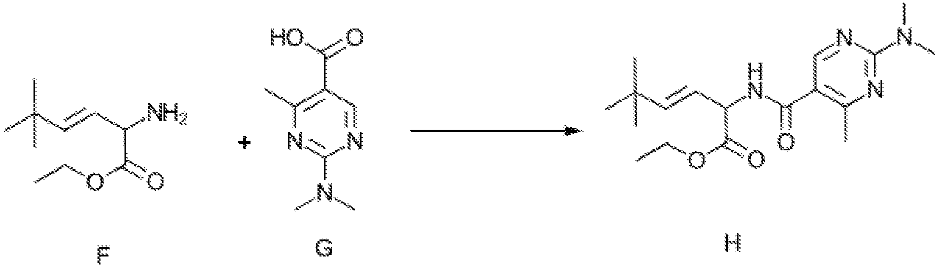

2-(Dimethylamino)-4-methylpyrimidine-5-carboxylic acid (G) (244.4 mg, 1.3493 mmol) was added to a stirred solution compound F (250 mg, 1.3493 mmol) in dimethylformamide (DMF, 6.25 ml) followed by N,N-diisopropylethylamine (DIPEA, 0.94 mL, 5.3972 mmol) and propyl phosphonic anhydride (T₃P, ~50% solution in ethyl acetate) (2.57 mL, 4.0479 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution until pH ~7-8 and the product was then extracted into ethyl acetate (2 × 50 mL). The combined organic layer was washed with saturated sodium chloride solution, dried over anhydrous sodium sulphate and concentrated in vacuum at 35°C to obtain the crude product. The crude product was purified by CombiFlash^{®} using ethyl acetate/hexane as eluent to obtain the pure product (H) as a pale brown thick oil.
Yield: 62 mg, (13.2 %)
¹H-NMR- CD₃OD (400 MHz): δ: 1.02 (9H, s), 1.25 (3H, t, J=7.2 Hz), 2.44 (3H, s), 3.18 (6H, s), 4.19 (2H, t, *J*=7.2 Hz), 4.94-4.92 (1H, m), 5.47-5.54 (1H, m), 5.87-5.91 (1H, m), 8.33 (1H, s);
LCMS: 349.3 [M+H]⁺

### Step 5: Synthesis of (E)-2-[2-(dimethylamino)-4-methyl-5-pyrimidinylcarbonylamino]-5,5-dimethyl-3-hexenoic acid (I)

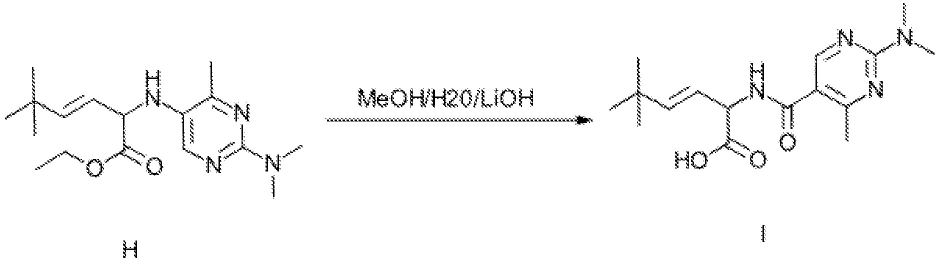

Lithium hydroxide monohydrate (33.11 mg, 0.7892 mmol) was added to a stirred solution of compound H (55 mg, 0.1578 mmol) in tetrahydrofuran (THF)/methanol/water (1.65 ml, 1:1:1, 0.55 ml each). The resulting reaction mixture was stirred over a period of 18 h at 25-30°C. The progress of the reaction was monitored by TLC.

After 18 h, the reaction mixture was concentrated, the remaining residue was dissolved in water and washed with ethyl acetate, and the aqueous layer was acidified to pH ~ 2-3 using 1.5 N HCl and the product was extracted with ethyl acetate (3 × 5 mL). The combined organic layer was washed with saturated sodium chloride solution, dried over sodium sulphate and concentrated at 35°C to obtain the crude product. The crude solid was triturated with hexanes and dried at 40°C to afford pure final product (I) as off white solid.
Yield: 37 mg, (73.2 %)
¹H-NMR-DMSO-*d*₆ (400 MHz): δ: 0.98 (9H, s), 2.40 (3H, s), 3.13 (6H, s), 4.78-4.82 (1H, m), 5.45-5.50 (1H, m), 5.80-5.84 (1H, m), 8.37 (1H, s), 8.57 (1H, d, *J*=7.2 Hz), 12.59 (1H, bs);
LCMS: 321.3 [M+H]⁺;
HPLC purity: 98.6 %

### Separation of enantiomers of (E)-2-[2-(dimethylamino)-4-methyl-5-pyrimidinylcarbonylamino]-5,5-dimethyl-3-hexenoic acid (SI1)

(*E*)*-*2-[2-(dimethylamino)-4-methyl-5-pyrimidinylcarbonylamino]-5,5-dimethyl-3-hexenoic acid as synthesized in Example 1 (12 mg, 34 mmol) was dissolved in methanol (7 mL) and purified by preparative supercritical fluid chromatography (SFC) using a CHIRALPAK-ID column (250 mm × 10 mm, 5 µM) eluting with methanol, 15% in CO₂, flow 15 mL/min. Baseline separation was obtained. Enantiomer 1 eluted at 29.7-30.1 min and enantiomer 2 eluted at 30.2-30.9 min. The fractions for each enantiomer were combined and evaporated to give enantiomer 1 (3.2 mg) and enantiomer 2 (3.2 mg).

### EXAMPLE 2 - Synthesis of (E)-5,5-dimethyl-2-[p-(2,2,2-trifluoroacetyl)benzoylaminol-3-hexenoic acid (SI2)

### Step 1: Synthesis of (E)-5,5-dimethyl-2-[p-(2,2,2-trifluoroacetyl)benzoylamino]-3-hexenoic acid (C)

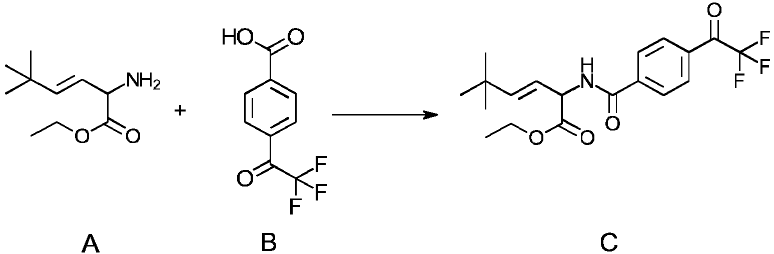

4-(Trifluoroacetyl)benzoic acid (B) (82.41 mg, 0.38 mmol) was added to a stirred solution of ethyl (E)-2-amino-5,5-dimethyl-3-hexenoate (A) (70 mg, 0.38 mmol) in DMF (1.75 mL) followed by DIPEA (0.263 mL, 1.52 mmol) and propyl phosphonic anhydride (T₃P, ~50% solution in ethyl acetate) (0.72 mL, 1.14 mmol) at 25-30°C. The resulting reaction mixture was stirred over a period of 48 h at 40°C. The progress of the reaction was monitored by TLC.

After completion of the reaction, the reaction mixture was quenched by addition of saturated NaHCO₃ solution to pH ~7-8 and product was then extracted into ethyl acetate (2 × 20 mL). The combined organic layer was washed with saturated sodium chloride solution, dried over anhydrous sodium sulphate and concentrated in vacuo at 35°C to obtain the crude product. The crude product was purified by CombiFlash^{®} using ethyl acetate/hexane as eluent to afford the pure product (C) as a pale brown thick oil.
Yield: 68 mg, (46.7 %)
¹H-NMR- CD₃OD (400 MHz): δ: 1.03 (9H, s), 1.27 (3H, t, *J*=7.2 Hz), 4.21 (2H, t, *J=7.2* Hz), 4.85-4.89 (1H, m), 5.57-5.59 (1H, m), 5.90-5.94 (1H, m), 7.71-7.80 (2H, m), 7.78-7.93 (2H, m);
LCMS: 404.3 [M+H+H₂O]⁺

### Step 2: Synthesis of (3E)-5,5-dimethyl-2-{[4-(2,2,2-trifluoroacetyl) phenyl(]formamido-hex-3-enoic acid (D)

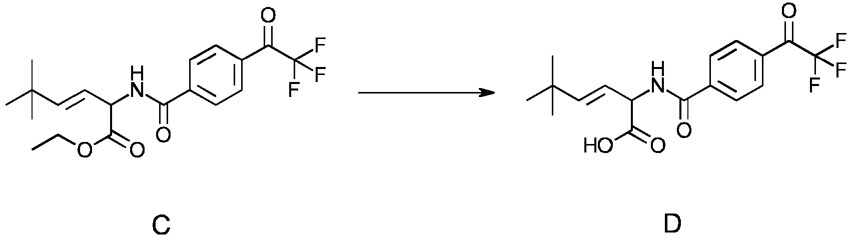

Lithium hydroxide monohydrate (35.38 mg, 0.85 mmol) was added to a stirred solution of compound C (65 mg, 0.17 mmol) in THF/methanol/water (1.95 mL, 1:1:1, 0.65 mL each). The resulting reaction mixture was stirred over a period of 18 h at 25-30°C. The progress of the reaction was monitored by TLC.

After 18 h, the reaction mixture was concentrated, the residue was dissolved in water and washed with ethyl acetate (10 mL), the aqueous layer was acidified to pH ~ 3.4 using 1.5 N HCI, and the product was then extracted with ethyl acetate (3 × 5 mL). The combined organic layer was dried over sodium sulphate and concentrated at 40°C to obtain a crude solid. The crude solid was washed with hexane (5 mL) and DCM (5 mL) to afford the final product D as an off white solid.
Yield: 18 mg, (29.9 %)
¹H-NMR-DMSO-*d*₆ (400 MHz): δ: 1.0 (9H, s), 4.91 (1H, d, *J*=9.6 Hz), 5.52-5.56 (1H, m), 5.81-5.88 (1H, m), 7.68-7.81 (1H, m), 8.09-8.21 (3H, m), 9.13 (1H, m), 12.67 (1H, bs);
LCMS: 376.2 [M+H+H₂O]⁺
HPLC purity: 86.6 %

### EXAMPLE 3 - Synthesis of (E)-5,5-dimethyl-2-[4-(2-pyridyl)-2-piperazinylcarbonylaminol-3-hexenoic acid (SI3)

### Step 1: Synthesis of tert-butyl 2-methoxycarbonyl-4-(2-pyridyl)-1-piperazinecarboxylate (C)

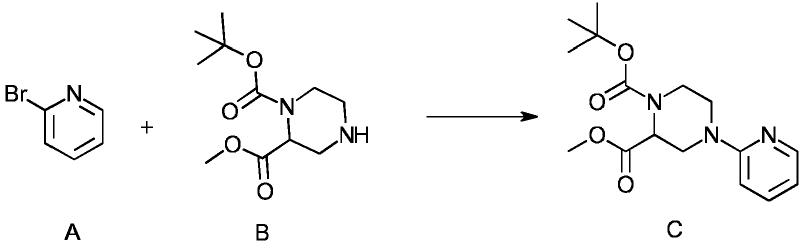

2,2'-Bis(diphenylphosphino)-1,1'-binaphthyl (BINAP, 0.084 g, 0.00013 mol), Pd(OAc)₂ (0.03 g, 0.00013 mol) and potassium t-butoxide (0.30 g, 0.0027 mol) were added to a degassed solution of 2-bromopyridine (A) (0.21 g, 0.0013 mol) and N-Boc-piperazine-2-carboxylic acid methyl ester (B) (1.0 g, 0.0040 mol) in toluene (10 mL) at 25-30°C. Degassing was continued for another 5 mins. The resulting reaction mixture was heated at 85°C over a period of 18 h. The progress of the reaction was monitored by TLC.

After completion of the reaction, the reaction mixture was diluted with water (10 mL), ethyl acetate (10 mL) and the product was extracted into ethyl acetate (2 × 20 mL). The combined organic layer was washed with saturated sodium chloride solution, dried over anhydrous sodium sulphate and concentrated in vacuo at 35°C to obtain the crude product. The crude product was purified by CombiFlash^{®} using ethyl acetate/hexane as eluent to afford the product C as pale yellow thick oil.
Yield: 0.18 g, (Impure)
LCMS: 322.2 [M+H]⁺

### Step 2: Synthesis of 1-tert-butoxycarbonyl-4-(2-pyridyl)-2-piperazinecarboxylic acid (D)

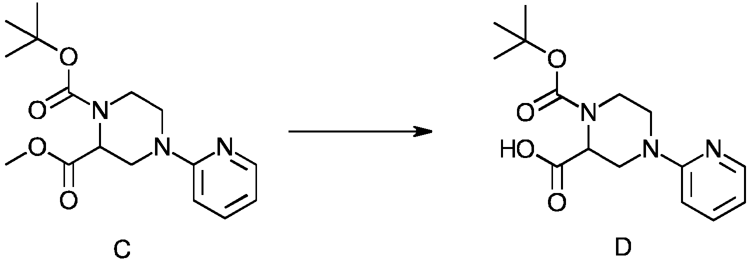

Lithium hydroxide monohydrate (0.11 g, 0.0028 mol) was added to a stirred solution of compound C (0.18 g, 0.00056 mol) in THF/methanol/water (5.4 mL, 1:1:1, 1.8 mL each). The resulting reaction mixture was stirred over a period of 18 h at 25-30°C. The progress of the reaction was monitored by TLC.

After 18 h, the reaction mixture was concentrated, the residue was dissolved in water and washed with methyl tert-butyl ether (MTBE, 20 mL), aqueous layer was neutralized to pH ~ 6-7 using 1.5 N HCl and concentrated to obtain the crude product. The crude product was purified by reverse-phase HPLC (RP-HPLC) to obtain pure D as a white solid.
Yield: 60 mg, (35.3 %)
¹H-NMR-DMSO-*d*₆ (400 MHz): δ: 1.39 (9H, d, J=19.2 Hz), 2.78-2.80 (1H, m), 2.99-3.07 (2H, m), 3.77-3.79 (1H, m), 4.11-4.15 (1H, m), 4.59-4.69 (2H, m), 6.64-6.68 (1H, m), 6.78-6.81 (1H, m), 7.51-7.55 (1H, m), 8.08-8.10 (1H, m), 12.9 (1H, bs);
LCMS: 308.2 [M+H]⁺;

### Step 3: Synthesis of tert-butyl 2-{[(3E)-1-ethoxy-5,5-dimethyl-1-oxohex-3-en-2-yl]carbamoyl}-4-(pyridin-2-yl)piperazine-1-carboxylate (F)

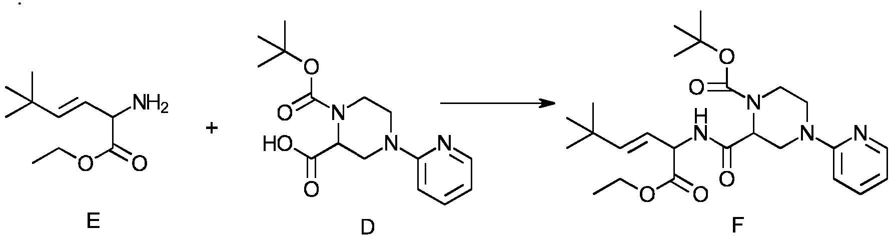

Ethyl (E)-2-amino-5,5-dimethyl-3-hexenoate (E) (39.18 mg, 0.22 mmol) was added to a stirred solution of compound D (65 mg, 0.2114 mmol) in DMF (1.62 mL) followed by DIPEA (0.14 mL, 0.85 mmol) and propyl phosphonic anhydride (T₃P, ~50% solution in ethyl acetate) (0.40 mL, 0.64 mmol) at 25-30°C. The resulting reaction mixture was stirred over a period of 48 h at 40°C. Progress of the reaction was monitored by TLC.

After completion of the reaction, the reaction mixture was quenched by addition of saturated NaHCO₃ solution until pH ~7-8 and product was extracted into ethyl acetate (2 × 20 mL). The combined organic layer was washed with saturated sodium chloride solution, dried over anhydrous sodium sulphate and concentrated in vacuo at 35°C to obtain the crude product. The crude product was purified by CombiFlash^{®} using ethyl acetate/hexane as eluent to obtain the product F as a pale-yellow, thick oil.
Yield: 50 mg, (Impure)
¹H-NMR- CD₃OD (400 MHz): δ: 0.87- 0.89 (9H, m), 1.24 (3H, t, *J*=3.6 Hz), 1.46 (9H, s), 3.46-3.51 (2H, m), 3.93 - 4.2 (5H, m), 4.67 - 4.79 (2H, m), 5.42 - 5.49 (2H, m), 5.69 - 5.82 (1H, m), 6.65 - 6.67 (1H, m), 6.80 - 6.86 (1H, m), 7.51 - 7.55 (1H, m), 8.08 - 8.10 (1H, m);
LCMS: 475.4 [M+H]⁺

### Step 4: Synthesis of (E)-2-[1-tert-butoxycarbonyl-4-(2-pyridyl)-2-piperazinylcarbonylamino]-5,5-dimethyl-3-hexenoic acid (G)

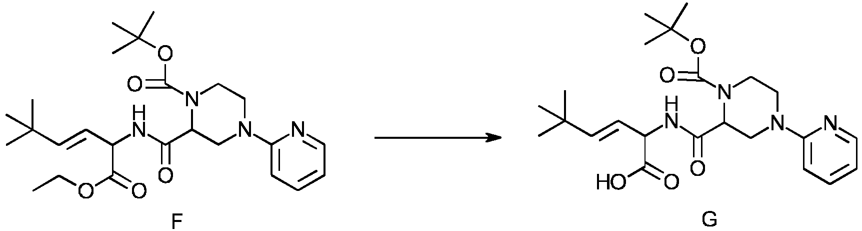

Lithium hydroxide monohydrate (22.1 mg, 0.53 mmol) was added to a stirred solution of compound F (50 mg, 0.11 mmol) in THF/methanol/water (1.5 mL, 1:1:1, 0.5 mL each). The resulting reaction mixture was stirred over a period of 18 h at 25-30°C. The progress of the reaction was monitored by TLC.

After 18 h, the reaction mixture was concentrated, the residue was dissolved in water and washed with ethyl acetate (10 mL), the aqueous layer was acidified to pH ~ 3.4 using 1.5 N HCI and the product was extracted with ethyl acetate (3 × 5 mL). The combined organic layer was dried over sodium sulphate and concentrated at 40°C to obtain a crude solid product. The crude solid product was washed with hexane (5 mL) and DCM (5 mL) to obtain G as a pale brown solid.
Yield: 35 mg, (57.8 %)
¹H-NMR- CD₃OD (400 MHz): δ: 0.98 (9H, s), 1.46 (9H, s), 3.59 - 3.65 (2H, m), 3.91 - 3.99 (1H, m), 4.01 - 4.06 (1H, m), 4.69 - 4.72 (2H, m), 5.44 - 5.51 (1H, m), 5.67 - 5.79 (1H, m), 6.66 - 6.69 (1H, m), 6.81 - 6.85 (1H, m), 7.55 - 7.59 (1H, m), 8.02 - 8.06 (1H, m);
LCMS: 447.4 [M+H] ⁺

### Step 5: Synthesis of (E)-5,5-dimethyl-2-[4-(2-pyridyl)-2-piperazinylcarbonylamino]-3-hexenoic acid (H)

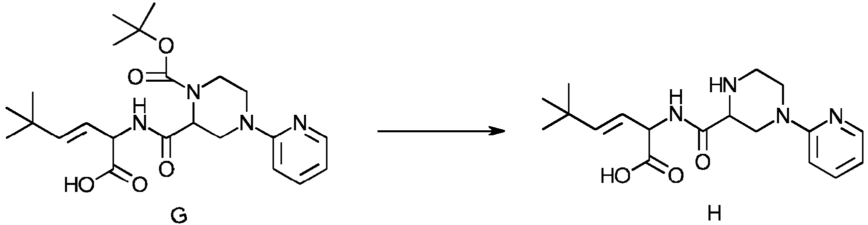

4 M HCI in a 1,4-dioxane solution was added to a stirred solution of compound G (35 mg, 0.0783 mmol) in 1,4-dioxane (0.2 mL) at 25-30°C. The resulting reaction mixture was stirred over a period of 5 h at 25-30°C. The progress of the reaction was monitored by TLC.

After 5 h, the reaction mixture was concentrated; the solid obtained was washed with hexane (5 mL) and diethyl ether (5 mL) to obtain a crude mixture of two diastereomers of the product H. The crude mixture was purified by RP-HPLC to afford two pure diastereomers of H as white solids.
Yield: 4.2 mg (diastereomer-1) and 5.3 mg (diastereomer-2)
LCMS: 347.3 [M+H] ⁺;

### EXAMPLE 4 - Synthesis of (E)-5,5-dimethyl-2-[4-(4-pyridyl)-2-piperazinylcarbonylaminol-3-hexenoic acid (SI4)

### Step 1 Synthesis of tert-butyl 2-methoxycarbonyl-4-phenyl-1-piperazinecarboxylate (C)

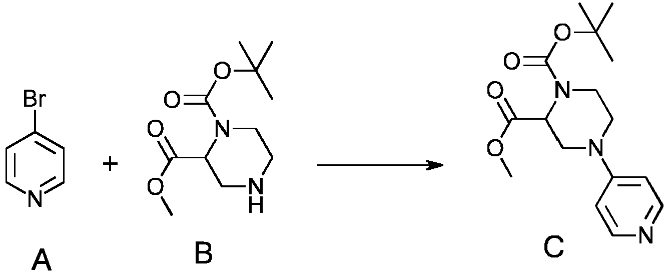

BINAP (0.015 g, 0.00002 mol), Pd₂(dba)₃ (0.011 g, 0.00001 mol) and cesium carbonate (0.32 g, 0.0010 mol) were added to a solution of 4-bromopyridine (A) (0.1 g, 0.0006 mol) and *tert*-butyl 2-methoxycarbonyl-1-piperazinecarboxylate (B) (0.15 g, 0.0006 mol) in toluene (4 mL) at 25-30°C. The resulting reaction mixture was heated at 80°C over a period of 40 h. The progress of the reaction was monitored by TLC.

After completion of the reaction, the reaction mixture was diluted with ethyl acetate (10 mL) and filtered through a plug of celite. The filtrate was concentrated under reduced pressure at 35°C to afford the crude product. This was purified by CombiFlash^{®} using DCM/methanol as eluent to afford compound C as a yellow thick oil.
Yield: 0.19 g, (96.4 %)
¹H-NMR-DMSO-*d*₆ (400 MHz): δ: 1.22 - 1.42 (9H, m), 2.87 - 3.08 (1H, m), 3.13 - 3.18 (2H, m), 3.55 - 3.59 (3H, m), 3.75 - 3.80 (2H, m), 4.23 - 4.33 (1H, m), 4.66 - 4.74 (1H, m), 6.81 (2H, d, *J*=6.4Hz), 8.17 (2H, d, *J*=6.4Hz);
LCMS: 322.2 [M+H]⁺

### Step 2 Synthesis of 1-tert-butoxycarbonyl-4-(4-pyridyl)-2-piperazinecarboxylic acid (D)

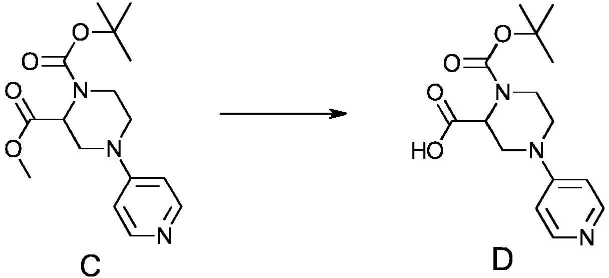

Lithium hydroxide monohydrate (0.12 g, 0.0029 mol) was added to a stirred solution of compound C (0.19 g, 0.0005 mol) in THF/methanol/water (5.7 mL, 1:1:1, 1.9 mL each). The resulting reaction mixture was stirred over a period of 18 h at 25-30°C. The progress of the reaction was monitored by TLC.

After 18 h, the reaction mixture was concentrated, the residue was dissolved in water and washed with MTBE (20 mL). The aqueous layer was neutralized to pH ~ 6-7 using 1.5 N HCI and concentrated to afford crude product. This was purified by RP-HPLC to afford pure compound D as white solid.
Yield: 0.15 g, (87.0 %)
¹H-NMR-DMSO-*d*₆ (400 MHz): δ: 1.37 - 1.42 (9H, m), 3.25 - 3.27 (4H, m), 3.13 - 3.18 (11H, m), 3.55 - 3.61 (2H, m), 4.62 - 4.72 (2H, m), 7.16 - 7.32 (2H, m), 8.26 - 8.37 (2H, m) 13.45 (2H, bs);
LCMS:308.0 [M+H] ⁺

### Step 3 Synthesis of tert-butyl 2-{[(3E)-1-ethoxy-5,5-dimethyl-1-oxohex-3-en-2-yl]carbamoyl}-4-(pyridin-4-yl)piperazine-1-carboxylate (F)

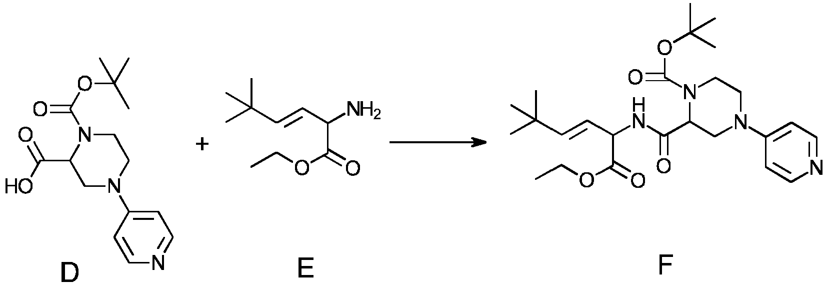

Ethyl (*E*)-2-amino-5,5-dimethyl-3-hexenoate (E) (90.42 mg, 0.4880 mmol) followed by DIPEA (0.33 mL, 1.9521 mmol) and propyl phosphonic anhydride (T₃P, ~50% solution in ethyl acetate) (0.93 mL, 1.4640 mmol) were added to a stirred solution of compound D (150 mg, 0.4880 mmol) in DMF (3.75 mL) at 25-30°C. The resulting reaction mixture was stirred over a period of 48 h at 40°C. The progress of the reaction was monitored by TLC.

After completion of the reaction, the reaction mixture was quenched by addition of saturated NaHCO₃ solution until pH ~7-8 and the product was extracted out using ethyl acetate (2 × 20 mL). The combined organic layer was washed with saturated sodium chloride solution, dried over anhydrous sodium sulphate and concentrated under reduced pressure at 35°C to afford crude material. This was purified by CombiFlash^{®} using DCM/methanol as eluent to afford compound F as a pale-yellow, thick oil.
Yield: 105 mg, (45.3 %)
¹H-NMR-DMSO-*d*₆ (400 MHz): δ: 0.87 - 0.92 (9H, m), 1.18-1.21 (m, 3H), 1.34 - 1.41 (9H, m), 3.50 - 3.12 (1H, m), 3.47 - 3.52 (2H, m), 3.74 - 3.79 (2H, m), 4.04 - 4.23 (3H, m), 4.19 - 4.23 (0.5H, m), 4.49 - 4.51 (0.5H, m), 4.68 - 4.70 (1H, m), 5.36 - 5.41 (1H, m), 5.74 - 5.76 (1H, m), 6.76 (2H, bs), 8.13 (2H, bs), 8.67 - 8.68 (1H, m);
LCMS: 475.4 [M+H]⁺

### Step 4 Synthesis of (E)-2-[1-tert-butoxycarbonyl-4-(4-pyridyl)-2-piperazinylcarbonylamino]-5,5-dimethyl-3-hexenoic acid (G)

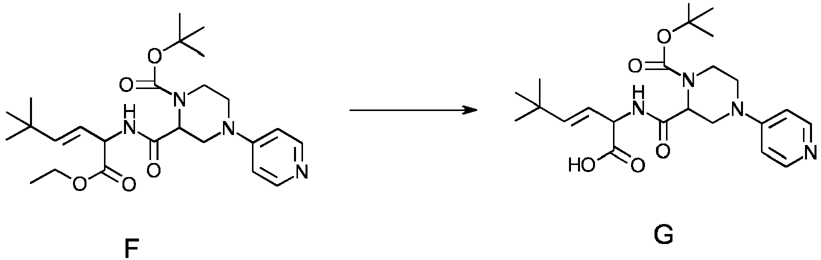

Lithium hydroxide monohydrate (44.20 mg, 1.05 mmol) was added to a stirred solution of compound F (100 mg, 0.21 mmol) in THF/methanol/water (3 mL, 1:1:1, 1.0 mL each). The resulting reaction mixture was stirred over a period of 18 h at 25-30°C. The progress of the reaction was monitored by TLC.

After 18 h, the reaction mixture was concentrated, the residue was dissolved in water and washed with ethyl acetate (10 mL). The aqueous layer was acidified to pH ~ 3-4 using 1.5 N HCI and the product was extracted out with ethyl acetate (3 × 5 mL). The combined organic layer was dried over anhydrous sodium sulphate and concentrated under reduced pressure at 40°C to afford crude solid material. This was triturated with hexane (5 mL) and DCM (5 mL) respectively to afford compound G as an off-white solid.
Yield: 27 mg
¹H-NMR- DMSO-*d*₆ (400 MHz): δ: 0.88 - 0.98 (9H, m), 1.37 - 1.42 (9H, m), 3.59 - 3.65 (2H, m), 3.91 - 3.99 (1H, m), 4.01 - 4.06 (1H, m), 4.63 - 4.71 (1H, m), 5.36 - 5.44 (1H, m), 6.88 - 6.91 (2H, m), 8.14 - 8.17 (2H, m);
LCMS: 447.4 [M+H] ⁺

### Step 5 Synthesis of (E)-5,5-dimethyl-2-[4-(4-pyridyl)-2-piperazinylcarbonylamino]-3-hexenoic acid (H)

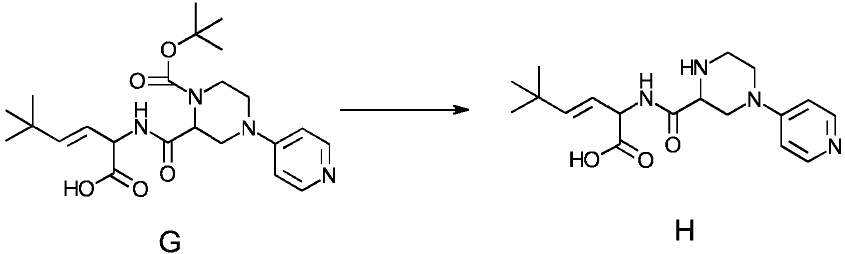

4 M HCI in 1,4-dioxane solution (0.5 mL) was added to a stirred solution of compound G (25 mg, 0.0559 mmol) in 1,4-dioxane (0.3 mL) at 25-30°C. The resulting reaction mixture was stirred over a period of 5 h at 25-30°C. The progress of the reaction was monitored by TLC.

After 5 h, the reaction mixture was concentrated, the solid material obtained was triturated with hexane (5 mL) and diethyl ether (5 mL) to afford a crude mixture. This was purified by RP-HPLC to afford two diastereomers as white solids.
Yield: 2 mg (isomer-1) and 1.9 mg (isomer-2)
LCMS: 347.3 [M+H] ⁺

### EXAMPLE 5 - Synthesis of (E)-5,5-dimethyl-2-(6-phenoxynicotinoylamino)-3-hexenoic acid (SI5)

### Step 1 Synthesis of ethyl (E)-5,5-dimethyl-2-(6-phenoxynicotinoylamino)-3-hexenoate (C)

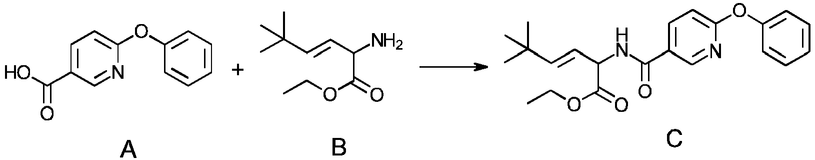

6-Phenoxynicotinic acid (A) (100 mg, 0.23 mmol) followed by DIPEA (0.32 mL, 0.92 mmol) and propyl phosphonic anhydride (T₃P, ~50% solution in ethyl acetate) (0.88 mL, 0.69 mmol) were added to a stirred solution of ethyl (E)-2-amino-5,5-dimethyl-3-hexenoate (B) (86.08 mg, 0.23 mmol) in DMF (2.5 mL) at 25-30°C. The resulting reaction mixture was stirred over a period of 48 h at 40°C. The progress of the reaction was monitored by TLC.

After completion of reaction, the reaction mixture was quenched by addition of saturated NaHCO₃ solution to pH ~7-8 and the product was extracted to ethyl acetate (2 × 20 mL). The combined organic layer was washed with saturated sodium chloride solution, dried over anhydrous sodium sulphate and concentrated in vacuum at 35°C to afford the crude product. The crude product was purified by CombiFlash^{®} using ethyl acetate/hexanes as eluent to afford compound C as yellow thick oil.
Yield: 71.3 mg, (40.2 %)
¹H-NMR- DMSO-*d*₆ (400 MHz): δ: 0.99 (9H, s), 1.16 (3H, t, *J*=7.2 Hz), 4.10 (2H, m), 4.89-4.91 (1H, m), 5.52 - 5.54 (1H, m), 5.85 (1H, d, *J*=14.4 Hz), 7.08- 7.10 (1H, m), 7.17 - 7.23 (2H, m), 7.24 - 7.27 (1H, m), 7.41 - 7.45 (2H, m), 8.28 (1H, d, *J₁*=2.8 Hz, *J₂*=8.8 Hz), 8.62 - 8.63 (1H, m), 8.96 (1H, s)
LCMS: 383.3 [M+H]⁺

### Step 2 Synthesis of (E)-5,5-dimethyl-2-(6-phenoxynicotinoylamino)-3-hexenoic acid (D)

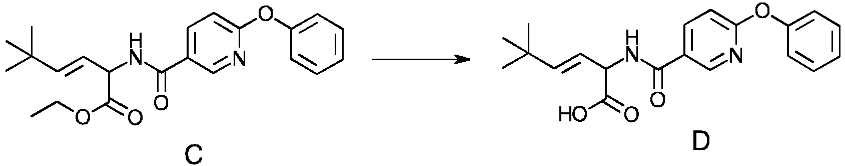

Lithium hydroxide monohydrate (21.94 mg, 0.52 mmol) was added to a stirred solution of compound C (100 mg, 0.26 mmol) in THF/methanol/water (3 mL, 1:1:1, 1.0 mL each). The resulting reaction mixture was stirred over a period of 5 h at 25-30°C. The progress of the reaction was monitored by TLC.

After 18 h, reaction mixture was concentrated, the residue was dissolved in water and washed with diethyl ether (10 mL), the aqueous layer was acidified to pH ~ 5-6 using 1.5 N HCI and the product was extracted with ethyl acetate (3 × 5 mL). The combined organic layer was dried over sodium sulphate and concentrated at 40°C to afford a crude solid material. This was washed with hexane (5 mL) to afford the crude product (mixture of two isomers) as an off white solid. The crude material was purified by RP-HPLC to afford compound D as a white solid.
Yield: 19 mg
¹H-NMR- DMSO-*d*₆ (400 MHz): δ: 0.96 (9H, s), 4.68 (1H, t, J=6.0 Hz), 5.51-5.64 (2H, m), 7.06 (1H, d, J=8.8 Hz), 7.15 - 7.17 (2H, m), 7.22 - 7.25 (1H, m), 7.41 - 7.45 (2H, m), 8.26 - 8.28 (2H, m), 8.41 (1H, d, J=6.4 Hz), 8.61 (1H, d, J=2.4 Hz);
LCMS: 355.3 [M+H] ⁺; HPLC purity - 99.5 %.

### Separation of enantiomers of (E)-5,5-dimethyl-2-(6-phenoxynicotinoylamino)-3-hexenoic acid (SI5)

This separation was carried out in a similar way as described above in Example 1 starting with 250 mg of crude racemic (*E*)-2-[2-(dimethylamino)-4-methyl-5-pyrimidinylcarbonylamino]-5,5-dimethyl-3-hexenoic acid. The preparative column was CHIRALPAK-IG (250×30 mm) and the elution was carried out with hexanes/EtOAc/MeOH//TFA in a ratio of 70/15/15/0.1. The flow rate was 40 mL/min, detection UV 245 mm. The feed concentration was 10 mg/mL and the injection volume was 5 mL (on column 50 mg). The retention time for enantiomer 1 was 4.2 min and for enantiomer 2 4.6 min. CHIRALPAK-IG (250×4.6 mm) was used as analytical column.

The separation resulted in 60 mg of enantiomer 1 with HPLC purity of 99.9 % and 58 mg of enantiomer 2 with HPLC purity of 99.4 %.

### EXAMPLE 6 - Synthesis of (E)-5,5-dimethyl-2-[m-(4H-1,2,4-triazol-4-yl)benzoylaminol-3-hexenoic acid (SI6)

### Step 1 Synthesis of ethyl (E)-5,5-dimethyl-2-[m-(4H-1,2,4-triazol-4-yl)benzoylamino]-3-hexenoate (C)

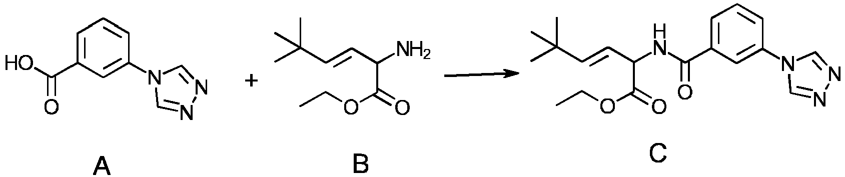

*m*-(4*H*-1,2,4-Triazol-4-yl)benzoic acid (A) (120 mg, 0.64 mmol) followed by DIPEA (0.45 mL, 2.59 mmol) and propyl phosphonic anhydride (T₃P, ~50% solution in ethyl acetate) (1.23 mL, 1.94 mmol) were added to a stirred solution of ethyl (E)-2-amino-5,5-dimethyl-3-hexenoate (B) (122.5 mg, 0.64 mmol) in DMF (3 mL) at 25-30°C. The resulting reaction mixture was stirred over a period of 48 h at 40°C. The progress of the reaction was monitored by TLC.

After the completion of the reaction, the reaction mixture was quenched by addition of saturated NaHCO₃ solution to pH ~7-8 and the product was extracted to ethyl acetate (2 × 20 mL). The combined organic layer was washed with saturated sodium chloride solution, dried over anhydrous sodium sulphate and concentrated in vacuo at 35°C to afford the crude product. This was purified by RP-HPLC to afford compound C as clear thick oil.
Yield: 70 mg, (31.0 %)
¹H-NMR- DMSO-*d*₆ (400 MHz): δ: 1.06 (9H, s), 1.17 (3H, t, *J*=7.2 Hz), 4.14 (2H, t, *J=7.2* Hz), 4.92-4.94 (1H, m), 5.54 - 5.57 (1H, m), 5.90 (1H, d, *J=*14.4 Hz), 7.66- 7.70 (1H, m), 7.87 - 7.93 (2H, m), 8.17 (1H, t, *J*=1.6 Hz), 9.02 (1H, m), 9.17 (2H, s);
LCMS: 357.3 [M+H]⁺

### Step 2 Synthesis of (E)-5,5-dimethyl-2-[m-(4H-1,2,4-triazol-4-yl)benzoylamino]-3-hexenoic acid (D)

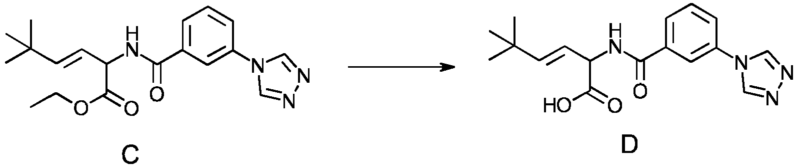

Lithium hydroxide monohydrate (16.57 mg, 0.39 mmol) was added to a stirred solution of compound C (70 mg, 0.19 mmol) in THF/methanol/water (2.1 mL, 1:1:1, 0.7 mL each). The resulting reaction mixture was stirred over a period of 5 h at 25-30°C. The progress of the reaction was monitored by TLC.

After 18 h, the reaction mixture was concentrated, the residue was dissolved in water and washed with diethyl ether (10 mL), and the aqueous layer was acidified to pH ~ 5-6 using 1.5 N HCI. The precipitate formed was collected by vacuum filtration, washed with water (5 mL) and hexane (5 mL) to give compound D as a white solid.
Yield: 28 mg (43.2 %)
¹H-NMR- DMSO-*d*₆ (400 MHz): δ: 1.00 (9H, s), 4.91 - 4.95 (1H, m), 5.52 - 5.58 (1H, m), 5.83 - 5.87 (1H, m), 7.65 - 7.69 (1H, m), 7.87 - 7.96 (2H, m), 8.16 (1H, t, *J*=1.6 Hz), 9.02 (1H, m), 9.17 (2H, s); 12.72 (1H, bs);
LCMS: 329.3 [M+H] ⁺ HPLC purity - 90.7%

### EXAMPLE 7 - Synthesis of (E)-5,5-dimethyl-2-(6-methyl-1H-1,5-diazainden-2-ylcarbonylamino)-3-hexenoic acid (SI7)

### Step 1 Synthesis of methyl 2-azido-3-(2-chloro-6-methyl-3-pyridyl)acrylate (C)

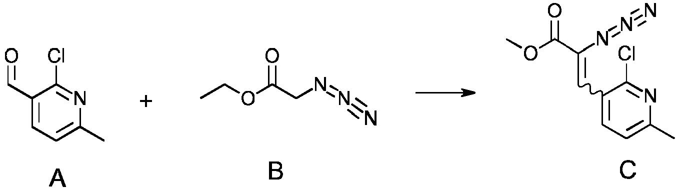

Sodium methoxide solution (25 wt. % in methanol) (62.49 mL, 0.2898 mol) was added dropwise to a stirred solution of 2-chloro-6-methylnicotinaldehyde (A) (5.0 g, 0.032 mol) and ethyl azido acetate (B) (9.26 mL, 0.080 mol) in methanol (50 mL) at -20°C to -30°C over a period of 20 min. The resulting reaction mixture was stirred at 0-5°C over a period of 5 h. The progress of the reaction was monitored by TLC.

After completion of the reaction, the reaction mixture was quenched with ice-cold NH₄Cl solution and stirred at 5-10°C over a period of 1 h to give a solid precipitation. The solid was collected by vacuum filtration, washed with water and dried to afford compound C as a pale yellow solid.
Yield: 1.88 g, (23.2 %)
¹H-NMR-DMSO-*d*₆ (400 MHz): δ: 2.46 (3H, s), 3.87 (3H, s), 6.99 (1H, s), 7.36 (1H, d, *J*=10.8 Hz), 8.46 (1H, d, *J*=10.8 Hz);
LCMS: 352.8 [M+H] ⁺

### Step 2 Synthesis of methyl 4-chloro-6-methyl-1H-1,5-diazaindene-2-carboxylate (D)

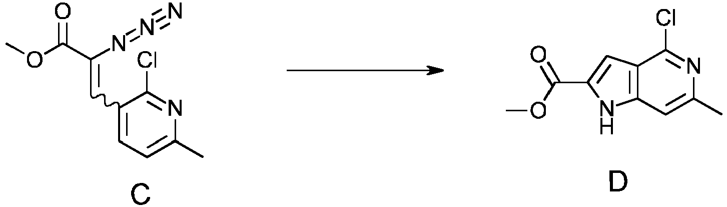

A solution of compound C (1.8 g, 0.0071 mol) in o-xylene (45 mL) was heated at 120°C over a period of 4 h. After 4 h, the heating was switched off and the reaction mixture was left to stand at 25-30°C for 16 h (solid precipitation was formed).

The solid was collected by vacuum filtration, washed with hexane (50 mL) and dried under reduced pressure to afford compound D as a pale yellow solid.
Yield: 0.85 g, (53.5 %)
¹H-NMR-DMSO-*d*₆ (400 MHz): δ: 3.88 (3H, s), 7.12 (1H, s), 7.22 (1H, s), 12.59 (1H, s); LCMS: 224.6 [M+H]⁺

### Step 3 Synthesis of methyl 6-methyl-1H-1,5-diazaindene-2-carboxylate (E)

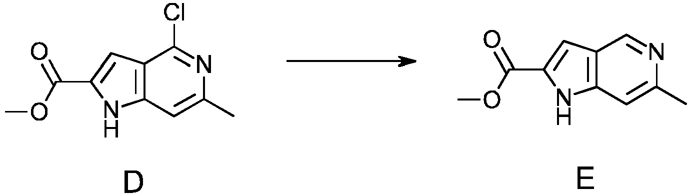

10 % Pd/C (0.17 g, 0.2 % w/w) was added to a degassed solution of compound D (0.85 g, 0.0037 mol) in MeOH (30.6 mL) and THF (10.2 mL) at 25-30°C. The resulting reaction mixture was stirred under H₂ atmosphere over a period of 4 h. The progress of the reaction was monitored by TLC.

After completion of the reaction, the reaction mixture was filtered through a plug of celite and the celite plug was then washed with MeOH (50 mL). The combined filtrate was evaporated to dryness to afford the crude product (HCI salt) (0.8 g). The crude solid product was stirred with saturated NaHCO₃ solution and DCM over a period of 1 h at 25-30°C. After 1 h, the layers were separated, the organic layer was dried over sodium sulphate and concentrated at 40°C to afford compound E as a pale yellow solid.
Yield: 0.69 mg, (95.9 %)
¹H-NMR-DMSO-*d*₆ (400 MHz): δ: 3.88 (3H, s), 7.12 (1H, s), 7.22 (1H, s), 12.59 (1H, s);
LCMS: 191.1 [M+H] ⁺

### Step 4 Synthesis of methyl 6-methyl-1-{[2-(trimethylsilyl)ethoxy]methyl}-1H-1,5-diazaindene-2-carboxylate (F)

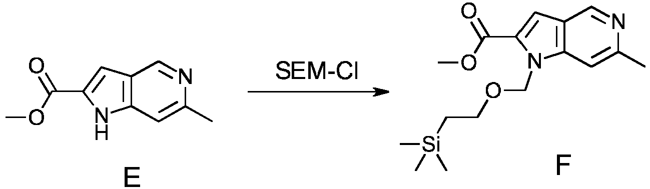

Sodium hydride (60% dispersion in mineral oil) (0.16 g, 0.0039 mol) was added in portions (4 portions) over a period of 15 min to a stirred solution of compound E (0.69 g, 0.0036 mol) in THF (13.8 mL) and DMF (6.9 mL) at 0-5°C. After 10 min of stirring, SEM-CI (0.70 mL, 0.039 mol) was added dropwise at 0-5°C. The resulting reaction mixture was stirred over a period of 18 h at 25-30°C. The progress of the reaction was monitored by TLC.

After 18 h, the reaction mixture was quenched by the addition of ice-cold water and the product was extracted to ethyl acetate (3 × 50 mL). The combined organic layer was dried over sodium sulphate and concentrated at 40°C to afford crude solid. The crude solid was purified by CombiFlash^{®} using ethyl acetate/hexane as eluent to afford pure product F as a brown thick oil.
Yield: 0.55 g (47.4 %)
¹H-NMR-DMSO-*d*₆ (300 MHz): δ: -0.13 (9H, s), 0.75 (2H, t, *J*=10.4 Hz), 2.55 (3H, s), 3.41 (2H, t, *J=*2.0 Hz), 3.85 (3H, s), 5.90 (2H, s), 7.43 (1H, s), 7.54 (1H, s), 8.86 (1H, s);
LCMS: 321.3 [M+H] ⁺

### Step 5 Synthesis of 6-methyl-1-{[2-(trimethylsilyl)ethoxy]methyl}-1H-1,5-diazaindene-2-carboxylic acid (G)

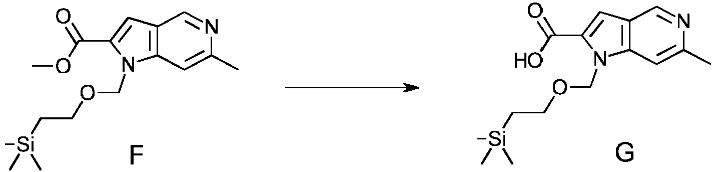

Lithium hydroxide monohydrate (0.19 g, 0.0046 mol) was added to a stirred solution of compound F (0.3 g, 0.0009 mol) in THF/methanol/water (9 mL, 1:1:1, 3 mL each). The resulting reaction mixture was stirred over a period of 18 h at 25-30°C. The progress of the reaction was monitored by TLC.

After 18 h, the reaction mixture was concentrated, and the remaining residue was dissolved in water and washed with MTBE (20 mL), the aqueous layer was neutralized to pH ~ 5-6 using 1.5 N HCI to afford a solid precipitate. The solid was collected by vacuum filtration, washed with water and dried at 40°C to afford compound G as a pale brown solid.
Yield: 0.24 g (83.9 %)
¹H-NMR-DMSO-*d*₆ (400 MHz): δ: -0.13 (9H, s), 0.80 (2H, t, *J*=8.0 Hz), 2.70 (3H, s), 3.47 (2H, t, *J*=7.6 Hz), 6.01 (2H, s), 7.59(1H, s), 8.02 (1H, s), 9.20 (1H, s), 14.56 (1H, bs)
LCMS: 307.2 [M+H] ⁺

### Step 6 Synthesis of ethyl (E)-5,5-dimethyl-2-(6-methyl-1-{[2-(trimethylsilyl)ethoxy]methyl}-1H-1,5-diazainden-2-ylcarbonylamino)-3-hexenoate (I)

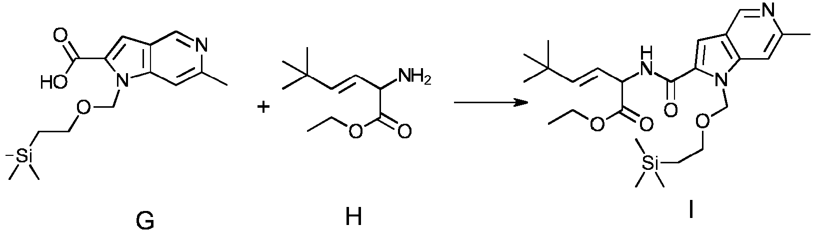

Ethyl (E)-2-amino-5,5-dimethyl-3-hexenoate (H) (96.73 mg, 0.5221 mmol), followed by HATU (297.8 mg, 0.7832 mmol), HOBt (105.8 mg, 0.7832 mmol) and DIPEA (0.18 mL, 1.0442 mmol) were added to a stirred solution of compound G (160 mg, 0.5221 mmol) in DMF (3.2 mL) at 25-30°C. The resulting reaction mixture was stirred over a period of 18 h at 25-30°C. The progress of the reaction was monitored by TLC.

After completion of the reaction, the reaction mixture was quenched by addition of saturated NaHCO₃ solution until pH ~7-8 and then the product was extracted to ethyl acetate (2 × 20 mL). The combined organic layer was washed with saturated sodium chloride solution, dried over anhydrous sodium sulphate and concentrated in vacuo at 35°C to afford crude product. The crude product was purified by CombiFlash^{®} using ethyl acetate/hexane as eluent to afford compound I as yellow thick oil.
Yield: 83 mg (29.4 %)
¹H-NMR-DMSO-*d*₆ (400 MHz): δ: -0.14 (9H, S), 0.73 (2H, t, *J*=10.4 Hz), 0.99 (9H, S), 1.18(3H, t, *J*= 7.22 Hz), 2.54 (3H, s), 3.37 (2H, m), 4.04 - 4.14 (2H, m), 4.87 (1H, t, *J*=7.2 Hz), 5.55 - 5.72 (1H, m), 5.87 - 5.90 (3H, m), 7.33 (1H, s), 7.45 (1H, s), 8.83 (1H, s), 9.10 (1H, d, *J=*7.2 Hz);
LCMS: 474.4 [M+H] ⁺

### Step 7 Synthesis of (E)-5,5-dimethyl-2-(6-methyl-1-{[2-(trimethylsilyl)ethoxy]methyl}-1H-1,5-diazainden-2-ylcarbonylamino)-3-hexenoic acid (J)

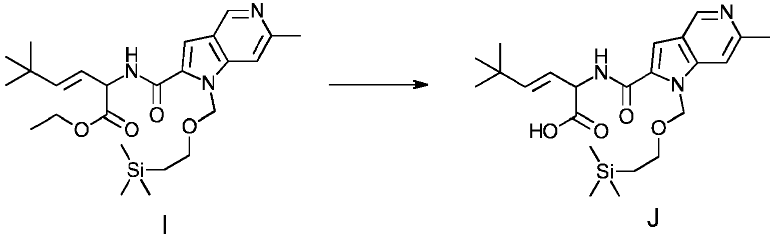

Lithium hydroxide monohydrate (35.4 mg, 0.8444 mmol) was added to a stirred solution of compound I (80 mg, 0.1688 mmol) in THF/methanol/water (2.4 mL, 1:1:1, 0.8 mL each). The resulting reaction mixture was stirred over a period of 18 h at 25-30°C. The progress of the reaction was monitored by TLC.

After 18 h, the reaction mixture was concentrated, the residue was dissolved in water and washed with ethyl acetate (10 mL) and the aqueous layer was acidified to pH ~ 5-6 using 1.5 N HCI at 0-5°C (solid precipitated). Solid was collected by vacuum filtration, washed with water and dried at 40°C to afford pure compound J as off white solid.
Yield: 44 mg (58.5 %)
¹H-NMR-DMSO-*d*₆ (400 MHz): δ: -0.13 (9H, S), 0.75 (2H, t, *J*=1.6 Hz), 1.0 (9H, S), 2.69 (3H, s), 3.44 (2H, t, *J*=6.8 Hz), 4.91 (1H, t, *J*=6.4 Hz), 5.51 - 5.55 (1H, m), 5.82 - 5.85 (1H, m), 6.0 - 6.02 (2H, m), 7.58 (1H, s), 7.97 (1H, s), 9.24 - 9.32 (1H, m), 12.87 (1H, bs), 15.34 (1H, bs);
LCMS: 446.2 [M+H]⁺

### Step 8 Synthesis of (E)-5,5-dimethyl-2-(6-methyl-1H-1,5-diazainden-2-ylcarbonylamino)-3-hexenoic acid (K)

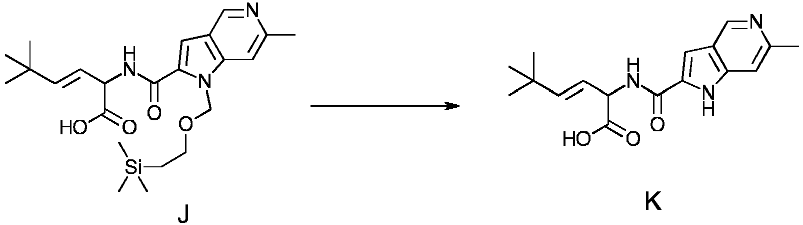

4 M HCI in 1,4-dioxane solution (0.8 mL) was added to a stirred solution of compound J (40 mg, 0.0897 mmol) in 1,4-dioxane (0.4 mL), at 25-30°C. The resulting reaction mixture was stirred over a period of 18 h at 25-30°C. The progress of the reaction was monitored by TLC.

After 18 h, the reaction mixture was concentrated; the solid obtained was washed with hexane (5 mL) and diethyl ether (5 mL) to afford the crude product. This was purified by RP-HPLC to afford the pure compound K as a white solid.
Yield: 8 mg (28.3 %)
¹H-NMR-DMSO-*d*₆ (400 MHz): δ: 1.0 (9H, S), 2.49 (3H, s), 4.90 (1H, t, *J*=6.0 Hz), 5.53 - 5.55 (1H, m), 5.79 (1H, t, *J*=15.6 Hz), 7.17 (1H, s), 7.35 (1H, s), 8.79 - 8.84 (3H, m), 11.83 (1H, bs);
LCMS: 316.3 [M+H] ⁺; HPLC purity 93.5 %.

### EXAMPLE 8 - Synthesis of (E)-2-(4-chloro-2-pyrrolylcarbonylamino)-5,5-dimethyl-3-hexenoic acid (SI8)

### Step 1 Synthesis of 4-chloro-1-{[2-(trimethylsilyl)ethoxy]methyl}-2-pyrrolecarboxylic acid (B)

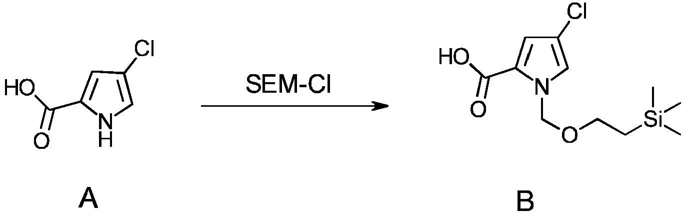

K₂CO₃ (0.99 g, 0.0072 mol) and 2-(trimethylsilyl)ethoxymethyl chloride (SEM-CI) (0.43 mL, 0.0024 mol) were added to a stirred solution of 4-chloro-1H-pyrrole-2-carboxylic acid (A) (0.3 g, 0.0020 mol) in DMF (3 mL) at 25-30°C. The resulting reaction mixture was stirred at 25-30°C over a period of 18 h. The progress of the reaction was monitored by TLC.

After completion of the reaction, the reaction mixture was filtered through a plug of celite. The filtrate was concentrated under reduced pressure at 40°C. Upon concentration, a thick oily mass was obtained, and it was dissolved in water and the pH of the solution was adjusted to 3-4 using 1.5 N HCI solution. The product was extracted using ethyl acetate (3 × 10 mL). The organic layer was dried over anhydrous sodium sulphate and concentrated under reduced pressure at 40°C to afford the crude product. This was further purified by using CombiFlash^{®} using ethyl acetate/hexane as eluent to afford the product B as a clear thick oil.
Yield: 0.22 g (38.7 %)
¹H-NMR-DMSO-*d*₆ (400 MHz): δ: 0.11 (9H, s), 0.89 (2H, t, *J*=9.4 Hz), 3.72 (2H, t, *J*=8.8 Hz), 5.38 (2H, s), 6.79 - 6.80 (1H, m), 7.17 - 7.18 (1H, m), 12.3 (1H, bs);
LCMS: No ionization was observed

### Step 2 Synthesis of ethyl (E)-2-(4-chloro-2-pyrrolylcarbonylamino)-5,5-dimethyl-3-hexenoate (D)

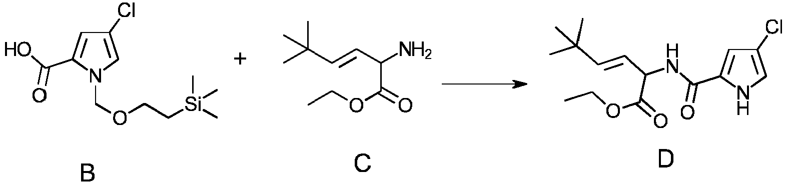

Oxalyl chloride (0.1 mL, 0.0011 mol) was added dropwise along with a catalytic amount of DMF (0.12 mL) to a stirred solution of compound B (0.3 g, 0.0009 mol) in DCM (6 mL) at 0-5°C. The resulting reaction mixture was stirred at 25-30°C over a period of 2 h. The progress of the reaction was monitored by TLC.

After 2 h, the reaction mixture was concentrated to dryness at 35°C and any remaining water was stripped off with toluene (3 × 5 mL). This resulted in a yellow thick oil. This was further dissolved in DCM (3 mL) and added to a stirred solution of ethyl (*E*)-2-amino-5,5-dimethyl-3-hexenoate (C) (0.16 g, 0.0009 mol) and DIPEA (0.62 mL, 0.0036 mol) in DCM (3 mL) at 25-30°C. The resulting reaction mixture was stirred over a period of 18 h at 25-30°C. The progress of the reaction was monitored by TLC.

After 18 h, the reaction mixture was quenched by the addition of saturated NaHCO₃ solution (15 mL) and the product was extracted with ethyl acetate (3 × 10 mL). The combined organic layer was dried over anhydrous sodium sulphate and concentrated under reduced pressure at 35°C to afford the crude product as a pale brown thick oil. The crude product was purified by CombiFlash^{®} using ethyl acetate/hexane as an eluent to afford the pure product D as a yellow thick oil.
Yield: 40 mg (14.3 % over two steps)
¹H-NMR-DMSO-*d*₆ (400 MHz): δ: 0.99 (9H, s), 1.15 (3H, t, *J*=7.2 Hz), 4.05 - 4.14 (2H, m), 4.84 - 4.88 (1H, m), 5.45 - 5.50 (1H, m), 5.74 - 5.83 (1H, m), 6.94 - 6.98 (2H, m), 8.47 (1H, t, *J*=6.8 Hz); 11.89 (1H, bs).

### Step 3 Synthesis of (E)-2-(4-chloro-2-pyrrolylcarbonylamino)-5,5-dimethyl-3-hexenoic acid (E)

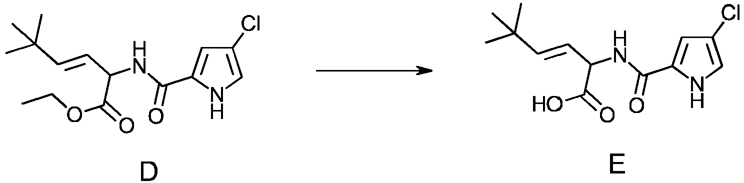

Lithium hydroxide monohydrate (10.73 mg, 0.255 mmol) was added to a stirred solution of compound D (40 mg, 0.1278 mmol) in THF/methanol/water (1.2 mL, 1:1:1, 0.4 mL each). The resulting reaction mixture was stirred over a period of 18 h at 25-30°C. The progress of the reaction was monitored by TLC.

After 18 h, the reaction mixture was concentrated, the remaining residue was dissolved in water and washed with ethyl acetate (10 mL), and the aqueous layer was acidified to pH ~ 5-6 using 1.5 N HCI at 0-5°C. The product was extracted with ethyl acetate (3 × 5 mL). The combined organic layer was dried over sodium sulphate and concentrated at 40°C to afford a crude solid. The crude mixture was purified by RP-HPLC to afford product E as a white solid.
Yield: 3.8 mg
LCMS: 285.2 [M+H] ⁺

### EXAMPLE 9 - Synthesis of (E)-2-(3-isobutyl-1-methyl-5-pyrazolylcarbonylamino)-5,5-dimethyl-3-hexenoic acid (SI9)

### Step 1 Synthesis of ethyl (E)-2-(3-isobutyl-1-methyl-5-pyrazolylcarbonylamino)-5,5-dimethyl-3-hexenoate (C)

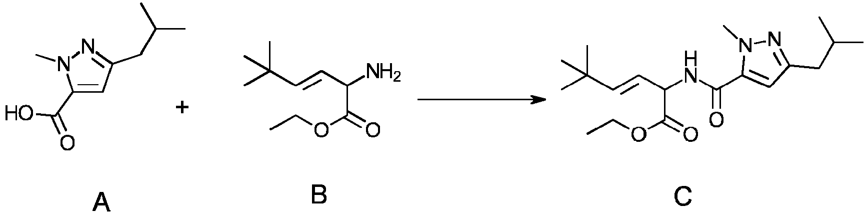

3-Isobutyl-1-methyl-5-pyrazolecarboxylic acid (A) (100 mg, 0.27 mmol) followed by DIPEA (0.38 mL, 1.09 mmol) and propyl phosphonic anhydride (T₃P, ~50% solution in ethyl acetate) (1.04 mL, 0.82 mmol) were added to a stirred solution of ethyl (E)-2-amino-5,5-dimethyl-3-hexenoate (B) (101.6 mg, 0.27 mmol) in DMF (2.5 mL) at 25-30°C. The reaction mixture was stirred over a period of 48 h at 40°C. The progress of the reaction was monitored by TLC.

After completion of the reaction, the reaction mixture was quenched by addition of saturated NaHCO₃ solution to pH ~7-8 and the product was extracted with ethyl acetate (2 × 20 mL). The combined organic layer was washed with saturated sodium chloride solution, dried over anhydrous sodium sulphate and concentrated under reduced pressure at 35°C to afford the crude product. This was purified by CombiFlash^{®} using ethyl acetate/hexane as eluent to afford a mixture of compound C and an isomer as a yellow thick oil.
Yield: 66.6 mg (34.8 %)
¹H-NMR-DMSO-*d*₆ (400 MHz): δ: (mixture of isomers) 0.88 - 0.90 (9H, s), 0.97 (9H, s), 1.20 - 1.23 (6H, m), 1.82 - 1.86 (1H, m), 2.37 - 2.40 (3H, m), 3.93 (3H, s), 4.07 - 4.14 (2H, m), 4.81 - 4.83 (1H, m), 5.49 - 5.51 (1H, m), 5.81 - 5.86 (1H, m), 6.58 (0.29H, m), 6.77 (1.31H, m), 8.79 (1H, m), 9.59 (0.29H, m);
LCMS: 350.3 [M+H]⁺

### Step 2 Synthesis of (E)-2-(3-isobutyl-1-methyl-5-pyrazolylcarbonylamino)-5,5-dimethyl-3-hexenoic acid (D)

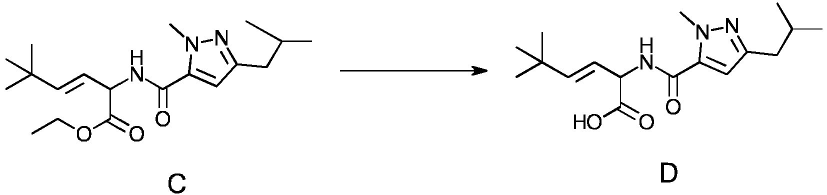

Lithium hydroxide monohydrate (24.01 mg, 0.57 mmol) was added to a stirred solution of compound C (100 mg, 0.28 mmol) in THF/methanol/water (3 mL, 1:1:1, 1.0 mL each). The resulting reaction mixture was stirred over a period of 5 h at 25-30°C. The progress of the reaction was monitored by TLC.

After 18 h, the reaction mixture was concentrated, the residue was dissolved in water and washed with diethyl ether (10 mL). The aqueous layer was acidified to pH ~ 5-6 using 1.5 N HCI and the product was extracted with ethyl acetate (3 × 5 mL). The combined organic layer was dried over sodium sulphate and concentrated at 40°C to afford a crude solid. This was washed with hexane (5 mL) to afford a crude mixture of two isomers as an off white solid. The mixture was purified by RP-HPLC to afford the pure compound D as a white solid.
Yield: 23.4 mg
¹H-NMR-DMSO-*d*₆ (400 MHz): δ: 0.83 (6H, d, *J*=6.4 Hz), 0.91 (9H, s), 1.78 - 1.82 (1H, m), 2.34 (2H, d, *J=7.2* Hz), 3.93 (3H, s), 4.50 - 4.53 (1H, m), 5.46 - 5.58 (2H, m), 6.64 (1H, s), 8.04 (1H, d, *J*=5.2 Hz); LCMS: 322.3 [M+H] ⁺
HPLC Purity - 99.76 %

### EXAMPLE 10 - Synthesis of (E)-2-(4-[amino(2-pyridyl)methyl]-2-pyrrolylcarbonylamino)-5,5-dimethyl-3-hexenoic acid (SI13)

### Step 1: Synthesis of 1-(2-pyridyl)-2-propyn-1-ol (B)

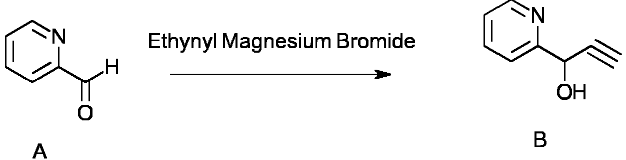

A pre-dissolved solution of 2-pyridinecarboxaldehyde (A) (2.0 g, 0.018 mol) in THF (6.0 mL) was added dropwise over a period of 15-20 min at -10°C to -15°C to a stirred solution of ethynyl magnesium bromide solution (0.5 M in THF) (44.7 mL, 0.0224 mol). The resulting reaction mixture was stirred over a period of 1 h at -5°C to -10°C and at 25-30°C for 1 h. The progress of the reaction was monitored by TLC.

After completion of the reaction, the reaction mixture was quenched with saturated NH₄Cl solution (100 mL) and the product was extracted with ethyl acetate (3 × 50 mL). The combined organic layer was dried over anhydrous sodium sulphate and concentrated in vacuo at 40°C to afford crude product. The crude solid was purified by CombiFlash^{®} using ethyl acetate/hexane as eluent to afford the product B as a brown oil.
Yield: 1.56 g (63.2 %)
¹H-NMR-CDCl₃ (300 MHz): δ: 2.59 (1H, d, *J*=2.8 Hz), 5.53 (1H, d, *J*=2.8 Hz), 7.31 - 7.37 (1H, m), 7.56 (1H, d, *J=*10.4 Hz), 7.77 - 7.82 (1H, m), 8.58 (1H, t, J=5.2 Hz);
LCMS: 134.23 [M+H]⁺

### Step 2: Synthesis of ethyl 4-[(2-pyridyl)carbonyl]-2-pyrrolecarboxylate (C)

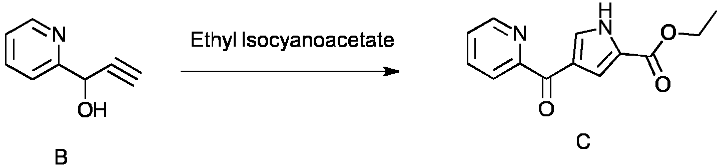

Silver carbonate (0.414 g, 0.0015 mol) was added to stirred solution of compound B (1.5 g, 0.011 mol) and ethyl isocyanoacetate (0.849 ml, 0.011 mol) in 1,4-dioxane at 80°C. The resulting reaction mixture was stirred over a period of 16 h at 80°C. The progress of the reaction was monitored by TLC.

After completion, the reaction mixture was evaporated to dryness at 40°C. The obtained residue was dissolved in DCM (50 mL), and the solution was filtered through a celite plug. The DCM layer was washed with saturated sodium chloride solution (2 × 50 mL). The organic layer was dried over anhydrous sodium sulphate and concentrated in vacuo at 40°C to afford a crude product. The crude product was purified by CombiFlash^{®} using ethyl acetate/hexane as eluent to afford the product (C) as a brown thick oil.
Yield: 0.16 g (6 %)
¹H-NMR-CDCl₃ (400 MHz): δ: 1.33 (3H, t, *J*=6.8 Hz), 4.35 (2H, q, *J*=5.6 Hz), 7.27 - 7.49 (1H, m), 7.71 (1H, d, *J*=1.2 Hz), 7.86 - 7.90 (1H, m), 8.13 - 8.15 (1H, m), 8.32 (1H, d, J=3.2 Hz), 8.71 - 8.74 (1H, m), 9.55 (1H, bs);
LCMS: 245.28 [M+H]⁺

### Step 3: Synthesis of ethyl 4-[(2-pyridyl)carbonyl]-1-{[2-(trimethylsilyl)ethoxy]methyl}-2-pyrrolecarboxylate

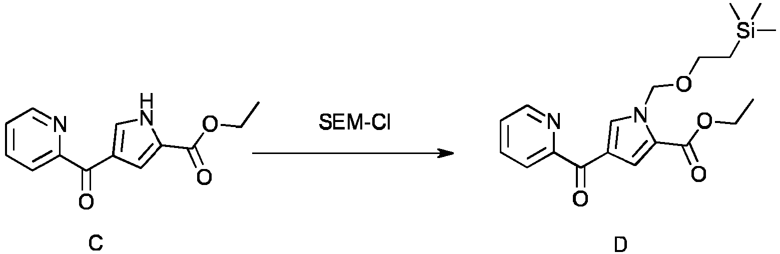

K₂CO₃ (226.3 mg, 1.6377 mmol) followed by 2-(trimethylsilyl)ethoxymethyl chloride (SEM-CI) (0.13 ml, 0.786 mmol) were added to a stirred solution of compound C (160 mg, 0.655 mmol) in DMF (3.2 mL) at 25-30°C. The resulting reaction mixture was stirred at 25-30°C over a period of 18 h. The progress of the reaction was monitored by TLC.

After completion of the reaction, the mixture was diluted with water (10 mL) and the product was extracted with ethyl acetate (3 ×10 mL). The organic layer was dried over anhydrous sodium sulphate and concentrated in vacuo at 40°C to afford a crude product. The crude product was purified by CombiFlash^{®} using ethyl acetate/hexane as eluent to afford the product D as a clear thick oil.
Yield: 50 mg, (20.4 %)
¹H-NMR-CDCl₃ (400 MHz): δ: - 0.08 (9H, s), 0.94 (2H, t, *J*=8.4 Hz), 1.38 (3H, t, *J*=6.8 Hz), 3.60 (2H, t, *J*=8.0 Hz), 4.34 (2H, q, *J=*7.2 Hz), 5.77 (2H, s), 7.46 - 7.49 (1H, m), 7.86 (1H, d, *J*=1.6 Hz), 7.88 - 7.90 (1H, m), 8.13 - 8.15 (1H, m), 8.72 (1H, d, *J*=1.6 Hz), 8.73 (1H, d, *J*=2.0 Hz);
LCMS: 375.3 [M+H] ⁺

### Step 4: Synthesis of ethyl 4-[(tert-butylsulfinylimino)(2-pyridyl)methyl]-1-{[2-(trimethylsilyl)-ethoxy]methyl}-2-pyrrolecarboxylate (E)

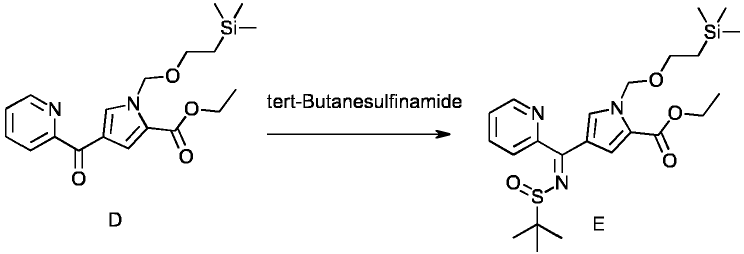

*tert*-Butanesulfinamide (126.2 mg, 1.041 mmol) and Ti(OEt)₄ (0.36 mL, 1.735 mmol) were added to a stirred solution of compound D (130 mg, 0.347 mmol) in dry THF (1.95 mL) at 25-30°C. The resulting reaction mixture was heated at 80°C over a period of 18 h. The progress of the reaction was monitored by TLC.

After completion of the reaction, the reaction mixture was quenched with ice-cold water (5 mL) and the product was extracted to ethyl acetate (3 × 5 mL). The organic layer was dried over anhydrous sodium sulphate and concentrated in vacuo at 40°C to afford a crude product. The crude product was purified by CombiFlash^{®} using ethyl acetate/hexane as eluent to afford compound E as a yellow oil.
Yield: 110 mg, (66.3 %)
¹H-NMR-DMSO-*d*₆ (400 MHz): δ: - 0.09 (9H, s), 0.92 (2H, t, *J*=8.2 Hz), 1.15 (9H, s), 1.25 (3H, t, *J*=6.8 Hz), 3.44 (2H, t, *J*=7.6 Hz), 4.23 (2H, q, *J=7.2* Hz), 5.62 (2H, s), 7.06 (1H, bs), 7.51 - 7.56 (3H, m), 7.93 (1H, t, *J*=7.6 Hz), 8.66 (1H, d, *J=* 4.4 Hz);
LCMS: 478.2 [M+H]⁺

### Step 5: Synthesis of ethyl 4-[(tert-butylsulfinylamino)(2-pyridyl)methyl]-1-{[2-(trimethylsilyl)-ethoxy]methyl}-2-pyrrolecarboxylate

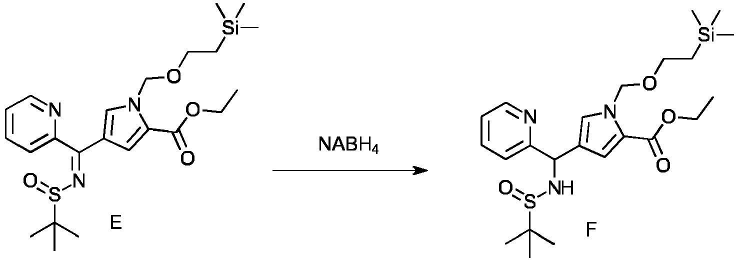

NaBH₄ (435 mg, 1.151 mmol) was added to a stirred solution of compound E (110 mg, 0.230 mmol) in dry THF (2.2 mL) at -78°C. The resulting reaction mixture was stirred at -40°C to -60°C over a period of 5 h and at 25-30°C for 6 h. The progress of the reaction was monitored by TLC.

After completion of the reaction, the reaction mixture was quenched with ice-cold water (5 mL) and the product was extracted to ethyl acetate (3 × 5 mL). The organic layer was dried over anhydrous sodium sulphate and concentrated in vacuo at 40°C to afford the crude compound F as pale yellow oil. The crude material was used as such for the next reaction.
Yield: 100 mg, (crude)
¹H-NMR-DMSO-*d*₆ (300 MHz): δ: -0.10 (9H, s), 0.75 (2H, t, *J*=10.4 Hz), 1.06 - 1.26 (12H, m), 3.37 (2H, t, *J*=2.8 Hz), 4.16 (2H, q, *J*=3.6 Hz), 5.38 - 5.42 (1H, s), 5.54 (2H, s), 5.89 - 5.92 (1H, s), 6.76 - 6.78 (1H, m), 7.15 (1H, d, *J*=2.8 Hz), 7.26 - 7.31 (1H, m) , 7.49 - 7.55 (1H, m) , 7.77 - 7.71 (1H, m), 8.48 - 8.51 (1H, m);
LCMS: 480.3 [M+H]⁺

### Step 6: Synthesis of 4-[(tert-butylsulfinylamino)(2-pyridyl)methyl]-1-{[2-(trimethylsilyl)ethoxy]-methyl}-2-pyrrolecarboxylic acid

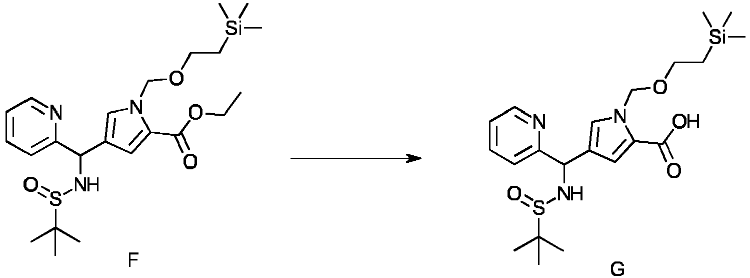

Lithium hydroxide monohydrate (41.54 mg, 0.990 mmol) was added to a stirred solution of compound F (95 mg, 0.198 mmol) in a mixture of THF/methanol/water (2.85 mL, 0.95 ml each) at room temperature (20-25°C). The resulting reaction mixture was stirred over a period of 18 h at 25-30°C. The progress of the reaction was monitored by TLC.

After completion of the reaction, the reaction mixture was evaporated to dryness. The obtained residue was dissolved in purified water (5 mL) and washed with MTBE (2 × 10 mL). The aqueous layer was acidified to pH ~ 5-6 using 0.5 N HCI solution and the product was extracted with ethyl acetate (3 × 15 mL). The combined organic layer was dried over anhydrous sodium sulphate and concentrated at 40 °C to afford a crude solid. The crude solid was washed with hexane (5 mL) and diethyl ether (10 mL) to afford crude compound G as an off white solid. The crude compound was used as such for next reaction.
Yield: 54 mg, (crude)
¹H-NMR-DMSO-*d*₆ (400 MHz): δ : -0.10 (9H, s), 0.75 (2H, t, *J*=7.6 Hz), 1.06 - 1.13 (9H, m), 3.39 (2H, t, *J*=5.6 Hz), 5.42 - 5.44 (1H, s), 5.54 (2H, s), 5.89 - 5.91 (1H, s), 6.72 - 6.79 (1H, m), 7.04 - 7.08 (1H, m), 7.25 - 7.28 (1H, m) , 7.45 - 7.49 (1H, m) , 7.52 - 7.61 (1H, m), 7.76 - 7.79 (1H, m), 8.46 - 8.49 (1H, m);
LCMS: 452.3 [M+H] ⁺

### Step 7: Synthesis of (E)-2-{4-[amino(2-pyridyl)methyl]-2-pyrrolylcarbonylamino}-5,5-dimethyl-3-hexenoic acid (I) (SI13)

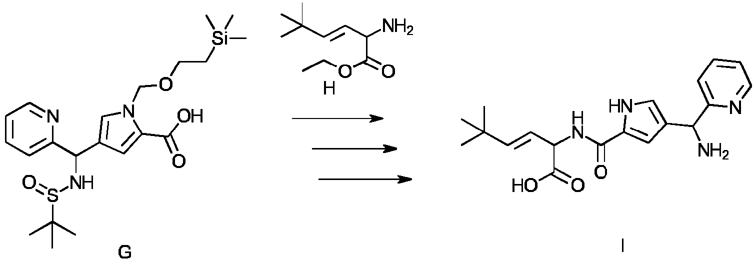

The final step in the synthesis of compound I was carried in the same way as described above for Example 7, steps 7 and 8, starting with the coupling of ethyl (*E*)-2-amino-5,5-dimethyl-3-hexenoate (H) with the prepared compound G, followed by subsequent hydrolysis of the resulting ethyl ester and removal of the two remaining protecting group. The final product was isolated as a mixture of diastereomers.

### EXAMPLE 11 - Synthesis of (E)-5,5-dimethyl-2-[1-(2-oxo-4-pyridyl)-4-pyrazolylcarbonylamino]-3-hexenoic acid (SI16)

### Step 1: Synthesis of 1-(2-oxo-4-pyridyl)-4-pyrazolecarboxylic acid (C)

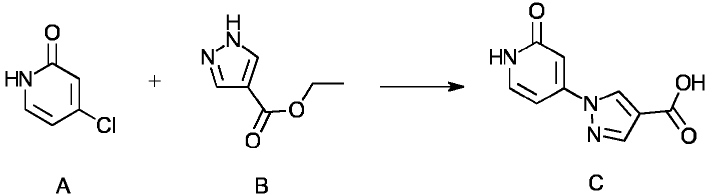

4-Chloro-1*H*-pyridin-2-one (A) (200 mg, 1.54 mmol), ethyl 1*H*-pyrazole-4-carboxylate (B) (325 mg, 2.32 mmol) and cesium carbonate (760 mg, 2.33 mmol) were mixed in dry N-methyl-2-pyrrolidone (0.5 ml). The mixture was heated at 120°C for 16 h.

After the reaction was complete, ethyl acetate (3 mL) was added. The mixture was shaken and then centrifuged. The supernatant was removed and more ethyl acetate (3 mL) was added and the mixture was again shaken, centrifuged and the supernatant was removed. Water and NaOH (0.5 mL) were added and the mixture was heated at 50°C overnight. The mixture was then centrifuged and the supernatant was removed. Water was added, and the mixture was again shaken and centrifuged. The supernatant was removed. The solid material obtained was dried under vacuum at 45°C to give a beige solid, 439 mg. Two compounds were detected in a 1:1 ratio according to LC-MS and NMR and assigned to a mixture of compound C and its corresponding ethyl ester.

The crude mixture (50 mg) was dissolved in methanol (0.5 mL) and aqueous (aq) NaOH (5 M, 0.5 mL) was added and the mixture was heated at 60°C for 2 h. The resulting mixtures was acidified to pH 4-5 by addition of aq HCl (5 M). The mixture was centrifuged and the supernatant was removed. Water and acetonitrile were added and the solvents were evaporated. The crude solid compound C formed was used directly in the next step.

### Step 2: Synthesis of (E)-5,5-dimethyl-2-[1-(2-oxo-4-pyridyl)-4-pyrazolylcarbonylamino]-3-hexenoic acid (E) (SI16)

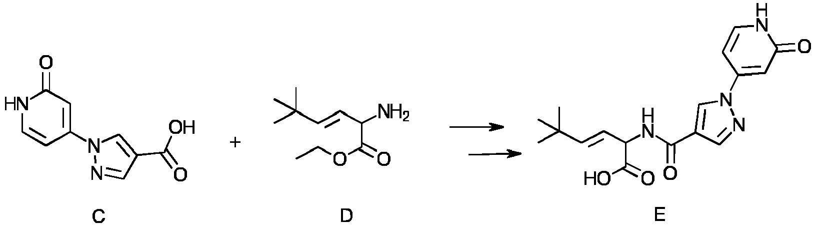

The final step in the synthesis of compound E was carried in the same way as described above for Example 5, starting with the coupling of ethyl (*E*)-2-amino-5,5-dimethyl-3-hexenoate (D) with the prepared compound C, followed by subsequent hydrolysis of the resulting ethyl ester giving the expected product.

### EXAMPLE 12 - Synthesis of (E)-2-[2-(dimethylamino)-5-pyrimidinylcarbonylamino]-5,5-dimethyl-3-hexenoic acid (SI17)

### Step 1: Synthesis of ethyl (E)-2-[2-(dimethylamino)-5-pyrimidinylcarbonylamino]-5,5-dimethyl-3-hexenoate (C)

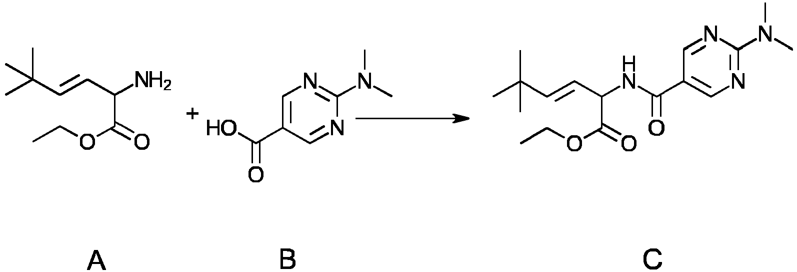

2-(Dimethylamino)-5-pyrimidinecarboxylic acid (B) (90.10 mg, 0.53 mmol) was added to a stirred solution of (*E*)-2-amino-5,5-dimethyl-3-hecenoate (A) (100 mg, 0.53 mmol) in DMF (2.5 mL) followed by DIPEA (0.376 mL, 2.15 mmol) and propylphosphonic anhydride (T₃P, ~50% solution in ethyl acetate) (1.028 mL, 1.61 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted to ethyl acetate (2 × 20 mL). The combined organic layer was washed with saturated sodium chloride solution (15 mL), dried over anhydrous sodium sulphate and concentrated in vacuo at 35°C to afford crude product. The crude material was purified by RP-HPLC to afford pure product C as an off white solid.
Yield: 25 mg, 13.8 %
LCMS: 335.26 [M+H]⁺

### Step 2: Synthesis of (E)-2-[2-(dimethylamino)-5-pyrimidinylcarbonylamino]-5,5-dimethyl-3-hexenoic acid (D)

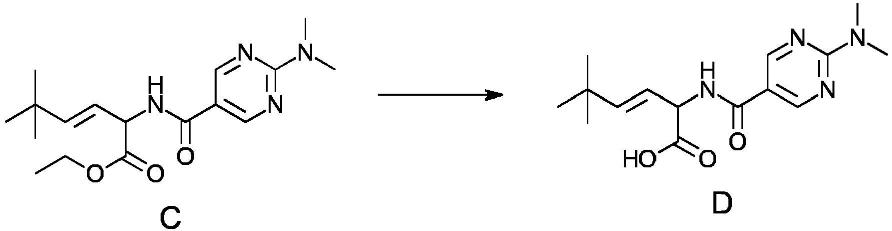

Lithium hydroxide monohydrate (15.69 mg, 0.37 mmol) was added to a stirred solution of compound C (25 mg, 0.07 mmol) in THF/methanol/water (0.75 mL, 1:1:1, 0.25 mL each). The resulting reaction mixture was stirred over a period of 18 h at 25-30°C. The progress of the reaction was monitored by TLC.

After 18 h, the reaction mixture was concentrated, the residue was dissolved in water and washed with diethyl ether (10 mL). The aqueous layer was acidified to pH ~ 5-6 using 1.5 N HCI and the product was extracted with ethyl acetate (3 × 5 mL). The combined organic layer was dried over sodium sulphate and concentrated at 40°C to afford a crude solid. The crude material was washed with hexane (5 mL) to afford pure compound D as white solid.
Yield: 16.4 mg
¹H-NMR- DMSO-*d*₆ (400 MHz): δ: 0.98 (9H, s), 3.17 (6H, s), 4.78-4.81 (1H, m), 5.49-5.54 (1H, m), 5.76 (1H, d, *J=*16.4 Hz), 8.50 (1H, bs) 8.78 (2H, s);
LCMS: 307.3 [M+H] ⁺; HPLC purity- 94.39 %

### EXAMPLE 13 - Synthesis of (E)-2-[2-(dimethylamino)-5-pyrimidinylcarbonylamino]-5,5-dimethyl-3-hexenoic acid (SI49)

### Step 1: Synthesis of ethyl (E)-2-(2-isopropyl-4-methyl-5-pyrimidinylcarbonylamino)-5,5-dimethyl-3-hexenoate (C)

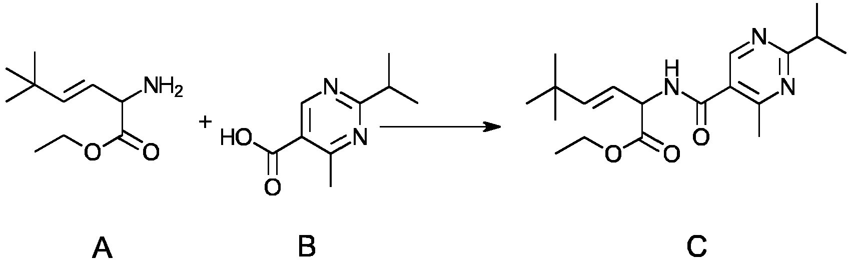

2-Isopropyl-4-methyl-5-pyrimidinecarboxylic acid (B) (144.16 mg, 0.80 mmol) was added to a stirred solution of (*E*)-2-amino-5,5-dimethyl-3-hecenoate (A) (150 mg, 0.80 mmol) in DMF (3.75 mL) followed by DIPEA (0.55 mL, 3.2 mmol) and propylphosphonic anhydride (T3P, ~50% olution in ethyl acetate) (1.52 mL, 2.4 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted to ethyl acetate (2 × 20 mL). The combined organic layer was washed with saturated sodium chloride solution (20 mL), dried over anhydrous sodium sulphate and concentrated in vacuo at 35°C to afford crude product. The crude material was purified by CombiFlash^{®} using ethyl acetate: hexanes as eluent to afford pure product C as a yellow, thick oil.
Yield: 66 mg (35.9 %)
¹H-NMR-CD3OD (400 MHz): δ: 1.12 (9H, s), 1.39-1.46 (9H, m), 2.73 (3H, s), 3.28-3.32 (1H, m), 4.35-4.38 (2H, m), 5.14 (1H, d, *J*=7.5 Hz), 5.62-5.70 (1H, m), 6.07 (1H, d, *J*=15.3 Hz), 6.94-7.26 (1H, m), 8.76 (1H, s);
LCMS: 348.3 [M+H]⁺

### Step 2: Synthesis of (E)-2-(2-isopropyl-4-methyl-5-pyrimidinylcarbonylamino)-5,5-dimethyl-3-hexenoic acid (D)

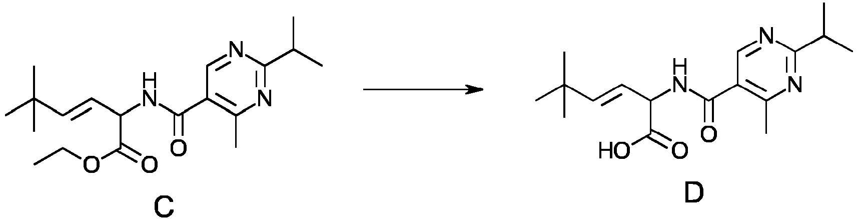

Lithium hydroxide monohydrate (39.9 mg, 1.04 mmol) was added to a stirred solution of compound C (66 mg, 0.19 mmol) in THF/methanol/water (2.4 mL, 1:1:1, 0.8 mL each). The resulting reaction mixture was stirred over a period of 18 h at 25-30°C. The progress of the reaction was monitored by TLC.

After 18 h, the reaction mixture was concentrated, the residue was dissolved in water and washed with diethyl ether (10 mL), the aqueous layer was acidified to pH ~ 5-6 using 1.5 N HCI and the product was extracted with ethyl acetate (3 × 5 mL). The combined organic layer was dried over sodium sulphate and concentrated at 40°C to afford a crude solid. The crude material was washed with hexane (5 mL) to afford a crude mixture of two isomers as an off white solid. The crude mixture was purified by RP-HPLC to afford pure product D as a white solid.
Yield: 12 mg
¹H-NMR- DMSO-*d*₆ (400 MHz): δ: 0.98 (9H, s), 1.24 (6H, d), 2.48 (3H, s), 3.05-3.12 (1H, m), 4.81-4.84 (1H, m), 5.42-5.48 (1H, m), 5.81-5.85 (1H, m), 8.59 (1H, s), 8.94 (1H, d, *J*=7.2 Hz), 12.74 (1H, bs); LCMS: 320.3 [M+H]⁺; HPLC purity: 94.9 %.

### EXAMPLE 14 - Synthesis of (E)-5,5-dimethyl-2-[p-(4H-1,2,4-triazol-4-yl)benzoylaminol-3-hexenoic acid (SI23)

### Step 1: Synthesis of ethyl (E)-5,5-dimethyl-2-[p-(4H-1,2,4-triazol-4-yl)benzoylamino]-3-hexenoate (C)

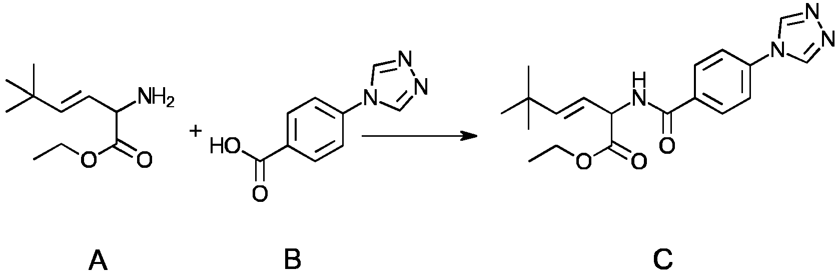

*p*-(4*H*-1,2,4-Triazol-4-yl)benzoic acid (B) (50.88 mg, 0.26 mmol) was added to a stirred solution of (*E*)-2-amino-5,5-dimethyl-3-hecenoate (A) (50 mg, 0.26 mmol) in DMF (1.25 mL) followed by DIPEA (0.18 mL, 1.07 mmol) and propylphosphonic anhydride (T₃P, ~50% solution in ethyl acetate) (0.51 mL, 0.807mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted to ethyl acetate (2 × 20 mL). the combined organic layer was washed with saturated sodium chloride solution (10 mL), dried over anhydrous sodium sulphate and concentrated in vacuo at 35°C to afford crude product. The crude was purified by CombiFlash^{®} using ethyl acetate: hexanes as eluent to afford pure compound C as thick yellow liquid.
Yield: 50 mg (52,0%)
¹H-NMR- CD₃OD (300 MHz): δ: 0.98 (9H, s), 1.30-1.34 (3H, m), 4.19-4.25 (2H, m), 5.07 -4.89 (1H, m), 5.58-5.65 (1H, m), 5.92-5.97 (1H, m), 7.80 (2H, d, *J*=8.7 Hz), 8.09 (2H, d, *J*=9 Hz), 9.13 (2H, s); LCMS: 357.4 [M+H]⁺

### Step 2: Synthesis of (E)-5,5-dimethyl-2-[p-(4H-1,2,4-triazol-4-yl)benzoylamino]-3-hexenoic acid (D)

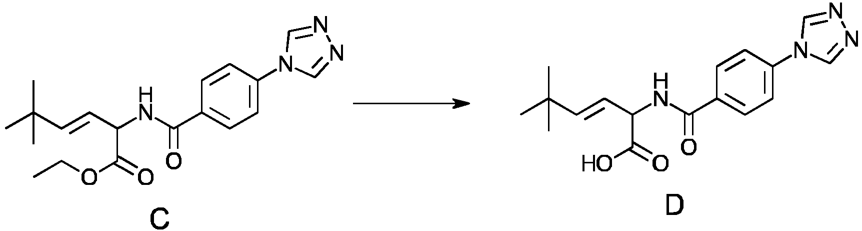

Lithium hydroxide monohydrate (29.46 mg, 0.70 mmol) was added to a stirred solution of compound C (50 mg, 0.14 mmol) in THF/methanol/water (1.5 mL, 1:1:1, 0.5 mL each). The resulting reaction mixture was stirred over a period of 18 h at 25-30°C. The progress of the reaction was monitored by TLC.

After 18 h, the reaction mixture was concentrated, the residue was dissolved in water and washed with diethyl ether (10 mL). The aqueous layer was acidified to pH ~ 5-6 using 1.5 N HCI and the product was extracted with ethyl acetate (3 × 5 mL). The combined organic layer was dried over sodium sulphate and concentrated at 40°C to afford a crude solid. The crude material was washed with hexane (5 mL) to afford a mixture of two isomers as an off white solid. The crude material was purified by RP-HPLC to afford the pure compound D as a white solid.
Yield: 4.4 mg;
¹H-NMR- DMSO-*d*₆ (400 MHz): δ: 0.9 (9H, s), 4.89-4.90 (1H, t, J=6.4 Hz), 5.57-5.59 (1H, m), 5.80-5.84 (1H, m), 7.85 - 7.86 (2H, dd, *J*₁=2 Hz, *J*₂=7.2 Hz), 8.07-8.08 (1H, dd, *J*₁=1.6 Hz, *J*₂=6.8 Hz), 8.89-8.91(1H, d), 9.23 (2H, s), 12.63 (1H, bs),
LCMS: 329.4 [M+H] ⁺; HPLC purity 99.4 %

### EXAMPLE 15 - Synthesis of (E)-5,5-dimethyl-2-[m-(1H-1,2 4-triazol-1-yl)benzoylamino]-3-hexenoic acid (S124)

### Step 1: Synthesis of ethyl (E)-5,5-dimethyl-2-[m-(1H-1,2,4-triazol-1-yl)benzoylamino]-3-hexenoate (C)

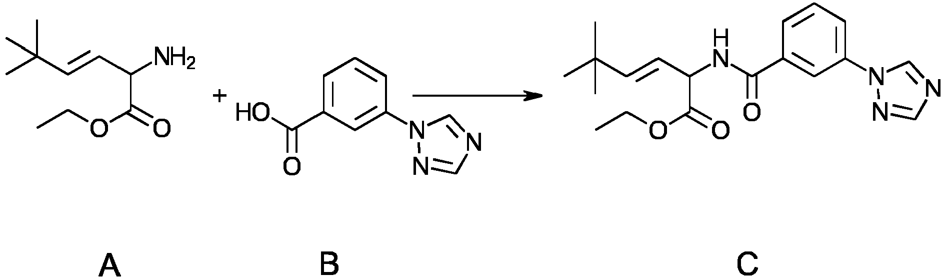

*m*-(1*H*-1,2,4-Triazol-1-yl)benzoic acid (B) (101.96 mg, 0.53 mmol) was added to a stirred solution of (*E*)-2-amino-5,5-dimethyl-3-hecenoate (A) (100 mg, 0.53 mmol) in DMF (2.5 mL) followed by DIPEA (0.37 mL, 2.15 mmol) and propylphosphonic anhydride (T₃P, ~50% solution in ethyl acetate) (1.02 mL, 1.61 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted to ethyl acetate (2 × 20 mL). The combined organic layer was washed with saturated sodium chloride solution (15 mL), dried over anhydrous sodium sulphate and concentrated in vacuo at 35°C to afford crude product. The crude material was purified by CombiFlash^{®} using ethyl acetate: hexanes as eluent to afford compound C as a thick yellow liquid.
Yield: 64 mg, (33.3 %)
¹H-NMR- CD₃OD (400 MHz): δ: 1.07 (9H, s), 1.24-1.30 (3H, m), 4.21- 4.24 (2H,m), 5.05 - 5.07 (1H, d, *J*=7.6 Hz), 5.60 - 5.64 (1H, m), 5.93-5.97 (1H, m), 7.66-7.70 (1H, m), 7.94 (1H, d, *J*=7.6Hz), 8.03-8.05 (1H, m), 8.21 (1H, s), 8.34 (1H, s), 9.17 (1H, s);
LCMS : 357.6 [M+H]⁺

### Step 2: Synthesis of (E)-5,5-dimethyl-2-[m-(1H-1,2,4-triazol-1-yl)benzoylamino]-3-hexenoic acid (D)

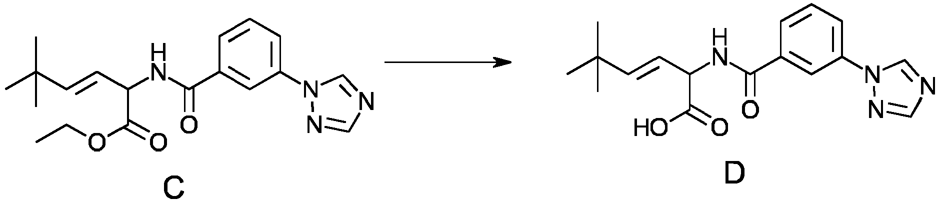

Lithium hydroxide monohydrate (37.69 mg, 0.8975 mmol) was added to a stirred solution of compound C) (64 mg, 0.1795 mmol) in THF/methanol/water (1.92 mL, 1:1:1, 0.64 mL each). The resulting reaction mixture was stirred over a period of 18 h at 25-30°C. The progress of the reaction was monitored by TLC.

After 18 h, the reaction mixture was concentrated, the residue was dissolved in water and washed with diethyl ether (10 mL). The aqueous layer was acidified to pH ~ 5-6 using 1.5 N HCI and the product was extracted with ethyl acetate (3 × 5 mL). The combined organic layer was dried over sodium sulphate and concentrated at 40°C to afford a crude solid. The crude material was washed with hexane (5 mL) to afford a mixture of two isomer as an off white solid. The mixture was purified by RP-HPLC to afford compound D as a white solid.
Yield: 29 mg
¹H-NMR- DMSO-*d*₆ (400 MHz): δ: 1.0 (9H, s), 4.90 - 4.94 (1H, m), 5.54-5.60 (1H, m), 5.83-5.87 (1H, m), 7.67(1H, t, *J*=8 Hz), 7.94 (1H, d, *J*=8Hz), 8.02 - 8.05 (1H, m), 8.28 (1H, s), 8.34 (1H, t, *J*=1.6Hz), 9.00 (1H, d, *J=* 7.2 Hz), 9.36 (1 H, s), 12.60 (1H, bs);
LCMS: 329.3 [M+H] ⁺; HPLC purity - 99.6 %

### EXAMPLE 16 - Synthesis of (E)-2-[m-(1-imidazolyl)benzoylamino]-5,5-dimethyl-3-hexenoic acid (SI25)

### Step 1: Synthesis of ethyl (E)-2-[m-(1-imidazolyl)benzoylamino]-5,5-dimethyl-3-hexenoate (C)

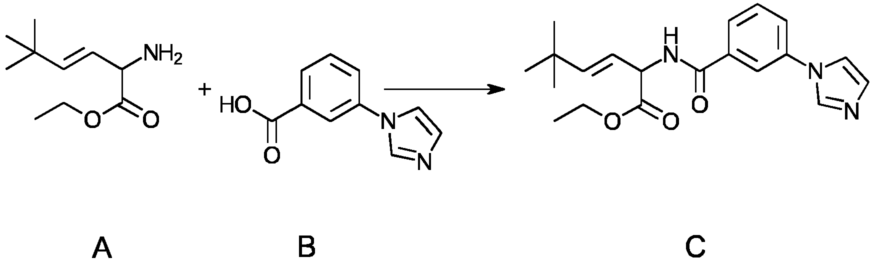

*m*-(1-Imidazolyl)benzoic acid (B) (101.5 mg, 0.53 mmol) was added to a stirred solution of (E)-2-amino-5,5-dimethyl-3-hecenoate (A) (100 mg, 0.53 mmol) in DMF (2.5 mL) followed by DIPEA (0.37 mL, 2.15 mmol) and propylphosphonic anhydride (T3P, ~50% solution in ethyl acetate) (1.03 mL, 1.61 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted with ethyl acetate (2 × 20 mL). The combined organic layer was washed with saturated sodium chloride solution (20 mL), dried over anhydrous sodium sulphate and concentrated in vacuo at 35°C to afford a crude product. The crude material was purified by CombiFlash^{®} using ethyl acetate: hexanes as eluent to afford pure compound C as a brown, thick oil.
Yield: 65 mg, (33.9 %)
¹H-NMR-CD₃OD (400 MHz): δ: 1.04 (9H, s), 1.24 (3H, t, *J*=7.2 Hz), 4.18 - 4.21 (2H, m), 5.02 - 5.04 (1H, m), 5.69 - 5.61 (1H, m), 5.90 - 5.94 (1H, m), 7.16 (1H, s), 7.62 - 7.64 (2H, m), 7.77-7.79 (1H, m), 7.87 - 7.89 (1H, m), 8.05 (1H, s), 8.21 (1H, s);
LCMS: 356.3 [M+H]⁺

### Step 2: Synthesis of (E)-2-[m-(1-imidazolyl)benzoylamino]-5,5-dimethyl-3-hexenoic acid (D)

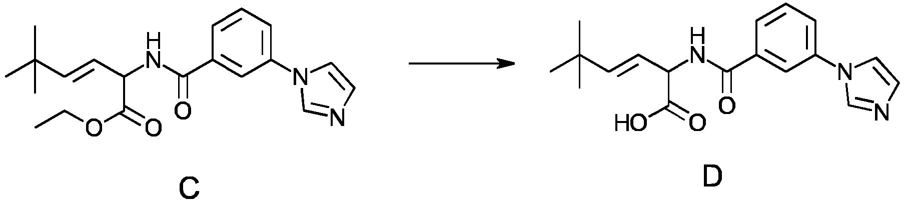

Lithium hydroxide monohydrate (32.5 mg,0.77 mmol) was added to a stirred solution of compound C (61 mg, 0.17 mmol) in THF/methanol/water (1.83 mL, 1:1:1, 0.61 mL each) was added. The resulting reaction mixture was stirred over a period of 18 h at 25-30°C. The progress of the reaction was monitored by TLC.

After 18 h, the reaction mixture was concentrated, the residue was dissolved in water and washed with diethyl ether (10 mL), The aqueous layer was acidified to pH ~ 5-6 using 1.5 N HCI and the product was extracted with ethyl acetate (3 × 5 mL). The combined organic layer was dried over sodium sulphate and concentrated at 40°C to afford a crude solid. The obtained crude solid was washed with hexane (5 mL) to afford a crude mixture of two isomers as a white solid. The crude mixture was purified by RP-HPLC to afford the pure product D as a white solid.
Yield: 20 mg
¹H-NMR-DMSO-*d*₆ (400 MHz): δ: 1.22 (9H, s), 4.93-4.97 (1H, m), 5.52 - 5.60 (1H, m), 5.82 - 5.89(1H, m), 7.23 (1H, s), 7.61 - 7.67 (1H, m), 7.83-7.91 (3H, m), 8.13 (1H, s), 8.47 (1H, s), 8.94 (1H, d, *J*=9.2 Hz), 12.63 (1H, bs);
LCMS: 328.3 [M+H] ⁺; HPLC purity- 99.6 %

### EXAMPLE 17 - Synthesis of (E)-5,5-dimethyl-2-[p-(2-thienyl)benzoylamino]-3-hexenoic acid (SI51)

### Step 1: Synthesis of ethyl (E)-5,5-dimethyl-2-[p-(2-thienyl)benzoylamino]-3-hexenoate (C)

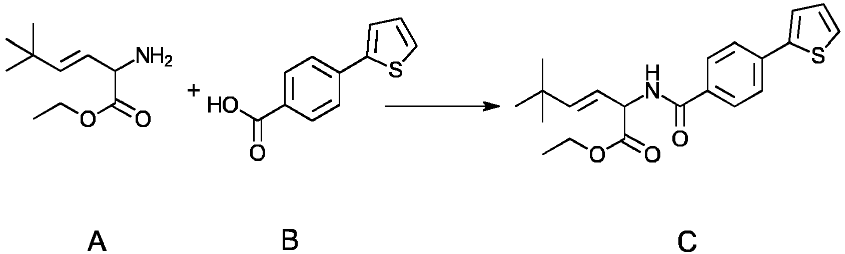

*p*-(2-Thienyl)benzoic acid (B) (108.1 mg, 0.53 mmol) was added to a stirred solution of (*E*)-2-amino-5,5-dimethyl-3-hecenoate (A) (100 mg, 0.53 mmol) in DMF (2.5 mL) followed by DIPEA (0.37 mL, 2.15 mmol) and propylphosphonic anhydride (T₃P, ~50% solution in ethyl acetate) (1.03 mL, 1.61 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted with ethyl acetate (2 × 20 mL). The combined organic layer was washed with saturated sodium chloride solution, dried over anhydrous sodium sulphate and concentrated in vacuo at 35°C to afford crude product. The crude material was purified by CombiFlash^{®} using ethyl acetate: hexanes as eluent to afford compound C as a pale yellow thick oil.
Yield: 117 mg (59.4 %)
¹H-NMR- CD₃OD (400 MHz): δ: 1.04 (9H, s), 1.24 (3H, t, *J*=7.2 Hz), 4.19 (2H, d, *J*=5.6 Hz), 5.01 - 5.03 (1H, m), 5.48 - 5.53 (1H, m), 5.88 - 5.92 (1H, m), 7.10 - 7.14 (1H, m), 7.42 - 7.50 (2H, m), 7.71 - 7.73 (2H, m), 7.85 - 7.88 (2H, m);
LCMS: 372.10 [M+H] ⁺

### Step 2: Synthesis of (E)-5,5-dimethyl-2-[p-(2-thienyl)benzoylamino]-3-hexenoic acid (D)

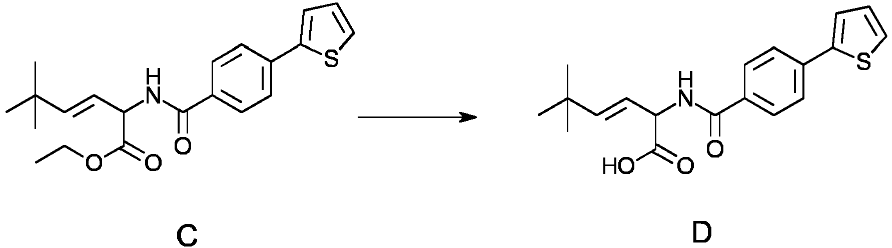

Lithium hydroxide monohydrate (31.6 mg, 0.75 mmol) was added to a stirred solution of compound C (112 mg, 0.30 mmol) in MeOH: THF: water (3.3 mL, 1:1:1, 1.1 mL each). The resulting reaction mixture was stirred over a period of 7 h at 25-30°C. The progress of the reaction was monitored by TLC.

After 18 h, the reaction mixture was concentrated, the residue was dissolved in water and washed with diethyl ether (10 mL). The aqueous layer was acidified to pH ~ 4-5 using saturated solution of potassium hydrogen sulphate and the product was extracted with ethyl acetate (3 × 5 mL). The combined organic layer was dried over sodium sulphate and concentrated at 40°C to afford a crude solid. The obtained solid was washed with hexane (5 mL) to afford a mixture of two isomers as a white solid. The crude product was purified by RP-HPLC to afford pure compound D as a white solid.
Yield: 48 mg;
¹H-NMR- DMSO-*d*₆ (400 MHz): δ: 1.00 (9H, s), 4.87 - 4.91 (1H, m), 5.53 - 5.61 (1H, m), 5.84 (1H, d, *J*=20.8 Hz), 7.16 - 7.19 (1H, m), 7.62 - 7.65 (2H, m), 7.74 - 7.77 (2H, m), 7.93 - 7.96 (2H, m), 8.81 (1H, d, *J*=9.6 Hz), 12.62 (1H, bs);
LCMS: 344.4 [M+H] ⁺; HPLC purity- 99.1 %.

### EXAMPLE 18 - Synthesis of (E)-5,5-dimethyl-2-[m-(2-thienyl)benzoylamino]-3-hexenoic acid (SIS2)

### Step 1: Synthesis of ethyl (E)-5,5-dimethyl-2-[m-(2-thienyl)benzoylamino]-3-hexenoate (C)

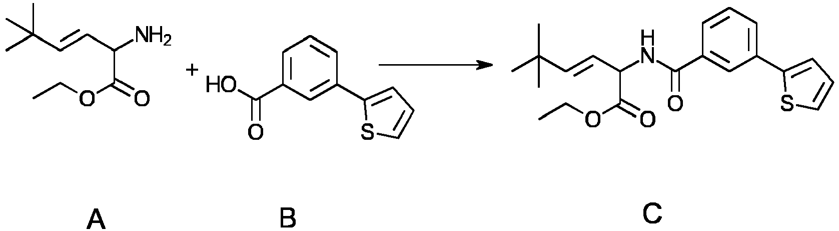

*m*-(2-Thienyl)benzoic acid (B) (109.96 mg, 0.53 mmol) was added to a stirred solution of (*E*)-2-amino-5,5-dimethyl-3-hecenoate (A) (100 mg, 0.53 mmol) in DMF (2.5 mL) followed by DIPEA (0.37 mL, 2.15 mmol) and propylphosphonic anhydride (T₃P, ~50% solution in ethyl acetate) (1.02 mL, 1.61 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted to ethyl acetate (2 × 20 mL). The combined organic layer was washed with saturated sodium chloride solution (15 mL), dried over anhydrous sodium sulphate and concentrated in vacuum at 35°C to afford the crude product, The crude material was purified by CombiFlash^{®} using ethyl acetate: hexanes as eluent to afford compound C as a thick yellow liquid.
Yield: 100 mg, (49.8 %)
¹H-NMR- CD₃OD (400 MHz): δ: 1.04 (9H, s), 1.25 (3H, t, J=6.8 Hz), 4.18 - 4.21 (2H, m), 5.03 (1H, m), 5.57- 5.62 (1H, m), 5.89 - 5.94 (1H, m), 7.09 - 7.11 (1H, m), 7.39 - 7.41 (1H, m), 7.45 - 7.49 (2H, m), 7.73 - 7.79 (2H, m), 8.11 - 8.12 (1H, m);
LCMS: 372.08 [M+H]⁺

### Step 2: Synthesis of (E)-5,5-dimethyl-2-[m-(2-thienyl)benzoylamino]-3-hexenoic acid (D)

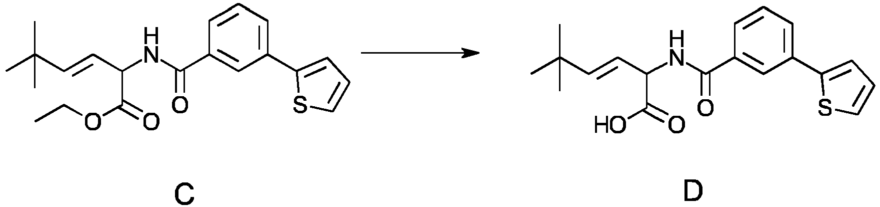

Lithium hydroxide monohydrate (56.51 mg, 1.34 mmol) was added to a stirred solution of compound C (100 mg, 0.26 mmol) in THF/methanol/water (3.0 mL, 1:1:1, 1.0 mL each). The resulting reaction mixture was stirred over a period of 18 h at 25-30°C. The progress of the reaction was monitored by TLC.

After 18 h, the reaction mixture was concentrated, the residue was dissolved in water and washed with diethyl ether (10 mL). The aqueous layer was acidified to pH ~ 5-6 using 1.5 N HCI and the product was extracted with ethyl acetate (3 × 5 mL). The combined organic layer was dried over sodium sulphate and concentrated at 40°C to afford crude solid. The obtained solid was washed with hexane (5 mL) to afford a crude mixture of two isomers as an off white solid. The crude mixture was purified by RP-HPLC to afford pure compound D
Yield: 52.8 mg;
¹H-NMR- DMSO-*d*₆ (400 MHz): δ: 1.01 (9H, s), 4.89 - 4.94 (1H, m), 5.54- 5.62 (1H, m), 5.81-5.87 (1H, d, *J*= 15.6 Hz), 7.16-7.19 (1H, m), 7.49- 7.54 (1H, m), 7.60 (2H, d, *J =* 5.7 Hz), 7.83 (2H, d, *J*= 7.5 Hz), 8.14 (1H, s), 8.93 (1H, d, *J*= 6.9 Hz), 12.72 (1H, bs);
LCMS: 344.2 [M+H] ⁺; HPLC purity - 99.8 %.

### EXAMPLE 19 - Synthesis of (E)-2-(4-bromo-2-pyrrolylcarbonylamino)-5,5-dimethyl-3-hexenoic acid (SI28)

### Step 1: Synthesis of 4-bromo-1-{[2-(trimethylsilyl)ethoxy]methyl}-2-pyrrolecarboxylic acid (B)

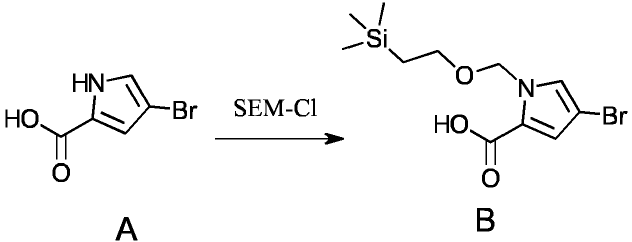

SEM-CI (0.279 mL, 1.57 mmol) was added to a stirred solution of 4-bromo-2-pyrrolecarboxylic acid (A) (250 mg, 1.31 mmol) in DMF (2.5 mL) followed by K₂CO₃ (653.6 mg, 4.71 mmol). The resulting reaction mixture was stirred over a period of 2 h at 25-30°C. The progress of the reaction was monitored by TLC.

After completion of the reaction, the reaction mixture was quenched by addition of water and adjusted to pH ~5-6 with 1.5 N HCI and the product was extracted to ethyl acetate (2 × 40 mL). The combined organic layer was washed with saturated sodium chloride solution (20 mL), dried over anhydrous sodium sulphate and concentrated in vacuo at 35°C to afford crude product. The crude material was purified by CombiFlash^{®} using ethyl acetate: hexanes as eluent to afford compound B as a thick, colorless liquid.
Yield: 239 mg (56.7 %)
¹H-NMR- DMSO-*d*₆ (300 MHz): δ: - 0.01 (9H, s), 0.89 (2H, t, *J*=7.8 Hz), 3.73 (2H, t, *J*=7.8 Hz), 5.37 (2H, s), 6.86 (1H, s), 7.21 (1H, s), 12.37 (1H, bs)

### Step 2: Synthesis of ethyl (E)-2-(4-bromo-1-{[2-(trimethylsilyl)ethoxy]methyl}-2-pyrrolylcarbonylamino)-5,5-dimethyl-3-hexenoate (D)

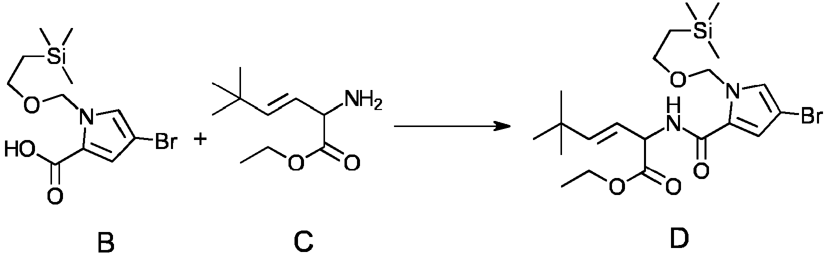

Oxalyl chloride (0.092 mL, 1.12 mmol) was added dropwise along with catalytic amount of DMF (0.12 mL) to a stirred solution of compound B (300 mg, 0.94 mmol) in DCM (6.0 mL) at 0-5°C. The resulting reaction mixture was stirred at 25-30°C over a period of 2 h. The progress of the reaction was monitored by TLC (aliquot was diluted with MeOH and formation of methyl ester was confirmed).

After 2 h, the reaction mixture was concentrated to dryness at 35°C and stripped off with toluene (5 mL × 3). After complete concentration, a yellow, thick oil was obtained. This was further dissolved in DCM (3 mL) and was then added to a stirred solution of (*E*)-2-amino-5,5-dimethyl-3-hecenoate (C) (0.16 g, 0.0009 mol) and DIPEA (0.62 mL, 0.0036 mol) in DCM (3 mL) at 25-30°C. The resulting reaction mixture was stirred over a period of 18 h at 25-30°C. The progress of the reaction was monitored by TLC.

After completion of the reaction, the reaction mixture was quenched by addition of saturated NaHCO₃ solution to pH ~7-8 and the product was extracted to ethyl acetate (2 × 20 mL). The combined organic layer was washed with saturated sodium chloride solution (20 mL), dried over anhydrous sodium sulphate and concentrated in vacuo at 35°C to afford crude material. The crude material was purified by CombiFlash^{®} using ethyl acetate: hexanes as eluent to afford compound D as a thick yellow liquid.
Yield: 109 mg (21.9%);
¹H-NMR- DMSO-*d*₆ (400 MHz): δ: 1.02 (9H, s), 1.14- 1.18 (3H, m), 4.10 - 4.13 (2H, m), 4.83 - 4.88 (1H, m), 5.44 - 5.52 (1H, m), 5.75 - 5.85 (1H, m), 7.02 (2H, s), 8.49 (1H, d, *J*= 6.9 Hz), 11.88 (1H, bs); LCMS: 381.1 [M+Na] ⁺;

### Step 3: Synthesis of (E)-2-(4-bromo-2-pyrrolylcarbonylamino)-5,5-dimethyl-3-hexenoic acid (E)

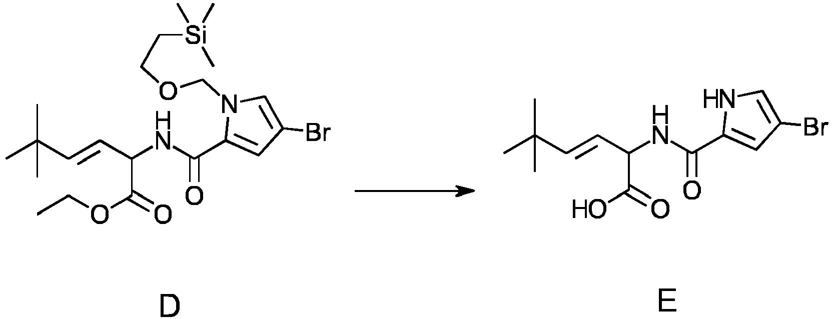

Lithium hydroxide monohydrate (58.8 mg, 1.39 mmol) was added to a stirred solution of compound D (100 mg, 0.27 mmol) in THF/methanol/water (3.0 mL, 1:1:1, 1.0 mL each). The resulting reaction mixture was stirred over a period of 18 h at 25-30°C. The progress of the reaction was monitored by TLC.

After 18 h, the reaction mixture was concentrated, and the residue was dissolved in water and washed with diethyl ether (10 mL). The aqueous layer was acidified to pH ~ 5-6 using 1.5 N HCI and the product was extracted with ethyl acetate (3 × 5 mL). The combined organic layer was dried over sodium sulphate and concentrated at 40°C to afford a crude solid. The obtained solid was washed with hexane (5 mL) to afford a crude mixture of two isomers as an off white solid. The crude material was purified by RP-HPLC to afford pure compound E as a pale, yellow solid.
Yield: 5.2 mg;
¹H-NMR- DMSO-*d*₆ (300 MHz): δ: 0.99 (9H, s), 4.79 (1H, m), 5.48-5.55 (1H, m), 5.71-5.76 (1H, m), 6.99 (2H, s), 8.26 (1H, bs), 11.87 (1H, bs);
LCMS: 353.20 [M+Na]⁺; HPLC purity- 98.5 %

### EXAMPLE 20 - Synthesis of (E)-2-(5-chloro-2-pyrrolylcarbonylamino)-5,5-dimethyl-3-hexenoic acid (SI29)

### Step 1: Synthesis of methyl 5-chloro-2-pyrrolecarboxylate (B)

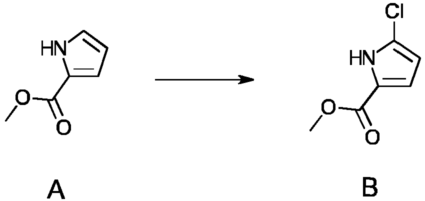

A pre-dissolved solution of t-butyl hypochlorite solution in CCl₄ (40.1 mL) was added dropwise to a stirred solution of methyl 2-pyrrolecarboxylate (A) (1.18 g, 9.4 mmol) in CCl₄ (200.6 mL) at 25-30°C. The resulting mixture was stirred over a period of 24 h at 25-30°C. The progress of the reaction was monitored by TLC.

After 18 h, the reaction mixture was concentrated at 40°C to afford a crude residue. The residue was purified by CombiFlash^{®} using ethyl acetate: hexanes as eluent to afford compound B as a white solid.
Yield: 0.62 g (41.3 %);
¹H-NMR-DMSO-*d*₆ (400 MHz): δ: 3.74 (3H, s), 6.13 - 6.15 (1H, m), 6.76 - 6.78 (1H, m), 12.70 (1H, bs); LCMS: 159.85 [M+H]⁺

### Step 2: Synthesis of methyl 5-chloro-1-{[2-(trimethylsilyl)ethoxy]methyl}-2-pyrrolecarboxylate (C)

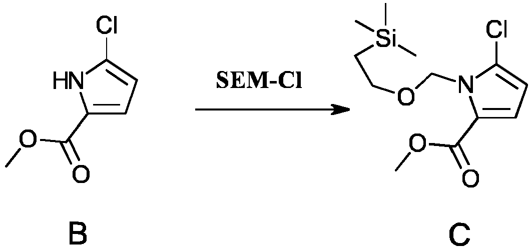

K₂CO₃ (0.46 g, 3.3 mmol) was added to a stirred solution of compound B (0.3 g, 1.8 mmol) in DMF (3 mL) at 25-30°C. After 5 min, SEM-chloride (0.39 mL, 2.2 mmol) was added dropwise at 25-30°C. The resulting mixture was stirred over a period of 18 h at 25-30°C. The progress of the reaction was monitored by TLC.

After completion of the reaction, the reaction mixture was quenched by addition of saturated NaHCO₃ solution to pH ~7-8 and the product was extracted with ethyl acetate (2 × 20 mL). The combined organic layer was washed with saturated sodium chloride solution (20 mL), dried over anhydrous sodium sulphate and concentrated in vacuo at 35°C to afford a crude residue. The residue was purified by CombiFlash^{®} using ethyl acetate: hexanes as eluent to afford compound C as a colorless oil.
Yield: 0.47 g (86.4 %)
¹H-NMR-DMSO-*d*₆ (400 MHz): δ: - 0.09 (9H, s), 0.79 (2H, t, *J*=7.6 Hz), 3.49 (2H, t, *J*=7.6 Hz), 3.74 (3H, s), 5.69 (2H, s), 6.33(1H, d, *J*=4.0 Hz), 6.98 (1H, d, *J*=4.0 Hz);
LCMS: 312.2 [M+Na] ⁺

### Step 3: Synthesis of 5-chloro-1-{[2-(trimethylsilyl)ethoxy]methyl}-2-pyrrolecarboxylic acid (D)

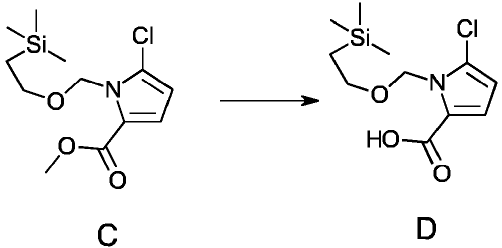

Lithium hydroxide monohydrate (0.16 g, 4.01 mmol) was added to a stirred solution compound C (0.46 g, 1.6 mmol) in MeOH/THF/water (13.8 mL, 1:1:1, 4.6 mL each). The resulting reaction mixture was stirred over a period of 18 h at 25-30°C. The progress of the reaction was monitored by TLC.

After 18 h, the reaction mixture was concentrated, the resulting residue was dissolved in water and washed with diethyl ether (10 mL). The aqueous layer was acidified to pH ~ 4-5 using saturated solution of potassium hydrogen sulphate and the product was extracted with ethyl acetate (3 × 5 mL). The combined organic layer was dried over sodium sulphate and concentrated at 40°C to afford a crude solid. The solid was washed with hexane (5 mL) to afford compound D as a white solid.
Yield: 0.42 g (35.5 %);
¹H-NMR-DMSO-*d*₆ (400 MHz): δ: -0.08 (9H, s), 0.79 (2H, t, *J*=7.6 Hz), 3.49 (2H, t, *J*=7.6 Hz), 5.72 (2H, s), 6.27 (1H, d, *J*=4.0 Hz), 6.90 (1H, d, *J*=4.0 Hz), 12.64 (1H, bs);
LCMS: 298.2 [M+Na] ⁺

### Step 4: Synthesis of ethyl (E)-2-(5-chloro-1-{[2-(trimethylsilyl)ethoxy]methyl}-2-pyrrolylcarbonylamino)-5,5-dimethyl-3-hexenoate (F)

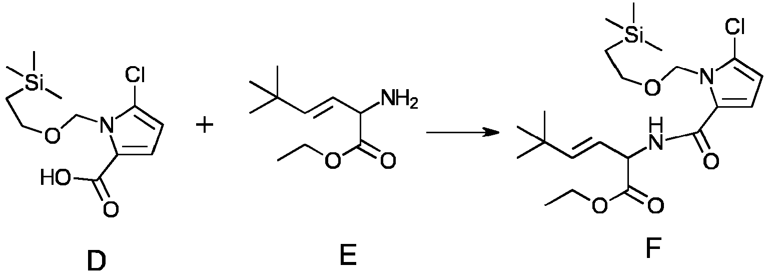

Oxalyl chloride at 0-5°C was added to a stirred solution of compound D (0.2 g, 0.72 mmol) in DMF (4.0 mL) followed by DMF (0.08 mL). The resulting mixture was stirred over a period of 2 h at 25-30°C. The progress of the reaction was monitored by TLC.

After completion of the reaction, the reaction mixture was concentrated and dried at 40°C. The pre-dissolved solution of the resulting residue in DCM (4.0 mL) was added to (E)-2-amino-5,5-dimethyl-3-hecenoate (E) (0.13 g, 0.72 mmol) followed by DIPEA (0.5 mL, 2.8 mmol) at 25-30 °C. The resulting mixture was stirred over a period of 18 h at 25-30°C.

After completion of the reaction, the reaction mixture was quenched by addition of saturated NaHCO₃ solution (20 mL) to pH ~7-8 and the product was extracted with DCM (2 × 20 mL). The combined organic layer was washed with saturated sodium chloride solution (20 mL), dried over anhydrous sodium sulphate and concentrated in vacuo at 35°C to afford a crude material. The crude material was purified by CombiFlash^{®} using ethyl acetate: hexanes as eluent to afford compound F as a pale brown oil.
Yield: 0.228 g (70.9 %);
¹H-NMR-DMSO-*d*₆ (400 MHz): δ: -0.09 (9H, s), 0.76 (2H, t, *J*=9.6 Hz), 0.98 (9H, s), 1.15 (3H, t, *J*=9.6 Hz), 3.44 (2H, t, *J=*10.4 Hz), 4.10 (2H, q, *J*=9.6 Hz), 4.82 (2H, t, *J*=9.6 Hz), 5.45 - 5.53 (1H, m), 5.74-5.83 (3H, m), 6.26 (1H, d, *J*=5.2 Hz), 7.01 (1H, d, *J*=5.6 Hz), 8.61 (1H, d, *J*=9.2 Hz); LCMS: 462.17 [M+Na] ⁺

### Step 5: Synthesis of (E)-2-(5-chloro-1-{[2-(trimethylsilyl)ethoxy]methyl}-2-pyrrolylcarbonylamino)-5,5-dimethyl-3-hexenoic acid (G)

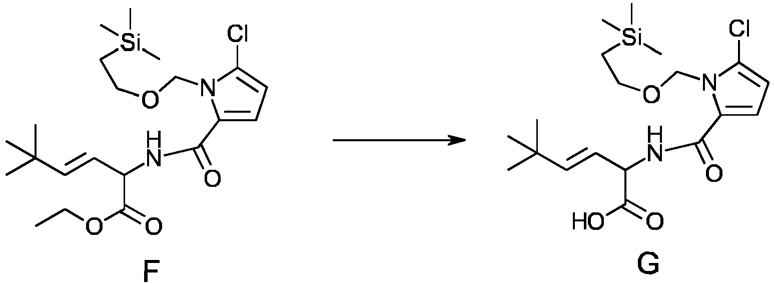

Lithium hydroxide monohydrate (0.052 g, 1.2 mmol) was added to a stirred solution of compound F (0.22 g, 0.49 mmol) in MeOH/THF/water (6.6 mL, 1:1:1, 2.2 mL each) was added. The resulting reaction mixture was stirred over a period of 18 h at 25-30°C. The progress of the reaction was monitored by TLC.

After completion of the reaction, the reaction mixture was concentrated, the resulting residue was dissolved in water and washed with diethyl ether (10 mL). The aqueous layer was acidified to pH ~ 4-5 using saturated solution of potassium hydrogen sulphate and the product was extracted with ethyl acetate (3 × 5 mL). The combined organic layer was dried over sodium sulphate and concentrated at 40°C to afford a crude solid. The resulting solid was washed with hexane (5 mL) to compound G as a white, gummy solid.
Yield: 0.19 g (95.9 %);
¹H-NMR-DMSO-*d*₆ (400 MHz): δ: -0.09 (9H, s), 0.78 (2H, t, *J*=9.6 Hz), 0.98 (9H, s), 3.44 (2H, t, *J*=7.6 Hz), 4.78 (1H, t, *J*=6.4 Hz), 5.48 - 5.53 (1H, m), 5.69-5.79 (3H, m), 6.23 (1H, d, *J*=4.0 Hz), 6.99 (1H, d, *J*=4.0 Hz), 8.40 (1H, d, *J*=6.8 Hz), 12.71 (1H, s);
LCMS: 437.10 [M+Na]⁺

### Step 6: Synthesis of (E)-2-(5-chloro-2-pyrrolylcarbonylamino)-5,5-dimethyl-3-hexenoic acid (H)

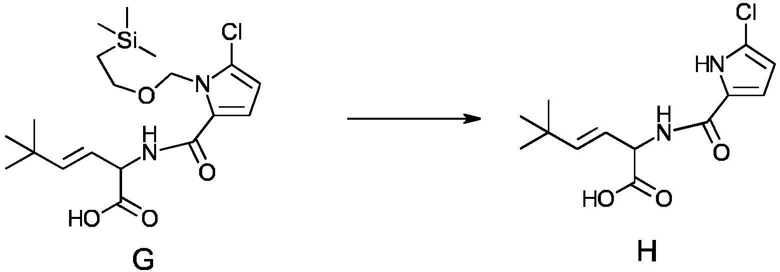

4 M HCI in 1,4-dioxane (3.8 mL) was added to a stirred solution of compound G (0.19 g, 0.45 mmol) in 1,4-dioxane (0.95 mL) at 25-30°C. The resulting mixture was stirred over a period of 18 h at 25-30°C. The progress of the reaction was monitored by TLC.

After 18 h, the reaction mixture was concentrated at 40°C to afford a crude solid. The crude solid was washed with hexane (5 mL) a white solid (0.13 g).

Ammonium hydroxide solution (0.9 mL) was added to a crude solution of the solid material (90 mg, 0.28 mmol) in ACN (1.8 mL) at 25-30°C. The resulting mixture was stirred over a period of 18 h at 25-30°C. The progress of the reaction was monitored by TLC.

After 18 h, the reaction mixture was concentrated at 40°C to afford a crude residue. The residue was washed with hexane (5 mL) to afford the pure product H, as a white solid.
Yield: 19.7 mg;
¹H-NMR-DMSO-*d*₆ (400 MHz): δ: 0.97 (9H, s), 4.81 - 4.85(1H, m), 5.45 - 5.51 (1H, m), 5.74 - 5.78 (1H, m), 6.04 (1H, d, *J*=4.0 Hz), 6.88 (1H, d, *J*=4.0 Hz), 8.26 (1H, d, *J*=7.8 Hz); 12.2 4(1H, bs), 12.60 (1H, bs);
LCMS: 285.3 [M+H] ⁺; HPLC purity- 98.4 %

### EXAMPLE 21 - Synthesis of (E)-2-(4,5-dichloro-2-thienylcarbonylamino)-5,5-dimethyl-3-hexenoic acid (SI62)

### Step 1: Synthesis of ethyl (E)-2-(4,5-dichloro-2-thienylcarbonylamino)-5,5-dimethyl-3-hexenoate (C)

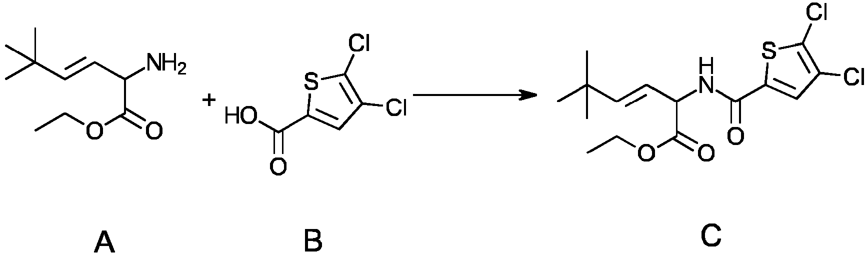

4,5-Dichloro-2-thenoic acid (B) (103.8 mg, 0.53 mmol) was added to a stirred solution of (*E*)-2-amino-5,5-dimethyl-3-hecenoate (A) (100 mg, 0.53 mmol) in DMF (2.5 mL) followed by DIPEA (0.37 mL, 2.15 mmol) and propylphosphonic anhydride (T3P, ~50% solution in ethyl acetate) (1.03 mL, 1.61 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted with ethyl acetate (2 × 20 mL). The combined organic layer was washed with saturated sodium chloride solution, dried over anhydrous sodium sulphate and concentrated in vacuo at 35°C to afford a crude product. The crude material was purified by CombiFlash^{®} using ethyl acetate : hexanes as eluent to afford compound C as a pale yellow, thick oil.
Yield: 148 mg (76.6 %);
¹H-NMR- CD₃OD (400 MHz): δ: 1.02 (9H, s), 1.22 - 1.26 (3H, m), 4.16 -4.17 (2H, m), 4.93 - 4.96 (1H, m), 5.50 - 5.53 (1H, m), 5.86 - 5.90 (1H, m), 7.68 - 7.69 (1H, m);
LCMS: 363.97 [M+H] ⁺

### Step 2: Synthesis (E)-2-(4,5-dichloro-2-thienylcarbonylamino)-5,5-dimethyl-3-hexenoic acid (D)

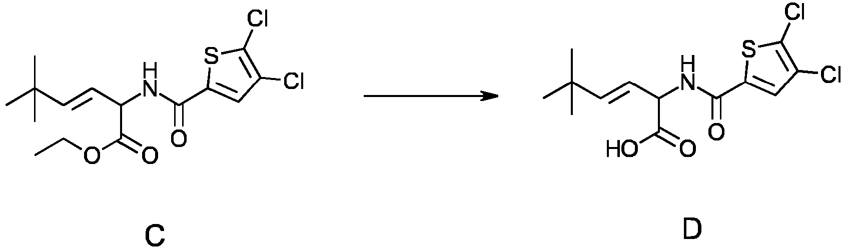

Lithium hydroxide monohydrate (40.9 mg, 0.97 mmol) was added to a stirred solution of compound C (142 mg, 0.38 mmol) in MeOH/THF/water (4.26 mL, 1:1:1, 1.42 mL each). The resulting reaction mixture was stirred over a period of 2.5 h at 25-30°C. The progress of the reaction was monitored by TLC.

After 18 h, the reaction mixture was concentrated, and the resulting residue was dissolved in water and washed with diethyl ether (10 mL). The aqueous layer was acidified to pH ~ 4-5 using a saturated solution of potassium hydrogen sulphate and the product was extracted with ethyl acetate (3 × 5 mL). The combined organic layer was dried over sodium sulphate and concentrated at 40°C to afford a crude solid. The obtained solid was washed with hexane (5 mL) to afford a mixture of two as a white solid. The crude mixture was purified by RP-HPLC to afford the pure product D as a white solid.
Yield: 65 mg;
¹H-NMR- DMSO-*d*₆ (400 MHz): δ: 0.99 (9H, s), 4.81 - 4.86 (1H, m), 5.44 - 5.52 (1H, m), 5.84 (1H, d, *J*=21.2 Hz), 8.01 (1H, s), 9.02 (1H, d, *J*=9.6 Hz), 12.81 (1H, bs);
LCMS: 336.3 [M+H]⁺; HPLC purity- 98.4 %.

### EXAMPLE 22 - Synthesis of (E)-2-(4-chloro-1-methyl-2-pyrrolylcarbonylamino)-5,5-dimethyl-3-hexenoic acid (SI31)

### Step 1: Synthesis of ethyl (E)-2-(4-chloro-1-methyl-2-pyrrolylcarbonylamino)-5,5-dimethyl-3-hexenoate (C)

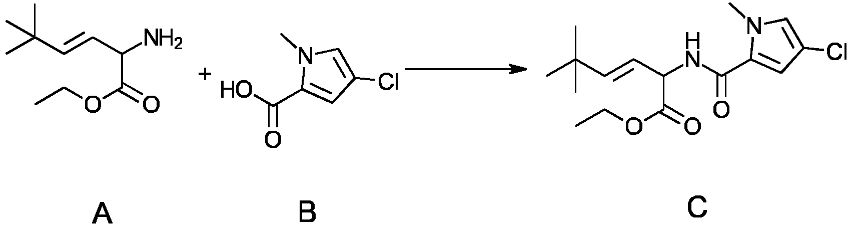

4-Chloro-1-methyl-2-pyrrolecarboxylic acid (B) (86 mg, 0.53 mmol) was added to a stirred solution of (*E*)-2-amino-5,5-dimethyl-3-hecenoate (A) (100 mg, 0.53 mmol) in DMF (2.5 mL) followed by DIPEA (0.35 mL, 2.12 mmol) and propylphosphonic anhydride (T3P, ~50% solution in ethyl acetate) (1.03 mL, 1.61 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted to ethyl acetate (2 × 20 mL). The combined organic layer was washed with saturated sodium chloride solution, dried over anhydrous sodium sulphate and concentrated in vacuo at 35°C to afford crude product. The crude material was purified by RP-HPLC to afford pure compound C as a colorless, thick oil.
Yield: 11 mg, (6.5 %)
LCMS: 327.5 [M+H]⁺

### Step 2: Synthesis of (E)-2-(4-chloro-1-methyl-2-pyrrolylcarbonylamino)-5,5-dimethyl-3-hexenoic acid (D)

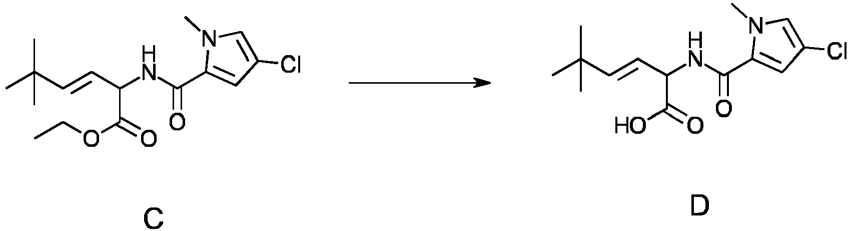

Lithium hydroxide monohydrate (7 mg, 0.15 mmol) was added to a stirred solution of compound C (11 mg, 0.03 mmol) in MeOH/THF/water (0.33 mL, 1:1:1). The resulting reaction mixture was stirred over a period of 18 h at 25-30°C. The progress of the reaction was monitored by TLC.

After 18 h, the reaction mixture was concentrated, the residue was dissolved in water and washed with diethyl ether (10 mL). The aqueous layer was acidified to pH ~ 5-6 using 1.5 N HCl and the product was extracted with ethyl acetate (3 × 5 mL). The combined organic layer was dried over sodium sulphate and concentrated at 40°C to afford a crude solid. The solid material was washed with hexane (5 mL) to afford a crude mixture of two isomers as a white solid. The crude material was purified by RP-HPLC to afford the product D as a white solid.
Yield: 0.4 mg
LCMS: 299.2 [M+H]⁺

### EXAMPLE 23 - Synthesis of (E)-2-(4-chloro-2-thienylcarbonylamino)-5,5-dimethyl-3-hexenoic acid (SI32)

### Step 1: Synthesis of ethyl (E)-2-(4-chloro-2-thienylcarbonylamino)-5,5-dimethyl-3-hexenoate (C)

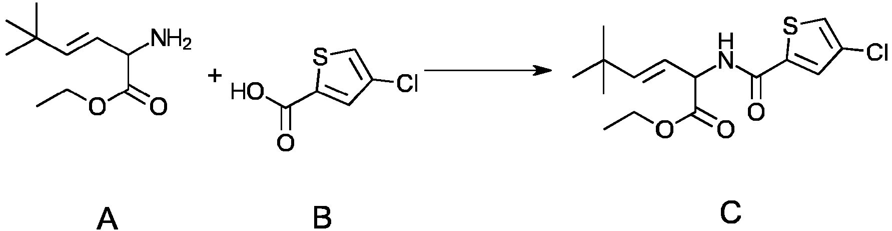

4-Chloro-2-thenoic acid (B) (97.26 mg, 0.53 mmol) was added to a stirred solution of (E)-2-amino-5,5-dimethyl-3-hecenoate (A) (100 mg, 0.53 mmol) in DMF (2.5 mL) followed by DIPEA (0.35 mL, 2.12 mmol) and propylphosphonic anhydride (T₃P, ~50% solution in ethyl acetate) (1.03 mL, 1.61 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted to ethyl acetate (2 × 20 mL). The combined organic layer was washed with saturated sodium chloride solution (15 mL), dried over anhydrous sodium sulphate and concentrated in vacuo at 35°C to afford a crude material. The crude material was purified by CombiFlash^{®} using ethyl acetate: hexanes as eluent to afford compound C as a brown, thick oil.
Yield: 47 mg (27.0 %)
¹H-NMR- CD₃OD (400 MHz): δ: 0.96 (9H, s), 1.26 (3H, t, J=7.2 Hz), 4.16-4.24 (2H, m), 4.96-4.98 (1H, m), 5.53-5.59 (1H, m), 5.93 (1H, d, *J=1.2* Hz), 7.56 (1H, s), 7.73 (1H, s);
LCMS: 330.3 [M+H]⁺

### Step 2: Synthesis of (E)-2-(4-chloro-2-thienylcarbonylamino)-5,5-dimethyl-3-hexenoic acid (D)

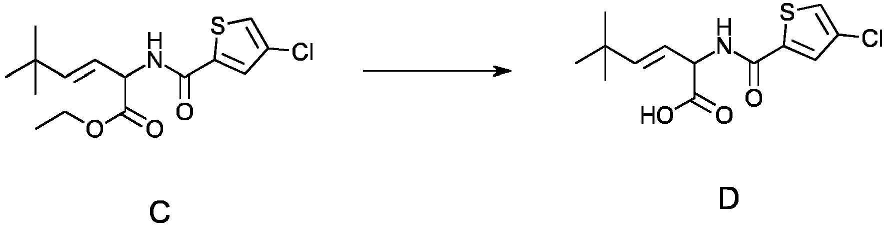

Lithium hydroxide monohydrate (39.9 mg, 1.04 mmol) was added to a stirred solution compound C (47 mg, 0.14 mmol) in THF/methanol/water (1.41 mL, 1:1:1, 0.47 mL each). The resulting reaction mixture was stirred over a period of 18 h at 25-30°C. The progress of the reaction was monitored by TLC.

After 18 h, the reaction mixture was concentrated, the resulting residue was dissolved in water and washed with diethyl ether (10 mL). The aqueous layer was acidified to pH ~ 5-6 using 1.5 N HCl and the product was extracted with ethyl acetate (3 × 5 mL). The combined organic layer was dried over sodium sulphate and concentrated at 40°C to afford a crude solid. The obtained solid was washed with hexane (5 mL) to afford a crude mixture of two isomers as an off white solid. The crude mixture was purified by RP-HPLC to afford pure compound D as a white solid.
Yield: 22 mg;
¹H-NMR- DMSO-*d*₆ (400 MHz): δ: 0.97 (9H, s), 4.76 (1H, t, J=6.8 Hz), 5.46-5.51 (1H, m), 5.74 (1H, d, *J=16.0* Hz), 7.80 (1H, s), 7.92 (1H, s), 8.76 (1H, bs);
LCMS: 302.6 [M+H]⁺; HPLC purity- 99.2 %.

### EXAMPLE 24 - Synthesis of (E)-5,5-dimethyl-2-[p-(3-pyridyloxy)benzoylaminol-3-hexenoic acid (SI34)

### Step 1: Synthesis of ethyl (E)-5,5-dimethyl-2-[p-(3-pyridyloxy)benzoylamino]-3-hexenoate (C)

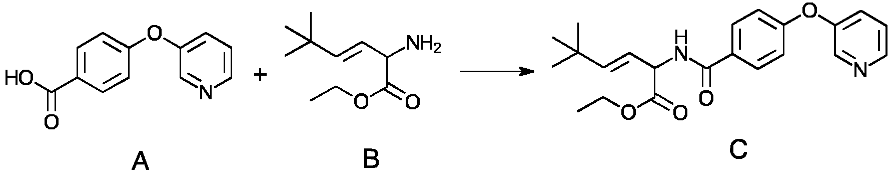

p-(3-Pyridyloxy)benzoic acid (A) (116 mg, 0.53 mmol) was added to a stirred solution of (E)-2-amino-5,5-dimethyl-3-hecenoate (B) (100 mg, 0.53 mmol) in DMF (2.5 mL) followed by DIPEA (0.35 mL, 2.12 mmol) and propylphosphonic anhydride (T₃P, ~50% solution in ethyl acetate) (1.03 mL, 1.61 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted to ethyl acetate (2 × 20 mL). The combined organic layer was washed with saturated sodium chloride solution (15 mL), dried over anhydrous sodium sulphate and concentrated in vacuo at 35°C to afford crude material. The crude material was purified by CombiFlash^{®} using ethyl acetate: hexanes as eluent to afford compound C as a yellow, thick oil.
Yield: 50.0 mg, (24.2 %);
¹H-NMR- CD₃OD (400 MHz): δ: 1.02 (9H, s), 1.24-1.31 (3H, m), 4.19-4.22 (2H, m), 5.04 (1H, d, J=1.2 Hz), 5.61-5.62 (1H, m), 5.94 (1H, d, *J*=1.2 Hz), 7.11 (2H, d, J=8.8 Hz), 7.46-7.66 (2H,m), 7.91-7.94 (2H, m), 8.37-8.38 (2H, m);
LCMS: 383.4 [M+H]⁺

### Step 2: Synthesis of (E)-5,5-dimethyl-2-[p-(3-pyridyloxy)benzoylamino]-3-hexenoic acid (D)

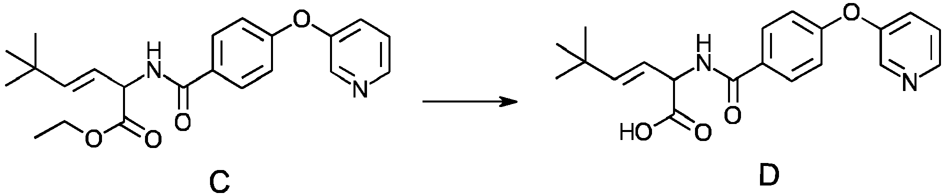

Lithium hydroxide monohydrate (43.9 mg, 1.04 mmol) was added to a stirred solution of compound C (80 mg, 0.20 mmol) in THF/methanol/water (2.4 mL, 1:1:1, 0.8 mL each). The resulting reaction mixture was stirred over a period of 18 h at 25-30°C. The progress of the reaction was monitored by TLC.

After 18 h, the reaction mixture was concentrated, the resulting residue was dissolved in water and washed with diethyl ether (10 mL): The aqueous layer was acidified to pH ~ 5-6 using 1.5 N HCl and the product was extracted with ethyl acetate (3 × 5 mL). The combined organic layer was dried over sodium sulphate and concentrated at 40°C to afford a crude solid. The solid was washed with hexane (5 mL) to afford a crude mixture of two isomers as an off white solid. The crude product was purified by RP-HPLC to afford pure compound D as a white solid.
Yield: 3.0 mg;
¹H-NMR- DMSO-*d*₆ (300 MHz): δ: 1.1 (9H, s), 4.99 (1H, t, J=7.2 Hz), 5.62-5.70 (1H, m), 5.93 (1H, d, J=15.6 Hz), 7.21 (2H, d, J=8.7 Hz), 7.55-7.66 (2H, m), 8.06 (2H, d, J=8.7 Hz), 8.53 (2H, d, J=4.2 Hz), 8.88 (1H, d, J=7.2 Hz), 12.67 (1H, bs);
LCMS: 355.3 [M+H]⁺; HPLC purity- 98.9 %.

### EXAMPLE 25 - Synthesis of (E)-5,5-dimethyl-2-(2-phenoxyisonicotinoylamino)-3-hexenoic acid (SI36)

### Step 1: Synthesis of ethyl (E)-5,5-dimethyl-2-(2-phenoxyisonicotinoylamino)-3-hexenoate (C)

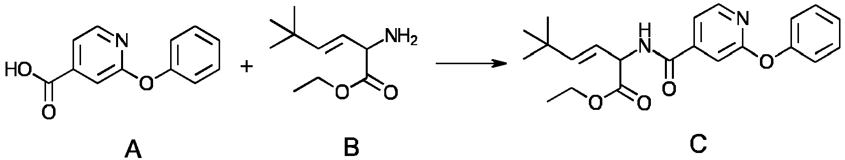

2-Phenoxyisonicotinic acid (A) (116.15 mg, 0.53 mmol) was added to a stirred solution of (E)-2-amino-5,5-dimethyl-3-hecenoate (B) (100 mg, 0.53 mmol) in DMF (2.5 mL) followed by DIPEA (0.35 mL, 2.12 mmol) and propylphosphonic anhydride (T₃P, ~50% solution in ethyl acetate) (1.03 mL, 1.61 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted to ethyl acetate (2 × 20 mL). The combined organic layer was washed with saturated sodium chloride solution (15 mL), dried over anhydrous sodium sulphate and concentrated in vacuo at 35°C to afford crude product. The crude product was purified by CombiFlash^{®} using ethyl acetate: hexanes as eluent to afford compound C as a brown, thick oil.
Yield: 103 mg, (49.9 %);
¹H-NMR- CD₃OD (400 MHz): δ: 1.02 (9H, s), 1.23 (3H, t, J=6.8 Hz), 4.16-4.19 (2H, m), 5.00 (1H, d, J=1.2 Hz), 5.53 (1H, m), 5.89 (1H, d, J=1.2 Hz), 7.10 (2H, t, J=0.8 Hz), 7.12 (1H, d, J=0.8 Hz), 7.21 (1H, s), 7.29-7.46 (3H, m), 8.22 (1H, d, J=0.8 Hz);
LCMS: 383.3 [M+H]⁺

### Step 2: Synthesis of (E)-5,5-dimethyl-2-(2-phenoxyisonicotinoylamino)-3-hexenoic acid (D)

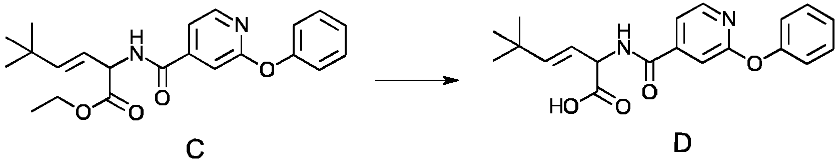

Lithium hydroxide monohydrate (54 mg, 1.3 mmol) was added to a stirred solution of compound C (100 mg, 0.26 mmol) in THF/methanol/water (3.0 mL, 1:1:1, 1.0 mL each) was added. The resulting reaction mixture was stirred over a period of 18 h at 25-30°C. The progress of the reaction was monitored by TLC.

After 18 h, the reaction mixture was concentrated, and the resulting residue was dissolved in water and washed with diethyl ether (10 mL). The aqueous layer was acidified to pH ~ 5-6 using 1.5 N HCl and the product was extracted with ethyl acetate (3 × 5 mL). The combined organic layer was dried over sodium sulphate and concentrated at 40°C to afford a crude solid. The crude solid was washed with hexane (5 mL) to afford a crude mixture of two isomers as an off white solid. The crude mixture was purified by RP-HPLC to afford pure compound D as a white solid.
Yield: 45 mg;
¹H-NMR- DMSO-*d*₆ (400 MHz): δ: 0.97 (9H, s), 4.84 (1H, d, J=1.2 Hz), 5.54 (1H, d, J=1.2 Hz), 7.14 (2H, d, J=0.8 Hz), 7.20-7.24 (1H, m), 7.40-7.45 (3H, m), 7.52-7.54 (1H, d, J=1.2 Hz), 8.27 (1H, d, J=5.2 Hz), 9.11 (1H, bs), 12.68 (1H, bs);
LCMS: 355.3 [M+H]⁺; HPLC purity- 98.4 %.

### EXAMPLE 26 - Synthesis of (E)-2-[6-(benzyloxy)nicotinoylamino]-5,5-dimethyl-3-hexenoic acid (SI39)

### Step 1: Synthesis of ethyl (E)-2-[6-(benzyloxy)nicotinoylamino]-5,5-dimethyl-3-hexenoate (C)

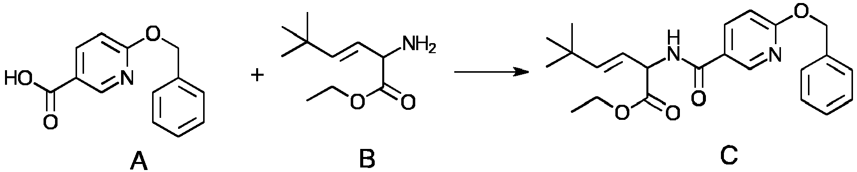

6-(Benzyloxy)nicotinic acid (A) (123.7 mg, 0.53 mmol) was added to a stirred solution (E)-2-amino-5,5-dimethyl-3-hecenoate (B) (100 mg, 0.53 mmol) in DMF (2.5 mL) followed by DIPEA (0.35 mL, 2.12 mmol) and propylphosphonic anhydride (T3P, ~50% solution in ethyl acetate) (1.03 mL, 1.61 mmol) at 25-30°C. The resulting reaction mixture was stirred over a period of 48 h at 40°C. The progress of the reaction was monitored by TLC.

After completion of the reaction, the reaction mixture was quenched by addition of saturated NaHCO3 solution to pH ~7-8 and the product was extracted to ethyl acetate (2 × 20 mL). The combined organic layer was washed with saturated sodium chloride solution (15 mL), dried over anhydrous sodium sulphate and concentrated in vacuo at 35°C to afford crude material. The crude product was purified by CombiFlash^{®} using ethyl acetate: hexanes as eluent to afford compound C as a brown, thick oil.
Yield: 65 mg (30.4 %);
¹H-NMR- CD₃OD (400 MHz): δ: 1.03 (9H, s), 1.22-1.26 (3H, m), 4.16-4.20 (2H, m), 5.01 (1H, m), 5.40 (2H, s), 5.58 (2H, d, *J=7.2* Hz), 5.89 (2H, d, *J=1.2* Hz), 6.88 (1H, m), 7.31-7.35 (3H, m), 7.41-7.43 (2H, m), 8.12 (1H, d, J=2.8 Hz), 8.66 (1H, s);
LCMS: 397.3 [M+H]⁺

### Step 2: Synthesis of (E)-2-[6-(benzyloxy)nicotinoylamino]-5,5-dimethyl-3-hexenoic acid (D)

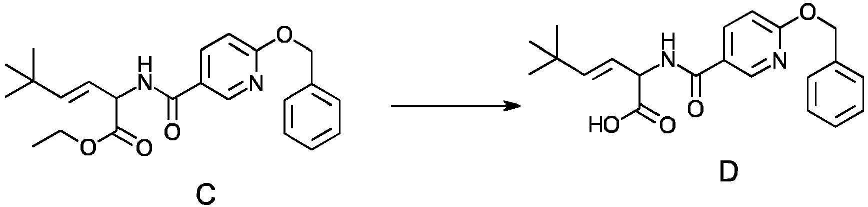

Lithium hydroxide monohydrate (32.8 mg,0.78 mmol) was added to a stirred solution of compound C (62 mg, 0.15 mmol) in THF/methanol/water (1.24 mL, 1:1:1, 0.62 mL each) The resulting reaction mixture was stirred over a period of 18 h at 25-30°C. The progress of the reaction was monitored by TLC.

After 18 h, the reaction mixture was concentrated, the residue was dissolved in water and washed with diethyl ether (10 mL). The aqueous layer was acidified to pH ~ 5-6 using 1.5 N HCl and the product was extracted with ethyl acetate (3 × 5 mL). The combined organic layer was dried over sodium sulphate and concentrated at 40°C to afford crude solid. The resulting solid was washed with hexane (5 mL) to afford a crude mixture of two isomers as an off white solid. The crude material was purified by RP-HPLC to afford pure compound D as a white solid.
Yield: 16 mg;
¹H-NMR- DMSO-*d*₆ (400 MHz): δ: 0.99 (9H, s), 4.87 (1H, d, J=7.2 Hz), 5.40 (2H, s), 5.53 (1H, d, J=7.2 Hz), 5.82 (1H, d, J=1.2 Hz), 6.95 (1H, d, J=8.8 Hz), 7.3 (3H, t, J=4.8 Hz), 7.35-7.46 (2H, m), 8.18 (1H, d, J=2.4 Hz), 8.70 (1H, s), 8.81 (1H, bs), 12.66 (1H, bs);
LCMS: 369.3 [M+H]⁺; HPLC purity 98.6 %

### EXAMPLE 27 - Synthesis of (E)-5,5-dimethyl-2-[p-(2-pyrimidinyloxy)benzoylaminol-3-hexenoic acid (SI41)

### Step 1: Synthesis of ethyl (E)-5,5-dimethyl-2-[p-(2-pyrimidinyloxy)benzoylamino]-3-hexenoate (C)

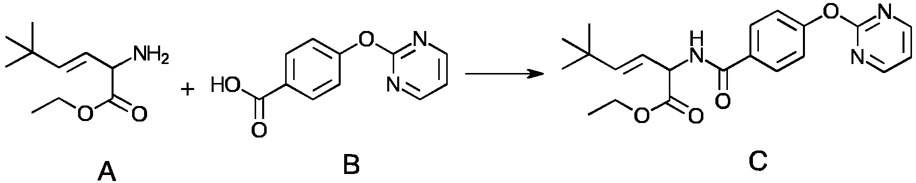

p-(2-Pyrimidinyloxy)benzoic acid (B) (116.6 mg, 0.53 mmol) was added to a stirred solution of (E)-2-amino-5,5-dimethyl-3-hecenoate (A) (100 mg, 0.53 mmol) in DMF (2.5 mL) followed by DIPEA (0.37 mL, 2.15 mmol) and propylphosphonic anhydride (T3P, ~50% solution in ethyl acetate) (1.03 mL, 1.61 mmol) at 25-30°C. The resulting reaction mixture was stirred over a period of 18 h at 40°C. The progress of the reaction was monitored by TLC.

After the completion of the reaction, the reaction mixture was quenched by addition of saturated NaHCO₃ solution to pH ~7-8 and the product was extracted with ethyl acetate (2 × 20 mL). The combined organic layer was washed with saturated sodium chloride solution, dried over anhydrous sodium sulphate and concentrated in vacuo at 35°C to afford crude material. The crude material was purified by CombiFlash^{®} using ethyl acetate: hexanes as eluent to afford compound C as a brown, thick oil.
Yield: 65 mg, (31.6 %);
¹H-NMR- DMSO-*d*₆ (400 MHz): δ: 1.02 (9H, s), 1.16 (3H, t, J=7.2 Hz), 4.10 (2H, q, J=3.6 Hz), 4.88 - 4.92 (1H, m), 5.52 - 5.57 (1H, m), 5.83 - 5.87 (1H, m), 7.28 - 7.31 (3H, m), 7.95 (2H, d, J=8.4 Hz), 8.65-8.68 (2H, m), 8.91 (1H, d, J=6.8 Hz); LCMS: 384.3 [M+H] ⁺

### Step 2: Synthesis of (E)-5,5-dimethyl-2-[p-(2-pyrimidinyloxy)benzoylamino]-3-hexenoic acid (D)

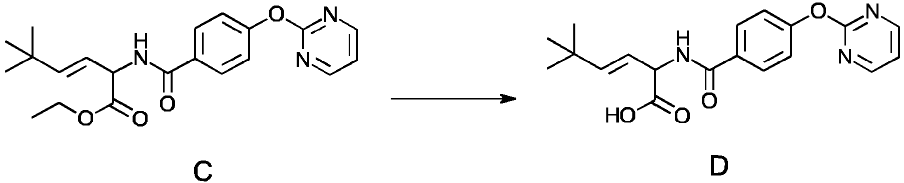

Lithium hydroxide monohydrate (16.7 mg,0.39 mmol) was added to a stirred solution of compound C (61 mg, 0.15 mmol) in THF : water (1.83 mL, 2:1). The resulting reaction mixture was stirred over a period of 18 h at 25-30°C. The progress of the reaction was monitored by TLC.

After 18 h, the reaction mixture was concentrated, the resulting residue was dissolved in water and washed with diethyl ether (10 mL). The aqueous layer was acidified to pH ~ 5-6 using 1.5 N HCl and the product was extracted with ethyl acetate (3 × 5 mL). The combined organic layer was dried over sodium sulphate and concentrated at 40°C to afford a crude solid. The obtained solid was washed with hexane (5 mL) to afford a crude mixture of two isomers. as a white solid. The crude solid was purified by RP-HPLC to afford pure product D as a white solid.
Yield: 20.0 mg;
¹H-NMR- DMSO-*d*₆ (400 MHz): δ: 1.02 (9H, s), 4.87 - 4.91 (1H, m), 5.53 - 5.59 (1H, m), 5.80 - 5.85 (1H, m), 7.28 - 7.30 (3H, m), 7.95 (2H, d, J=8.4 Hz), 8.65-8.68 (2H, m), 8.81 (1H, d, J=7.2 Hz), 12.64 (1H, bs);
LCMS: 356.03 [M+H] ⁺; HPLC purity- 99.8 %.

### EXAMPLE 28 - Synthesis of (E)-5,5-dimethyl-2-(2-phenoxy-5-pyrimidinylcarbonylamino)-3-hexenoic acid (SI44)

### Step 1: Synthesis of ethyl 2-phenoxy-5-pyrimidinecarboxylate (B)

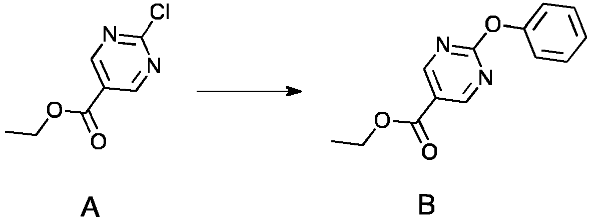

K₂CO₃ (1.1 g, 8.02 mmol) at 25-30°C was added to a stirred solution of ethyl 2-chloro-5-pyrimidinecarboxylate (A) (1 g, 5.35 mmol) and phenol (0.55 g, 5.89 mmol) in DMF (20 mL). The resulting reaction mixture was stirred over a period of 2 h at 60°C. The progress of the reaction was monitored by TLC.

After completion of the reaction, the reaction mixture was quenched by addition of ice-cold water and the product was extracted with ethyl acetate (2 × 20 mL). The combined organic layer was washed with saturated sodium chloride solution, dried over anhydrous sodium sulphate and concentrated in vacuo at 40°C to afford a crude residue. The crude residue was purified by CombiFlash^{®} using ethyl acetate: hexanes as eluent to afford compound B as a colorless, thick oil.
Yield: 1.1 g (84.6 %);
¹H-NMR- DMSO-*d*₆ (400 MHz): δ: 1.30 (3H, t, J=7.2 Hz), 4.30 (2H, q, *J=7.2* Hz), 7.23-7.30 (3H, m), 7.44-7.48 (2H, m), 9.06 (2H, m);
LCMS: 245.1 [M+H] ⁺

### Step 2: Synthesis of 2-phenoxy-5-pyrimidinecarboxylic acid (C)

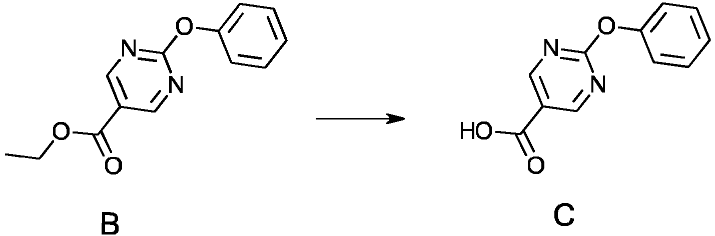

1 M aqueous NaOH (0.98 mL, 0.9 mmol) was added to a stirred solution of compound B (0.2 g, 0.81 mmol) in THF (2 mL) at 0-5°C. The resulting reaction mixture was stirred over a period of 4 h at 25-30°C. The progress of the reaction was monitored by TLC.

After 4 h, the reaction mixture was concentrated, the resulting residue was dissolved in water and washed with diethyl ether (10 mL). The aqueous layer was acidified to pH ~ 4-5 using saturated solution of potassium hydrogen sulphate and the product was extracted with ethyl acetate (3 × 5 mL). The combined organic layer was dried over sodium sulphate and concentrated at 40°C to afford a crude solid. The crude solid was washed with hexane (5 mL) to afford compound C as a white solid.
Yield: 0.14 g (83.4 %);
¹H-NMR- DMSO-*d*₆ (400 MHz): δ: 7.23-7.31 (3H, m), 7.43-7.48 (2H, m), 9.03 (2H, m), 13.76 (1H, bs); LCMS: 216.88 [M+H] ⁺

### Step 3: Synthesis of ethyl (E)-5,5-dimethyl-2-(2-phenoxy-5-pyrimidinylcarbonylamino)-3-hexenoate (E)

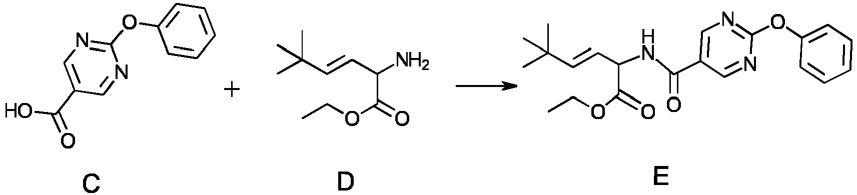

Compound C (116.6 mg, 0.53 mmol) was added to a stirred solution of (E)-2-amino-5,5-dimethyl-3-hecenoate (D) (100 mg, 0.53 mmol) in DMF (2.5 mL) followed by DIPEA (0.37 mL, 2.15 mmol) and propylphosphonic anhydride (T3P, ~50% solution in ethyl acetate) (1.03 mL, 1.61 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted with ethyl acetate (2 × 20 mL). The combined organic layer was washed with saturated sodium chloride solution, dried over anhydrous sodium sulphate and concentrated in vacuo at 35°C to afford a crude residue. The crude residue was purified by CombiFlash^{®} using ethyl acetate: hexanes as eluent to afford compound E as a pale green, thick oil.
Yield: 75 mg (43.6 %);
¹H-NMR- DMSO-*d*₆ (400 MHz): δ: 1.02 (9H, s), 1.32 (3H, t, J=7.6 Hz), 4.13 (2H, q, *J=7.2* Hz), 4.93-4.96 (1H, m), 5.47-5.55 (1H, m), 5.86-5.91 (1H, m), 7.23-7.41 (3H, m), 7.44-7.49 (2H, m), 9.04 (2H, s), 9.15 (1H, d, J=8.8 Hz);
LCMS: 384.3 [M+H] ⁺

### Step 4: Synthesis of (E)-5,5-dimethyl-2-(2-phenoxy-5-pyrimidinylcarbonylamino)-3-hexenoic acid (F)

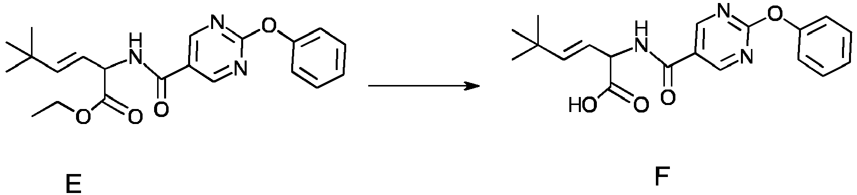

1 M aqueous NaOH (0.21 mL, 0.21 mmol) was added to a stirred solution of compound E (70 mg, 0.18 mmol) in THF (0.7 mL) at 0-5°C. The resulting reaction mixture was stirred over a period of 2 h at 25-30°C. The progress of the reaction was monitored by TLC.

After 2 h, the reaction mixture was concentrated, the resulting residue was dissolved in water and washed with diethyl ether (10 mL). The aqueous layer was acidified to pH ~ 4-5 using saturated solution of potassium hydrogen sulphate and the product was extracted with ethyl acetate (3 × 5 mL). The combined organic layer was dried over sodium sulphate and concentrated at 40°C to afford a crude solid. The solid was washed with hexane (5 mL) to afford a white solid. The crude solid was purified by RP-HPLC to afford the pure product F as a white solid.
Yield: 19 mg;
¹H-NMR- DMSO-*d*₆ (400 MHz): δ: 1.02 (9H, s), 4.82 - 4.93 (1H, m), 5.49 - 5.55 (1H, m), 5.81 - 5.85 (1H, m), 7.22 - 7.29 (3H, m), 7.43 - 7.47 (2H, m), 9.03-9.05 (3H, m), 12.39 (1H, bs);
LCMS: 356.07 [M+H] ⁺; HPLC purity- 98.9 %.

### EXAMPLE 29 - Synthesis of (E)-2-[p-(p-chlorophenoxy)benzoylaminol-5,5-dimethyl-3-hexenoic acid (SI53)

### Step 1: Synthesis of ethyl (E)-2-[p-(p-chlorophenoxy)benzoylamino]-5,5-dimethyl-3-hexenoate (C)

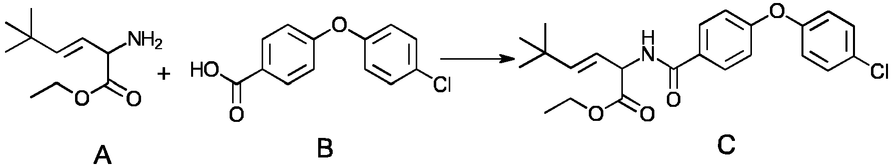

p-(p-Chlorophenoxy)benzoic acid (B) (131.7 mg, 0.53 mmol) was added to a stirred solution of (E)-2-amino-5,5-dimethyl-3-hecenoate (A) (100 mg, 0.53 mmol) in DMF (2.5 mL) followed by DIPEA (0.37 mL, 2.15 mmol) and propylphosphonic anhydride (T₃P, ~50% solution in ethyl acetate) (1.03 mL, 1.61 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted with ethyl acetate (2 × 20 mL). The combined organic layer was washed with saturated sodium chloride solution, dried over anhydrous sodium sulphate and concentrated in vacuo at 35°C to afford a crude product. The crude material was purified by CombiFlash^{®} using ethyl acetate: hexanes as eluent to afford compound C as a pale yellow, thick oil.
Yield: 35 mg (15.8 %);
¹H-NMR- CD₃OD (400 MHz): δ: 1.02 (9H, s), 1.24 (3H, t, J=7.2 Hz), 4.17 (2H, d, J=5.2 Hz), 5.02 - 5.04 (1H, m), 5.58 - 5.60 (1H, m), 5.87 - 5.91 (1H, m), 7.00 - 7.04 (4H, m), 7.36 - 7.39 (2H, m), 7.85 - 7.87 (2H, m);
LCMS: 416.13 [M+H] ⁺

### Step 2: Synthesis of (E)-2-[p-(p-chlorophenoxy)benzoylamino]-5,5-dimethyl-3-hexenoic acid (D)

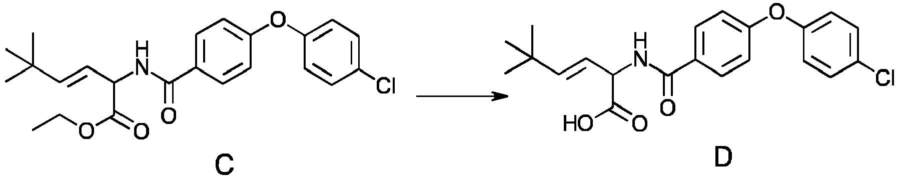

Lithium hydroxide monohydrate (7.56 mg, 0.18 mmol) was added to a stirred solution of compound C (30 mg, 0.07 mmol) in MeOH/THF/water (0.9 mL, 1:1:1, 0.3 mL each) was added. The resulting reaction mixture was stirred over a period of 7 h at 25-30°C. The progress of the reaction was monitored by TLC.

After 18 h, the reaction mixture was concentrated, the resulting residue was dissolved in water and washed with diethyl ether (10 mL). The aqueous layer was acidified to pH ~ 4-5 using 1.5 N HCl and the product was extracted with ethyl acetate (3 × 5 mL). The combined organic layer was dried over sodium sulphate and concentrated at 40°C to afford a crude solid. The obtained solid was washed with hexane (5 mL) to afford the crude product as a white solid. The crude material was purified by RP-HPLC to afford the product D as a white solid.
Yield: 10 mg;
¹H-NMR- DMSO-*d*₆ (400 MHz): δ: 1.00 (9H, s), 4.84 - 4.88 (1H, m), 5.52 - 5.59 (1H, m), 5.81 (1H, d, J=20.8 Hz), 7.06 - 7.13 (4H, m), 7.48 (2H, d, J=12.0 Hz), 7.94 (2H, d, J=11.6 Hz), 8.70 (1H, d, J=9.2 Hz), 12.67 (1H, bs);
LCMS: 388.10 [M+H] ⁺; HPLC purity- 96.5 %.

### EXAMPLE 30 - Synthesis of (E)-2-[p-(m-chlorophenoxy)benzoylaminol-5,5-dimethyl-3-hexenoic acid (SI54)

### Step 1: Synthesis of ethyl (E)-2-[p-(m-chlorophenoxy)benzoylamino]-5,5-dimethyl-3-hexenoate (C)

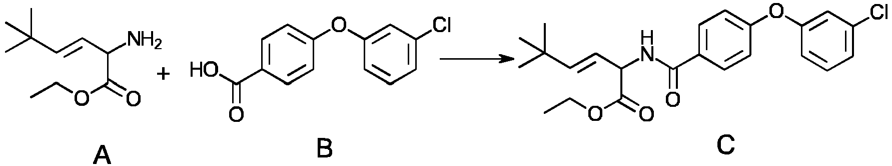

p-(m-Chlorophenoxy)benzoic acid (B) (100 mg, 0.40 mmol) was added to a stirred solution of (E)-2-amino-5,5-dimethyl-3-hecenoate (A) (74.50 mg, 0.40 mmol) in DMF (2.5 mL) followed by DIPEA (0.28 mL, 1.60 mmol) and propylphosphonic anhydride (T3P, ~50% solution in ethyl acetate) (0.76 mL, 1.2 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted with ethyl acetate (2 × 20 mL). The combined organic layer was washed with saturated sodium chloride solution (10 mL), dried over anhydrous sodium sulphate and concentrated in vacuo at 35°C to afford crude material. The crude material was purified by CombiFlash^{®} using ethyl acetate: hexanes as eluent to afford compound C as a pale yellow, thick oil.
Yield: 29 mg (17.3 %);
¹H-NMR- CD₃OD (400 MHz): δ: : 0.96 (9H, s), 1.22-1.28 (3H, m), 4.17-4.20 (2H, m), 4.99-5.01 (1H, m), 5.57 (1H, t, J=7.6 Hz), 5.90 (1H, d, J=1.2 Hz), 7.03-7.06 (5H, m), 7.35 (1H, d, J=8.0 Hz), 7.87 (2H, t, J=1.2 Hz);
LCMS: 416.4 [M+H]⁺

### Step 2: Synthesis of (E)-2-[p-(m-chlorophenoxy)benzoylamino]-5,5-dimethyl-3-hexenoic acid (D)

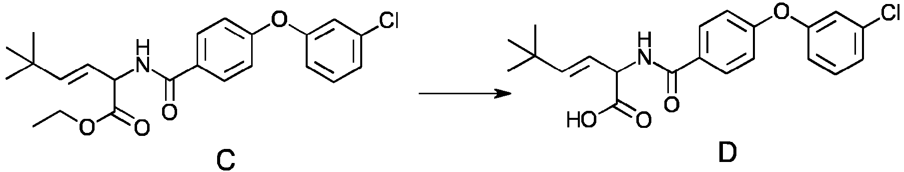

Lithium hydroxide monohydrate (6.30 mg, 0.15 mmol) was added to a stirred solution of compound C in MeOH/THF/water (0.75 mL, 1:1:1, 0.25 mL each) was added. The resulting reaction mixture was stirred over a period of 18 h at 25-30°C. The progress of the reaction was monitored by TLC.

After 18 h, the reaction mixture was concentrated, the resulting residue was dissolved in water and acidified to pH ~ 4-5 using 1.5 N HCl at 0-5 °C and the product was extracted with ethyl acetate (3 × 5 mL). The combined organic layer was dried over sodium sulphate and concentrated at 40°C to afford a crude solid. The obtained solid was washed with hexane (5 mL) to afford a crude mixture of two isomers as a white solid. The crude product was purified by RP-HPLC to afford the pure product D as a white solid.
Yield: 8.4 mg;
¹H-NMR- DMSO-*d*₆ (400 MHz): δ: 0.97 (9H, s), 5.50-5.56 (1H, m), 5.77-5.81 (1H, m), 7.01-7.09 (1H, m), 7.10-7.14 (3H, m), 7.22-7.25 (1H, m), 7.42 (1H, t, J=8.0 Hz), 7.92-7.94 (2H, m), 8.8 (1H, bs); LCMS: 388.5 [M+H]⁺; HPLC purity: 97.9%.

### EXAMPLE 31 - Synthesis of (E)-5,5-dimethyl-2-[p-(phenoxymethyl)benzoylaminol-3-hexenoic acid (SI55)

### Step 1: Synthesis of ethyl (E)-5,5-dimethyl-2-[p-(phenoxymethyl)benzoylamino]-3-hexenoate (C)

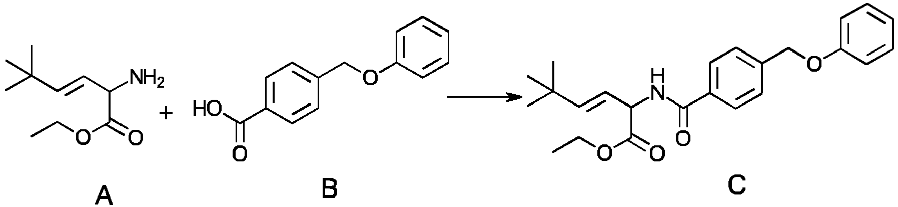

p-(Phenoxymethyl)benzoic acid (B) (120 mg, 0.64 mmol) was added to a stirred solution of (E)-2-amino-5,5-dimethyl-3-hecenoate (A) (147.83 mg, 0.64 mmol) in DMF (3.0 mL) was added followed by DIPEA (0.45 mL, 2.59 mmol) and propylphosphonic anhydride (T₃P, ~50% solution in ethyl acetate) (1.23 mL, 1.94 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted with ethyl acetate (2 × 20 mL). the combined organic layer was washed with saturated sodium chloride solution (20 mL), dried over anhydrous sodium sulphate and concentrated in vacuo at 35°C to afford a crude material. The crude material was purified by CombiFlash^{®} using ethyl acetate: hexanes as eluent to afford pure compound C as a brown, thick oil.
Yield: 34 mg (13.3 %)

### Step 2: Synthesis of (E)-5,5-dimethyl-2-[p-(phenoxymethyl)benzoylamino]-3-hexenoic acid (D)

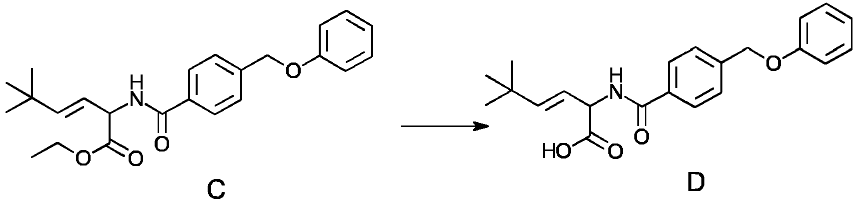

Lithium hydroxide monohydrate (8.73 mg, 0.20 mmol) was added to a stirred solution of compound C (32 mg, 0.08 mmol) in MeOH/THF/water (0.96 mL, 1:1:1, 0.32 mL each) was added. The resulting reaction mixture was stirred over a period of 18 h at 25-30°C. The progress of the reaction was monitored by TLC.

After 18 h, the reaction mixture was concentrated, the resulting residue was dissolved in water and acidified to pH ~ 4-5 using 1.5 N HCl at 0-5°C and the product was extracted with ethyl acetate (3 × 5 mL). The combined organic layer was dried over sodium sulphate and concentrated at 40°C to afford a solid material. The solid material was washed with hexane (5 mL) to afford a crude mixture of two isomers as a white solid. The crude mixture was purified by RP-HPLC to afford pure product D as a pure white solid.
Yield: 12.5 mg;
¹H-NMR- CDCl₃ (400 MHz): δ: 1.00 (9H, s), 4.86-4.90 (1H, m), 5.18 (2H, s), 5.53-5.59 (1H, m), 5.81 (1H, d, J=1.2 Hz), 6.92-7.02 (3H, m), 7.27-7.31 (2H, m), 7.53 (2H, d, J=8.4 Hz), 7.91 (2H, d, J=8.4 Hz), 8.79 (1H, s);
LCMS: 368.3 [M+H] ⁺; HPLC purity 99.0 %.

### EXAMPLE 32 - Synthesis of (E)-2-(1-ethyl-3-isobutyl-5-pyrazolylcarbonylamino)-5,5-dimethyl-3-hexenoic acid (SI56)

### Step 1: Synthesis of ethyl (E)-2-(1-ethyl-3-isobutyl-5-pyrazolylcarbonylamino)-5,5-dimethyl-3-hexenoate (C)

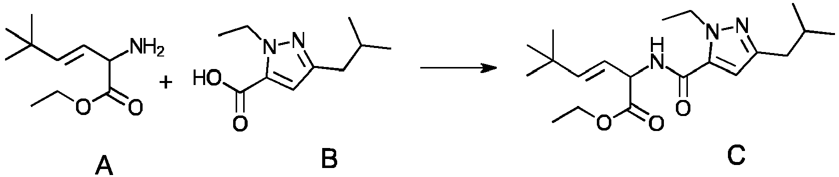

1-Ethyl-3-isobutyl-5-pyrazolecarboxylic acid (B) (105.7 mg, 0.53 mmol) was added to a stirred solution (*E*)-2-amino-5,5-dimethyl-3-hecenoate (A) (100 mg, 0.53 mmol) in DMF (2.5 mL) followed by DIPEA (0.376 mL, 2.15 mmol) and propylphosphonic anhydride (T₃P, ~50% solution in ethyl acetate) (1.028 mL, 1.61 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted to ethyl acetate (2 × 20 mL). The combined organic layer was washed with saturated sodium chloride solution (15 mL), dried over anhydrous sodium sulphate and concentrated in vacuo at 35°C to afford crude material. The crude material was purified by CombiFlash^{®} using ethyl acetate: hexanes as eluent to afford compound C as a thick, yellow liquid.
Yield: 47 mg, (24.0 %);
¹H-NMR- DMSO-*d*₆ (400 MHz): δ: 0.94-0.97 (6H, m), 1.05 (9H, s), 1.23-1.36 (6H, m), 1.90-1.95 (1H, m), 2.48 (2H, d, *J=7.2* Hz), 4.16-4.24 (2H, m), 4.43-4.48 (2H, m), 4.96 (1H, d, J=7.2 Hz), 5.54-5.59 (1H, m), 5.88-5.92 (1H, m), 6.66 (1H, s);
LCMS- 363.49 [M+H]⁺

### Step 2: Synthesis of (E)-2-(1-ethyl-3-isobutyl-5-pyrazolylcarbonylamino)-5,5-dimethyl-3-hexenoic acid (D)

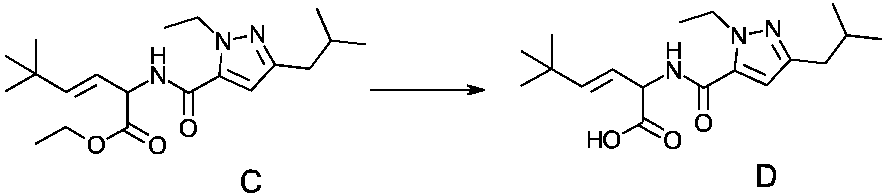

Lithium hydroxide monohydrate (27.15 mg, 0.64 mmol) was added to a stirred solution of compound C (47 mg, 0.12 mmol) in THF/methanol/water (1.41 mL, 1:1:1, 0.47 mL each) was added. The resulting reaction mixture was stirred over a period of 18 h at 25-30°C. The progress of the reaction was monitored by TLC.

After 18 h, the reaction mixture was concentrated, the resulting residue was dissolved in water and washed with diethyl ether (10 mL). The aqueous layer was acidified to pH ~ 5-6 using 1.5 N HCl and the product was extracted with ethyl acetate (3 × 5 mL). The combined organic layer was dried over sodium sulphate and concentrated at 40°C to afford a crude solid. The obtained solid was washed with hexane (5 mL) to afford a crude mixture of two isomers as an off white solid. The mixture was purified by RP-HPLC to afford pure product D as a white solid.
Yield: 28.9 mg;
¹H-NMR- DMSO-*d*₆ (400 MHz): δ: 0.90 - 0.88 (6H, m), 0.99 (9H, s), 1.23 (3H, t, J= 6.8 Hz), 1.83 - 1.86 (1H, m), 2.49 (2H, d, *J* =2.0 Hz), 4.34 - 4.41 (2H, m), 4.80-4.82 (1H, m), 5.33-5.45 (1H, m), 5.79 - 5.83 (1H, m), 6.74 (1H, s) 8.67 (1H, d, *J* = 7.2 Hz), 12.6 (1H, bs);
LCMS: 336.4 [M+H] ⁺; HPLC purity - 99.2 %.

### EXAMPLE 33 - Synthesis of (E)-2-(3-chloro-1-methyl-5-pyrazolylcarbonylamino)-5,5-dimethyl-3-hexenoic acid (SI57)

### Step 1: Synthesis of ethyl (E)-2-(3-chloro-1-methyl-5-pyrazolylcarbonylamino)-5,5-dimethyl-3-hexenoate (C)

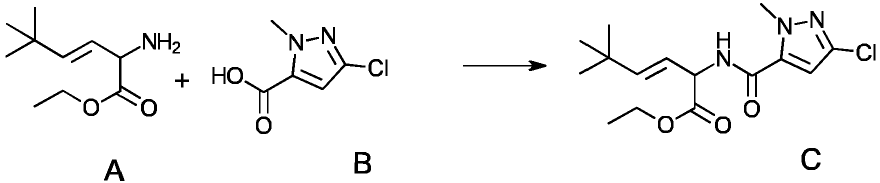

3-Chloro-1-methyl-5-pyrazolecarboxylic acid (B) (86.53 mg, 0.53 mmol) was added to a stirred solution of (*E*)-2-amino-5,5-dimethyl-3-hecenoate (A) (100 mg, 0.53 mmol) in DMF (2.5 mL) followed by DIPEA (0.376 mL, 2.15 mmol) and propylphosphonic anhydride (T₃P, ~50% solution in ethyl acetate) (1.02 mL, 1.61 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted to ethyl acetate (2 × 20 mL). The combined organic layer was washed with saturated sodium chloride solution (15 mL), dried over anhydrous sodium sulphate and concentrated in vacuum at 35°C to afford a crude residue. The crude residue was purified by CombiFlash^{®} using ethyl acetate: hexanes as eluent to afford compound C as a thick, yellow liquid.
Yield: 52 mg, (29.4 %);
¹H-NMR- CD₃OD (300 MHz): δ: 1.06 (9H, s), 1.25-1.35 (3H, m), 4.04 (3H, s), 4.15 - 4.25 (2H, m), 4.97 (1H, d, *J*=7.5 Hz), 5.50 - 5.58 (1H, m), 5.88 -5.94 (1H, m), 6.83 (1H, s);
LCMS: 328.3 [M+H]⁺

### Step 2: Synthesis of (E)-2-(3-chloro-1-methyl-5-pyrazolylcarbonylamino)-5,5-dimethyl-3-hexenoic acid (D)

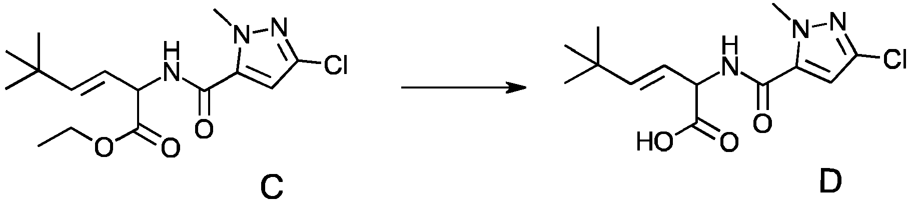

Lithium hydroxide monohydrate (33.3 mg, 0.79 mmol) was added to a stirred solution of compound C (52 mg, 0.15 mmol) in THF/methanol/water (1.56 mL, 1:1:1, 0.52 mL each) was added. The resulting reaction mixture was stirred over a period of 18 h at 25-30°C. The progress of the reaction was monitored by TLC.

After 18 h, the reaction mixture was concentrated, the resulting residue was dissolved in water and washed with diethyl ether (10 mL). The aqueous layer was acidified to pH ~ 5-6 using 1.5 N HCl and the product was extracted with ethyl acetate (3 × 5 mL). The combined organic layer was dried over sodium sulphate and concentrated at 40°C to afford a crude solid. The crude solid was washed with hexane (5 mL) to afford a crude mixture of two isomers as an off white solid. The mixture was purified by RP-HPLC to afford pure product D as a white solid.
Yield: 28.5 mg;
¹H-NMR- DMSO-*d*₆ (400 MHz): δ: 0.97 (9H, s), 3.96 (3H, s), 4.80 - 4.84 (1H, m), 5.45- 5.50 (1H, m), 5.77-5.81 (1H, m), 7.02 (1H, s), 8.84 (1 H, d, J=7.6 Hz);
LCMS: 300.3 [M+H] ⁺; HPLC purity - 98.4 %

### EXAMPLE 34 - Synthesis of (E)-2-(3-isopropyl-1-methyl-5-pyrazolylcarbonylamino)-5,5-dimethyl-3-hexenoic acid (SI58)

### Step 1: Synthesis of ethyl (E)-2-(3-isopropyl-1-methyl-5-pyrazolylcarbonylamino)-5,5-dimethyl-3-hexenoate (C)

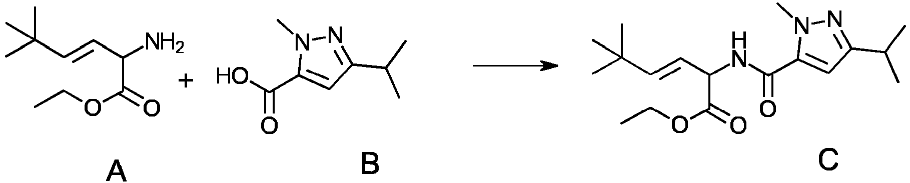

3-Isopropyl-1-methyl-5-pyrazolecarboxylic acid (B) (90.7 mg, 0.53 mmol) followed by DIPEA (0.37 mL, 2.15 mmol) and propylphosphonic anhydride (T3P, ~50% solution in ethyl acetate) (1.03 mL, 1.61 mmol) were added to a stirred solution of (*E*)-2-amino-5,5-dimethyl-3-hecenoate (A) (100 mg, 0.53 mmol) in DMF (2.5 mL) was added at 25-30 °C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted to ethyl acetate (2 × 20 mL). The combined organic layer was washed with saturated sodium chloride solution (20 mL), dried over anhydrous sodium sulphate and concentrated in vacuo at 35°C to afford a crude residue. The crude residue was purified by CombiFlash^{®} using ethyl acetate: hexanes as eluent to afford compound C as a brown, thick oil.
Yield: 56 mg, (31.0 %);
¹H-NMR-CD₃OD (400 MHz): δ: 1.05 (9H, s), 1.25 - 1.28 (9H, m), 2.90 - 2.97 (1H, m), 4.02 (3H, s), 4.23 (2H, q, J=3.6 Hz), 4.95 - 4.98 (1H, m), 5.53-5.58 (1H, m), 5.92 (1H, d, J=14.4 Hz), 6.73 (1H, s); LCMS: 366.5 [M+H] ⁺

### Step 2: Synthesis of (E)-2-(3-isopropyl-1-methyl-5-pyrazolylcarbonylamino)-5,5-dimethyl-3-hexenoic acid (D)

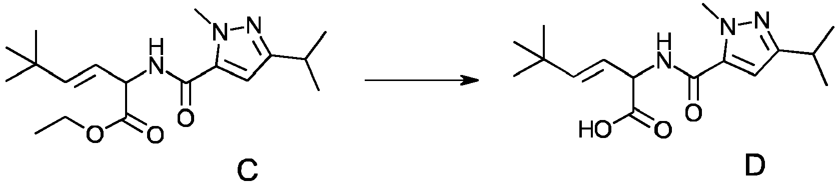

Lithium hydroxide monohydrate (32.5 mg, 0.77 mmol) was added to a stirred solution of compound C (52 mg, 0.15 mmol) in THF/methanol/water (1.56 mL, 1:1:1, 0.52 mL each) was added. The resulting reaction mixture was stirred over a period of 18 h at 25-30°C. The progress of the reaction was monitored by TLC.

After 18 h, the reaction mixture was concentrated, the residue was dissolved in water and washed with diethyl ether (10 mL). The aqueous layer was acidified to pH ~ 5-6 using 1.5 N HCl and the product was extracted with ethyl acetate (3 × 5 mL). The combined organic layer was dried over sodium sulphate and concentrated at 40°C to afford a crude solid. The crude solid was washed with hexane (5 mL) to afford a crude mixture of two isomers as a white solid. The mixture was purified by RP-HPLC to afford pure product D as a white solid.
Yield: 23 mg;
¹H-NMR- DMSO-*d*₆ (400 MHz): δ: 1.0 (9H, s), 1.19 (6H, d, J=9.2 Hz), 2.84-2.89 (1H, m), 3.95 (3H, s), 4.81 (1H, t, J=9.6 Hz), 5.47 - 5.54 (1H, m), 5.82 (1H, d, J=16.4 Hz), 6.84 (1H, s), 8.68 (1H, d, J=9.6 Hz), 12.66 (1H, bs);
LCMS: 308.4 [M+H] ⁺; HPLC purity- 99.4 %.

### EXAMPLE 35 - Synthesis of (E)-5,5-dimethyl-2-[1-methyl-3-(3-pyridyl)-5-pyrazolylcarbonylaminol-3-hexenoic acid (SI59)

### Step 1: Synthesis of ethyl nicotinoylacetate (C)

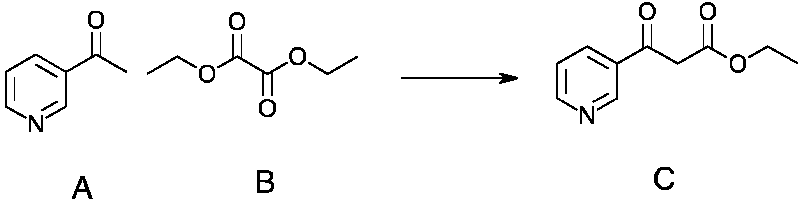

Diethyl oxalate (B) (6.7 g, 0.049 mmol) was washed with dry THF (4.5 mL) and added to a stirred solution of NaH (60 % w/w, 2.95 g, 0.074 mmol) in dry THF (51 mL). The resulting mixture was heated to 75°C, then a solution of 1-(3-pyridyl)-1-ethanone (A) (3 g, 0.024 mmol) in dry THF (4.5 mL) was added slowly with stirring at reflux. The resulting reaction mixture was stirred over a period of 15 min at 25-30°C. The progress of the reaction was monitored by TLC.

The resulting reaction mixture was added slowly to ice cold 1.5 N HCl solution and then solid NaHCO₃ was added until slightly basic. The resulting mixture was extracted with ethyl acetate (2 × 75 mL). The combined organic layer was washed with saturated sodium chloride solution (100 mL), dried over anhydrous sodium sulphate and concentrated in vacuo at 35°C to afford crude material The obtained solid was dissolved in warm ethanol (100 mL) and 1g of charcoal was added. After 5 min, the solution was filtered hot through a celite bed. The filtrate was concentrated under vacuo to afford compound C as a reddish brown solid.
Yield: 890 mg, Yield 16.3 %

### Step 2: Synthesis of ethyl 1-methyl-3-(3-pyridyl)-5-pyrazolecarboxylate (D)

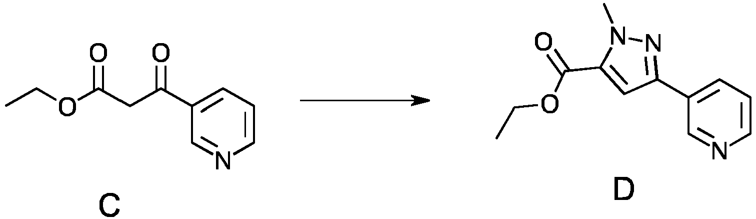

Methyl hydrazine (0.118 mL) was added to a stirred solution of compound C (450 mg, 2.034 mmol) in ethanol (5.4 mL) followed by p-toluensulfonic acid (PTSA) (699.69 mg, 4.06 mmol). The resulting reaction mixture was stirred over a period of 18 h at 80°C. The progress of the reaction was monitored by TLC.

After completion of the reaction, the reaction mixture was quenched by addition of saturated NaHCO₃ solution, and the product was extracted to ethyl acetate (2 × 30 mL). The combined organic layer was dried over anhydrous sodium sulphate and concentrated in vacuo at 35°C to afford crude material. The crude material was purified by CombiFlash^{®} to afford compound D as an off white solid.
Yield: 43 mg (9.2 %);
¹H-NMR- CDCl₃ (400 MHz): δ: 1.42 (3H, t, J=7.2 Hz), 4.25 (3H, s), 4.39 (2H, q, *J=7.2* Hz), 7.17 (1H, s), 7.34-7.37 (1H, m), 8.11- 8.14 (1H, m), 8.57 (1H, d, *J=* 4 Hz), 9.03 (1H, s);
LCMS: 232.1 [M+H] ⁺

### Step 3: Synthesis of 1-methyl-3-(3-pyridyl)-5-pyrazolecarboxylic acid (E)

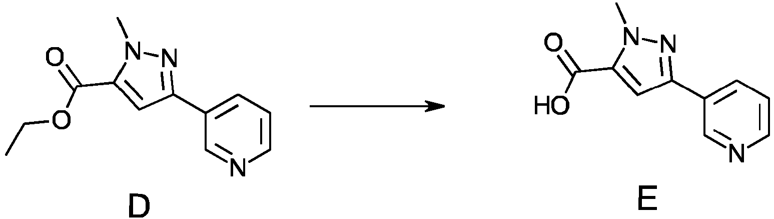

A stirred solution of compound D (34 mg, 0.14 mmol) in 1,4-dioxane (0.24 mL) was cooled to 0°C. NaOH (14.7 mg, 0.36 mmol) was dissolved in water (0.04 mL) and added to the above solution dropwise. The resulting reaction mixture was stirred over a period of 2 h at 25-30°C. The progress of the reaction was monitored by TLC.

After completion of the reaction, the reaction mixture was quenched by addition of 1.5 N HCl solution to pH ~2-3. The reaction mixture was concentrated in vacuum at 45°C and stripped with toluene to afford crude compound E as a white solid.

Yield: 50 mg (crude):
¹H-NMR- DMSO-*d*₆ (300 MHz): δ: 4.16 (3H, s), 7.55 (1H, s), 7.73-7.77 (1H, m), 8.56 (1H, d, J= 8.4 Hz), 8.67 (1H, d, J= 5.1 Hz), 9.18 (1H, d, J=2.0 Hz), 13.65 (1H, bs);
LCMS: 204.0 [M+H] ⁺

### Step 4: Synthesis of ethyl (E)-5,5-dimethyl-2-[1-methyl-3-(3-pyridyl)-5-pyrazolylcarbonylamino]-3-hexenoate (G)

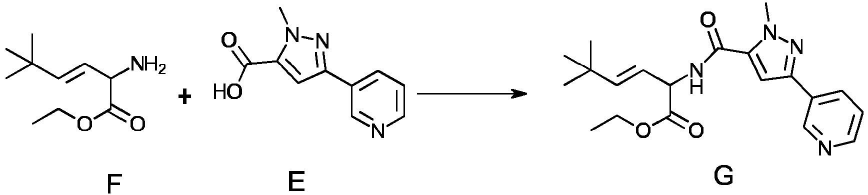

Compound E (54.65 mg, 0.26 mmol) was added to a stirred solution of (E)-2-amino-5,5-dimethyl-3-hecenoate (F) (50 mg, 0.26 mmol) in DMF (2.5 mL) followed by DIPEA (0.18 mL, 1.07 mmol) and propylphosphonic anhydride (T₃P, ~50% solution in ethyl acetate) (0.51 mL, 0.80 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted to ethyl acetate (2 × 20 mL). the combined organic layer was washed with saturated sodium chloride solution (10 mL), dried over anhydrous sodium sulphate and concentrated in vacuo at 35°C to afford crude material. The crude material was purified by CombiFlash^{®} using ethyl acetate: hexanes as eluent to afford compound G as a white solid.
Yield: 15 mg, (7.5 %);
H-NMR- DMSO-*d*₆ (400 MHz): δ: 1.01 (9H, s), 1.15 - 1.17 (3H, m), 4.08 (3H, s), 4.11- 4.16 (2H, m), 4.86- 4.90 (1H, m), 5.47 - 5.51 (1H, m), 5.86 - 5.90 (1H, m), 7.40 - 7.45 (2H, m), 8.12 (1H, d, J= 8.0 Hz), 8.53 (1H, d, *J* =2 Hz), 8.96 (1H, s), 9.00 - 9.02 (1H, m),;
LCMS: 383.3 [M+H]⁺

### Step 5: Synthesis of (E)-5,5-dimethyl-2-[1-methyl-3-(3-pyridyl)-5-pyrazolylcarbonylamino]-3-hexenoic acid (H)

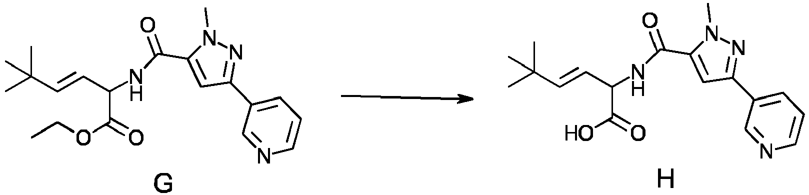

Lithium hydroxide monohydrate (8.58 mg, 0.20 mmol) was added to a stirred solution of compound G (15 mg, 0.040 mmol) in THF/methanol/water (0.45 mL, 1:1:1, 0.15 mL each). The resulting reaction mixture was stirred over a period of 18 h at 25-30°C. The progress of the reaction was monitored by TLC.

After 18 h, the reaction mixture was concentrated, the resulting residue was dissolved in water and washed with diethyl ether (10 mL). The aqueous layer was acidified to pH ~ 5-6 using 1.5 N HCl and the product was extracted with ethyl acetate (3 × 5 mL). The combined organic layer was dried over sodium sulphate and concentrated at 40°C to afford a crude solid. The obtained solid was washed with hexane (5 mL) to afford a crude mixture of two isomers as an off white solid. The crude mixture was purified by RP-HPLC to afford pure product H, as a white solid.
Yield: 7.1 mg;
¹H-NMR- DMSO-*d*₆ (400 MHz): δ: 0.99 (9H, s), 4.08 (3H, s), 4.77 -4.81 (1H, m), 5.50-5.55 (1H, m), 5.79 (1H, d, J=16 Hz), 8.11 - 8.13 (1H, m), 8.14 (1H, s), 8.51 - 8.53 (1H, m), 8.71 (1H, bs), 8.97- 8.99 (1H, m), 12.87 (1H, bs);
LCMS: 343.2 [M+H] ⁺; HPLC purity - 99.4 %.

### EXAMPLE 36 - Synthesis of (E)-2-[5-(3,4-dichlorophenyl)-1-methyl-3-pyrazolylcarbonylaminol-5,5-dimethyl-3-hexenoic acid (SI61)

### Step 1: Synthesis of methyl 5-(3,4-dichlorophenyl)-1-methyl-3-pyrazolecarboxylate (B)

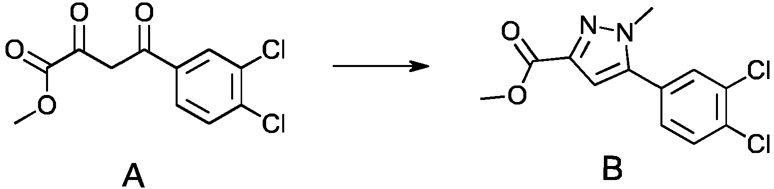

Methyl hydrazine (50.0 mg, 1.01 mmol) was added to a stirred solution of methyl 4-(3,4-dichlorophenyl)-2,4-dioxobutyrate (A) (300 mg, 1.01 mmol) in MeOH (3.0 mL) at 25-30°C. The resulting reaction mixture was heated at 70°C for 2 h and allowed to stir at 25-30°C over a period 18 h. The progress of the reaction was monitored by TLC

After completion of the reaction, the reaction mixture was evaporated to afford crude material containing two isomeric products. The crude material was purified by CombiFlash^{®} using ethyl acetate : hexanes as eluent to afford compound B as an off white solid.
Yield: 0.11 g;
Int-2.2: ¹H-NMR- DMSO-*d*₆ (400 MHz): δ: 3.78 (3H, s), 3.92 (3H, s), 7.00 (1H, s), 7.58 (1H, d, J=2.0 Hz), 7.76 (1H, d, *J*=8.4 Hz), 7.89 (1H, s);
LCMS: 284.93 [M+H]⁺

### Step 2: Synthesis of 5-(3,4-dichlorophenyl)-1-methyl-3-pyrazolecarboxylic acid (C)

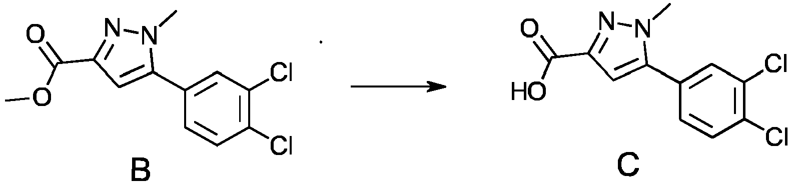

Sodium hydroxide (61.72 mg, 1.54 mmol) was added to a stirred solution of compound B (2.2) (110 mg, 0.38 mmol) in MeOH: water (8.8 mL, 1:1, 4.4 mL each). The resulting reaction mixture was stirred over a period of 4 h at 75°C. The progress of the reaction was monitored by TLC.

After completion of the reaction, most of the methanol was evaporated. The aqueous mixture was acidified to pH ~ 3-4 using 1.5 N HCl and the product was extracted with ethyl acetate (3 × 10 mL). The combined organic layer was dried over sodium sulphate and concentrated at 40°C to afford a crude solid. The solid was washed with hexane (15 mL) to afford crude compound C as a white solid. The crude material was used as such for next reaction.
Yield: 99 mg (crude);
¹H-NMR- DMSO-*d*₆ (400 MHz): δ: 3.90 (3H, s), 6.92 (1H, s), 7.56 (1H, d, J=2.0 Hz), 7.75 (1H, d, J=8.4 Hz), 7.88 (1H, s), 12.74 (1H. bs);
LCMS: 270.91 [M+H]⁺

### Step 3: Synthesis of ethyl (E)-2-[5-(3,4-dichlorophenyl)-1-methyl-3-pyrazolylcarbonylamino]-5,5-dimethyl-3-hexenoate (E)

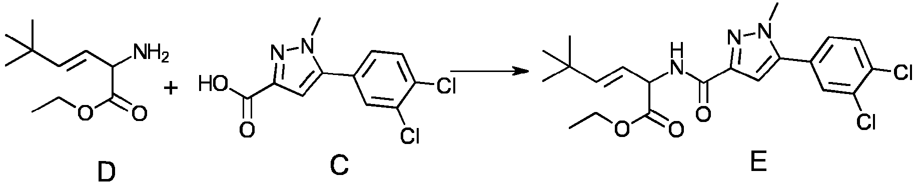

Compound C (95 mg, 0.35 mmol) was added to a stirred solution of (E)-2-amino-5,5-dimethyl-3-hecenoate (D) (64.9 mg, 0.40 mmol) in DMF (2.37 mL) followed by DIPEA (0.24 mL, 1.40 mmol) and propylphosphonic anhydride (T₃P, ~50% solution in ethyl acetate) (0.66 mL, 1.05 mmol) at 25-30°C. The resulting reaction mixture was stirred over a period of 18 h at 40°C. The progress of the reaction was monitored by TLC.

After completion of the reaction, the reaction mixture was quenched by addition of saturated NaHCO₃ solution to pH ~7-8 and the product was extracted with ethyl acetate (2 × 20 mL). The combined organic layer was washed with saturated sodium chloride solution (10 mL), dried over anhydrous sodium sulphate and concentrated in vacuo at 35°C to afford crude material. The crude material was purified by CombiFlash^{®} using ethyl acetate: hexanes as eluent to afford compound E as a brown thick oil.
Yield: 60 mg 39.0 %;
¹H-NMR- CD₃OD (300 MHz): δ: 0.99 (9H, s), 1.28 (3H, t, J=6.9 Hz), 3.96 (3H, s), 4.20-4.27 (2H, m), 5.07 (1H, d, J=6.0 Hz), 5.56-5.63 (1H, m), 5.91 (1H, d, J=15.6 Hz), 6.87 (1H, s), 7.48 (1H, d, J=1.2 Hz), 7.68 (1H, d, J=8.4 Hz), 7.75 (1H, s);
LCMS: 438.5 [M+H]⁺

### Step 4: Synthesis of (E)-2-[5-(3,4-dichlorophenyl)-1-methyl-3-pyrazolylcarbonylamino]-5,5-dimethyl-3-hexenoic acid (F)

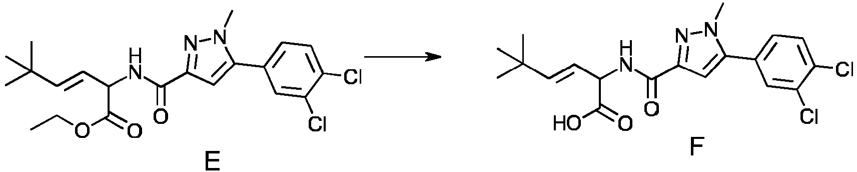

Lithium hydroxide monohydrate (13.16 mg, 0.31 mmol) was added to a stirred solution of compound E (4.2) (55 mg, 0.12 mmol) in MeOH/THF/water (1.65 mL, 1:1:1, 0.55 mL each). The resulting reaction mixture was stirred over a period of 18 h at 25-30°C. The progress of the reaction was monitored by TLC.

After 18 h, the reaction mixture was concentrated, the resulting residue was dissolved in water and washed with diethyl ether (10 mL). The aqueous layer was acidified to pH ~ 4-5 using 1.5 N HCl at 0-5 °C and the product was extracted with ethyl acetate (3 × 5 mL). The combined organic layer was dried over sodium sulphate and concentrated at 40°C to afford a crude solid. The crude solid was purified by RP-HPLC to afford pure product F.as a white solid.
Yield: 42 mg (81.6 %);
¹H-NMR- DMSO-*d*₆ (400 MHz): δ: 0.99 (9H, s), 3.94 (3H, s), 4.87 (1H, t, J=6.9 Hz), 5.58 (1H, d, J=6.9 Hz, 5.77 (1H, d, J=15.6 Hz), 6.92 (1H, s), 7.60 (1H, d, J=8.1 Hz), 7.79 (1H, d, J=8.4 Hz), 7.91 (1H, s), 8.17 (1H, d, J=7.5 Hz), 12.78 (1H, bs);
LCMS: 410.3 [M+H]⁺; HPLC purity- 98.6 %.

### EXAMPLE 37 - Synthesis of S10-S12, S14-S15, SI18-SI22, SI26-SI27, S130, S133, S135, SI37-SI38, SI40, S142-S143, S145-S148, S163-S84

These compounds were prepared in the same way as described above for Example 5 starting with the coupling of ethyl (E)-2-amino-5,5-dimethyl-3-hexenoate with a suitable, commercially available aromatic, heteroaromatic or heterocyclic carboxylic acid, followed by subsequent hydrolysis of the resulting ethyl esters and giving the expected products.

### EXAMPLE 38 - Synthesis of 2-[2-(dimethylamino)-4-methyl-5-pyrimidinylcarbonylaminol-5,5-dimethylhexanoic acid (reference compound 1 (RC1))

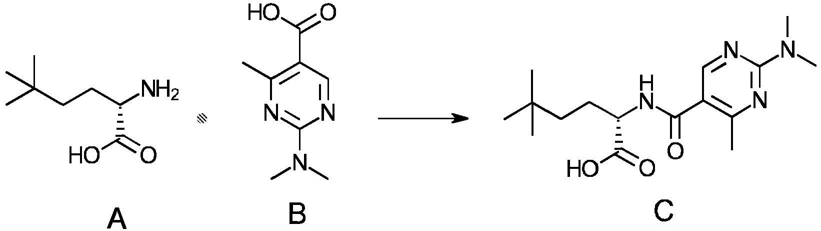

2-Hydroxypyridine-1-oxide (26 mg, 0.276 mmol) and N,N'-diisopropylcarbodiimide (35 mg, 0.276 mmol) were added to a solution of 2-(dimethylamino)-4-methyl-5-pyrimidinecarboxylic acid (B) (50 mg, 0.276 mmol) in DMSO (1 mL) and the reaction mixture was stirred for 10 min at 0°C. A preformed solution of (S)-2-amino-5,5-dimethylhexanoic acid (A) (44 mg, 0.276 mmol) in acetonitrile : water (1:1, 0.5 mL) was added and the mixture was stirred at room temperature for 4 h. The crude material was purified by reverse phase HPLC (C18 column, gradient of 5-100% MeCN/H₂O containing 0.05% HCOOH). The solvents were evaporated by freeze drying to provide pure compound C.
Yield: 6.0 mg (6.7 %)
¹H NMR (400 MHz, DMSO-*d*₆) δ: 8.35 (s, 1H), 8.30 (d, J3=7.7 Hz, 1H), 4.25 - 4.13 (m, 1H), 3.14 (s, 6H), 2.41 (s, 3H), 1.82 - 1.57 (m, 2H), 1.34 - 1.16 (m, 2H), 0.86 (s, 9H).

### EXAMPLE 39 - Synthesis of (S)-5,5-dimethyl-2-(6-phenoxynicotinoylamino)hexanoic acid (RC2)

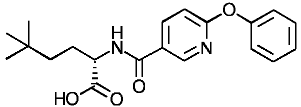

This compound was prepared as described in the Supplementary data for the paper of Shawn J Stachel et al., Bioorganic & Medicinal Chemistry Letters (2020), 30(17): 127403.

### EXAMPLE 40 Synthesis of 2-(N-6-Methyl-2-pyridylcarbamoyl)-5-(trifluoromethyl)benzoic acid (RC3)

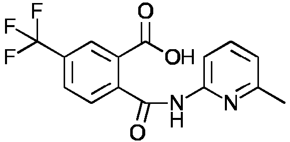

This compound was prepared as described in WO 2014/114779

### EXAMPLE 41 - Binding of compounds to sortilin using neurotensin competitive fluorescence polarization binding assay (FPA)

### Material and Methods

### Synthesis of secreted sortilin (sSORT) and Neurotensin

The extracellular part of human sortilin (NCBI reference sequence: NM_002959.7), amino acids 1-756 in SEQ ID NO: 1 plus a C-terminal His6-tag, was produced in CHO-S cells by transient transfection as a secreted protein. Supernatant from two different transfection reagents were pooled; 150 ml FectoPro and 150 ml NovaCHOice. Purification was performed by using Immobilized Metal Ion Affinity Chromatography (IMAC) in buffer (50 mM HEPES pH 7.4, 100 mM NaCl and 2 mM CaCl₂). Proteins were eluted using an imidazole-gradient (125-500 mM), fractions containing sSORT were pooled and protein size was confirmed by Western blot. Buffer was exchanged to 50 mM HEPES, pH 7.4; 100 mM NaCl; 2.0 mM CaCl₂ prior to storage in -80°C. Neurotensin, amino acid sequence LYENKPRRPYIL, SEQ ID NO: 4, (Genescript), and Neurotensin-Ahx-FITC containing the same sequence with an additional N-terminal modification FITC-Ahx (Genescript) were used as competitive ligand for sSORT.

### Fluorescence polarization assay conditions

Fresh 0.1% bovine serum albumin (BSA) was added to the assay buffer to obtain the final concentration: 50 mM HEPES, pH 7.4; 100 mM NaCl; 2.0 mM CaCl₂; 0.1% BSA; 0.1% TWEEN^{®} 20. The sortilin inhibitors of the invention and reference compounds (see Table 1) analyzed in the screen and neurotensin (used as an assay control) were serially diluted in ten different concentrations in assay buffer. In each well of a Nunc^{®} MaxiSorp^{™} 384-well plate (Sigma Aldrich), 100 nM sSORT was mixed with pre-diluted sortilin inhibitors and 10 nM Neurotensin-Ahx-FITC in assay buffer adjusted to contain 1% DMSO in a final volume of 20 µl. The plate was then briefly centrifuged prior to 1 h incubation at room temperature in the dark. The mPolarization values were obtained from a CLARIOstar plate reader (excitation at 482 nm and emission at 530-540 nm) with each well flashed 200 times. The Z' value was calculated from a total of 16 positive controls and 16 negative controls.

### Results

To demonstrate binding of sortilin inhibitors to sortilin, a competitive binding assay was performed for sortilin using a fluorescence polarization-based (FPA) method with neurotensin as competitor ligand (Figs. 1A-1S). Mean IC₅₀ values are shown in Table 1. All compounds in Table 1, except SI1, SI5 and RC2, are only tested as racemates.

**Table 1 - mean IC₅₀ values for sortilin inhibitors**

| **Sortilin inhibitor** | **Diastereomer No** | **IC₅₀ (nM)** |
|---|---|---|
| SI1 (racemate) | - | 59 |
| SI1 (active enantiomer) | - | 9 |
| SI2 | - | 234 |
| SI3 | 1 | 135 |
| SI3 | 2 | 339 |
| SI4 | 1 | 229 |
| SI4 | 2 | 912 |
| SI5 (racemate) | - | 17 |
| SI (active enantiomer) | - | 5 |
| SI6 | - | 62 |
| SI7 | - | 89 |
| SI8 | - | 43 |
| SI9 | - | 102 |
| SI17 | - | 149 |
| SI19 | - | 851 |
| SI20 | - | 1000 |
| SI23 | - | 158 |
| SI24 | - | 52 |
| SI25 | - | 31 |
| SI28 | - | 55 |
| SI29 | - | 80 |
| SI31 | - | 71 |
| SI32 | - | 52 |
| SI34 | - | 56 |
| SI39 | - | 65 |
| SI44 | - | 158 |
| SI49 | | 68 |
| SI50 | | 668 |
| SI51 | - | 80 |
| SI52 | - | 31 |
| SI53 | - | 148 |
| SI54 | - | 158 |
| SI55 | - | 116 |
| SI56 | - | 95 |
| SI57 | - | 45 |
| SI58 | - | 141 |
| SI59 | - | 316 |
| SI61 | - | 117 |
| SI62 | - | 61 |
| RC1 | - | 123 |
| RC2 (active enantiomer) | - | 28 |
| RC3 | - | 191 |

As can be seen by comparing sortilin inhibitor SI5 of the invention with reference compound RC2 and sortilin inhibitor SI1 of the invention with reference compound RC1, the sortilin inhibitors of the invention having an unsaturated aliphatic moiety instead of a saturated aliphatic moiety of the reference compounds had lower mean IC₅₀ values and thereby a higher binding affinity to sortilin. This is even more accentuated by comparing the pure enantiomer SI5 with the pure enantiomer RC2.

### EXAMPLE 42 - Reducing the progranulin induced secondary sphere formation as well as baseline sphere formation in MDA-MB-231 breast cancer cell line, HT-29 colon cancer cell line and SK-MEL-30 melanoma cell line

### Material and Methods

### Peptide treatments

C-terminal Progranulin (progranulin) (Caslo, peptide sequence: EAPRWDAPLRDPALRQL, SEQ ID NO: 5) were reconstituted in sterile PBS upon delivery, aliquoted and stored at -20°C. Working stock concentrations were achieved by dilution in culture media. The MDA-MB-231, HT-29 or SK-MEL-30 cell lines were treated with or without 500 nM progranulin with and without indicated concentrations of sortilin inhibitors SI1, SI5, SI8, SI25, SI32, SI39, SI51 and SI62 of the invention or RC3 (AF38469, Bioorganic & Medicinal Chemistry Letters, 2014, 24(1): 177-180) for 48 hours at 37°C 5% CO₂ and 21% O₂ before performing the primary followed by secondary sphere formation assay.

### Secondary sphere formation

The sphere formation assay was performed as described previously *(*Mammary Gland Biol Neoplasia, 2012, 17(2): 111-117). Briefly, single cell suspensions were obtained following treatment with respective sortilin inhibitors SI1, S15, S18, SI25, SI32, SI39, SI51 and SI62 of the invention and seeded in phenol red-free DMEM/F-12 (Gibco^{®}, Life Technologies), supplemented with 1% B27 supplement (Fisher Scientific, Invitrogen), 1% P/S and 20 ng/ml EGF (BD Biosciences) onto non-adherent polyhema-coated plates. After cultivation for five days, single cell suspensions were obtained from the primary spheres and seeded again in phenol red-free DMEM/F-12 supplemented with 1% B27 supplement, 1% P/S and 20 ng/ml EGF onto non-adherent polyhema-coated plates for 5-7 days. Subsequently, spheres greater than 50 µm in diameter were counted manually in the microscope.

### Results

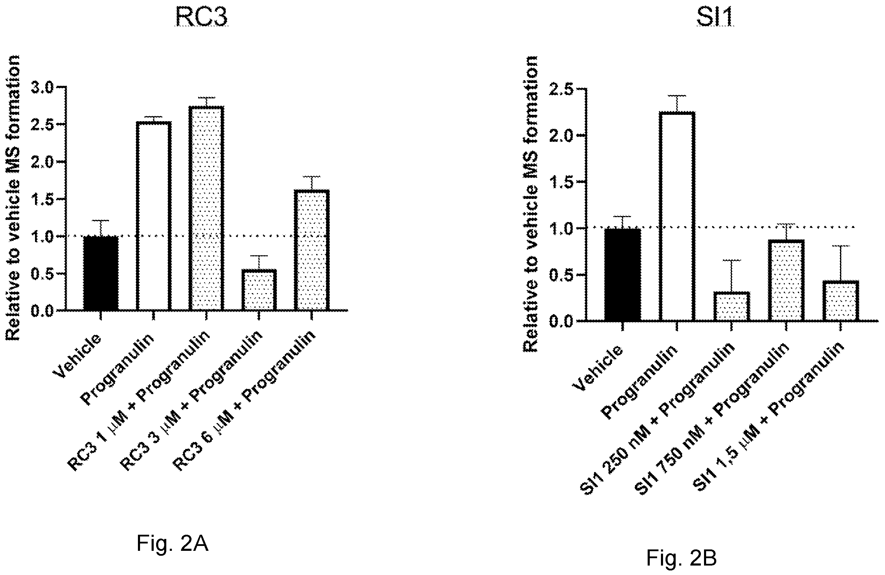

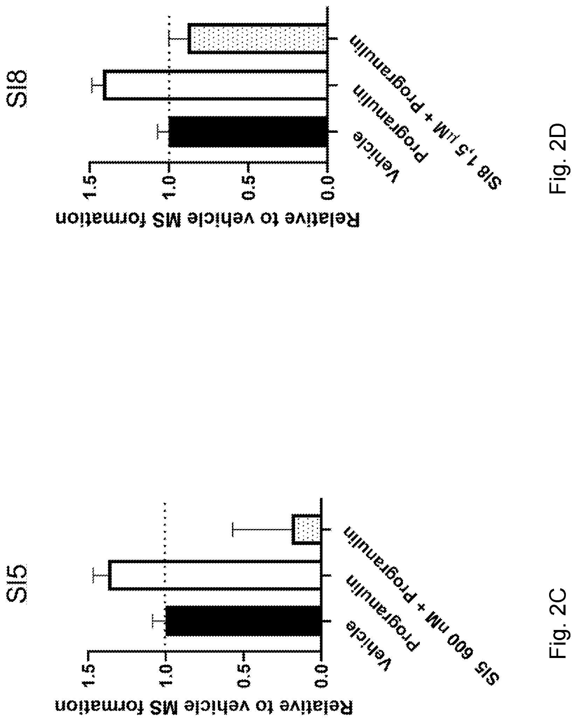

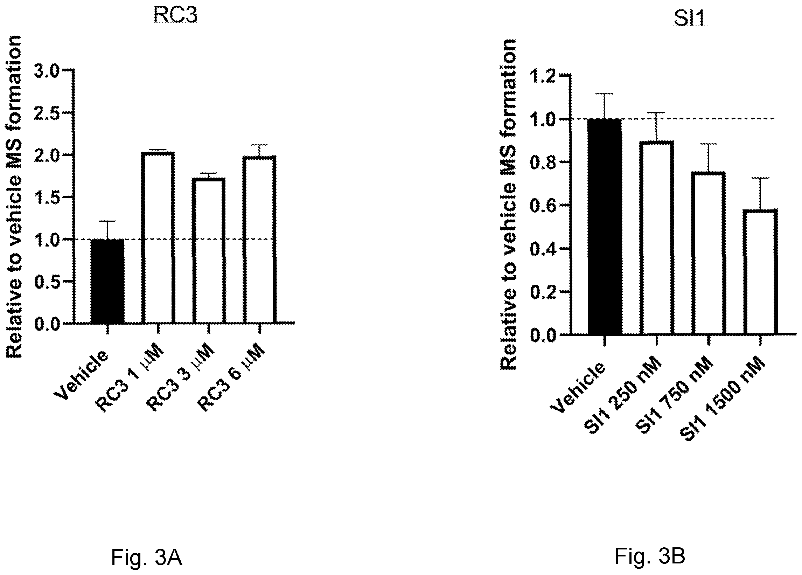

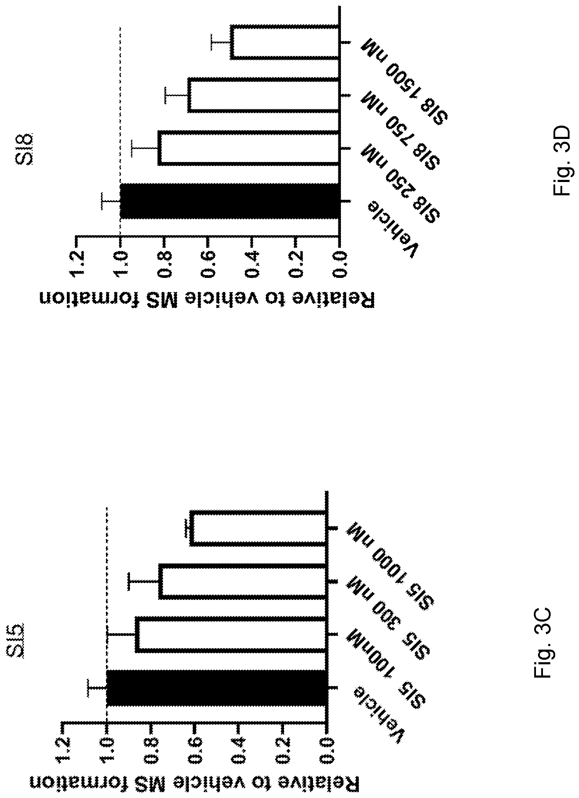

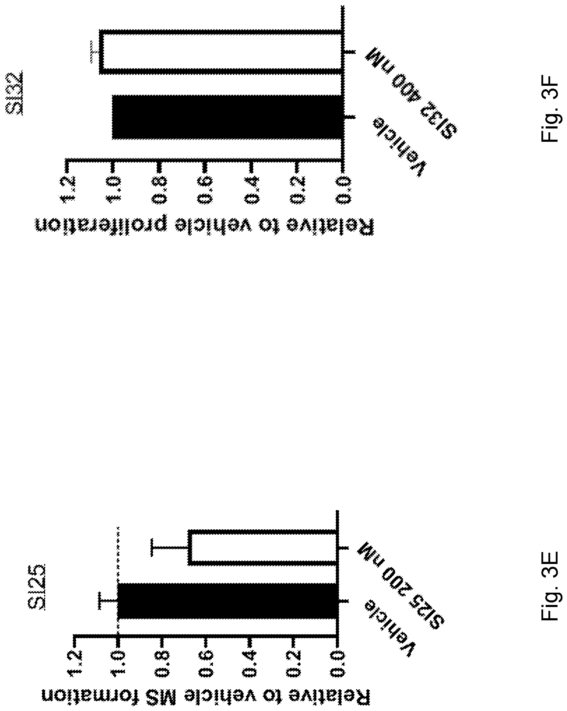

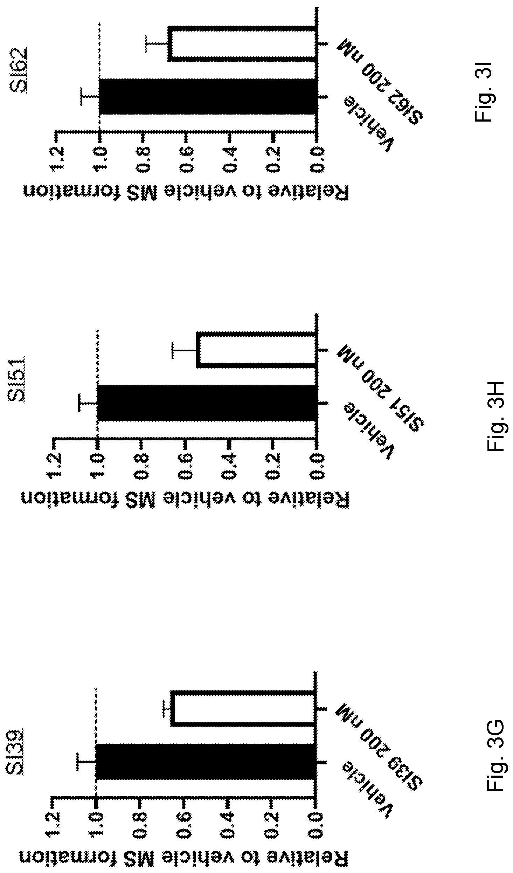

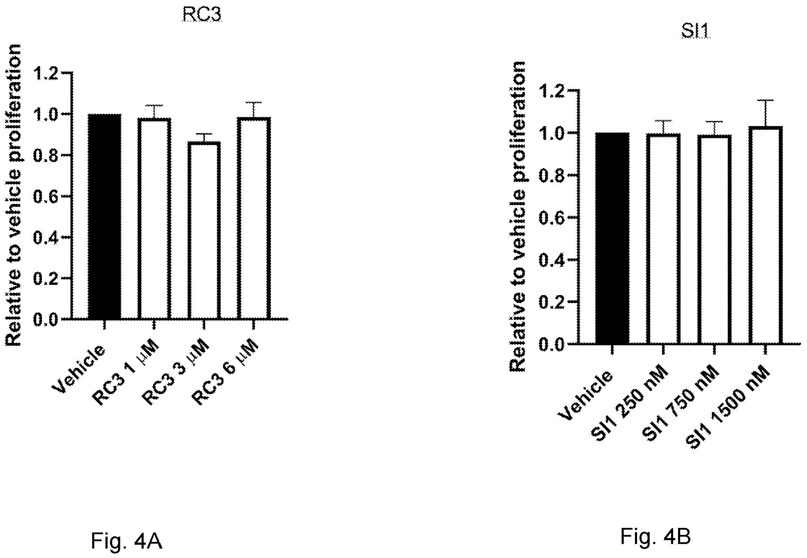

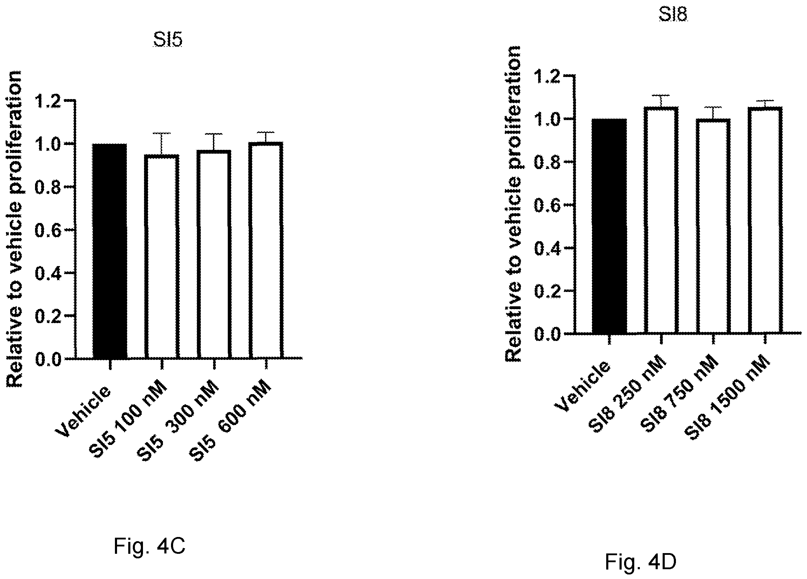

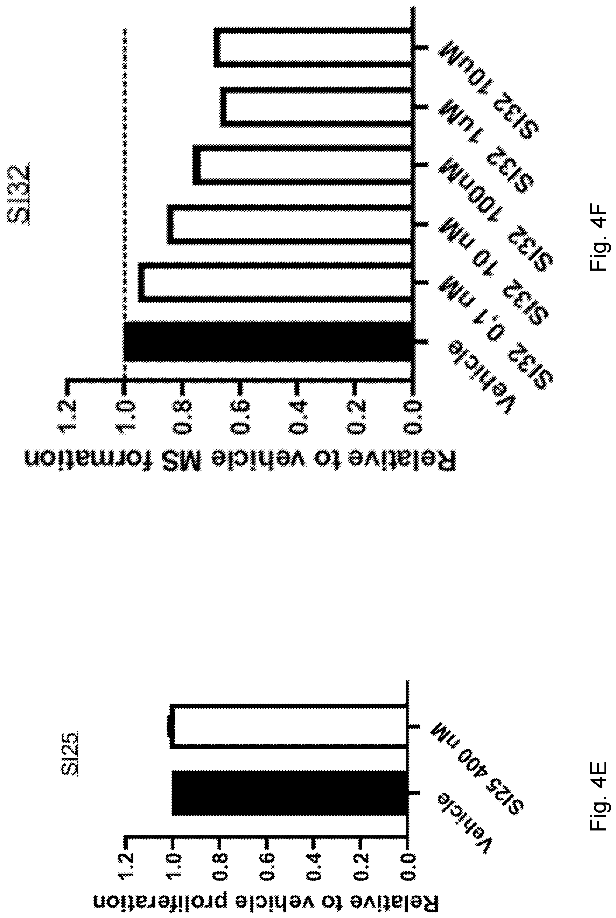

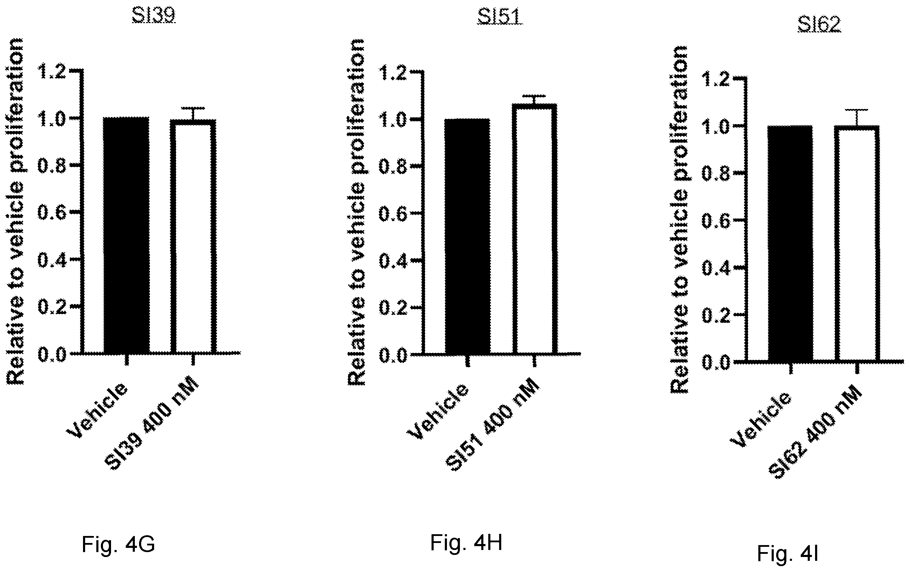

To determine whether the increase in sphere formation triggered by progranulin could be blocked using sortilin inhibitors SI1, SI5 and SI8 of the invention, the triple negative breast cancer cell line MDA-MB-231 was treated with progranulin, alone or in combination with the sortilin inhibitors SI1, SI5 and SI8 of the invention, or the published sortilin binding small molecule RC3 *(*Breast Cancer Research 2018 20: 137). Results show that as expected, progranulin increased secondary sphere formation (Figs. 2A to 2D). Further, progranulin in combination with RC3 reduced sphere formation (Fig. 2A). However, RC3 possessed agonistic properties when not used in combination with progranulin, illustrated as increased sphere formation with the use of RC3 alone (Fig. 3A). The sortilin inhibitors SI1, SI5 and SI8 of the invention demonstrated reduced sphere formation in combination with progranulin (Figs. 2B-2D) without agonistic properties (Figs. 3B-3D) when used alone. The preferred antagonistic properties were also demonstrated with SI25, SI32, SI39, SI51 and SI62 (Figs. 3E-3I). In contrast to RC3, SI1, S15, S18, SI25, SI32, SI39, SI51 and SI62 reduced baseline sphere formation suggesting potent sortilin antagonistic properties. Importantly, viability of cells were not affected by the compounds (Figs. 4A-4I).

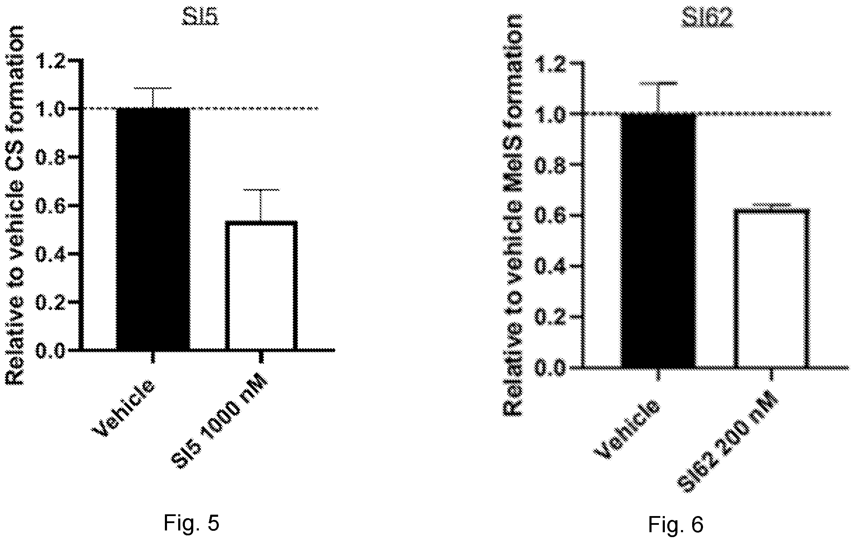

In addition to breast cancer, the sphere formation capacity using the colon cancer cell line HT-29 and melanoma cell line SK-MEL-30 were investigated. As is shown in Figs. 5 and 6, sphere formation is reduced with SI5 and SI62, respectively.

### EXAMPLE 43 - Increased metastasis formation by the reference compound RC3 and reduced metastasis formation by SI5 in MDA-MB-231 xenograft in vivo model

### Material and Methods

### In vivo studies

Luciferase tagged MDA-MB-231 cells were injected at a concentration of 0.2×10⁶ cells subcutaneously into two sites of the flank of NOD SCID gamma mice (Taconic, Denmark). Cells were prepared in a 60% mixture of matrigel (growth factor reduced, BD Bioscience) and 40% complete media. The size of the tumors were determined by calliper measurement of the subcutaneous tumor mass two times per week and tumor volume was calculated according to the formula volume = (length × (width)²/2). Assessments of metastasis were performed using the IVIS^{®} whole body imager (PerkinElmer) based on luciferase expression from stably transfected cell lines. For sortilin inhibition studies, mice were given vehicle (DMSO) or 5-50 µg RC3 (MedChem Express)/day/mouse in drinking water with a weekly refill for 21 days before assessments of metastasis were performed.

### Results

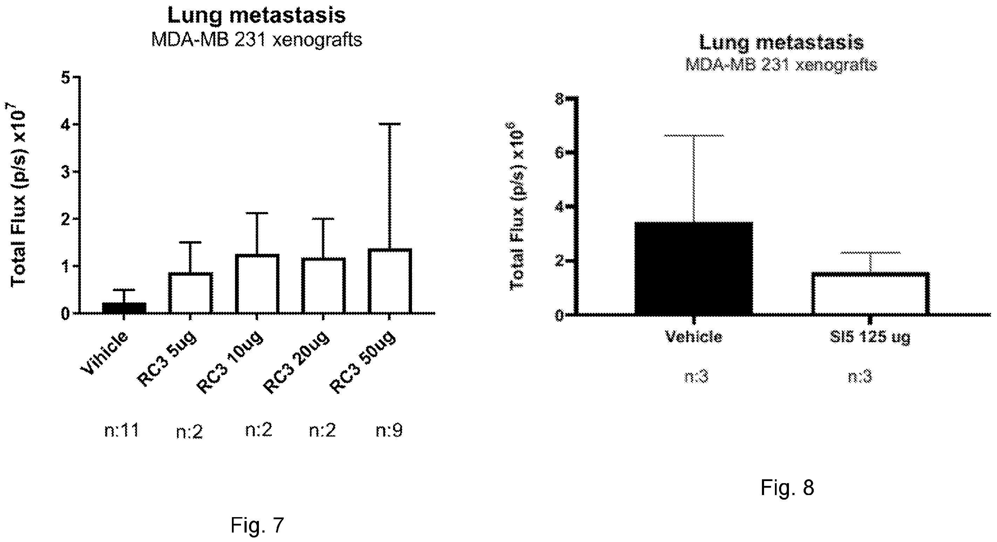

The agonistic property of RC3, observed in the sphere assay (Fig. 3A) in Example 42, was also observed *in vivo* where it induced lung metastasis in MDA-MB-231 xenograft model (Fig. 7). This should be compared to the sortilin inhibitor SI5 of the invention, which resulted in reduced metastasis formation as compared to vehicle control (Fig. 8). Furthermore, S15, and SI1 and SI8, did not induce such agonistic effect on sphere formation (Figs. 3B-3D) in Example 42.

### EXAMPLE 44 - Sequence information

SEQ ID NO: 1 
MERPWGAADGLSRWPHGLGLLLLLQLLPPSTLSQDRLDAPPPPAAPLPRWSGPIGVSWGLRAAAAGGAFPRGGRW
RRSAPGEDEECGRVRDFVAKLANNTHQHVFDDLRGSVSLSWVGDSTGVILVLTTFHVPLVIMTFGQSKLYRSEDY
GKNFKDITDLINNTFIRTEFGMAIGPENSGKVVLTAEVSGGSRGGRIFRSSDFAKNFVQTDLPFHPLTQMMYSPQ
NSDYLLALSTENGLWVSKNFGGKWEEIHKAVCLAKWGSDNTIFFTTYANGSCKADLGALELWRTSDLGKSFKTIG
VKIYSFGLGGRFLFASVMADKDTTRRIHVSTDQGDTWSMAQLPSVGQEQFYSILAANDDMVFMHVDEPGDTGFGT
IFTSDDRGIVYSKSLDRHLYTTTGGETDFTNVTSLRGVYITSVLSEDNSIQTMITFDQGGRWTHLRKPENSECDA
TAKNKNECSLHIHASYSISQKLNVPMAPLSEPNAVGIVIAHGSVGDAISVMVPDVYISDDGGYSWTKMLEGPHYY
TILDSGGIIVAIEHSSRPINVIKFSTDEGQCWQTYTFTRDPIYFTGLASEPGARSMNISIWGFTESFLTSQWVSY
TIDFKDILERNCEEKDYTIWLAHSTDPEDYEDGCILGYKEQFLRLRKSSVCQNGRDYVVTKQPSICLCSLEDFLC
DFGYYRPENDSKCVEQPELKGHDLEFCLYGREEHLTTNGYRKIPGDKCQGGVNPVREVKDLKKKCTSNFLSPEKQ
NSKSNSVPIILAIVGLMLVTVVAGVLIVKKYVCGGRFLVHRYSVLQQHAEANGVDGVDALDTASHTNKSGYHDDS
DEDLLE
SEQ ID NO: 2 
TFGQSKLYRSEDYGKNFKDITDLINNTFIRTEFGMAIGPENSGKVVLTAEVSGGSRGGRIFRSSDFAKNFVQTDL
PFHPLTQMMYSPQNSDYLLALSTENGLWVSKNFGGKWEEIHKAVCLAKWGSDNTIFFTTYANGSCKADLGALELW
 
RTSDLGKSFKTIGVKIYSFGLGGRFLFASVMADKDTTRRIHVSTDQGDTWSMAQLPSVGQEQFYSILAANDDMVF
MHVDEPGDTGFGTIFTSDDRGIVYSKSLDRHLYTTTGGETDFTNVTSLRGVYITSVLSEDNSIQTMITFDQGGRW
THLRKPENSECDATAKNKNECSLHIHASYSISQKLNVPMAPLSEPNAVGIVIAHGSVGDAISVMVPDVYISDDGG
YSWTKMLEGPHYYTILDSGGIIVAIEHSSRPINVIKFSTDEGQCWQTYTFTRDPIYFTGLASEPGARSMNISIWG
FTESFLTSQWVSYTIDFKDILERNCEEKDYTIWLAHSTDPEDYEDGCILGYKEQFLRLRKSSVCQNGRDYVVTKQ
PSICLCSLEDFLCDFGYYRPENDSKCVEQPELKGHDLEFCLYGREEHLTTNGYRKIPGDKCQGGVNPVREVKDLK
KKCTSNFLSPEKQNSKSNSVPIILAIVGLMLVTVVAGVLIVKKYVCGGRFLVHRYSVLQQHAEANGVDGVDALDT
ASHTNKSGYHDDSDEDLLE
SEQ ID NO: 3
SEQ ID NO: 4
   LYENKPRRPYIL
SEQ ID NO: 5
   EAPRWDAPLRDPALRQL

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

## Claims

1. A compound of formula I: 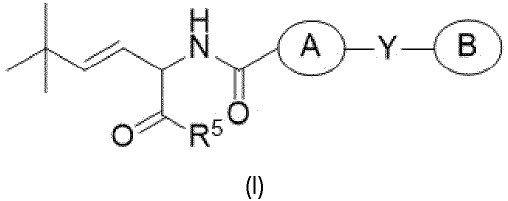 wherein
A is a 5 or 6 membered aromatic, heteroaromatic or heterocyclic ring with 0 to 2 heteroatom(s) selected among N, O and S, or a 8 to 10 membered bicyclic heterocyclic or heteroaromatic ring with 1 or 2 heteroatom(s) selected among N, O and S, the aromatic, heteroaromatic, heterocyclic or bicyclic ring A is optionally substituted with one or more substituents independently selected from the group consisting of C₁-C₄ alkyl, -NR¹R², -C(O)CZ₃, -OR⁴, halogen and =O;
Y is absent, -O-, -OCH₂-, -CH₂-, -NR³-, or -CH(NH₂)-;
B is absent or a 5 or 6 membered aromatic or heteroaromatic ring with 0 to 4 heteroatom(s) selected among N, O and S, the aromatic or heteroaromatic ring B is optionally substituted with one or more substituents independently selected from the group consisting of C₁-C₄ alkyl, -NR¹R², -C(O)CZ₃, - OR⁴, halogen and =O;
R¹, R², R³ and R⁴ are independently hydrogen or C₁-C₄ alkyl;
Z is halogen; and
R⁵ is hydroxyl or C₁-C₄ alkoxy,
or an enantiomer thereof, a diastereomer thereof, a racemate thereof, or a pharmaceutically acceptable salt thereof.

2. The compound according to claims 1, wherein A is a 5 or 6 membered aromatic, heteroaromatic or heterocyclic ring with 0 to 2 heteroatom(s) selected among N and O, preferably N, or a 9 membered bicyclic heterocyclic ring with 1 or 2 heteroatom(s) selected among N and O, preferably N, the aromatic, heteroaromatic, heterocyclic or bicyclic ring A is optionally substituted with one or more substituents independently selected from the group consisting of C₁-C₄ alkyl, -NR¹R², -C(O)CZ₃, -OR⁴, halogen, and =O.

3. The compound according to claim 1 or 2, wherein the aromatic, heteroaromatic, heterocyclic or bicyclic ring A is optionally substituted with one or more substituents independently selected from the group consisting of -CH₃, -CH₂CH(CH₃)₂, -C(CH₃)₃, -N(CH₃)₂, C(O)CZ₃, and halogen, preferably independently selected from the group consisting of -CH₃, -CH₂CH(CH₃)₂, -C(CH₃)₃, -N(CH₃)₂, -C(O)CF₃, -Cl, and -Br.

4. The compound according to any of the claims 1 to 3, wherein Y is absent, -O-, -OCH₂-, -CH₂-, - NH-, or -CH(NH₂)-, preferably Y is absent or O, and more preferably Y is absent

5. The compound according to any of the claims 1 to 4, wherein B is absent or a 5 or 6 membered aromatic or heteroaromatic ring with 0 to 4 heteroatom(s) selected among N and O, preferably N, the aromatic or heteroaromatic ring B is optionally substituted with one or more substituents independently selected from the group consisting of -OR⁴ and =O, preferably independently selected from the group consisting of hydroxyl, methoxy and =O.

6. The compound according to any of the claims 1 to 5, wherein R⁵ is selected from the group consisting of hydroxyl, methoxy and ethoxy, preferably R⁵ is hydroxyl.

7. The compound according to any of the claims 1 to 6, wherein the compound is selected from the group consisting of:
(*E*)-2-[2-(dimethylamino)-4-methyl-5-pyrimidinylcarbonylamino]-5,5-dimethyl-3-hexenoic acid;
(*E*)-5,5-dimethyl-2-[*p*-(2,2,2-trifluoroacetyl)benzoylamino]-3-hexenoic acid;
(*E*)-5,5-dimethyl-2-[4-(2-pyridyl)-2-piperazinylcarbonylamino]-3-hexenoic acid;
(*E*)-5,5-dimethyl-2-[4-(4-pyridyl)-2-piperazinylcarbonylamino]-3-hexenoic acid;
(*E*)-5,5-dimethyl-2-(6-phenoxynicotinoylamino)-3-hexenoic acid;
(*E*)-5,5-dimethyl-2-[*m*-(4*H*-1,2,4-triazol-4-yl)benzoylamino]-3-hexenoic acid;
(*E*)-5,5-dimethyl-2-(6-methyl-1*H*-1,5-diazainden-2-ylcarbonylamino)-3-hexenoic acid;
(*E*)-2-(4-chloro-2-pyrrolylcarbonylamino)-5,5-dimethyl-3-hexenoic acid;
(*E*)-2-(3-isobutyl-1-methyl-5-pyrazolylcarbonylamino)-5,5-dimethyl-3-hexenoic acid;
(*E*)-5,5-dimethyl-2-[4-(2-pyridyl)-2-pyrrolylcarbonylamino]-3-hexenoic acid;
(*E*)-5,5-dimethyl-2-[4-(4-pyridyl)-2-pyrrolylcarbonylamino]-3-hexenoic acid;
(*E*)-5,5-dimethyl-2-[4-(3-pyridyl)-2-pyrrolylcarbonylamino]-3-hexenoic acid;
(*E*)-2-{4-[amino(2-pyridyl)methyl]-2-pyrrolylcarbonylamino}-5,5-dimethyl-3-hexenoic acid;
(*E*)-2-(3-isobutyl-5-isoxazolylcarbonylamino)-5,5-dimethyl-3-hexenoic acid;
(*E*)-5,5-dimethyl-2-[1-(4-pyridyl)-4-pyrazolylcarbonylamino]-3-hexenoic acid;
(*E*)-5,5-dimethyl-2-[1-(2-oxo-4-pyridyl)-4-pyrazolylcarbonylamino]-3-hexenoic acid;
(*E*)-2-[2-(dimethylamino)-5-pyrimidinylcarbonylamino]-5,5-dimethyl-3-hexenoic acid;
(*E*)-2-(2-isopropyl-5-pyrimidinylcarbonylamino)-5,5-dimethyl-3-hexenoic acid;
(*E*)-2-[2-(dimethylamino)-4-pyrimidinylcarbonylamino]-5,5-dimethyl-3-hexenoic acid;
(*E*)-2-[2-(dimethylamino)-6-methyl-4-pyrimidinylcarbonylamino]-5,5-dimethyl-3-hexenoic acid;
(*E*)-2-[2-(diethylamino)-6-methyl-4-pyrimidinylcarbonylamino]-5,5-dimethyl-3-hexenoic acid;
(*E*)-5,5-dimethyl-2-[*p*-(1*H*-1,2,4-triazol-1-yl)benzoylamino]-3-hexenoic acid;
(*E*)-5,5-dimethyl-2-[*p*-(4*H*-1,2,4-triazol-4-yl)benzoylamino]-3-hexenoic acid;
(E)-5,5-dimethyl-2-[m-(1H-1,2,4-triazol-1-yl)benzoylamino]-3-hexenoic acid;
(*E*)-5,5-dimethyl-2[*m*-(1-imidazolyl)benzoylamino]--3-hexenoic acid;
(E)-5,5-dimethyl-2-[m-(1H-tetraazol-1-yl)benzoylamino]-3-hexenoic acid;
(*E*)-5,5-dimethyl-2-[2-(4*H*-1,2,4-triazol-4-yl)isonicotinoylamino]-3-hexenoic acid;
(*E*)-2-(4-bromo-2-pyrrolylcarbonylamino)-5,5-dimethyl-3-hexenoic acid;
(*E*)-2-(5-chloro-2-pyrrolylcarbonylamino)-5,5-dimethyl-3-hexenoic acid;
(*E*)-2-(4,5-dichloro-2-pyrrolylcarbonylamino)-5,5-dimethyl-3-hexenoic acid;
(*E*)-2-(4-chloro-1-methyl-2-pyrrolylcarbonylamino)-5,5-dimethyl-3-hexenoic acid;
(*E*)-2-(4-chloro-2-thienylcarbonylamino)-5,5-dimethyl-3-hexenoic acid;
(*E*)-2-(5-isopropyl-3-pyrazolylcarbonylamino)-5,5-dimethyl-3-hexenoic acid;
(*E*)-5,5-dimethyl-2-[*p*-(3-pyridyloxy)benzoylamino]-3-hexenoic acid;
(*E*)-5,5-dimethyl-2-(5-phenoxynicotinoylamino)-3-hexenoic acid;
(*E*)-5,5-dimethyl-2-(2-phenoxyisonicotinoylamino)-3-hexenoic acid;
(*E*)-5,5-dimethyl-2-[*p*-(2-pyridyloxy)benzoylamino]-3-hexenoic acid;
(*E*)-5,5-dimethyl-2-[*m*-(2-pyridyloxy)benzoylamino]-3-hexenoic acid;
(*E*)-2-[6-(benzyloxy)nicotinoylamino]-5,5-dimethyl-3-hexenoic acid;
(*E*)-5,5-dimethyl-2-[*p*-(6-methyl-2-pyrazinyloxy)benzoylamino]-3-hexenoic acid;
(*E*)-5,5-dimethyl-2-[*p*-(2-pyrimidinyloxy)benzoylamino]-3-hexenoic acid;
(*E*)-2-[*m*-(5-chloro-2-pyridyloxy)benzoylamino]-5,5-dimethyl-3-hexenoic acid;
(*E*)-2-[6-(*p*-bromophenoxy)nicotinoylamino]-5,5-dimethyl-3-hexenoic acid;
(*E*)-5,5-dimethyl-2-(2-phenoxy-5-pyrimidinylcarbonylamino)-3-hexenoic acid;
(*E*)-2-{6-[*p*-(*tert*-Butyl)phenoxy]nicotinoylamino}-5,5-dimethyl-3-hexenoic acid;
(*E*)-2-[2-(*p*-cumenyloxy)isonicotinoylamino]-5,5-dimethyl-3-hexenoic acid;
(*E*)-5,5-Dimethyl-2-(5-phenoxy-2-pyrazinylcarbonylamino)-3-hexenoic acid;
(*E*)-5,5-dimethyl-2-[*p*-(5-methyl-2-pyridyloxy)benzoylamino]-3-hexenoic acid;
(*E*)-2-(2-isopropyl-5-pyrimidinylcarbonylamino)-5,5-dimethyl-3-hexenoic acid;
(E)-5,5-dimethyl-2-[6-(4*H*-1,2,4-triazol-4-yl)-2-pyridylcarbonylamino]-3-hexenoic acid;
(*E*)-5,5-dimethyl-2-[*p*-(2-thienyl)benzoylamino]-3-hexenoic acid;
(*E*)-5,5-dimethyl-2-[*m*-(2-thienyl)benzoylamino]-3-hexenoic acid;
(*E*)-2-[*p*-(*p*-chlorophenoxy)benzoylamino]-5,5-dimethyl-3-hexenoic acid;
(*E*)-2-[*p*-(*m*-chlorophenoxy)benzoylamino]-5,5-dimethyl-3-hexenoic acid;
(*E*)-5,5-dimethyl-2-[*p*-(phenoxymethyl)benzoylamino]-3-hexenoic acid;
(*E*)-2-(1-ethyl-3-isobutyl-5-pyrazolylcarbonylamino)-5,5-dimethyl-3-hexenoic acid;
(*E*)-2-(3-chloro-1-methyl-5-pyrazolylcarbonylamino)-5,5-dimethyl-3-hexenoic acid;
(*E*)-2-(3-isopropyl-1-methyl-5-pyrazolylcarbonylamino)-5,5-dimethyl-3-hexenoic acid;
(*E*)-5,5-dimethyl-2-[1-methyl-3-(3-pyridyl)-5-pyrazolylcarbonylamino]-3-hexenoic acid;
(*E*)-2-[3-(3,4-dichlorophenyl)-1-methyl-5-pyrazolylcarbonylamino]-5,5-dimethyl-3-hexenoic acid;
(*E*)-2-[5-(3,4-dichlorophenyl)-1-methyl-3-pyrazolylcarbonylamino]-5,5-dimethyl-3-hexenoic acid;
(*E*)-2-(4,5-dichloro-2-thienylamino)-5,5-dimethyl-3-hexenoic acid;
(*E*)-2-(2-Benzyl-4-methyl-5-pyrimidinylcarbonylamino)-5,5-dimethyl-3-hexenoic acid;
(*E*)-5,5-dimethyl-2-[4-methyl-2-(*p*-toluidino)-5-pyrimidinylcarbonylamino]-3-hexenoic acid;
(*E*)-2-[2-(p-chlorophenylamino)-4-methyl-5-pyrimidinylcarbonylamino]-5,5-dimethyl-3-hexenoic acid;
(*E*)-2-[6-(*p*-chlorophenoxy)nicotinoylamino]-5,5-dimethyl-3-hexenoic acid;
(*E*)-5,5-dimethyl-2-[6-(*p*-tolyloxy)nicotinoylamino]-3-hexenoic acid;
(*E*)-2-(6-benzylnicotinoylamino)-5,5-dimethyl-3-hexenoic acid;
(*E*)-2-(*p*-benzylbenzoylamino)-5,5-dimethyl-3-hexenoic acid;
(*E*)-5,5-dimethyl-2-(p-phenoxybenzoylamino)-3-hexenoic acid;
(*E*)-5,5-dimethyl-2-(6-phenylnicotinoylamino)-3-hexenoic acid;
(*E*)-5,5-dimethyl-2-(2-phenylisonicotinoylamino)-3-hexenoic acid;
(*E*)-5,5-dimethyl-2-[6-(2-thienyloxy)nicotinoylamino]-3-hexenoic acid;
(*E*)-2-(5-chloro-1-benzothiophen-2-ylcarbonylamino)-5,5-dimethyl-3-hexenoic acid;
(*E*)-2-(5-chloro-2-indolylcarbonylamino)-5,5-dimethyl-3-hexenoic acid;
(*E*)-5,5-dimethyl-2-(1-thia-5-aza-2-indenylcarbonylamino)-3-hexenoic acid;
(*E*)-2-(2-chloro-1-benzothiophen-6-ylcarbonylamino)-5,5-dimethyl-3-hexenoic acid;
(*E*)-2-(2-chloro-6-indolylcarbonylamino)-5,5-dimethyl-3-hexenoic acid;
(*E*)-2-(4-chloro-1,3-thiazol-2-ylcarbonylamino)-5,5-dimethyl-3-hexenoic acid;
(*E*)-2-(4,5-dichloro-1,3-thiazol-2-ylcarbonylamino)-5,5-dimethyl-3-hexenoic acid;
(*E*)-2-(3-chloro-5-isothiazolylcarbonylamino)-5,5-dimethyl-3-hexenoic acid;
(*E*)-2-(3,4-dichloro-5-isothiazolylcarbonylamino)-5,5-dimethyl-3-hexenoic acid;
(*E*)-5,5-dimethyl-2-[*m*-(3-pyridyl)benzoylamino]-3-hexenoic acid;
(*E*)-2-[m-(6-chloro-2-methyl-3-pyridyl)benzoylamino]-5,5-dimethyl-3-hexenoic acid;
or an enantiomer thereof, a diastereomer thereof, a racemate thereof, or a pharmaceutically acceptable salt thereof, preferably selected from the group consisting of:
(*E*)-2-[2-(dimethylamino)-4-methyl-5-pyrimidinylcarbonylamino]-5,5-dimethyl-3-hexenoic acid;
(*E*)-5,5-dimethyl-2-[*p*-(2,2,2-trifluoroacetyl)benzoylamino]-3-hexenoic acid;
(*E*)-5,5-dimethyl-2-[4-(2-pyridyl)-2-piperazinylcarbonylamino]-3-hexenoic acid;
(*E*)-5,5-dimethyl-2-[4-(4-pyridyl)-2-piperazinylcarbonylamino]-3-hexenoic acid;
(*E*)-5,5-dimethyl-2-(6-phenoxynicotinoylamino)-3-hexenoic acid;
(*E*)-5,5-dimethyl-2-[m-(4*H*-1,2,4-triazol-4-yl)benzoylamino]-3-hexenoic acid;
(*E*)-5,5-dimethyl-2-(6-methyl-1*H*-1,5-diazainden-2-ylcarbonylamino)-3-hexenoic acid;
(*E*)-2-(4-chloro-2-pyrrolylcarbonylamino)-5,5-dimethyl-3-hexenoic acid;
(*E*)-2-(3-isobutyl-1-methyl-5-pyrazolylcarbonylamino)-5,5-dimethyl-3-hexenoic acid;
(*E*)-5,5-dimethyl-2-[4-(2-pyridyl)-2-pyrrolylcarbonylamino]-3-hexenoic acid;
(*E*)-5,5-dimethyl-2-[4-(4-pyridyl)-2-pyrrolylcarbonylamino]-3-hexenoic acid;
(*E*)-5,5-dimethyl-2-[4-(3-pyridyl)-2-pyrrolylcarbonylamino]-3-hexenoic acid;
(*E*)-2-{4-[amino(2-pyridyl)methyl]-2-pyrrolylcarbonylamino}-5,5-dimethyl-3-hexenoic acid;
(*E*)-2-(3-isobutyl-5-isoxazolylcarbonylamino)-5,5-dimethyl-3-hexenoic acid;
(*E*)-5,5-dimethyl-2-[1-(4-pyridyl)-4-pyrazolylcarbonylamino]-3-hexenoic acid;
(*E*)-5,5-dimethyl-2-[1-(2-oxo-4-pyridyl)-4-pyrazolylcarbonylamino]-3-hexenoic acid;
(*E*)-2-[2-(dimethylamino)-5-pyrimidinylcarbonylamino]-5,5-dimethyl-3-hexenoic acid;
(*E*)-2-(2-isopropyl-5-pyrimidinylcarbonylamino)-5,5-dimethyl-3-hexenoic acid;
(*E*)-2-[2-(dimethylamino)-4-pyrimidinylcarbonylamino]-5,5-dimethyl-3-hexenoic acid;
(*E*)-2-[2-(dimethylamino)-6-methyl-4-pyrimidinylcarbonylamino]-5,5-dimethyl-3-hexenoic acid;
(*E*)-2-[2-(diethylamino)-6-methyl-4-pyrimidinylcarbonylamino]-5,5-dimethyl-3-hexenoic acid;
(*E*)-5,5-dimethyl-2-[*p*-(1*H*-1,2,4-triazol-1-yl)benzoylamino]-3-hexenoic acid;
(*E*)-5,5-dimethyl-2-[p-(4H-1,2,4-triazol-4-yl)benzoylamino]-3-hexenoic acid;
(*E*)-5,5-dimethyl-2-[m-(1H-1,2,4-triazol-1-yl)benzoylamino]-3-hexenoic acid;
(*E*)-5,5-dimethyl-2[*m*-(1-imidazolyl)benzoylamino]--3-hexenoic acid;
(*E*)-5,5-dimethyl-2-[m-(1*H*-tetraazol-1-yl)benzoylamino]-3-hexenoic acid;
(*E*)-5,5-dimethyl-2-[2-(4*H*-1,2,4-triazol-4-yl)isonicotinoylamino]-3-hexenoic acid;
(*E*)-2-(4-bromo-2-pyrrolylcarbonylamino)-5,5-dimethyl-3-hexenoic acid;
(*E*)-2-(5-chloro-2-pyrrolylcarbonylamino)-5,5-dimethyl-3-hexenoic acid;
(*E*)-2-(4,5-dichloro-2-pyrrolylcarbonylamino)-5,5-dimethyl-3-hexenoic acid;
(*E*)-2-(4-chloro-1-methyl-2-pyrrolylcarbonylamino)-5,5-dimethyl-3-hexenoic acid;
(*E*)-2-(4-chloro-2-thienylcarbonylamino)-5,5-dimethyl-3-hexenoic acid;
(*E*)-2-(5-isopropyl-3-pyrazolylcarbonylamino)-5,5-dimethyl-3-hexenoic acid;
(*E*)-5,5-dimethyl-2-[*p*-(3-pyridyloxy)benzoylamino]-3-hexenoic acid;
(*E*)-5,5-dimethyl-2-(5-phenoxynicotinoylamino)-3-hexenoic acid;
(*E*)-5,5-dimethyl-2-(2-phenoxyisonicotinoylamino)-3-hexenoic acid;
(*E*)-5,5-dimethyl-2-[*p*-(2-pyridyloxy)benzoylamino]-3-hexenoic acid;
(*E*)-5,5-dimethyl-2-[*m*-(2-pyridyloxy)benzoylamino]-3-hexenoic acid;
(*E*)-2-[6-(benzyloxy)nicotinoylamino]-5,5-dimethyl-3-hexenoic acid;
(*E*)-5,5-dimethyl-2-[*p*-(6-methyl-2-pyrazinyloxy)benzoylamino]-3-hexenoic acid;
(*E*)-5,5-dimethyl-2-[*p*-(2-pyrimidinyloxy)benzoylamino]-3-hexenoic acid;
(*E*)-2-[*m*-(5-chloro-2-pyridyloxy)benzoylamino]-5,5-dimethyl-3-hexenoic acid;
(*E*)-2-[6-(*p*-bromophenoxy)nicotinoylamino]-5,5-dimethyl-3-hexenoic acid;
(*E*)-5,5-dimethyl-2-(2-phenoxy-5-pyrimidinylcarbonylamino)-3-hexenoic acid;
(*E*)-2-{6-[*p*-(*tert*-Butyl)phenoxy]nicotinoylamino}-5,5-dimethyl-3-hexenoic acid;
(*E*)-2-[2-(*p*-cumenyloxy)isonicotinoylamino]-5,5-dimethyl-3-hexenoic acid;
(*E*)-5,5-Dimethyl-2-(5-phenoxy-2-pyrazinylcarbonylamino)-3-hexenoic acid;
(*E*)-5,5-dimethyl-2-[*p*-(5-methyl-2-pyridyloxy)benzoylamino]-3-hexenoic acid;
or an enantiomer thereof, a diastereomer thereof, a racemate thereof, or a pharmaceutically acceptable salt thereof.

8. The compound according to any of the claims 1 to 6, wherein the compound is selected from the group consisting of:
(*E*)-2-[2-(dimethylamino)-4-methyl-5-pyrimidinylcarbonylamino]-5,5-dimethyl-3-hexenoic acid;
(*E*)-5,5-dimethyl-2-[*p*-(2,2,2-trifluoroacetyl)benzoylamino]-3-hexenoic acid;
(*E*)-5,5-dimethyl-2-[4-(2-pyridyl)-2-piperazinylcarbonylamino]-3-hexenoic acid;
(*E*)-5,5-dimethyl-2-[4-(4-pyridyl)-2-piperazinylcarbonylamino]-3-hexenoic acid;
(*E*)-5,5-dimethyl-2-(6-phenoxynicotinoylamino)-3-hexenoic acid;
(*E*)-5,5-dimethyl-2-[m-(4H-1,2,4-triazol-4-yl)benzoylamino]-3-hexenoic acid;
(*E*)-5,5-dimethyl-2-(6-methyl-1*H*-1,5-diazainden-2-ylcarbonylamino)-3-hexenoic acid;
(*E*)-2-(4-chloro-2-pyrrolylcarbonylamino)-5,5-dimethyl-3-hexenoic acid;
(*E*)-2-(3-isobutyl-1-methyl-5-pyrazolylcarbonylamino)-5,5-dimethyl-3-hexenoic acid;
(*E*)-2-{4-[amino(2-pyridyl)methyl]-2-pyrrolylcarbonylamino}-5,5-dimethyl-3-hexenoic acid;
(*E*)-5,5-dimethyl-2-[1-(2-oxo-4-pyridyl)-4-pyrazolylcarbonylamino]-3-hexenoic acid;
(*E*)-2-[2-(dimethylamino)-5-pyrimidinylcarbonylamino]-5,5-dimethyl-3-hexenoic acid;
(*E*)-5,5-dimethyl-2-[*p*-(4*H*-1,2,4-triazol-4-yl)benzoylamino]-3-hexenoic acid;
(E)-5,5-dimethyl-2-[m-(1H-1,2,4-triazol-1-yl)benzoylamino]-3-hexenoic acid;
(*E*)-5,5-dimethyl-2[*m*-(1-imidazolyl)benzoylamino]--3-hexenoic acid;
(*E*)-2-(4-bromo-2-pyrrolylcarbonylamino)-5,5-dimethyl-3-hexenoic acid;
(*E*)-2-(5-chloro-2-pyrrolylcarbonylamino)-5,5-dimethyl-3-hexenoic acid;
(*E*)-2-(4-chloro-1-methyl-2-pyrrolylcarbonylamino)-5,5-dimethyl-3-hexenoic acid;
(*E*)-2-(4-chloro-2-thienylcarbonylamino)-5,5-dimethyl-3-hexenoic acid;
(*E*)-5,5-dimethyl-2-[*p*-(3-pyridyloxy)benzoylamino]-3-hexenoic acid;
(*E*)-5,5-dimethyl-2-(2-phenoxyisonicotinoylamino)-3-hexenoic acid;
(*E*)-2-[6-(benzyloxy)nicotinoylamino]-5,5-dimethyl-3-hexenoic acid;
(*E*)-5,5-dimethyl-2-[*p*-(2-pyrimidinyloxy)benzoylamino]-3-hexenoic acid;
(*E*)-5,5-dimethyl-2-(2-phenoxy-5-pyrimidinylcarbonylamino)-3-hexenoic acid;
(*E*)-2-(2-isopropyl-5-pyrimidinylcarbonylamino)-5,5-dimethyl-3-hexenoic acid;
(*E*)-5,5-dimethyl-2-[*p*-(2-thienyl)benzoylamino]-3-hexenoic acid;
(E)-5,5-dimethyl-2-[m-(2-thienyl)benzoylamino]-3-hexenoic acid;
(*E*)-2-[*p*-(*p*-chlorophenoxy)benzoylamino]-5,5-dimethyl-3-hexenoic acid;
(E)-2-[p-(m-chlorophenoxy)benzoylamino]-5,5-dimethyl-3-hexenoic acid;
(*E*)-5,5-dimethyl-2-[*p*-(phenoxymethyl)benzoylamino]-3-hexenoic acid;
(*E*)-2-(1-ethyl-3-isobutyl-5-pyrazolylcarbonylamino)-5,5-dimethyl-3-hexenoic acid;
(*E*)-2-(3-chloro-1-methyl-5-pyrazolylcarbonylamino)-5,5-dimethyl-3-hexenoic acid;
(*E*)-2-(3-isopropyl-1-methyl-5-pyrazolylcarbonylamino)-5,5-dimethyl-3-hexenoic acid;
(*E*)-5,5-dimethyl-2-[1-methyl-3-(3-pyridyl)-5-pyrazolylcarbonylamino]-3-hexenoic acid;
(*E*)-2-[5-(3,4-dichlorophenyl)-1-methyl-3-pyrazolylcarbonylamino]-5,5-dimethyl-3-hexenoic acid;
(*E*)-2-(4,5-dichloro-2-thienylamino)-5,5-dimethyl-3-hexenoic acid;
or an enantiomer thereof, a diastereomer thereof, a racemate thereof, or a pharmaceutically acceptable salt thereof, preferably selected from the group consisting of:
(*E*)-2-[2-(dimethylamino)-4-methyl-5-pyrimidinylcarbonylamino]-5,5-dimethyl-3-hexenoic acid;
(*E*)-5,5-dimethyl-2-[*p*-(2,2,2-trifluoroacetyl)benzoylamino]-3-hexenoic acid;
(*E*)-5,5-dimethyl-2-[4-(2-pyridyl)-2-piperazinylcarbonylamino]-3-hexenoic acid;
(*E*)-5,5-dimethyl-2-[4-(4-pyridyl)-2-piperazinylcarbonylamino]-3-hexenoic acid;
(*E*)-5,5-dimethyl-2-(6-phenoxynicotinoylamino)-3-hexenoic acid;
(*E*)-5,5-dimethyl-2-[m-(4H-1,2,4-triazol-4-yl)benzoylamino]-3-hexenoic acid;
(*E*)-5,5-dimethyl-2-(6-methyl-1*H*-1,5-diazainden-2-ylcarbonylamino)-3-hexenoic acid;
(*E*)-2-(4-chloro-2-pyrrolylcarbonylamino)-5,5-dimethyl-3-hexenoic acid;
(*E*)-2-(3-isobutyl-1-methyl-5-pyrazolylcarbonylamino)-5,5-dimethyl-3-hexenoic acid;
or an enantiomer thereof, a diastereomer thereof, a racemate thereof, or a pharmaceutically acceptable salt thereof.

9. The compound according to any of the claims 1 to 6, wherein the compound is selected from the group consisting of:
(*E*)-2-[2-(dimethylamino)-4-methyl-5-pyrimidinylcarbonylamino]-5,5-dimethyl-3-hexenoic acid;
(*E*)-5,5-dimethyl-2-(6-phenoxynicotinoylamino)-3-hexenoic acid;
(*E*)-5,5-dimethyl-2-[m-(4H-1,2,4-triazol-4-yl)benzoylamino]-3-hexenoic acid;
(*E*)-5,5-dimethyl-2-(6-methyl-1*H*-1,5-diazainden-2-ylcarbonylamino)-3-hexenoic acid;
(*E*)-2-(4-chloro-2-pyrrolylcarbonylamino)-5,5-dimethyl-3-hexenoic acid;
(*E*)-5,5-dimethyl-2-[*p*-(4*H*-1,2,4-triazol-4-yl)benzoylamino]-3-hexenoic acid;
(*E*)-5,5-dimethyl-2-[m-(1H-1,2,4-triazol-1-yl)benzoylamino]-3-hexenoic acid;
(*E*)-5,5-dimethyl-2[*m*-(1-imidazolyl)benzoylamino]--3-hexenoic acid;
(*E*)-2-(4-bromo-2-pyrrolylcarbonylamino)-5,5-dimethyl-3-hexenoic acid;
(*E*)-2-(5-chloro-2-pyrrolylcarbonylamino)-5,5-dimethyl-3-hexenoic acid;
(*E*)-2-(4-chloro-1-methyl-2-pyrrolylcarbonylamino)-5,5-dimethyl-3-hexenoic acid;
(*E*)-2-(4-chloro-2-thienylcarbonylamino)-5,5-dimethyl-3-hexenoic acid;
(*E*)-5,5-dimethyl-2-[*p*-(3-pyridyloxy)benzoylamino]-3-hexenoic acid;
(*E*)-2-[6-(benzyloxy)nicotinoylamino]-5,5-dimethyl-3-hexenoic acid;
(*E*)-5,5-dimethyl-2-(2-phenoxy-5-pyrimidinylcarbonylamino)-3-hexenoic acid;
(*E*)-2-(2-isopropyl-5-pyrimidinylcarbonylamino)-5,5-dimethyl-3-hexenoic acid;
(*E*)-5,5-dimethyl-2-[*p*-(2-thienyl)benzoylamino]-3-hexenoic acid;
(*E*)-5,5-dimethyl-2-[*m*-(2-thienyl)benzoylamino]-3-hexenoic acid;
(*E*)-2-[*p*-(*p*-chlorophenoxy)benzoylamino]-5,5-dimethyl-3-hexenoic acid;
(*E*)-2-[*p*-(m-chlorophenoxy)benzoylamino]-5,5-dimethyl-3-hexenoic acid;
(*E*)-5,5-dimethyl-2-[*p*-(phenoxymethyl)benzoylamino]-3-hexenoic acid;
(*E*)-2-(1-ethyl-3-isobutyl-5-pyrazolylcarbonylamino)-5,5-dimethyl-3-hexenoic acid;
(*E*)-2-(3-chloro-1-methyl-5-pyrazolylcarbonylamino)-5,5-dimethyl-3-hexenoic acid;
(*E*)-2-(3-isopropyl-1-methyl-5-pyrazolylcarbonylamino)-5,5-dimethyl-3-hexenoic acid;
(*E*)-2-[5-(3,4-dichlorophenyl)-1-methyl-3-pyrazolylcarbonylamino]-5,5-dimethyl-3-hexenoic acid;
(*E*)-2-(4,5-dichloro-2-thienylamino)-5,5-dimethyl-3-hexenoic acid;
or an enantiomer thereof, a diastereomer thereof, a racemate thereof, or a pharmaceutically acceptable salt thereof, preferably selected from the group consisting of:
(*E*)-2-[2-(dimethylamino)-4-methyl-5-pyrimidinylcarbonylamino]-5,5-dimethyl-3-hexenoic acid;
(*E*)-5,5-dimethyl-2-(6-phenoxynicotinoylamino)-3-hexenoic acid;
(*E*)-5,5-dimethyl-2-[m-(4*H*-1,2,4-triazol-4-yl)benzoylamino]-3-hexenoic acid;
(*E*)-2-(4-chloro-2-pyrrolylcarbonylamino)-5,5-dimethyl-3-hexenoic acid;
(E)-5,5-dimethyl-2-[*m*-(1H-1,2,4-triazol-1-yl)benzoylamino]-3-hexenoic acid;
(*E*)-5,5-dimethyl-2[*m*-(1-imidazolyl)benzoylamino]--3-hexenoic acid;
(*E*)-2-(4-bromo-2-pyrrolylcarbonylamino)-5,5-dimethyl-3-hexenoic acid;
(*E*)-2-(4-chloro-1-methyl-2-pyrrolylcarbonylamino)-5,5-dimethyl-3-hexenoic acid;
(*E*)-2-(4-chloro-2-thienylcarbonylamino)-5,5-dimethyl-3-hexenoic acid;
(*E*)-5,5-dimethyl-2-[*p*-(3-pyridyloxy)benzoylamino]-3-hexenoic acid;
(*E*)-2-[6-(benzyloxy)nicotinoylamino]-5,5-dimethyl-3-hexenoic acid;
(*E*)-2-(2-isopropyl-5-pyrimidinylcarbonylamino)-5,5-dimethyl-3-hexenoic acid;
(*E*)-5,5-dimethyl-2-[*p*-(2-thienyl)benzoylamino]-3-hexenoic acid;
(E)-5,5-dimethyl-2-[m-(2-thienyl)benzoylamino]-3-hexenoic acid;
(*E*)-2-(3-chloro-1-methyl-5-pyrazolylcarbonylamino)-5,5-dimethyl-3-hexenoic acid;
(*E*)-2-(4,5-dichloro-2-thienylamino)-5,5-dimethyl-3-hexenoic acid;
or an enantiomer thereof, a diastereomer thereof, a racemate thereof, or a pharmaceutically acceptable salt thereof, and more preferably
(E)-5,5-dimethyl-2[m-(1-imidazolyl)benzoylamino]--3-hexenoic acid, or an enantiomer thereof, a diastereomer thereof, a racemate thereof, or a pharmaceutically acceptable salt thereof; or
(*E*)-2-(4-chloro-1-methyl-2-pyrrolylcarbonylamino)-5,5-dimethyl-3-hexenoic acid, or an enantiomer thereof, a diastereomer thereof, a racemate thereof, or a pharmaceutically acceptable salt thereof; or
(*E*)-2-(4-chloro-2-thienylcarbonylamino)-5,5-dimethyl-3-hexenoic acid, or an enantiomer thereof, a diastereomer thereof, a racemate thereof, or a pharmaceutically acceptable salt thereof; or
(*E*)-2-[6-(benzyloxy)nicotinoylamino]-5,5-dimethyl-3-hexenoic acid, or an enantiomer thereof, a diastereomer thereof, a racemate thereof, or a pharmaceutically acceptable salt thereof; or
(*E*)-5,5-dimethyl-2-[*p*-(2-thienyl)benzoylamino]-3-hexenoic acid, or an enantiomer thereof, a diastereomer thereof, a racemate thereof, or a pharmaceutically acceptable salt thereof; or
(*E*)-5,5-dimethyl-2-[*m*-(2-thienyl)benzoylamino]-3-hexenoic acid, or an enantiomer thereof, a diastereomer thereof, a racemate thereof, or a pharmaceutically acceptable salt thereof; or
(*E*)-2-(4,5-dichloro-2-thienylamino)-5,5-dimethyl-3-hexenoic acid, or an enantiomer thereof, a diastereomer thereof, a racemate thereof, or a pharmaceutically acceptable salt thereof.

10. The compound according to any one of claims 1 to 6, wherein the compound is selected from the group consisting of:
(*E*)-2-[2-(dimethylamino)-4-methyl-5-pyrimidinylcarbonylamino]-5,5-dimethyl-3-hexenoic acid;
(*E*)-5,5-dimethyl-2-(6-phenoxynicotinoylamino)-3-hexenoic acid;
(*E*)-5,5-dimethyl-2-[m-(4H-1,2,4-triazol-4-yl)benzoylamino]-3-hexenoic acid;
(*E*)-5,5-dimethyl-2-(6-methyl-1*H*-1,5-diazainden-2-ylcarbonylamino)-3-hexenoic acid;
(*E*)-2-(4-chloro-2-pyrrolylcarbonylamino)-5,5-dimethyl-3-hexenoic acid;
or an enantiomer thereof, a diastereomer thereof, a racemate thereof, or a pharmaceutically acceptable salt thereof, preferably
(*E*)-2-[2-(dimethylamino)-4-methyl-5-pyrimidinylcarbonylamino]-5,5-dimethyl-3-hexenoic acid or an enantiomer thereof, a diastereomer thereof, a racemate thereof, or a pharmaceutically acceptable salt thereof; or
(*E*)-5,5-dimethyl-2-(6-phenoxynicotinoylamino)-3-hexenoic acid or an enantiomer thereof, a diastereomer thereof, a racemate thereof, or a pharmaceutically acceptable salt thereof.

11. A pharmaceutical composition comprising a compound according to any of the claims 1 to 10 and at least one pharmaceutically acceptable excipient, carrier or diluent.

12. A compound according to any of the claims 1 to 10 for use as a medicament.

13. A compound according to any of the claims 1 to 10 for use in prevention or treatment of cancer.

14. A compound according to any of the claims 1 to 10 for use in prevention or treatment of a neurodegenerative disease, a psychiatric disease, a motor neuron disease, peripheral neuropathies, pain, neuroinflammation, atherosclerosis, hyperlipidemia, cardiovascular diseases, dermatology related diseases, autoimmune diseases, preferably Alzheimer's disease and Parkinson's disease.

15. An intermediate for the production of a compound according to claim 1, wherein the intermediate is selected from the group consisting of:
(*E*)-2-{[(p-methoxyphenyl)methyl]amino}-5,5-dimethyl-3-hexenoic acid;
ethyl (*E*)-2-{[(*p*-methoxyphenyl)methyl]amino}-5,5-dimethyl-3-hexenoate; and
ethyl (*E*)-2-amino-5,5-dimethyl-3-hexenoate, preferably wherein the intermediate is ethyl (*E*)-2-amino-5,5-dimethyl-3-hexenoate.

## Patentansprüche

1. Verbindung der Formel I: 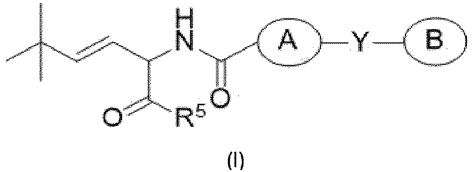 wobei
A ein 5- oder 6-gliedriger aromatischer, heteroaromatischer oder heterocyclischer Ring mit 0 bis 2 Heteroatom(en), ausgewählt aus N, O und S, oder ein 8- bis 10-gliedriger bicyclischer, heterocyclischer oder heteroaromatischer Ring mit 1 oder 2 Heteroatom(en), ausgewählt aus N, O und S ist, wobei der aromatische, heteroaromatische, heterocyclische oder bicyclische Ring A gegebenenfalls mit einem oder mehreren Substituenten substituiert ist, unabhängig ausgewählt aus der Gruppe, bestehend aus C₁-C₄-Alkyl, -NR¹R², -C(O)CZ₃, -OR⁴, Halogen und =O;
Y fehlt, -O-, -OCH₂-, -CH₂-, -NR³- oder -CH(NH₂)- ist;
B fehlt oder ein 5- oder 6-gliedriger aromatischer oder heteroaromatischer Ring mit 0 bis 4 Heteroatom(en) ist, ausgewählt aus N, O und S, wobei der aromatische oder heteroaromatische Ring B gegebenenfalls mit einem oder mehreren Substituenten substituiert ist, unabhängig ausgewählt aus der Gruppe, bestehend aus C₁-C₄-Alkyl, -NR¹R², -C(O)CZ₃, -OR⁴, Halogen und =O;
R¹, R², R³ und R⁴ unabhängig Wasserstoff oder C₁-C₄-Alkyl sind;
Z Halogen ist; und
R⁵ Hydroxyl oder C₁-C₄-Alkoxy ist,
oder ein Enantiomer davon, ein Diastereomer davon, ein Racemat davon oder ein pharmazeutisch verträgliches Salz davon.

2. Verbindung nach Anspruch 1, wobei A ein 5- oder 6-gliedriger aromatischer, heteroaromatischer oder heterocyclischer Ring mit 0 bis 2 Heteroatom(en), ausgewählt aus N und O, vorzugsweise N, oder ein 9-gliedriger bicyclischer, heterocyclischer Ring mit 1 oder 2 Heteroatom(en), ausgewählt aus N und O, vorzugsweise N, ist, wobei der aromatische, heteroaromatische, heterocyclische oder bicyclische Ring A gegebenenfalls mit einem oder mehreren Substituenten substituiert ist, unabhängig ausgewählt aus der Gruppe, bestehend aus C₁-C₄-Alkyl, -NR¹R², -C(O)CZ₃, -OR⁴, Halogen und =O.

3. Verbindung nach Anspruch 1 oder 2, wobei der aromatische, heteroaromatische, heterocyclische oder bicyclische Ring A gegebenenfalls mit einem oder mehreren Substituenten substituiert ist, unabhängig ausgewählt aus der Gruppe, bestehend aus -CH₃, -CH₂CH(CH₃)₂, -C(CH₃)₃, -N(CH₃)₂, C(O)CZ₃ und Halogen, vorzugsweise unabhängig ausgewählt aus der Gruppe, bestehend aus -CH₃, -CH₂CH(CH₃)₂, -C(CH₃)₃, -N(CH₃)₂, -C(O)CF₃, - Cl und -Br.

4. Verbindung nach einem der Ansprüche 1 bis 3, wobei Y fehlt, -O-, -OCH₂-, -CH₂-, -NH- oder -CH(NH₂)- ist, wobei Y vorzugsweise fehlt oder O ist, und mehr bevorzugt Y fehlt.

5. Verbindung nach einem der Ansprüche 1 bis 4, wobei B fehlt oder ein 5- oder 6-gliedriger aromatischer oder heteroaromatischer Ring mit 0 bis 4 Heteroatom(en) ausgewählt aus N und O, vorzugsweise N, ist, wobei der aromatische oder heteroaromatische Ring B gegebenenfalls mit einem oder mehreren Substituenten substituiert ist, unabhängig ausgewählt aus der Gruppe, bestehend aus -OR⁴ und =O, vorzugsweise unabhängig ausgewählt aus der Gruppe, bestehend aus Hydroxyl, Methoxy und =O.

6. Verbindung nach einem der Ansprüche 1 bis 5, wobei R⁵ ausgewählt ist aus der Gruppe, bestehend aus Hydroxyl, Methoxy und Ethoxy, wobei vorzugsweise R⁵ Hydroxyl ist.

7. Verbindung nach einem der Ansprüche 1 bis 6, wobei die Verbindung ausgewählt ist aus der Gruppe, bestehend aus:
(*E*)-2-[2-(Dimethylamino)-4-methyl-5-pyrimidinylcarbonylamino]-5,5-dimethyl-3-hexensäure;
(*E*)-5,5-Dimethyl-2-[*p*-(2,2,2-trifluoracetyl)benzoylamino]-3-hexensäure;
(*E*)-5,5-Dimethyl-2-[4-(2-pyridyl)-2-piperazinylcarbonylamino]-3-hexensäure;
(*E*)-5,5-Dimethyl-2-[4-(4-pyridyl)-2-piperazinylcarbonylamino]-3-hexensäure;
(*E*)-5,5-Dimethyl-2-(6-phenoxynicotinoylamino)-3-hexensäure;
(*E*)-5,5-Dimethyl-2-[*m*-(4*H*-1,2,4-triazol-4-yl)benzoylamino]-3-hexensäure;
(*E*)-5,5-Dimethyl-2-(6-methyl-1*H*-1,5-diazainden-2-ylcarbonylamino)-3-hexensäure;
(*E*)-2-(4-Chlor-2-pyrrolylcarbonylamino)-5,5-dimethyl-3-hexensäure;
(*E*)-2-(3-Isobutyl-1-methyl-5-pyrazolylcarbonylamino)-5,5-dimethyl-3-hexensäure;
(*E*)-5,5-Dimethyl-2-[4-(2-pyridyl)-2-pyrrolylcarbonylamino]-3-hexensäure;
(*E*)-5,5-Dimethyl-2-[4-(4-pyridyl)-2-pyrrolylcarbonylamino]-3-hexensäure;
(*E*)-5,5-Dimethyl-2-[4-(3-pyridyl)-2-pyrrolylcarbonylamino]-3-hexensäure;
(*E*)-2-{4-[Amino(2-pyridyl)methyl]-2-pyrrolylcarbonylamino}-5,5-dimethyl-3-hexensäure;
(*E*)-2-(3-Isobutyl-5-isoxazolylcarbonylamino)-5,5-dimethyl-3-hexensäure;
(*E*)-5,5-Dimethyl-2-[1-(4-pyridyl)-4-pyrazolylcarbonylamino]-3-hexensäure;
(*E*)-5,5-Dimethyl-2-[1-(2-oxo-4-pyridyl)-4-pyrazolylcarbonylamino]-3-hexensäure;
(*E*)-2-[2-(Dimethylamino)-5-pyrimidinylcarbonylamino]-5,5-dimethyl-3-hexensäure;
(*E*)-2-(2-Isopropyl-5-pyrimidinylcarbonylamino)-5,5-dimethyl-3-hexensäure;
(*E*)-2-[2-(Dimethylamino)-4-pyrimidinylcarbonylamino]-5,5-dimethyl-3-hexensäure;
(*E*)-2-[2-(Dimethylamino)-6-methyl-4-pyrimidinylcarbonylamino]-5,5-dimethyl-3-hexensäure;
(*E*)-2-[2-(Diethylamino)-6-methyl-4-pyrimidinylcarbonylamino]-5,5-dimethyl-3-hexensäure;
(*E*)-5,5-Dimethyl-2-[p-(1H-1,2,4-triazol-1-yl)benzoylamino]-3-hexensäure;
(*E*)-5,5-Dimethyl-2-[p-(4H-1,2,4-triazol-4-yl)benzoylamino]-3-hexensäure;
(*E*)-5,5-Dimethyl-2-[m-(1H-1,2,4-triazol-1-yl)benzoylamino]-3-hexensäure;
(*E*)-5,5-Dimethyl-2[m-(1-imidazolyl)benzoylamino]-3-hexensäure;
(*E*)-5,5-Dimethyl-2-[m-(1*H*-tetraazol-1-yl)benzoylamino]-3-hexensäure;
(*E*)-5,5-Dimethyl-2-[2-(4H-1,2,4-triazol-4-yl)isonicotinoylamino]-3-hexensäure;
(*E*)-2-(4-Brom-2-pyrrolylcarbonylamino)-5,5-dimethyl-3-hexensäure;
(*E*)-2-(5-Chlor-2-pyrrolylcarbonylamino)-5,5-dimethyl-3-hexensäure;
(*E*)-2-(4,5-Dichlor-2-pyrrolylcarbonylamino)-5,5-dimethyl-3-hexensäure;
(*E*)-2-(4-Chlor-1-methyl-2-pyrrolylcarbonylamino)-5,5-dimethyl-3-hexensäure;
(*E*)-2-(4-Chlor-2-thienylcarbonylamino)-5,5-dimethyl-3-hexensäure;
(*E*)-2-(5-Isopropyl-3-pyrazolylcarbonylamino)-5,5-dimethyl-3-hexensäure;
(*E*)-5,5-Dimethyl-2-[p-(3-pyridyloxy)benzoylamino]-3-hexensäure;
(*E*)-5,5-Dimethyl-2-(5-phenoxynicotinoylamino)-3-hexensäure;
(*E*)-5,5-Dimethyl-2-(2-phenoxyisonicotinoylamino)-3-hexensäure;
(*E*)-5,5-Dimethyl-2-[*p*-(2-pyridyloxy)benzoylamino]-3-hexensäure;
(E)-5,5-Dimethyl-2-[m-(2-pyridyloxy)benzoylamino]-3-hexensäure;
(*E*)-2-[6-(Benzyloxy)nicotinoylamino]-5,5-dimethyl-3-hexensäure;
(*E*)-5,5-Dimethyl-2-[p-(6-methyl-2-pyrazinyloxy)benzoylamino]-3-hexensäure;
(E)-5,5-Dimethyl-2-[p-(2-pyrimidinyloxy)benzoylamino]-3-hexensäure;
(*E*)-2-[*m*-(5-Chlor-2-pyridyloxy)benzoylamino]-5,5-dimethyl-3-hexensäure;
(*E*)-2-[6-(*p*-Bromphenoxy)nicotinoylamino]-5,5-dimethyl-3-hexensäure;
(*E*)-5,5-Dimethyl-2-(2-phenoxy-5-pyrimidinylcarbonylamino)-3-hexensäure;
(*E*)-2-{6-[*p*-(*tert*-Butyl)phenoxy]nicotinoylamino}-5,5-dimethyl-3-hexensäure;
(*E*)-2-[2-(*p*-Cumenyloxy)isonicotinoylamino]-5,5-dimethyl-3-hexensäure;
(*E*)-5,5-Dimethyl-2-(5-phenoxy-2-pyrazinylcarbonylamino)-3-hexensäure;
(E)-5,5-Dimethyl-2-[p-(5-methyl-2-pyridyloxy)benzoylamino]-3-hexensäure;
(*E*)-2-(2-Isopropyl-5-pyrimidinylcarbonylamino)-5,5-dimethyl-3-hexensäure;
(*E*)-5,5-Dimethyl-2-[6-(4H-1,2,4-triazol-4-yl)-2-pyridylcarbonylamino]-3-hexensäure;
(*E*)-5,5-Dimethyl-2-[*p*-(2-thienyl)benzoylamino]-3-hexensäure;
(*E*)-5,5-Dimethyl-2-[*m*-(2-thienyl)benzoylamino]-3-hexensäure;
(*E*)-2-[*p*-(*p*-Chlorphenoxy)benzoylamino]-5,5-dimethyl-3-hexensäure;
(*E*)-2-[*p*-(*m*-Chlorphenoxy)benzoylamino]-5,5-dimethyl-3-hexensäure;
(E)-5,5-Dimethyl-2-[p-(phenoxymethyl)benzoylamino]-3-hexensäure;
(*E*)-2-(1-Ethyl-3-isobutyl-5-pyrazolylcarbonylamino)-5,5-dimethyl-3-hexensäure;
(*E*)-2-(3-Chlor-1-methyl-5-pyrazolylcarbonylamino)-5,5-dimethyl-3-hexensäure;
(*E*)-2-(3-Isopropyl-1-methyl-5-pyrazolylcarbonylamino)-5,5-dimethyl-3-hexensäure;
(E)-5,5-Dimethyl-2-[1-methyl-3-(3-pyridyl)-5-pyrazolylcarbonylamino]-3-hexensäure;
(E)-2-[3-(3,4-Dichlorphenyl)-1-methyl-5-pyrazolylcarbonylamino]-5,5-dimethyl-3-hexensäure;
(E)-2-[5-(3,4-Dichlorphenyl)-1-methyl-3-pyrazolylcarbonylamino]-5,5-dimethyl-3-hexensäure;
(*E*)-2-(4,5-Dichlor-2-thienylamino)-5,5-dimethyl-3-hexensäure;
(*E*)-2-(2-Benzyl-4-methyl-5-pyrimidinylcarbonylamino)-5,5-dimethyl-3-hexensäure;
(*E*)-5,5-Dimethyl-2-[4-methyl-2-(p-toluidino)-5-pyrimidinylcarbonylamino]-3-hexensäure;
(*E*)-2-[2-(*p*-Chlorphenylamino)-4-methyl-5-pyrimidinylcarbonylamino]-5,5-dimethyl-3-hexensäure;
(*E*)-2-[6-(*p*-Chlorphenoxy)nicotinoylamino]-5,5-dimethyl-3-hexensäure;
(*E*)-5,5-Dimethyl-2-[6-(*p*-tolyloxy)nicotinoylamino]-3-hexensäure;
(*E*)-2-(6-Benzylnicotinoylamino)-5,5-dimethyl-3-hexensäure;
(*E*)-2-(*p*-Benzylbenzoylamino)-5,5-dimethyl-3-hexensäure;
(*E*)-5,5-Dimethyl-2-(p-phenoxybenzoylamino)-3-hexensäure;
(*E*)-5,5-Dimethyl-2-(6-phenylnicotinoylamino)-3-hexensäure;
(*E*)-5,5-Dimethyl-2-(2-phenylisonicotinoylamino)-3-hexensäure;
(*E*)-5,5-Dimethyl-2-[6-(2-thienyloxy)nicotinoylamino]-3-hexensäure;
(*E*)-2-(5-Chlor-1-benzothiophen-2-ylcarbonylamino)-5,5-dimethyl-3-hexensäure;
(*E*)-2-(5-Chlor-2-indolylcarbonylamino)-5,5-dimethyl-3-hexensäure;
(*E*)-5,5-Dimethyl-2-(1-thia-5-aza-2-indenylcarbonylamino)-3-hexensäure;
(*E*)-2-(2-Chlor-1-benzothiophen-6-ylcarbonylamino)-5,5-dimethyl-3-hexensäure;
(*E*)-2-(2-Chlor-6-indolylcarbonylamino)-5,5-dimethyl-3-hexensäure;
(*E*)-2-(4-Chlor-1,3-thiazol-2-ylcarbonylamino)-5,5-dimethyl-3-hexensäure;
(*E*)-2-(4,5-Dichlor-1,3-thiazol-2-ylcarbonylamino)-5,5-dimethyl-3-hexensäure;
(*E*)-2-(3-Chlor-5-isothiazolylcarbonylamino)-5,5-dimethyl-3-hexensäure;
(*E*)-2-(3,4-Dichlor-5-isothiazolylcarbonylamino)-5,5-dimethyl-3-hexensäure;
(*E*)-5,5-Dimethyl-2-[*m*-(3-pyridyl)benzoylamino]-3-hexensäure;
(*E*)-2-[*m*-(6-Chlor-2-methyl-3-pyridyl)benzoylamino]-5,5-dimethyl-3-hexensäure;
oder ein Enantiomer davon, ein Diastereomer davon, ein Racemat davon oder ein pharmazeutisch verträgliches Salz davon, vorzugsweise ausgewählt aus der Gruppe, bestehend aus:
(*E*)-2-[2-(Dimethylamino)-4-methyl-5-pyrimidinylcarbonylamino]-5,5-dimethyl-3-hexensäure;
(*E*)-5,5-Dimethyl-2-[*p*-(2,2,2-trifluoracety)benzoylamino]-3-hexensäure;
(*E*)-5,5-Dimethyl-2-[4-(2-pyridyl)-2-piperazinylcarbonylamino]-3-hexensäure;
(*E*)-5,5-Dimethyl-2-[4-(4-pyridyl)-2-piperazinylcarbonylamino]-3-hexensäure;
(*E*)-5,5-Dimethyl-2-(6-phenoxynicotinoylamino)-3-hexensäure;
(*E*)-5,5-Dimethyl-2-[m-(4H-1,2,4-triazol-4-yl)benzoylamino]-3-hexensäure;
(*E*)-5,5-Dimethyl-2-(6-methyl-1*H*-1,5-diazainden-2-ylcarbonylamino)-3-hexensäure;
(*E*)-2-(4-Chlor-2-pyrrolylcarbonylamino)-5,5-dimethyl-3-hexensäure;
(*E*)-2-(3-Isobutyl-1-methyl-5-pyrazolylcarbonylamino)-5,5-dimethyl-3-hexensäure;
(*E*)-5,5-Dimethyl-2-[4-(2-pyridyl)-2-pyrrolylcarbonylamino]-3-hexensäure;
(*E*)-5,5-Dimethyl-2-[4-(4-pyridyl)-2-pyrrolylcarbonylamino]-3-hexensäure;
(*E*)-5,5-Dimethyl-2-[4-(3-pyridyl)-2-pyrrolylcarbonylamino]-3-hexensäure;
(*E*)-2-{4-[Amino(2-pyridyl)methyl]-2-pyrrolylcarbonylamino}-5,5-dimethyl-3-hexensäure;
(*E*)-2-(3-Isobutyl-5-isoxazolylcarbonylamino)-5,5-dimethyl-3-hexensäure;
(*E*)-5,5-Dimethyl-2-[1-(4-pyridyl)-4-pyrazolylcarbonylamino]-3-hexensäure;
(*E*)-5,5-Dimethyl-2-[1-(2-oxo-4-pyridyl)-4-pyrazolylcarbonylamino]-3-hexensäure;
(*E*)-2-[2-(Dimethylamino)-5-pyrimidinylcarbonylamino]-5,5-dimethyl-3-hexensäure;
(*E*)-2-(2-Isopropyl-5-pyrimidinylcarbonylamino)-5,5-dimethyl-3-hexensäure;
(*E*)-2-[2-(Dimethylamino)-4-pyrimidinylcarbonylamino]-5,5-dimethyl-3-hexensäure;
(*E*)-2-[2-(Dimethylamino)-6-methyl-4-pyrimidinylcarbonylamino]-5,5-dimethyl-3-hexensäure;
(*E*)-2-[2-(Diethylamino)-6-methyl-4-pyrimidinylcarbonylamino]-5,5-dimethyl-3-hexensäure;
(*E*)-5,5-Dimethyl-2-[p-(1H-1,2,4-triazol-1-yl)benzoylamino]-3-hexensäure;
(*E*)-5,5-Dimethyl-2-[p-(4H-1,2,4-triazol-4-yl)benzoylamino]-3-hexensäure;
(*E*)-5,5-Dimethyl-2-[m-(1H-1,2,4-triazol-1-yl)benzoylamino]-3-hexensäure;
(*E*)-5,5-Dimethyl-2[*m*-(1-imidazolyl)benzoylamino]-3-hexensäure;
(*E*)-5,5-Dimethyl-2-[m-(1H-tetraazol-1-yl)benzoylamino]-3-hexensäure;
(*E*)-5,5-Dimethyl-2-[2-(4H-1,2,4-triazol-4-yl)isonicotinoylamino]-3-hexensäure;
(*E*)-2-(4-Brom-2-pyrrolylcarbonylamino)-5,5-dimethyl-3-hexensäure;
(*E*)-2-(5-Chlor-2-pyrrolylcarbonylamino)-5,5-dimethyl-3-hexensäure;
(*E*)-2-(4,5-Dichlor-2-pyrrolylcarbonylamino)-5,5-dimethyl-3-hexensäure;
(*E*)-2-(4-Chlor-1-methyl-2-pyrrolylcarbonylamino)-5,5-dimethyl-3-hexensäure;
(*E*)-2-(4-Chlor-2-thienylcarbonylamino)-5,5-dimethyl-3-hexensäure;
(*E*)-2-(5-Isopropyl-3-pyrazolylcarbonylamino)-5,5-dimethyl-3-hexensäure;
(*E*)-5,5-Dimethyl-2-[p-(3-pyridyloxy)benzoylamino]-3-hexensäure;
(*E*)-5,5-Dimethyl-2-(5-phenoxynicotinoylamino)-3-hexensäure;
(*E*)-5,5-Dimethyl-2-(2-phenoxyisonicotinoylamino)-3-hexensäure;
(*E*)-5,5-Dimethyl-2-[p-(2-pyridyloxy)benzoylamino]-3-hexensäure;
(*E*)-5,5-Dimethyl-2-[m-(2-pyridyloxy)benzoylamino]-3-hexensäure;
(*E*)-2-[6-(Benzyloxy)nicotinoylamino]-5,5-dimethyl-3-hexensäure;
(*E*)-5,5-Dimethyl-2-[p-(6-methyl-2-pyrazinyloxy)benzoylamino]-3-hexensäure;
(*E*)-5,5-Dimethyl-2-[p-(2-pyrimidinyloxy)benzoylamino]-3-hexensäure;
(*E*)-2-[*m*-(5-Chlor-2-pyridyloxy)benzoylamino]-5,5-dimethyl-3-hexensäure;
(*E*)-2-[6-(*p*-Bromphenoxy)nicotinoylamino]-5,5-dimethyl-3-hexensäure;
(*E*)-5,5-Dimethyl-2-(2-phenoxy-5-pyrimidinylcarbonylamino)-3-hexensäure;
(*E*)-2-{6-[*p*-(*tert*-Butyl)phenoxy]nicotinoylamino}-5,5-dimethyl-3-hexensäure;
(*E*)-2-[2-(*p*-Cumenyloxy)isonicotinoylamino]-5,5-dimethyl-3-hexensäure;
(*E*)-5,5-Dimethyl-2-(5-phenoxy-2-pyrazinylcarbonylamino)-3-hexensäure;
(*E*)-5,5-Dimethyl-2-[p-(5-methyl-2-pyridyloxy)benzoylamino]-3-hexensäure;
oder ein Enantiomer davon, ein Diastereomer davon, ein Racemat davon oder ein pharmazeutisch verträgliches Salz davon.

8. Verbindung nach einem der Ansprüche 1 bis 6, wobei die Verbindung ausgewählt ist aus der Gruppe, bestehend aus:
(*E*)-2-[2-(Dimethylamino)-4-methyl-5-pyrimidinylcarbonylamino]-5,5-dimethyl-3-hexensäure;
(*E*)-5,5-Dimethyl-2-[*p*-(2,2,2-trifluoracety)benzoylamino]-3-hexensäure;
(*E*)-5,5-Dimethyl-2-[4-(2-pyridyl)-2-piperazinylcarbonylamino]-3-hexensäure;
(*E*)-5,5-Dimethyl-2-[4-(4-pyridyl)-2-piperazinylcarbonylamino]-3-hexensäure;
(*E*)-5,5-Dimethyl-2-(6-phenoxynicotinoylamino)-3-hexensäure;
(*E*)-5,5-Dimethyl-2-[m-(4H-1,2,4-triazol-4-yl)benzoylamino]-3-hexensäure;
(*E*)-5,5-Dimethyl-2-(6-methyl-1*H*-1,5-diazainden-2-ylcarbonylamino)-3-hexensäure;
(*E*)-2-(4-Chlor-2-pyrrolylcarbonylamino)-5,5-dimethyl-3-hexensäure;
(*E*)-2-(3-Isobutyl-1-methyl-5-pyrazolylcarbonylamino)-5,5-dimethyl-3-hexensäure;
(*E*)-2-{4-[Amino(2-pyridyl)methyl]-2-pyrrolylcarbonylamino}-5,5-dimethyl-3-hexensäure;
(*E*)-5,5-Dimethyl-2-[1-(2-oxo-4-pyridyl)-4-pyrazolylcarbonylamino]-3-hexensäure;
(*E*)-2-[2-(Dimethylamino)-5-pyrimidinylcarbonylamino]-5,5-dimethyl-3-hexensäure;
(*E*)-5,5-Dimethyl-2-[*p*-(4H-1,2,4-triazol-4-yl)benzoylamino]-3-hexensäure;
(*E*)-5,5-Dimethyl-2-[*m*-(1H-1,2,4-triazol-1-yl)benzoylamino]-3-hexensäure;
(*E*)-5,5-Dimethyl-2[*m*-(1-imidazolyl)benzoylamino]-3-hexensäure;
(*E*)-2-(4-Brom-2-pyrrolylcarbonylamino)-5,5-dimethyl-3-hexensäure;
(*E*)-2-(5-Chlor-2-pyrrolylcarbonylamino)-5,5-dimethyl-3-hexensäure;
(*E*)-2-(4-Chlor-1-methyl-2-pyrrolylcarbonylamino)-5,5-dimethyl-3-hexensäure;
(*E*)-2-(4-Chlor-2-thienylcarbonylamino)-5,5-dimethyl-3-hexensäure;
(*E*)-5,5-Dimethyl-2-[*p*-(3-pyridyloxy)benzoylamino]-3-hexensäure;
(*E*)-5,5-Dimethyl-2-(2-phenoxyisonicotinoylamino)-3-hexensäure;
(*E*)-2-[6-(Benzyloxy)nicotinoylamino]-5,5-dimethyl-3-hexensäure;
(*E*)-5,5-Dimethyl-2-[*p*-(2-pyrimidinyloxy)benzoylamino]-3-hexensäure;
(*E*)-5,5-Dimethyl-2-(2-phenoxy-5-pyrimidinylcarbonylamino)-3-hexensäure;
(*E*)-2-(2-Isopropyl-5-pyrimidinylcarbonylamino)-5,5-dimethyl-3-hexensäure;
(*E*)-5,5-Dimethyl-2-[*p*-(2-thienyl)benzoylamino]-3-hexensäure;
(*E*)-5,5-Dimethyl-2-[*m*-(2-thienyl)benzoylamino]-3-hexensäure;
(*E*)-2-[*p*-(*p*-Chlorphenoxy)benzoylamino]-5,5-dimethyl-3-hexensäure;
(*E*)-2-[*p*-(*m*-Chlorphenoxy)benzoylamino]-5,5-dimethyl-3-hexensäure;
(*E*)-5,5-Dimethyl-2-[*p*-(phenoxymethyl)benzoylamino]-3-hexensäure;
(*E*)-2-(1-Ethyl-3-isobutyl-5-pyrazolylcarbonylamino)-5,5-dimethyl-3-hexensäure;
(*E*)-2-(3-Chlor-1-methyl-5-pyrazolylcarbonylamino)-5,5-dimethyl-3-hexensäure;
(*E*)-2-(3-Isopropyl-1-methyl-5-pyrazolylcarbonylamino)-5,5-dimethyl-3-hexensäure;
(*E*)-5,5-Dimethyl-2-[1-methyl-3-(3-pyridyl)-5-pyrazolylcarbonylamino]-3-hexensäure;
(*E*)-2-[5-(3,4-Dichlorphenyl)-1-methyl-3-pyrazolylcarbonylamino]-5,5-dimethyl-3-hexensäure;
(*E*)-2-(4,5-Dichlor-2-thienylamino)-5,5-dimethyl-3-hexensäure;
oder ein Enantiomer davon, ein Diastereomer davon, ein Racemat davon oder ein pharmazeutisch verträgliches Salz davon, vorzugsweise ausgewählt aus der Gruppe, bestehend aus:
(*E*)-2-[2-(Dimethylamino)-4-methyl-5-pyrimidinylcarbonylamino]-5,5-dimethyl-3-hexensäure;
(*E*)-5,5-Dimethyl-2-[*p*-(2,2,2-trifluoracetyl)benzoylamino]-3-hexensäure;
(*E*)-5,5-Dimethyl-2-[4-(2-pyridyl)-2-piperazinylcarbonylamino]-3-hexensäure;
(*E*)-5,5-Dimethyl-2-[4-(4-pyridyl)-2-piperazinylcarbonylamino]-3-hexensäure;
(*E*)-5,5-Dimethyl-2-(6-phenoxynicotinoylamino)-3-hexensäure;
(*E*)-5,5-Dimethyl-2-[*m*-(4*H*-1,2,4-triazol-4-yl)benzoylamino]-3-hexensäure;
(*E*)-5,5-Dimethyl-2-(6-methyl-1*H*-1,5-diazainden-2-ylcarbonylamino)-3-hexensäure;
(*E*)-2-(4-Chlor-2-pyrrolylcarbonylamino)-5,5-dimethyl-3-hexensäure;
(*E*)-2-(3-Isobutyl-1-methyl-5-pyrazolylcarbonylamino)-5,5-dimethyl-3-hexensäure;
oder ein Enantiomer davon, ein Diastereomer davon, ein Racemat davon oder ein pharmazeutisch verträgliches Salz davon.

9. Verbindung nach einem der Ansprüche 1 bis 6, wobei die Verbindung ausgewählt ist aus der Gruppe, bestehend aus:
(*E*)-2-[2-(Dimethylamino)-4-methyl-5-pyrimidinylcarbonylamino]-5,5-dimethyl-3-hexensäure;
(*E*)-5,5-Dimethyl-2-(6-phenoxynicotinoylamino)-3-hexensäure;
(*E*)-5,5-Dimethyl-2-[*m*-(4H-1,2,4-triazol-4-yl)benzoylamino]-3-hexensäure;
(*E*)-5,5-Dimethyl-2-(6-methyl-1*H*-1,5-diazainden-2-ylcarbonylamino)-3-hexensäure;
(*E*)-2-(4-Chlor-2-pyrrolylcarbonylamino)-5,5-dimethyl-3-hexensäure;
(*E*)-5,5-Dimethyl-2-[*p*-(4H-1,2,4-triazol-4-yl)benzoylamino]-3-hexensäure;
(E)-5,5-Dimethyl-2-[m-(1*H*-1,2,4-triazol-1-yl)benzoylamino]-3-hexensäure;
(*E*)-5,5-Dimethyl-2[*m*-(1-imidazolyl)benzoylamino]-3-hexensäure;
(*E*)-2-(4-Brom-2-pyrrolylcarbonylamino)-5,5-dimethyl-3-hexensäure;
(*E*)-2-(5-Chlor-2-pyrrolylcarbonylamino)-5,5-dimethyl-3-hexensäure;
(*E*)-2-(4-Chlor-1-methyl-2-pyrrolylcarbonylamino)-5,5-dimethyl-3-hexensäure;
(*E*)-2-(4-Chlor-2-thienylcarbonylamino)-5,5-dimethyl-3-hexensäure;
(*E*)-5,5-Dimethyl-2-[*p*-(3-pyridyloxy)benzoylamino]-3-hexensäure;
(*E*)-2-[6-(Benzyloxy)nicotinoylamino]-5,5-dimethyl-3-hexensäure;
(*E*)-5,5-Dimethyl-2-(2-phenoxy-5-pyrimidinylcarbonylamino)-3-hexensäure;
(*E*)-2-(2-Isopropyl-5-pyrimidinylcarbonylamino)-5,5-dimethyl-3-hexensäure;
(*E*)-5,5-Dimethyl-2-[*p*-(2-thienyl)benzoylamino]-3-hexensäure;
(*E*)-5,5-Dimethyl-2-[*m*-(2-thienyl)benzoylamino]-3-hexensäure;
(*E*)-2-[*p*-(*p*-Chlorphenoxy)benzoylamino]-5,5-dimethyl-3-hexensäure;
(*E*)-2-[*p*-(*m*-Chlorphenoxy)benzoylamino]-5,5-dimethyl-3-hexensäure;
(*E*)-5,5-Dimethyl-2-[*p*-(phenoxymethyl)benzoylamino]-3-hexensäure;
(*E*)-2-(1-Ethyl-3-isobutyl-5-pyrazolylcarbonylamino)-5,5-dimethyl-3-hexensäure;
(*E*)-2-(3-Chlor-1-methyl-5-pyrazolylcarbonylamino)-5,5-dimethyl-3-hexensäure;
(*E*)-2-(3-Isopropyl-1-methyl-5-pyrazolylcarbonylamino)-5,5-dimethyl-3-hexensäure;
(*E*)-2-[5-(3,4-Dichlorphenyl)-1-methyl-3-pyrazolylcarbonylamino]-5,5-dimethyl-3-hexensäure;
(*E*)-2-(4,5-Dichlor-2-thienylamino)-5,5-dimethyl-3-hexensäure;
oder ein Enantiomer davon, ein Diastereomer davon, ein Racemat davon oder ein pharmazeutisch verträgliches Salz davon, vorzugsweise ausgewählt aus der Gruppe, bestehend aus:
(*E*)-2-[2-(Dimethylamino)-4-methyl-5-pyrimidinylcarbonylamino]-5,5-dimethyl-3-hexensäure;
(*E*)-5,5-Dimethyl-2-(6-phenoxynicotinoylamino)-3-hexensäure;
(*E*)-5,5-Dimethyl-2-[m-(4H-1,2,4-triazol-4-yl)benzoylamino]-3-hexensäure;
(*E*)-2-(4-Chlor-2-pyrrolylcarbonylamino)-5,5-dimethyl-3-hexensäure;
(*E*)-5,5-Dimethyl-2-[*m*-(1*H*-1,2,4-triazol-1-yl)benzoylamino]-3-hexensäure;
(*E*)-5,5-Dimethyl-2[*m*-(1-imidazolyl)benzoylamino]-3-hexensäure;
(*E*)-2-(4-Brom-2-pyrrolylcarbonylamino)-5,5-dimethyl-3-hexensäure;
(*E*)-2-(4-Chlor-1-methyl-2-pyrrolylcarbonylamino)-5,5-dimethyl-3-hexensäure;
(*E*)-2-(4-Chlor-2-thienylcarbonylamino)-5,5-dimethyl-3-hexensäure;
(*E*)-5,5-Dimethyl-2-[*p*-(3-pyridyloxy)benzoylamino]-3-hexensäure;
(*E*)-2-[6-(Benzyloxy)nicotinoylamino]-5,5-dimethyl-3-hexensäure;
(*E*)-2-(2-Isopropyl-5-pyrimidinylcarbonylamino)-5,5-dimethyl-3-hexensäure;
(*E*)-5,5-Dimethyl-2-[*p*-(2-thienyl)benzoylamino]-3-hexensäure;
(*E*)-5,5-Dimethyl-2-[*m*-(2-thienyl)benzoylamino]-3-hexensäure;
(*E*)-2-(3-Chlor-1-methyl-5-pyrazolylcarbonylamino)-5,5-dimethyl-3-hexensäure;
(*E*)-2-(4,5-Dichlor-2-thienylamino)-5,5-dimethyl-3-hexensäure;
oder ein Enantiomer davon, ein Diastereomer davon, ein Racemat davon oder ein pharmazeutisch verträgliches Salz davon, und mehr bevorzugt
(*E*)-5,5-Dimethyl-2[*m*-(1-imidazolyl)benzoylamino]-3-hexensäure oder ein Enantiomer davon, ein Diastereomer davon, ein Racemat davon oder ein pharmazeutisch verträgliches Salz davon; oder
(*E*)-2-(4-Chlor-1-methyl-2-pyrrolylcarbonylamino)-5,5-dimethyl-3-hexensäure oder ein Enantiomer davon, ein Diastereomer davon, ein Racemat davon oder ein pharmazeutisch verträgliches Salz davon; oder
(*E*)-2-(4-Chlor-2-thienylcarbonylamino)-5,5-dimethyl-3-hexensäure oder ein Enantiomer davon, ein Diastereomer davon, ein Racemat davon oder ein pharmazeutisch verträgliches Salz davon; oder
(*E*)-2-[6-(Benzyloxy)nicotinoylamino]-5,5-dimethyl-3-hexensäure oder ein Enantiomer davon, ein Diastereomer davon, ein Racemat davon oder ein pharmazeutisch verträgliches Salz davon; oder
(*E*)-5,5-Dimethyl-2-[p-(2-thienyl)benzoylamino]-3-hexensäure oder ein Enantiomer davon, ein Diastereomer davon, ein Racemat davon oder ein pharmazeutisch verträgliches Salz davon; oder
(*E*)-5,5-Dimethyl-2-[m-(2-thienyl)benzoylamino]-3-hexensäure oder ein Enantiomer davon, ein Diastereomer davon, ein Racemat davon oder ein pharmazeutisch verträgliches Salz davon; oder
(*E*)-2-(4,5-Dichlor-2-thienylamino)-5,5-dimethyl-3-hexensäure oder ein Enantiomer davon, ein Diastereomer davon, ein Racemat davon oder ein pharmazeutisch verträgliches Salz davon.

10. Verbindung nach einem der Ansprüche 1 bis 6, wobei die Verbindung ausgewählt ist aus der Gruppe, bestehend aus:
(*E*)-2-[2-(Dimethylamino)-4-methyl-5-pyrimidinylcarbonylamino]-5,5-dimethyl-3-hexensäure;
(*E*)-5,5-Dimethyl-2-(6-phenoxynicotinoylamino)-3-hexensäure;
(*E*)-5,5-Dimethyl-2-[*m*-(4*H*-1,2,4-triazol-4-y)benzoylamino]-3-hexensäure;
(*E*)-5,5-Dimethyl-2-(6-methyl-1*H*-1,5-diazainden-2-ylcarbonylamino)-3-hexensäure;
(*E*)-2-(4-Chlor-2-pyrrolylcarbonylamino)-5,5-dimethyl-3-hexensäure;
oder ein Enantiomer davon, ein Diastereomer davon, ein Racemat davon oder ein pharmazeutisch verträgliches Salz davon, vorzugsweise
(*E*)-2-[2-(Dimethylamino)-4-methyl-5-pyrimidinylcarbonylamino]-5,5-dimethyl-3-hexensäure oder ein Enantiomer davon, ein Diastereomer davon, ein Racemat davon oder ein pharmazeutisch verträgliches Salz davon; oder
(*E*)-5,5-Dimethyl-2-(6-phenoxynicotinoylamino)-3-hexensäure oder ein Enantiomer davon, ein Diastereomer davon, ein Racemat davon oder ein pharmazeutisch verträgliches Salz davon.

11. Pharmazeutische Zusammensetzung, umfassend eine Verbindung nach einem der Ansprüche 1 bis 10 und mindestens einen pharmazeutisch verträglichen Hilfsstoff, einen pharmazeutisch verträglichen Träger oder ein pharmazeutisch verträgliches Verdünnungsmittel.

12. Verbindung nach einem der Ansprüche 1 bis 10 zur Verwendung als ein Medikament.

13. Verbindung nach einem der Ansprüche 1 bis 10 zur Verwendung bei der Prävention oder Behandlung von Krebs.

14. Verbindung nach einem der Ansprüche 1 bis 10 zur Verwendung bei der Prävention oder Behandlung einer neurodegenerativen Erkrankung, einer psychiatrischen Erkrankung, einer Motoneuronenerkrankung, peripherer Neuropathien, Schmerzen, Neuroinflammation, Atherosklerose, Hyperlipidämie, kardiovaskulären Erkrankungen, dermatologisch bedingten Erkrankungen, Autoimmunerkrankungen, vorzugsweise von Alzheimer-Krankheit und Parkinson-Krankheit.

15. Zwischenprodukt zur Herstellung einer Verbindung nach Anspruch 1, wobei das Zwischenprodukt ausgewählt ist aus der Gruppe, bestehend aus:
(*E*)-2-{[(*p*-Methoxyphenyl)methyl]amino}-5,5-dimethyl-3-hexensäure;
Ethyl-(*E*)-2-{[(*p*-methoxyphenyl)methyl]amino}-5,5-dimethyl-3-hexenoat; und
Ethyl-(*E*)-2-amino-5,5-dimethyl-3-hexenoat, vorzugsweise wobei das Zwischenprodukt Ethyl-(*E*)-2-amino-5,5-dimethyl-3-hexenoat ist.

## Revendications

1. Composé de formule I : 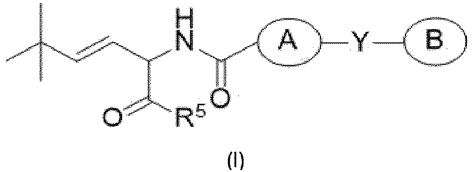 dans lequel
A est un cycle aromatique, hétéroaromatique ou hétérocyclique à 5 ou 6 chaînons avec 0 à 2 hétéroatome(s) choisi(s) parmi N, O et S, ou un cycle hétérocyclique ou hétéroaromatique bicyclique à 8 à 10 chaînons avec 1 ou 2 hétéroatome(s) choisi(s) parmi N, O et S, le cycle aromatique, hétéroaromatique, hétérocyclique ou bicyclique A étant éventuellement substitué par un ou plusieurs substituants indépendamment choisis dans le groupe constitué d'un alkyle en C₁-C₄, -NR¹R², -C(O)CZ₃, -OR⁴, un halogène et =O ;
Y est absent, -O-, -OCH₂-, -CH₂-, -NR³- ou -CH(NH₂)- ;
B est absent ou un cycle aromatique ou hétéroaromatique à 5 ou 6 chaînons avec 0 à 4 hétéroatome(s) choisi(s) parmi N, O et S, le cycle aromatique ou hétéroaromatique B est éventuellement substitué par un ou plusieurs substituants indépendamment choisis dans le groupe constitué d'un alkyle en C₁-C₄, -NR¹R², - C(O)CZ₃, -OR⁴, un halogène et =O ;
R¹, R², R³ et R⁴ sont indépendamment un hydrogène ou un alkyle en C₁-C₄ ;
Z est un halogène ; et
R⁵ est un hydroxyle ou un alcoxy en C₁-C₄,
ou un énantiomère de celui-ci, un diastéréoisomère de celui-ci, un racémate de celui-ci ou un sel acceptable sur le plan pharmaceutique de celui-ci.

2. Composé selon la revendication 1, dans lequel A est un cycle aromatique, hétéroaromatique ou hétérocyclique à 5 ou 6 chaînons avec 0 à 2 hétéroatome(s) choisi(s) parmi N et O, de préférence N, ou un cycle hétérocyclique bicyclique à 9 chaînons avec 1 ou 2 hétéroatome(s) choisis parmi N et O, de préférence N, le cycle aromatique, hétéroaromatique, hétérocyclique ou bicyclique A est éventuellement substitué par un ou plusieurs substituants indépendamment choisis dans le groupe constitué d'alkyle en C₁-C₄, -NR¹R², -C (O) CZ₃, -OR⁴, halogène, et =O.

3. Composé selon la revendication 1 ou 2, dans lequel le cycle aromatique, hétéroaromatique, hétérocyclique ou bicyclique A est éventuellement substitué par un ou plusieurs substituants indépendamment choisis dans le groupe constitué de -CH₃, - CH₂CH(CH₃)₂, -C(CH₃)₃, -N (CH₃)₂, C(O)CZ₃, et halogène, de préférence indépendamment choisis dans le groupe constitué de - CH₃, -CH₂CH(CH₃)₂, -C(CH₃)₃, -N(CH₃)₂, -C(O)CF₃, -Cl et -Br.

4. Composé selon l'une quelconque des revendications 1 à 3, dans lequel Y est absent, -O-, -OCH₂-, -CH₂-, -NH- ou -CH(NH₂)-, de préférence Y est absent ou O, et plus préférablement Y est absent

5. Composé selon l'une quelconque des revendications 1 à 4, dans lequel B est absent ou est un cycle aromatique ou hétéroaromatique à 5 ou 6 chaînons avec 0 à 4 hétéroatome(s) choisi(s) parmi N et O, de préférence N, le cycle aromatique ou hétéroaromatique B est éventuellement substitué par un ou plusieurs substituants indépendamment choisis dans le groupe constitué de -OR⁴ et =O, de préférence indépendamment choisis dans le groupe constitué d'hydroxyle, méthoxy et =O.

6. Composé selon l'une quelconque des revendications 1 à 5, dans lequel R⁵ est choisi dans le groupe constitué par hydroxyle, méthoxy et éthoxy, de préférence R⁵ est hydroxyle.

7. Composé selon l'une quelconque des revendications 1 à 6, dans lequel le composé est choisi dans le groupe constitué de :
l'acide (*E*)-2-[2-(diméthylamino)-4-méthyl-5-pyrimidinylcarbonylamino]-5,5-diméthyl-3-hexènoïque ;
l'acide (*E*)-5,5-diméthyl-2-[p-(2,2,2-trifluoroacétyl)benzoylamino]-3-hexènoïque ;
l'acide (*E*)-5,5-diméthyl-2-[4-(2-pyridyl)-2-pipérazinylcarbonylamino]-3-hexènoïque ;
l'acide (*E*)-5,5-diméthyl-2-[4-(4-pyridyl)-2-pipérazinylcarbonylamino]-3-hexènoïque ;
l'acide (*E*)-5,5-diméthyl-2-(6-phénoxynicotinoylamino)-3-hexènoïque ;
l'acide (*E*)-5,5-diméthyl-2-[*m*-(4H-1,2,4-triazol-4-yl)benzoylamino]-3-hexènoïque ;
l'acide (*E*)-5,5-diméthyl-2-(6-méthyl-1*H*-1,5-diazainden-2-ylcarbonylamino)-3-hexènoïque ;
l'acide (*E*)-2-(4-chloro-2-pyrrolylcarbonylamino)-5,5-diméthyl-3-hexènoïque ;
l'acide (*E*)-2-(3-isobutyl-1-méthyl-5-pyrazolylcarbonylamino)-5,5-diméthyl-3-hexènoïque ;
l'acide (*E*)-5,5-diméthyl-2-[4-(2-pyridyl)-2-pipérazinylcarbonylamino]-3-hexènoïque ;
l'acide (*E*)-5,5-diméthyl-2-[4-(4-pyridyl)-2-pipérazinylcarbonylamino]-3-hexènoïque ;
l'acide (*E*)-5,5-diméthyl-2-[4-(3-pyridyl)-2-pyrrolylcarbonylamino]-3-hexènoïque ;
l'acide (*E*)-2-{4-[amino(2-pyridyl)méthyl]-2-pyrrolylcarbonylamino}-5,5-diméthyl-3-hexènoïque ;
l'acide (*E*)-2-(3-isobutyl-5-isoxazolylcarbonylamino)-5,5-diméthyl-3-hexènoïque ;
l'acide (*E*)-5,5-diméthyl-2-[1-(4-pyridyl)-4-pyrazolylcarbonylamino]-3-hexènoïque ;
l'acide (*E*)-5,5-diméthyl-2-[1-(2-oxo-4-pyridyl)-4-pyrazolylcarbonylamino]-3-hexènoïque ;
l'acide (*E*)-2-[2-(diméthylamino)-5-pyrimidinylcarbonylamino]-5,5-diméthyl-3-hexènoïque ;
l'acide (*E*)-2-(2-isopropyl-5-pyrimidinylcarbonylamino)-5,5-diméthyl-3-hexènoïque ;
l'acide (*E*)-2-[2-(diméthylamino)-4-pyrimidinylcarbonylamino]-5,5-diméthyl-3-hexènoïque ;
l'acide (*E*)-2-[2-(diméthylamino)-6-méthyl-4-pyrimidinylcarbonylamino]-5,5-diméthyl-3-hexènoïque ;
l'acide (*E*)-2-[2-(diéthylamino)-6-méthyl-4-pyrimidinylcarbonylamino]-5,5-diméthyl-3-hexènoïque ;
l'acide (*E*)-5,5-diméthyl-2-[*p*-(1*H*-1,2,4-triazol-1-yl)benzoylamino]-3-hexènoïque ;
l'acide (*E*)-5,5-diméthyl-2-[*p*-(4*H*-1,2,4-triazol-4-yl)benzoylamino]-3-hexènoïque ;
l'acide (*E*)-5,5-diméthyl-2-[*m*-(1*H*-1,2,4-triazol-1-yl)benzoylamino]-3-hexènoïque ;
l'acide (*E*)-5,5-diméthyl-2[*m*-(1-imidazolyl)benzoylamino]--3-hexènoïque ;
l'acide (*E*)-5,5-diméthyl-2-[*m*-(1*H*-tétraazol-1-yl)benzoylamino]-3-hexènoïque ;
l'acide (*E*)-5,5-diméthyl-2-[2-(4*H*-1,2,4-triazol-4-yl)isonicotinoylamino]-3-hexènoïque ;
l'acide (*E*)-2-(4-bromo-2-pyrrolylcarbonylamino)-5,5-diméthyl-3-hexènoïque ;
l'acide (*E*)-2-(5-chloro-2-pyrrolylcarbonylamino)-5,5-diméthyl-3-hexènoïque ;
l'acide (*E*)-2-(4,5-dichloro-2-pyrrolylcarbonylamino)-5,5-diméthyl-3-hexènoïque ;
l'acide (*E*)-2-(4-chloro-1-méthyl-2-pyrrolylcarbonylamino)-5,5-diméthyl-3-hexènoïque ;
l'acide (*E*)-2-(4-chloro-2-thiénylcarbonylamino)-5,5-diméthyl-3-hexènoïque ;
l'acide (*E*)-2-(5-isopropyl-3-pyrazolylcarbonylamino)-5,5-diméthyl-3-hexènoïque ;
l'acide (*E*)-5,5-diméthyl-2-[*p*-(3-pyridyloxy)benzoylamino]-3-hexènoïque ;
l'acide (*E*)-5,5-diméthyl-2-(5-phénoxynicotinoylamino)-3-hexènoïque ;
l'acide (*E*)-5,5-diméthyl-2-(2-phénoxyisonicotinoylamino)-3-hexènoïque ;
l'acide (*E*)-5,5-diméthyl-2-[*p*-(2-pyridyloxy)benzoylamino]-3-hexènoïque ;
l'acide (*E*)-5,5-diméthyl-2-[*m*-(2-pyridyloxy)benzoylamino]-3-hexènoïque ;
l'acide (*E*)-2-[6-(benzyloxy)nicotinoylamino]-5,5-diméthyl-3-hexènoïque ;
l'acide (*E*)-5,5-diméthyl-2-[*p*-(6-méthyl-2-pyrazinyloxy)benzoylamino]-3-hexènoïque ;
l'acide (*E*)-5,5-diméthyl-2-[*p*-(2-pyrimidinyloxy)benzoylamino]-3-hexènoïque ;
l'acide (*E*)-2-[*m*-(5-chloro-2-pyridyloxy)benzoylamino]-5,5-diméthyl-3-hexènoïque ;
l'acide (*E*)-2-[6-(p-bromophénoxy)nicotinoylamino]-5,5-diméthyl-3-hexènoïque ;
l'acide (*E*)-5,5-diméthyl-2-(2-phénoxy-5-pyrimidinylcarbonylamino)-3-hexènoïque ;
l'acide (*E*)-2-{6-[*p*-(*tert*-Butyl)phénoxy]nicotinoylamino}-5,5-diméthyl-3-hexènoïque ;
l'acide (*E*)-2-[2-(*p*-cumenyloxy)isonicotinoylamino]-5,5-diméthyl-3-hexènoïque ;
l'acide (*E*)-5,5-Diméthyl-2-(5-phénoxy-2-pyrazinylcarbonylamino)-3-hexènoïque ;
l'acide (*E*)-5,5-diméthyl-2-[*p*-(5-méthyl-2-pyridyloxy)benzoylamino]-3-hexènoïque ;
l'acide (*E*)-2-(2-isopropyl-5-pyrimidinylcarbonylamino)-5,5-diméthyl-3-hexènoïque ;
l'acide (*E*)-5,5-diméthyl-2-[6-(4H-1,2,4-triazol-4-yl)-2-pyridylcarbonylamino]-3-hexènoïque ;
l'acide (*E*)-5,5-diméthyl-2-[*p*-(2-thiényl)benzoylamino]-3-hexènoïque ;
l'acide (*E*)-5,5-diméthyl-2-[*m*-(2-thiényl)benzoylamino]-3-hexènoïque ;
l'acide (*E*)-2-[*p*-(*p*-chlorophénoxy)benzoylamino]-5,5-diméthyl-3-hexènoïque ;
l'acide (*E*)-2-[*p*-(*m-*chlorophénoxy)benzoylamino]-5,5-diméthyl-3-hexènoïque ;
l'acide (*E*)-5,5-diméthyl-2-[*p*-(phénoxyméthyl)benzoylamino]-3-hexènoïque ;
l'acide (*E*)-2-(1-éthyl-3-isobutyl-5-pyrazolylcarbonylamino)-5,5-diméthyl-3-hexènoïque ;
l'acide (*E*)-2-(3-chloro-1-méthyl-5-pyrazolylcarbonylamino)-5,5-diméthyl-3-hexènoïque ;
l'acide (*E*)-2-(3-isopropyl-1-méthyl-5-pyrazolylcarbonylamino)-5,5-diméthyl-3-hexènoïque ;
l'acide (*E*)-5,5-diméthyl-2-[1-méthyl-3-(3-pyridyl)-5-pyrazolylcarbonylamino]-3-hexènoïque ;
l'acide (*E*)-2-[3-(3,4-dichlorophényl)-1-méthyl-5-pyrazolylcarbonylamino]-5,5-diméthyl-3-hexènoïque ;
l'acide (*E*)-2-[5-(3,4-dichlorophényl)-1-méthyl-3-pyrazolylcarbonylamino]-5,5-diméthyl-3-hexènoïque ;
l'acide (*E*)-2-(4,5-dichloro-2-thiénylamino)-5,5-diméthyl-3-hexènoïque ;
l'acide (*E*)-2-(2-Benzyl-4-méthyl-5-pyrimidinylcarbonylamino)-5,5-diméthyl-3-hexènoïque ;
l'acide (*E*)-5,5-diméthyl-2-[4-méthyl-2-(p-toluidino)-5-pyrimidinylcarbonylamino]-3-hexènoïque ;
l'acide (*E*)-2-[2-(*p*-chlorophénylamino)-4-méthyl-5-pyrimidinylcarbonylamino]-5,5-diméthyl-3-hexènoïque ;
l'acide (*E*)-2-[6-(*p*-chlorophénoxy)nicotinoylamino]-5,5-diméthyl-3-hexènoïque ;
l'acide (*E*)-5,5-diméthyl-2-[6-(p-tolyloxy)nicotinoylamino]-3-hexènoïque ;
l'acide (*E*)-2-(6-benzylnicotinoylamino)-5,5-diméthyl-3-hexènoïque ;
l'acide (*E*)-2-(*p*-benzylbenzoylamino)-5,5-diméthyl-3-hexènoïque ;
l'acide (*E*)-5,5-diméthyl-2-(*p*-phénoxybenzoylamino)-3-hexènoïque ;
l'acide (*E*)-5,5-diméthyl-2-(6-phénylnicotinoylamino)-3-hexènoïque ;
l'acide (*E*)-5,5-diméthyl-2-(2-phénylisonicotinoylamino)-3-hexènoïque ;
l'acide (*E*)-5,5-diméthyl-2-[6-(2-thiényloxy)nicotinoylamino]-3-hexènoïque ;
l'acide (*E*)-2-(5-chloro-1-benzothiophen-2-ylcarbonylamino)-5,5-diméthyl-3-hexènoïque ;
l'acide (*E*)-2-(5-chloro-2-indolylcarbonylamino)-5,5-diméthyl-3-hexènoïque ;
l'acide (*E*)-5,5-diméthyl-2-(1-thia-5-aza-2-indenylcarbonylamino)-3-hexènoïque ;
l'acide (*E*)-2-(2-chloro-1-benzothiophen-6-ylcarbonylamino)-5,5-diméthyl-3-hexènoïque ;
l'acide (*E*)-2-(2-chloro-6-indolylcarbonylamino)-5,5-diméthyl-3-hexènoïque ;
l'acide (*E*)-2-(4-chloro-1,3-thiazol-2-ylcarbonylamino)-5,5-diméthyl-3-hexènoïque ;
l'acide (*E*)-2-(4,5-dichloro-1,3-thiazol-2-ylcarbonylamino)-5,5-diméthyl-3-hexènoïque ;
l'acide (*E*)-2-(3-chloro-5-isothiazolylcarbonylamino)-5,5-diméthyl-3-hexènoïque ;
l'acide (*E*)-2-(3,4-dichloro-5-isothiazolylcarbonylamino)-5,5-diméthyl-3-hexènoïque ;
l'acide (*E*)-5,5-diméthyl-2-[*m*-(3-pyridyl)benzoylamino]-3-hexènoïque ;
l'acide (*E*)-2-[*m*-(6-chloro-2-méthyl-3-pyridyl)benzoylamino]-5,5-diméthyl-3-hexènoïque ;
ou un énantiomère de celui-ci, un diastéréoisomère de celui-ci, un racémate de celui-ci ou un sel acceptable sur le plan pharmaceutique de celui-ci, de préférence choisi dans le groupe constitué de :
l'acide (*E*)-2-[2-(diméthylamino)-4-méthyl-5-pyrimidinylcarbonylamino]-5,5-diméthyl-3-hexènoïque ;
l'acide (*E*)-5,5-diméthyl-2-[*p*-(2,2,2-trifluoroacéty)benzoylamino]-3-hexènoïque ;
l'acide (*E*)-5,5-diméthyl-2-[4-(2-pyridyl)-2-pipérazinylcarbonylamino]-3-hexènoïque ;
l'acide (*E*)-5,5-diméthyl-2-[4-(4-pyridyl)-2-pipérazinylcarbonylamino]-3-hexènoïque ;
l'acide (*E*)-5,5-diméthyl-2-(6-phénoxynicotinoylamino)-3-hexènoïque ;
l'acide (*E*)-5,5-diméthyl-2-[*m*-(4H-1,2,4-triazol-4-yl)benzoylamino]-3-hexènoïque ;
l'acide (*E*)-5,5-diméthyl-2-(6-méthyl-1*H*-1,5-diazainden-2-ylcarbonylamino)-3-hexènoïque ;
l'acide (*E*)-2-(4-chloro-2-pyrrolylcarbonylamino)-5,5-diméthyl-3-hexènoïque ;
l'acide (*E*)-2-(3-isobutyl-1-méthyl-5-pyrazolylcarbonylamino)-5,5-diméthyl-3-hexènoïque ;
l'acide (*E*)-5,5-diméthyl-2-[4-(2-pyridyl)-2-pyrrolylcarbonylamino]-3-hexènoïque ;
l'acide (*E*)-5,5-diméthyl-2-[4-(4-pyridyl)-2-pyrrolylcarbonylamino]-3-hexènoïque ;
l'acide (*E*)-5,5-diméthyl-2-[4-(3-pyridyl)-2-pyrrolylcarbonylamino]-3-hexènoïque ;
l'acide (*E*)-2-{4-[amino(2-pyridyl)méthyl]-2-pyrrolylcarbonylamino}-5,5-diméthyl-3-hexènoïque ;
l'acide (*E*)-2-(3-isobutyl-5-isoxazolylcarbonylamino)-5,5-diméthyl-3-hexènoïque ;
l'acide (*E*)-5,5-diméthyl-2-[1-(4-pyridyl)-4-pyrazolylcarbonylamino]-3-hexènoïque ;
l'acide (*E*)-5,5-diméthyl-2-[1-(2-oxo-4-pyridyl)-4-pyrazolylcarbonylamino]-3-hexènoïque ;
l'acide (*E*)-2-[2-(diméthylamino)-5-pyrimidinylcarbonylamino]-5,5-diméthyl-3-hexènoïque ;
l'acide (*E*)-2-(2-isopropyl-5-pyrimidinylcarbonylamino)-5,5-diméthyl-3-hexènoïque ;
l'acide (*E*)-2-[2-(diméthylamino)-4-pyrimidinylcarbonylamino]-5,5-diméthyl-3-hexènoïque ;
l'acide (*E*)-2-[2-(diméthylamino)-6-méthyl-4-pyrimidinylcarbonylamino]-5,5-diméthyl-3-hexènoïque ;
l'acide (*E*)-2-[2-(diéthylamino)-6-méthyl-4-pyrimidinylcarbonylamino]-5,5-diméthyl-3-hexènoïque ;
l'acide (*E*)-5,5-diméthyl-2-[*p*-(1*H*-1,2,4-triazol-1-yl)benzoylamino]-3-hexènoïque ;
l'acide (*E*)-5,5-diméthyl-2-[*p*-(4*H*-1,2,4-triazol-4-yl)benzoylamino]-3-hexènoïque ;
l'acide (*E*)-5,5-diméthyl-2-[*m*-(1*H*-1,2,4-triazol-1-yl)benzoylamino]-3-hexènoïque ;
l'acide (*E*)-5,5-diméthyl-2[*m*-(1-imidazolyl)benzoylamino]--3-hexènoïque ;
l'acide (*E*)-5,5-diméthyl-2-[m-(1*H*-tétraazol-1-yl)benzoylamino]-3-hexènoïque ;
l'acide (*E*)-5,5-diméthyl-2-[2-(4*H*-1,2,4-triazol-4-yl)isonicotinoylamino]-3-hexènoïque ;
l'acide (*E*)-2-(4-bromo-2-pyrrolylcarbonylamino)-5,5-diméthyl-3-hexènoïque ;
l'acide (*E*)-2-(5-chloro-2-pyrrolylcarbonylamino)-5,5-diméthyl-3-hexènoïque ;
l'acide (*E*)-2-(4,5-dichloro-2-pyrrolylcarbonylamino)-5,5-diméthyl-3-hexènoïque ;
l'acide (*E*)-2-(4-chloro-1-méthyl-2-pyrrolylcarbonylamino)-5,5-diméthyl-3-hexènoïque ;
l'acide (*E*)-2-(4-chloro-2-thiénylcarbonylamino)-5,5-diméthyl-3-hexènoïque ;
l'acide (*E*)-2-(5-isopropyl-3-pyrazolylcarbonylamino)-5,5-diméthyl-3-hexènoïque ;
l'acide (*E*)-5,5-diméthyl-2-[*p*-(3-pyridyloxy)benzoylamino]-3-hexènoïque ;
l'acide (*E*)-5,5-diméthyl-2-(5-phénoxynicotinoylamino)-3-hexènoïque ;
l'acide (*E*)-5,5-diméthyl-2-(2-phénoxyisonicotinoylamino)-3-hexènoïque ;
l'acide (*E*)-5,5-diméthyl-2-[*p*-(2-pyridyloxy)benzoylamino]-3-hexènoïque ;
l'acide (*E*)-5,5-diméthyl-2-[*m*-(2-pyridyloxy)benzoylamino]-3-hexènoïque ;
l'acide (*E*)-2-[6-(benzyloxy)nicotinoylamino]-5,5-diméthyl-3-hexènoïque ;
l'acide (*E*)-5,5-diméthyl-2-[*p*-(6-méthyl-2-pyrazinyloxy)benzoylamino]-3-hexènoïque ;
l'acide (*E*)-5,5-diméthyl-2-[*p*-(2-pyrimidinyloxy)benzoylamino]-3-hexènoïque ;
l'acide (*E*)-2-[*m*-(5-chloro-2-pyridyloxy)benzoylamino]-5,5-diméthyl-3-hexènoïque ;
l'acide (*E*)-2-[6-(*p*-bromophenoxy)nicotinoylamino]-5,5-diméthyl-3-hexènoïque ;
l'acide (*E*)-5,5-diméthyl-2-(2-phénoxy-5-pyrimidinylcarbonylamino)-3-hexènoïque ;
l'acide (*E*)-2-{6-[*p*-(*tert*-Butyl)phénoxy]nicotinoylamino}-5,5-diméthyl-3-hexènoïque ;
l'acide (*E*)-2-[2-(*p*-cumenyloxy)isonicotinoylamino]-5,5-diméthyl-3-hexènoïque ;
l'acide (*E*)-5,5-Diméthyl-2-(5-phénoxy-2-pyrazinylcarbonylamino)-3-hexènoïque ;
l'acide (*E*)-5,5-diméthyl-2-[*p*-(5-méthyl-2-pyridyloxy)benzoylamino]-3-hexènoïque ;
ou un énantiomère de celui-ci, un diastéréoisomère de celui-ci, un racémate de celui-ci ou un sel acceptable sur le plan pharmaceutique de celui-ci.

8. Composé selon l'une quelconque des revendications 1 à 6, dans lequel le composé est choisi dans le groupe constitué de :
l'acide (*E*)-2-[2-(diméthylamino)-4-méthyl-5-pyrimidinylcarbonylamino]-5,5-diméthyl-3-hexènoïque ;
l'acide (*E*)-5,5-diméthyl-2-[p-(2,2,2-trifluoroacéty)benzoylamino]-3-hexènoïque ;
l'acide (*E*)-5,5-diméthyl-2-[4-(2-pyridyl)-2-pipérazinylcarbonylamino]-3-hexènoïque ;
l'acide (*E*)-5,5-diméthyl-2-[4-(4-pyridyl)-2-pipérazinylcarbonylamino]-3-hexènoïque ;
l'acide (*E*)-5,5-diméthyl-2-(6-phénoxynicotinoylamino)-3-hexènoïque ;
l'acide (*E*)-5,5-diméthyl-2-[*m*-(4H-1,2,4-triazol-4-yl)benzoylamino]-3-hexènoïque ;
l'acide (*E*)-5,5-diméthyl-2-(6-méthyl-1*H*-1,5-diazainden-2-ylcarbonylamino)-3-hexènoïque ;
l'acide (*E*)-2-(4-chloro-2-pyrrolylcarbonylamino)-5,5-diméthyl-3-hexènoïque ;
l'acide (*E*)-2-(3-isobutyl-1-méthyl-5-pyrazolylcarbonylamino)-5,5-diméthyl-3-hexènoïque ;
l'acide (*E*)-2-{4-[amino(2-pyridyl)méthyl]-2-pyrrolylcarbonylamino}-5,5-diméthyl-3-hexènoïque ;
l'acide (*E*)-5,5-diméthyl-2-[1-(2-oxo-4-pyridyl)-4-pyrazolylcarbonylamino]-3-hexènoïque ;
l'acide (*E*)-2-[2-(diméthylamino)-5-pyrimidinylcarbonylamino]-5,5-diméthyl-3-hexènoïque ;
l'acide (*E*)-5,5-diméthyl-2-[*p*-(4*H*-1,2,4-triazol-4-yl)benzoylamino]-3-hexènoïque ;
l'acide (*E*)-5,5-diméthyl-2-[*m*-(1*H*-1,2,4-triazol-1-yl)benzoylamino]-3-hexènoïque ;
l'acide (*E*)-5,5-diméthyl-2[*m*-(1-imidazolyl)benzoylamino]--3-hexènoïque ;
l'acide (*E*)-2-(4-bromo-2-pyrrolylcarbonylamino)-5,5-diméthyl-3-hexènoïque ;
l'acide (*E*)-2-(5-chloro-2-pyrrolylcarbonylamino)-5,5-diméthyl-3-hexènoïque ;
l'acide (*E*)-2-(4-chloro-1-méthyl-2-pyrrolylcarbonylamino)-5,5-diméthyl-3-hexènoïque ;
l'acide (*E*)-2-(4-chloro-2-thiénylcarbonylamino)-5,5-diméthyl-3-hexènoïque ;
l'acide (*E*)-5,5-diméthyl-2-[*p*-(3-pyridyloxy)benzoylamino]-3-hexènoïque ;
l'acide (*E*)-5,5-diméthyl-2-(2-phénoxyisonicotinoylamino)-3-hexènoïque ;
l'acide (*E*)-2-[6-(benzyloxy)nicotinoylamino]-5,5-diméthyl-3-hexènoïque ;
l'acide (*E*)-5,5-diméthyl-2-[*p*-(2-pyrimidinyloxy)benzoylamino]-3-hexènoïque ;
l'acide (*E*)-5,5-diméthyl-2-(2-phénoxy-5-pyrimidinylcarbonylamino)-3-hexènoïque ;
l'acide (*E*)-2-(2-isopropyl-5-pyrimidinylcarbonylamino)-5,5-diméthyl-3-hexènoïque ;
l'acide (*E*)-5,5-diméthyl-2-[*p*-(2-thiényl)benzoylamino]-3-hexènoïque ;
l'acide (*E*)-5,5-diméthyl-2-[*m*-(2-thiényl)benzoylamino]-3-hexènoïque ;
l'acide (*E*)-2-[*p*-(*p*-chlorophénoxy)benzoylamino]-5,5-diméthyl-3-hexènoïque ;
l'acide (*E*)-2-[*p*-(*m*-chlorophénoxy)benzoylamino]-5,5-diméthyl-3-hexènoïque ;
l'acide (*E*)-5,5-diméthyl-2-[*p*-(phénoxyméthyl)benzoylamino]-3-hexènoïque ;
l'acide (*E*)-2-(1-éthyl-3-isobutyl-5-pyrazolylcarbonylamino)-5,5-diméthyl-3-hexènoïque ;
l'acide (*E*)-2-(3-chloro-1-méthyl-5-pyrazolylcarbonylamino)-5,5-diméthyl-3-hexènoïque ;
l'acide (*E*)-2-(3-isopropyl-1-méthyl-5-pyrazolylcarbonylamino)-5,5-diméthyl-3-hexènoïque ;
l'acide (*E*)-5,5-diméthyl-2-[1-méthyl-3-(3-pyridyl)-5-pyrazolylcarbonylamino]-3-hexènoïque ;
l'acide (*E*)-2-[5-(3,4-dichlorophényl)-1-méthyl-3-pyrazolylcarbonylamino]-5,5-diméthyl-3-hexènoïque ;
l'acide (*E*)-2-(4,5-dichloro-2-thiénylamino)-5,5-diméthyl-3-hexènoïque ;
ou un énantiomère de celui-ci, un diastéréoisomère de celui-ci, un racémate de celui-ci ou un sel acceptable sur le plan pharmaceutique de celui-ci, de préférence choisi dans le groupe constitué de :
l'acide (*E*)-2-[2-(diméthylamino)-4-méthyl-5-pyrimidinylcarbonylamino]-5,5-diméthyl-3-hexènoïque ;
l'acide (*E*)-5,5-diméthyl-2-[*p*-(2,2,2-trifluoroacétyl)benzoylamino]-3-hexènoïque ;
l'acide (*E*)-5,5-diméthyl-2-[4-(2-pyridyl)-2-pipérazinylcarbonylamino]-3-hexènoïque ;
l'acide (*E*)-5,5-diméthyl-2-[4-(4-pyridyl)-2-pipérazinylcarbonylamino]-3-hexènoïque ;
l'acide (*E*)-5,5-diméthyl-2-(6-phénoxynicotinoylamino)-3-hexènoïque ;
l'acide (*E*)-5,5-diméthyl-2-[*m*-(4*H*-1,2,4-triazol-4-yl)benzoylamino]-3-hexènoïque ;
l'acide (*E*)-5,5-diméthyl-2-(6-méthyl-1*H*-1,5-diazainden-2-ylcarbonylamino)-3-hexènoïque ;
l'acide (*E*)-2-(4-chloro-2-pyrrolylcarbonylamino)-5,5-diméthyl-3-hexènoïque ;
l'acide (*E*)-2-(3-isobutyl-1-méthyl-5-pyrazolylcarbonylamino)-5,5-diméthyl-3-hexènoïque ;
ou un énantiomère de celui-ci, un diastéréoisomère de celui-ci, un racémate de celui-ci ou un sel acceptable sur le plan pharmaceutique de celui-ci.

9. Composé selon l'une quelconque des revendications 1 à 6, dans lequel le composé est choisi dans le groupe constitué de :
l'acide (*E*)-2-[2-(diméthylamino)-4-méthyl-5-pyrimidinylcarbonylamino]-5,5-diméthyl-3-hexènoïque ;
l'acide (*E*)-5,5-diméthyl-2-(6-phénoxynicotinoylamino)-3-hexènoïque ;
l'acide (*E*)-5,5-diméthyl-2-[*m*-(4*H*-1,2,4-triazol-4-yl)benzoylamino]-3-hexènoïque ;
l'acide (*E*)-5,5-diméthyl-2-(6-méthyl-1*H*-1,5-diazainden-2-ylcarbonylamino)-3-hexènoïque ;
l'acide (*E*)-2-(4-chloro-2-pyrrolylcarbonylamino)-5,5-diméthyl-3-hexènoïque ;
l'acide (*E*)-5,5-diméthyl-2-[*p*-(4*H*-1,2,4-triazol-4-yl)benzoylamino]-3-hexènoïque ;
l'acide (*E*)-5,5-diméthyl-2-[*m*-(1*H*-1,2,4-triazol-1-yl)benzoylamino]-3-hexènoïque ;
l'acide (*E*)-5,5-diméthyl-2[*m*-(1-imidazolyl)benzoylamino]--3-hexènoïque ;
l'acide (*E*)-2-(4-bromo-2-pyrrolylcarbonylamino)-5,5-diméthyl-3-hexènoïque ;
l'acide (*E*)-2-(5-chloro-2-pyrrolylcarbonylamino)-5,5-diméthyl-3-hexènoïque ;
l'acide (*E*)-2-(4-chloro-1-méthyl-2-pyrrolylcarbonylamino)-5,5-diméthyl-3-hexènoïque ;
l'acide (*E*)-2-(4-chloro-2-thiénylcarbonylamino)-5,5-diméthyl-3-hexènoïque ;
l'acide (*E*)-5,5-diméthyl-2-[*p*-(3-pyridyloxy)benzoylamino]-3-hexènoïque ;
l'acide (*E*)-2-[6-(benzyloxy)nicotinoylamino]-5,5-diméthyl-3-hexènoïque ;
l'acide (*E*)-5,5-diméthyl-2-(2-phénoxy-5-pyrimidinylcarbonylamino)-3-hexènoïque ;
l'acide (*E*)-2-(2-isopropyl-5-pyrimidinylcarbonylamino)-5,5-diméthyl-3-hexènoïque ;
l'acide (*E*)-5,5-diméthyl-2-[*p*-(2-thiényl)benzoylamino]-3-hexènoïque ;
l'acide (*E*)-5,5-diméthyl-2-[*m*-(2-thiényl)benzoylamino]-3-hexènoïque ;
l'acide (*E*)-2-[*p*-(*p*-chlorophénoxy)benzoylamino]-5,5-diméthyl-3-hexènoïque ;
l'acide (*E*)-2-[*p*-(*m*-chlorophénoxy)benzoylamino]-5,5-diméthyl-3-hexènoïque ;
l'acide (*E*)-5,5-diméthyl-2-[*p*-(phénoxyméthyl)benzoylamino]-3-hexènoïque ;
l'acide (*E*)-2-(1-éthyl-3-isobutyl-5-pyrazolylcarbonylamino)-5,5-diméthyl-3-hexènoïque ;
l'acide (*E*)-2-(3-chloro-1-méthyl-5-pyrazolylcarbonylamino)-5,5-diméthyl-3-hexènoïque ;
l'acide (*E*)-2-(3-isopropyl-1-méthyl-5-pyrazolylcarbonylamino)-5,5-diméthyl-3-hexènoïque ;
l'acide (*E*)-2-[5-(3,4-dichlorophényl)-1-méthyl-3-pyrazolylcarbonylamino]-5,5-diméthyl-3-hexènoïque ;
l'acide (*E*)-2-(4,5-dichloro-2-thiénylamino)-5,5-diméthyl-3-hexènoïque ;
ou un énantiomère de celui-ci, un diastéréoisomère de celui-ci, un racémate de celui-ci ou un sel acceptable sur le plan pharmaceutique de celui-ci, de préférence choisi dans le groupe constitué de :
l'acide (*E*)-2-[2-(diméthylamino)-4-méthyl-5-pyrimidinylcarbonylamino]-5,5-diméthyl-3-hexènoïque ;
l'acide (*E*)-5,5-diméthyl-2-(6-phénoxynicotinoylamino)-3-hexènoïque ;
l'acide (*E*)-5,5-diméthyl-2-[*m*-(4*H*-1,2,4-triazol-4-yl)benzoylamino]-3-hexènoïque ;
l'acide (*E*)-2-(4-chloro-2-pyrrolylcarbonylamino)-5,5-diméthyl-3-hexènoïque ;
l'acide (*E*)-5,5-diméthyl-2-[*m*-(1*H*-1,2,4-triazol-1-yl)benzoylamino]-3-hexènoïque ;
l'acide (*E*)-5,5-diméthyl-2[*m*-(1-imidazolyl)benzoylamino]--3-hexènoïque ;
l'acide (*E*)-2-(4-bromo-2-pyrrolylcarbonylamino)-5,5-diméthyl-3-hexènoïque ;
l'acide (*E*)-2-(4-chloro-1-méthyl-2-pyrrolylcarbonylamino)-5,5-diméthyl-3-hexènoïque ;
l'acide (*E*)-2-(4-chloro-2-thiénylcarbonylamino)-5,5-diméthyl-3-hexènoïque ;
l'acide (*E*)-5,5-diméthyl-2-[*p*-(3-pyridyloxy)benzoylamino]-3-hexènoïque ;
l'acide (*E*)-2-[6-(benzyloxy)nicotinoylamino]-5,5-diméthyl-3-hexènoïque ;
l'acide (*E*)-2-(2-isopropyl-5-pyrimidinylcarbonylamino)-5,5-diméthyl-3-hexènoïque ;
l'acide (*E*)-5,5-diméthyl-2-[*p*-(2-thiényl)benzoylamino]-3-hexènoïque ;
l'acide (*E*)-5,5-diméthyl-2-[*m*-(2-thiényl)benzoylamino]-3-hexènoïque ;
l'acide (*E*)-2-(3-chloro-1-méthyl-5-pyrazolylcarbonylamino)-5,5-diméthyl-3-hexènoïque ;
l'acide (*E*)-2-(4,5-dichloro-2-thiénylamino)-5,5-diméthyl-3-hexènoïque ;
ou un énantiomère de celui-ci, un diastéréoisomère de celui-ci, un racémate de celui-ci ou un sel acceptable sur le plan pharmaceutique de celui-ci, et de préférence
l'acide (*E*)-5,5-diméthyl-2[*m*-(1-imidazolyl)benzoylamino]-3-hexènoïque, ou un énantiomère de celui-ci, un diastéréoisomère de celui-ci, un racémate de celui-ci ou un sel acceptable sur le plan pharmaceutique de celui-ci ; ou
l'acide (*E*)-2-(4-chloro-1-méthyl-2-pyrrolylcarbonylamino)-5,5-diméthyl-3-hexènoïque, ou un énantiomère de celui-ci, un diastéréoisomère de celui-ci, un racémate de celui-ci ou un sel acceptable sur le plan pharmaceutique de celui-ci ; ou
l'acide (*E*)-2-(4-chloro-2-thiénylcarbonylamino)-5,5-diméthyl-3-hexènoïque ou un énantiomère de celui-ci, un diastéréoisomère de celui-ci, un racémate de celui-ci ou un sel acceptable sur le plan pharmaceutique de celui-ci ; ou
l'acide (*E*)-2-[6-(benzyloxy)nicotinoylamino]-5,5-diméthyl-3-hexènoïque, ou un énantiomère de celui-ci, un diastéréoisomère de celui-ci, un racémate de celui-ci ou un sel acceptable sur le plan pharmaceutique de celui-ci ; ou
l'acide (*E*)-5,5-diméthyl-2-[*p*-(2-thiényl)benzoylamino]-3-hexènoïque, ou un énantiomère de celui-ci, un diastéréoisomère de celui-ci, un racémate de celui-ci ou un sel acceptable sur le plan pharmaceutique de celui-ci ; ou
l'acide (*E*)-5,5-diméthyl-2-[*m*-(2-thiényl)benzoylamino]-3-hexènoïque, ou un énantiomère de celui-ci, un diastéréoisomère de celui-ci, un racémate de celui-ci ou un sel acceptable sur le plan pharmaceutique de celui-ci ; ou
l'acide (*E*)-2-(4,5-dichloro-2-thiénylamino)-5,5-diméthyl-3-hexènoïque ou un énantiomère de celui-ci, un diastéréoisomère de celui-ci, un racémate de celui-ci ou un sel acceptable sur le plan pharmaceutique de celui-ci.

10. Composé selon l'une quelconque des revendications 1 à 6, dans lequel le composé est choisi dans le groupe constitué de :
l'acide (*E*)-2-[2-(diméthylamino)-4-méthyl-5-pyrimidinylcarbonylamino]-5,5-diméthyl-3-hexènoïque ;
l'acide (*E*)-5,5-diméthyl-2-(6-phénoxynicotinoylamino)-3-hexènoïque ;
l'acide (E)-5,5-diméthyl-2-[*m*-(4*H*-1,2,4-triazol-4-y)benzoylamino]-3-hexènoïque ;
l'acide (*E*)-5,5-diméthyl-2-(6-méthyl-1*H*-1,5-diazainden-2-ylcarbonylamino)-3-hexènoïque ;
l'acide (*E*)-2-(4-chloro-2-pyrrolylcarbonylamino)-5,5-diméthyl-3-hexènoïque ;
ou un énantiomère de celui-ci, un diastéréoisomère de celui-ci, un racémate de celui-ci ou un sel acceptable sur le plan pharmaceutique de celui-ci, de préférence
l'acide (*E*)-2-[2-(diméthylamino)-4-méthyl-5-pyrimidinylcarbonylamino]-5,5-diméthyl-3-hexènoïque, ou un énantiomère de celui-ci, un diastéréoisomère de celui-ci, un racémate de celui-ci ou un sel acceptable sur le plan pharmaceutique de celui-ci ; ou
l'acide (*E*)-5,5-diméthyl-2-(6-phénoxynicotinoylamino)-3-hexènoïque, ou un énantiomère de celui-ci, un diastéréoisomère de celui-ci, un racémate de celui-ci ou un sel acceptable sur le plan pharmaceutique de celui-ci.

11. Composition pharmaceutique comprenant un composé selon l'une quelconque des revendications 1 à 10 et au moins un excipient, support ou diluant acceptable sur le plan pharmaceutique.

12. Composé selon l'une quelconque des revendications 1 à 10 pour une utilisation en tant que médicament.

13. Composé selon l'une quelconque des revendications 1 à 10 destiné à une utilisation dans la prévention ou le traitement du cancer.

14. Composé selon l'une quelconque des revendications 1 à 10, destiné à une utilisation dans la prévention ou le traitement d'une maladie neurodégénérative, d'une maladie psychiatrique, d'une maladie des neurones moteurs, de neuropathies périphériques, de la douleur, de la neuro-inflammation, de l'athérosclérose, de l'hyperlipidémie, de maladies cardiovasculaires, de maladies liées à la dermatologie, de maladies auto-immunes, de préférence de la maladie d'Alzheimer et de la maladie de Parkinson.

15. Intermédiaire pour la production d'un composé selon la revendication 1, dans lequel l'intermédiaire est choisi dans le groupe constitué de :
l'acide (*E*)-2-{[(*p*-méthoxyphényl)méthyl]amino}-5,5-diméthyl-3-hexènoïque ;
(*E*)-2-{[(*p*-méthoxyphényl)méthyl]amino}-5,5-diméthyl-3-hexénoate d'éthyle ; et
(*E*)-2-amino-5,5-diméthyl-3-hexénoate d'éthyle, de préférence dans lequel l'intermédiaire est (*E*)-2-amino-5,5-diméthyl-3-hexénoate d'éthyle.
